(19)

Europäisches Patentamt
European Patent Office
Office européen des brevets

(11)  **EP 2 816 351 A2**

(12)  **EUROPEAN PATENT APPLICATION**

(43) Date of publication:
     **24.12.2014 Bulletin 2014/52**

(51) Int Cl.:
     ***G01N 33/48*** *(2006.01)*     ***G06F 19/00*** *(2011.01)*
     ***C07K 14/705*** *(2006.01)*    *G06F 19/20 (2011.01)*
     *G06F 19/22 (2011.01)*           *G06F 19/28 (2011.01)*

(21) Application number: **13005799.5**

(22) Date of filing: **27.01.2005**

(84) Designated Contracting States:
     **AT BE BG CH CY CZ DE DK EE ES FI FR GB GR HU IE IS IT LI LT LU MC NL PL PT RO SE SI SK TR**

(30) Priority: **27.01.2004 US 539129 P**

(62) Document number(s) of the earlier application(s) in accordance with Art. 76 EPC:
     **05703149.4 / 1 713 900**

(71) Applicant: **Compugen Ltd.**
     **69512 Tel Aviv (IL)**

(72) Inventors:
     • **Diber, Alex**
       **75502 Rishon-LeZion (IL)**
     • **Pollock, Sarah**
       **63506 Tel-Aviv (IL)**
     • **Levine, Zurit**
       **46420 Herzeliya (IL)**
     • **Nemzer, Sergey**
       **43352 RaAnana (IL)**
     • **Rosenberg, Avi**
       **44622 Kfar Saba (IL)**
     • **Zekharia, Tomer**
       **99725 Shimson (IL)**

     • **Novik, Amit**
       **30500 Binyamina (IL)**
     • **Cojocaru, Gad S.**
       **47248 Ramat-HaSharon (IL)**
     • **Haviv, Ami**
       **45217 Hod-HaSharon (IL)**
     • **Shaked, Zipi**
       **69102 Tel Aviv (IL)**
     • **Farkash, Ariel**
       **32712 Haifa (IL)**
     • **Privman, Eyal**
       **Haifa 3478605 (IL)**
     • **Dahary, Dvir**
       **69270 Tel Aviv (IL)**
     • **Beck, Nili**
       **44405 Kfar Saba (IL)**

(74) Representative: **Fuchs Patentanwälte**
     **Partnerschaft mbB**
     **Patentanwälte**
     **Westhafenplatz 1**
     **60327 Frankfurt am Main (DE)**

Remarks:
     This application was filed on 12-12-2013 as a divisional application to the application mentioned under INID code 62.

(54)  **Methods and systems for annotating biomolecular sequences**

(57)  Polypeptide sequences and polynucleotide sequences are provided. Also provided are annotative information concerning such sequences and uses for these sequences.

Fig. 1a

EP 2 816 351 A2

**Description**

FIELD AND BACKGROUND OF THE INVENTION

The present invention relates to systems and methods useful for annotating biomolecular sequences. More particularly, the present invention relates to computational approaches, which enable systemic characterization of biomolecular sequences and identification of differentially expressed biomolecular sequences such as sequences associated with a pathology.

In the post-genomic era, data analysis rather than data collection presents the biggest challenge to biologists. Efforts to ascribe biological meaning to genomic data, whether by identification of function, structure or expression pattern are lagging behind sequencing efforts [Boguski MS (1999) Science 286:453-455].

It is well recognized that elucidation of spatial and temporal patterns of gene expression in healthy and diseased states may contribute immensely to further understanding of disease mechanisms.

Therefore, any observational method that can rapidly, accurately and economically observe and measure the pattern of expression of selected individual genes or of whole genomes is of great value to scientists.

In recent years, a variety of techniques have been developed to analyze differential gene expression. However, current observation and measurement methods are inaccurate, time consuming, labor intensive or expensive, oftentimes requiring complex molecular and biochemical analysis of numerous gene sequences.

For example, observation methods for individual mRNA or cDNA molecules such as Northern blot analysis, RNase protection, or selective hybridization to arrayed cDNA libraries [see Sambrook et al. (1989) Molecular cloning, A laboratory manual, Cold Spring Harbor press, NY] depend on specific hybridization of a single oligonucleotide probe complementary to the known sequence of an individual molecule. Since a single human cell is estimated to express 10,000-30,000 genes [Liang et al. (1992) Science 257:967-971], single probe methods to identify all sequences in a complex sample are ineffective and laborious.

Other approaches for high throughput analysis of differential gene expression are summarized infra.

*EST sequencing -* The basic idea is to create cDNA libraries from tissues of interest, pick clones randomly from these libraries and then perform a single sequencing reaction from a large number of clones. Each sequencing reaction generates 300 base pairs or so of sequence that represents a unique sequence tag for a particular transcript. An EST sequencing project is technically simple to execute since it requires only a cDNA library, automated DNA sequencing capabilities and standard bioinformatics protocols.

To generate meaningful amounts of data, however, high throughput template preparation, sequencing and analysis protocols must be applied. As such, the number of new genes identified as well as the statistical significance of the data is proportional to the number of clones sequenced as well as the complexity of the tissue being analyzed [Adams et al. (1995) Nature 377:3-173; Hillier et al. (1996) Genome Res. 6:807-828].

*Subtractive cloning* -Subtractive cloning offers an inexpensive and flexible alternative to EST sequencing and cDNA array hybridization. In this approach, double-stranded cDNA is created from the two-cell or tissue populations of interest, linkers are ligated to the ends of the cDNA fragments and the cDNA pools are then amplified by PCR. The cDNA pool from which unique clones are desired is designated the "tester", and the cDNA pool that is used to subtract away shared sequences is designated the "driver". Following initial PCR amplification, the linkers are removed from both cDNA pools and unique linkers are ligated to the tester sample. The tester is then hybridized to a vast excess of driver DNA and sequences that are unique to the tester cDNA pool are amplified by PCR.

The primary limitation of subtractive methods is that they are not always comprehensive. The cDNAs identified are typically those, which differ significantly in expression level between cell-populations and subtle quantitative differences are often missed. In addition each experiment is a pair wise comparison and since subtractions are based on a series of sensitive biochemical reactions it is difficult to directly compare a series of RNA samples.

*Differential display -* Differential display is another PCR-based differential cloning method [Liang and Pardee (1992) Science 257:967-70; Welsh et al. (1992) Nucleic Acids Res. 20:4965-70]. In classical differential display, reverse transcription is primed with either oligo-dT or an arbitrary primer. Thereafter an arbitrary primer is used in conjunction with the reverse transcription primer to amplify cDNA fragments and the cDNA fragments are separated on a polyacrylamide gel. Differences in gene expression are visualized by the presence or absence of bands on the gel and quantitative differences in gene expression are identified by differences in the intensity of bands. Adaptation of differential display methods for fluorescent DNA sequencing machines has enhanced the ability to quantify differences in gene expression [Kato (1995) Nucleic Acids Res. 18:3685-90].

A limitation of the classical differential display approach is that false positive results are often generated during PCR or in the process of cloning the differentially expressed PCR products. Although a variety of methods have been developed to discriminate true from false positives, these typically rely on the availability of relatively large amounts of RNA.

*Serial analysis of gene expression (SAGE) -* this DNA sequence based method is essentially an accelerated version of EST sequencing [Valculescu et al. (1995) Science 270:484-8]. In this method a digestible unique sequence tag of 13 or more bases is generated for each transcript in the cell or tissue of interest, thereby generating a SAGE library.

Sequencing each SAGE library creates transcript profiles. Since each sequencing reaction yields information for twenty or more genes, it is possible to generate data points for tens of thousands of transcripts in modest sequencing efforts. The relative abundance of each gene is determined by counting or clustering sequence tags. The advantages of SAGE over many other methods include the high throughput that can be achieved and the ability to accumulate and compare SAGE tag data from a variety of samples, however the technical difficulties concerning the generation of good SAGE libraries and data analysis are significant.

Altogether, it is clear from the above that laboratory bench approaches are ineffective, time consuming, expensive and often times inaccurate in handling and processing the vast amount of genomic information which is now available.

It is appreciated, that much of the analysis can be effected by developing computational algorithms, which can be applied to mining data from existing databases, thereby retrieving and integrating valuable biological information.

To date, there are more than a hundred major biomolecule databases and application servers on the Internet and new sites are being introduced at an ever-increasing rates [Ashburner and Goodman (1997) Curr. Opin. Genet Dev. 7:750-756; Karp (1998) Trends Biochem. Sci. 23:114-116].

However, these databases are organized in extremely heterogeneous formats. These reflect the inherent complexity of biological data, ranging from plain-text nucleic acid and protein sequences, through the three dimensional structures of therapeutic drugs and macromolecules and high resolution images of cells and tissues, to microarray-chip outputs. Moreover data structures are constantly evolving to reflect new research and technology development

The heterogeneous and dynamic nature of these biological databases present major obstacles in mining data relevant to specific biological queries. Clearly, simple retrieval of data is not sufficient for data mining; efficient data retrieval requires flexible data manipulation and sophisticated data integration. Efficient data retrieval requires the use of complex queries across multiple heterogeneous data sources; data warehousing by merging data derived from multiple public sources and local (i.e., private) sources; and multiple data-analysis procedures that require feeding subsets of data derived from different sources into various application programs for gene finding, protein-structure prediction, functional domain or motif identification, phylogenetic tree construction, graphic presentation and so forth.

Current biological data retrieval systems are not fully up to the demand of smooth and flexible data integration [Etzold et al. (1996) Methods Enzymol 266:t14-t28; Schuler et al. (1996) Methods Enzymol. 266:141-162; Chung and Wong (1999) Trends Biotech. 17:351-355].

There is thus a widely recognized need for, and it would be highly advantageous to have, systems and methods which can be used for efficient retrieval and processing of data from biological databases thereby enabling annotation of previously un-annotated biomolecular sequences.

SUMMARY OF THE INVENTION

According to one aspect of the present invention there is provided a computer readable storage medium, comprising a database stored in a retrievable manner, the database including biomolecular sequence information as set forth in files "Franscripts.gz", and/or "Proteins.gz" of enclosed CD-ROM4, and biomolecular sequence annotations, as set forth in file "Annotations.gz" of enclosed CD-ROM4.

According to another aspect of the present invention there is provided a method of comparing an expression level of a gene of interest in at least two types of tissues, the method comprising: (a) obtaining a contig representing the gene of interest, the contig being assembled from a plurality of expressed sequences; and (b) comparing a number of the plurality of expressed sequences corresponding to the contig which are expressed in each of the at least two tissue types, to thereby compare the expression level of the gene of interest in the at least two tissue types.

According to further features in preferred embodiments of the invention described below, the method further comprises computationally aligning sequences expressed in each of the at least two types of tissue with the contig to thereby identify the expressed sequences corresponding to the contig prior to (b).

According to yet another aspect of the present invention there is provided a method of comparing an expression level of at least two splice variants of a gene of interest in a tissue, the method comprising: (a) obtaining a contig having exonal sequences of the at least two splice variants of the gene of interest, the contig being assembled from a plurality of expressed sequences; (b) identifying at least one contig sequence region unique to one of the at least two splice variants of the gene of interest; and (c) comparing a number of the plurality of expressed sequences in the tissue having the at least one contig sequence region with a number of the plurality of expressed sequences not-having the at least one contig sequence region, to thereby compare the expression level of the at least two splice variants of the gene of interest in the tissue.

According to still further features in the described preferred embodiments the plurality of expressed sequences present complete exonal coverage of the gene of interest.

According to still further features in the described preferred embodiments the plurality of expressed sequences present partial exonal coverage of the gene of interest.

According to still further features in the described preferred embodiments the obtaining the contig is effected by a sequence assembly software.

According to still further features in the described preferred embodiments the method further comprising scoring each of the plurality of the expressed sequences prior to (c), wherein the scoring is effected according to:

(i) expression level of each of the plurality of the expressed sequences; and
(ii) a quality of each of the plurality of the expressed sequences;

According to still further features in the described preferred embodiments comparing is effected using statistical pairing analysis.

According to still further features in the described preferred embodiments the statistical pairing analysis is Fisher exact test.

According to still further features in the described preferred embodiments the tissue is selected from the group consisting of a tissue of a pathological origin of interest, a tissue of a cellular composition of interest.

According to still further features in the described preferred embodiments the method further comprising comparing the number of the plurality of expressed sequences in the tissue having the at least one contig sequence region with a number of the plurality of expressed sequences of the contig.

According to still another aspect of the present invention there is provided a computer readable storage medium comprising data stored in a retrievable manner, the data including sequence information of differentially expressed mRNA sequences as set forth in files "Transcripts.gz", and/or "Proteins.gz" of enclosed CD-ROM4, and sequence annotations as set forth in annotation categories "#TS", "#TAA" and/or "#TAAT", in the file "Annotations.gz" of enclosed CD-ROM4.

According to still further features in the described preferred embodiments the database further includes information pertaining to generation of the data and potential uses of the data.

According to still further features in the described preferred embodiments the medium is selected from the group consisting of a magnetic storage medium, an optical storage medium and an optico-magnetic storage medium.

According to still further features in the described preferred embodiments the database further includes information pertaining to gain and/or loss of function of the differentially expressed mRNA splice variants or polypeptides encoded thereby.

According to an additional aspect of the present invention there is provided a kit useful for detecting differentially expressed polynucleotide sequences, the kit comprising at least one oligonucleotide being designed and configured to be specifically hybridizable with a polynucleotide sequence selected from the group consisting of sequence files "Transcripts.gz" of enclosed CD-ROM4 under moderate to stringent hybridization conditions.

According to still further features in the described preferred embodiments the at least one oligonucleotide is labeled.

According to still further features in the described preferred embodiments the at least one oligonucleotide is attached to a solid substrate.

According to still further features in the described preferred embodiments the solid substrate is configured as a microarray and whereas the at least one oligonucleotide includes a plurality of oligonucleotides each being capable of hybridizing with a specific polynucleotide sequence of the polynucleotide sequences set forth in the files "Transcripts.gz" of enclosed CD-ROM4 under moderate to stringent hybridization conditions.

According to still further features in the described preferred embodiments each of the plurality of oligonucleotides is being attached to the microarray in a regio-specific manner.

According to still further features in the described preferred embodiments the at least one oligonucleotide is designed and configured for DNA hybridization.

According to still further features in the described preferred embodiments the at least one oligonucleotide is designed and configured for RNA hybridization.

According to yet an additional aspect of the present invention there is provided a system for generating a database of differentially expressed genes, the system comprising a processing unit, the processing unit executing a software application configured for: (a) obtaining contigs representing genes of interest, each of the contigs being assembled from a plurality of expressed sequences; (b) comparing a number of the plurality of expressed sequences corresponding to each of the contigs, which are expressed in each of at least two tissue types, to thereby compare the expression level of the genes of interest in the at least two tissue types; and (c) storing contigs which are supported by different numbers of the plurality of expressed sequences in each of the at least two tissue types, to thereby generate the database of differentially expressed genes.

According to still an additional aspect of the present invention there is provided an isolated polynucleotide comprising a nucleic acid sequence being at least 80 % identical to a nucleic acid sequence of the sequences set forth in file "Transcripts.gz" of the enclosed CD-ROM4.

According to still further features in the described preferred embodiments the nucleic acid sequence is set forth in the file "Transcripts.gz" of the enclosed CD-ROM4.

According to a further aspect of the present invention there is provided an isolated polynucleotide comprising a nucleic acid sequence encoding a polypeptide having an amino acid sequence at least 80 % homologous to a sequence set forth in the file "Proteins.gz" of the enclosed CD-ROM4.

According to yet a further aspect of the present invention there is provided an isolated polynucleotide comprising a nucleic acid sequence at least 80 % identical to a sequence set forth in the file "Transcripts.gz" of the enclosed CD-ROM4.

According to still a further aspect of the present invention there is provided an isolated polypeptide having an amino acid sequence at least 80 % homologous to a sequence set forth in the file "Proteins.gz" of the enclosed CD-ROM4.

According to still a further aspect of the present invention there is provided use of a polynucleotide or polypeptide set forth in the file "Transcripts.gz" or "Proteins.gz" of the enclosed CD-ROM4 for the diagnosis and/or treatment of the diseases listed in herein.

The present invention successfully addresses the shortcomings of the presently known configurations by providing methods and systems useful for systematically uncovering and annotating biomolecular sequences.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

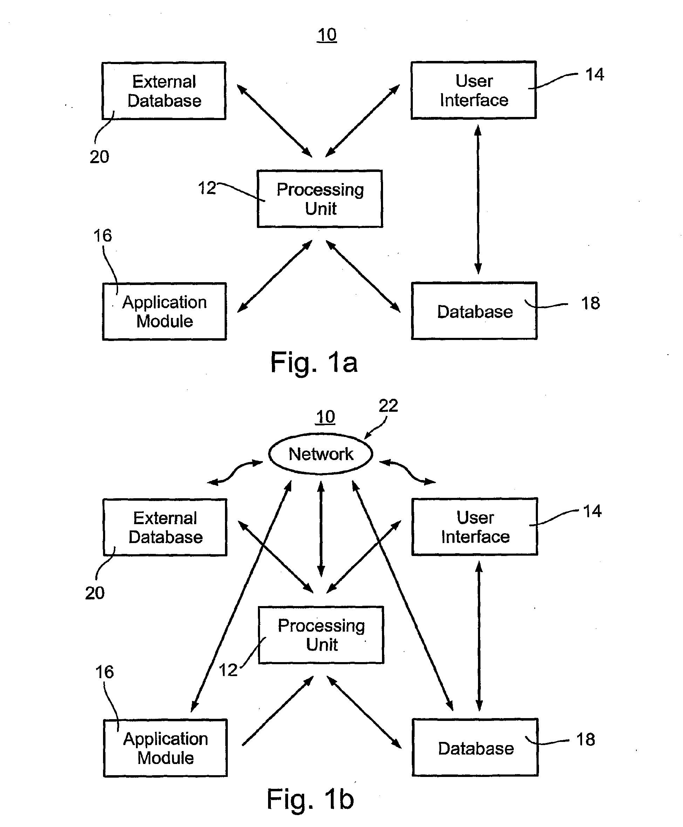

FIG. 1a illustrates a system designed and configured for generating a database of annotated biomolecular sequences according to the teachings of the present invention.

FIG. 1b illustrates a remote configuration of the system described in Figure 1a.

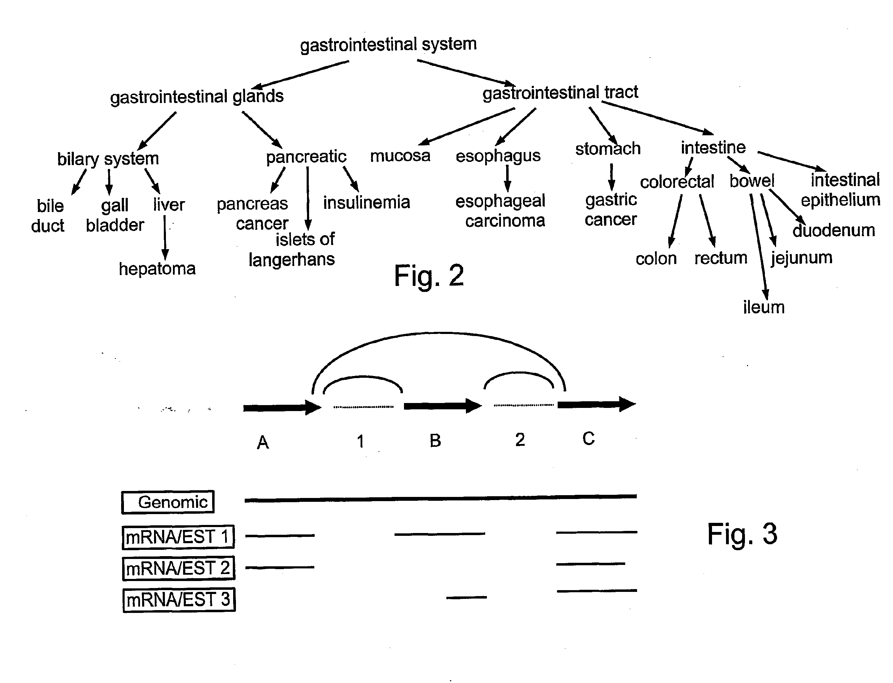

FIG. 2 illustrates a gastrointestinal tissue hierarchy dendrogram generated according to the teachings of the present invention.

FIG. 3 is a scheme illustrating multiple alignment of alternatively spliced expressed sequences with a genomic sequence including 3 exons (A, B and C) and two introns. Two alternative splicing events are described; One from the donor site, which involves an AB junction, between donor and proximal acceptor and an AC junction, between donor and distal acceptor; A Second alternative splicing event is described from the acceptor site, which involves AC junction, between distal donor and acceptor and BC junction, between proximal donor and acceptor.

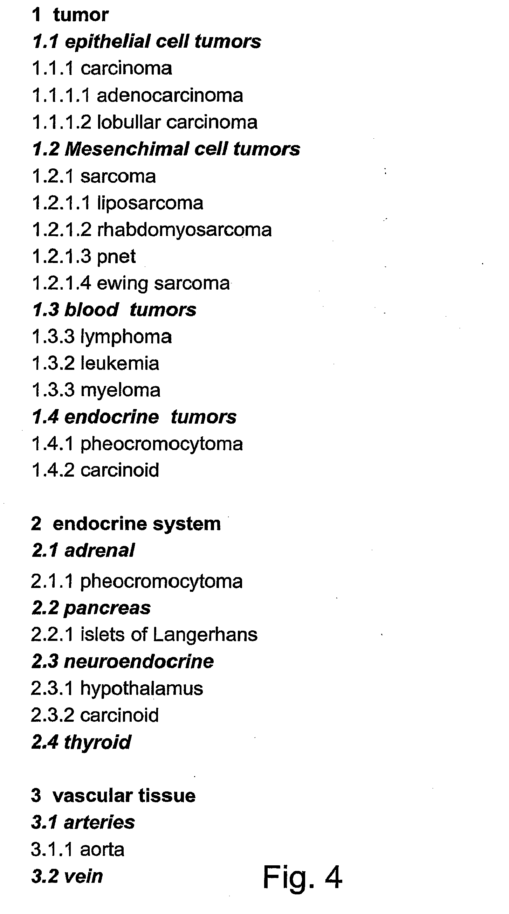

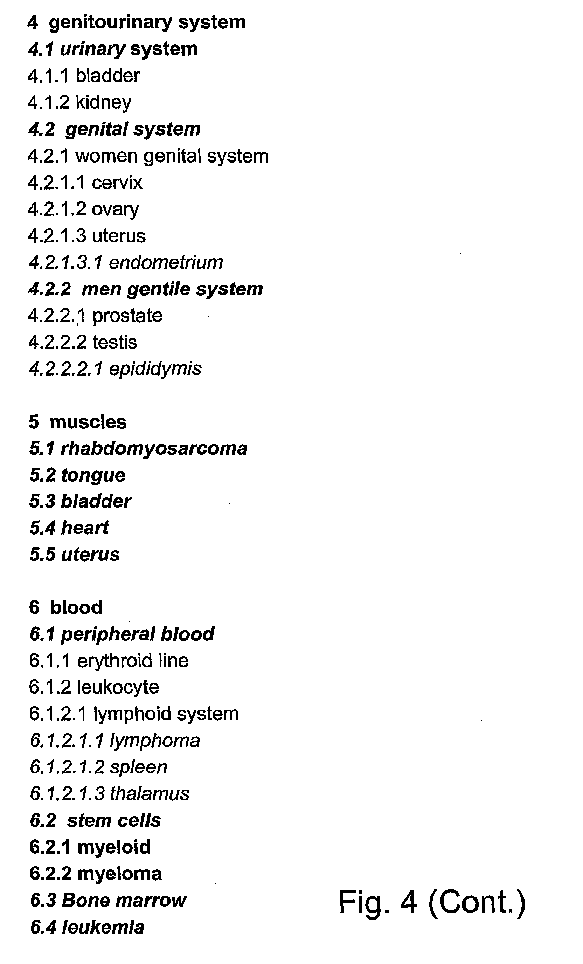

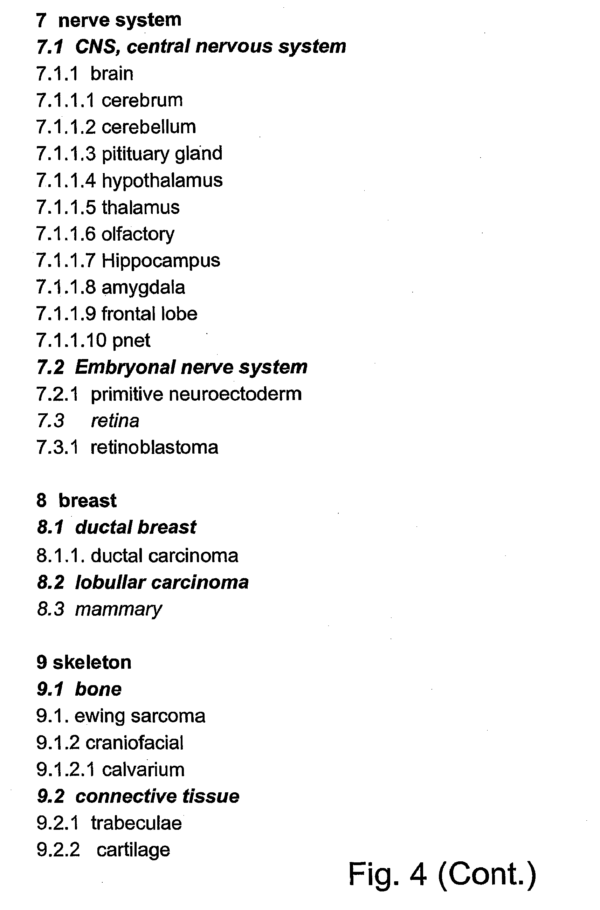

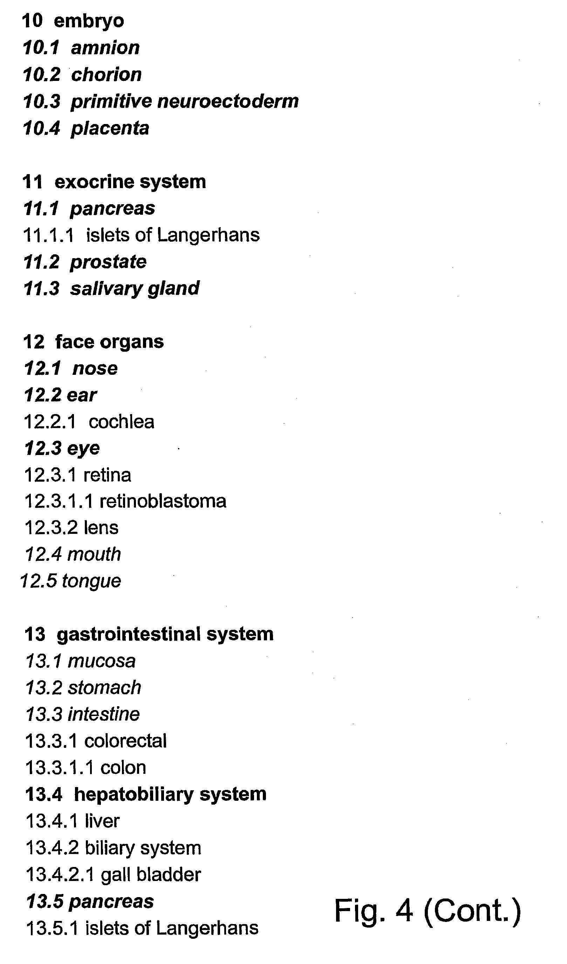

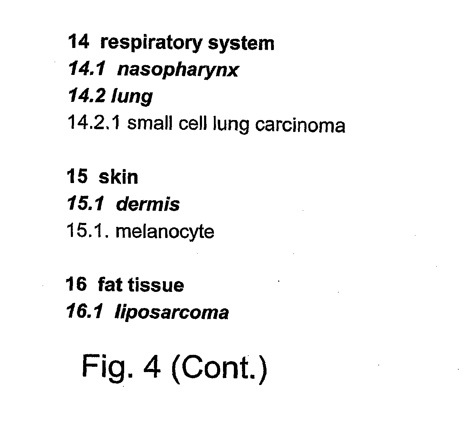

FIG. 4 is a tissue hierarchy dendogram generated according to the teachings of the present invention. The higher annotation levels are marked with a single number, i.e., 1-16. The lower annotation levels are marked within the relevant category as one - four numbers after the point (e.g. 4. genitourinary system; 4.2 genital system; 4.2.1 women genital system; 4.2.1.1 cervix).

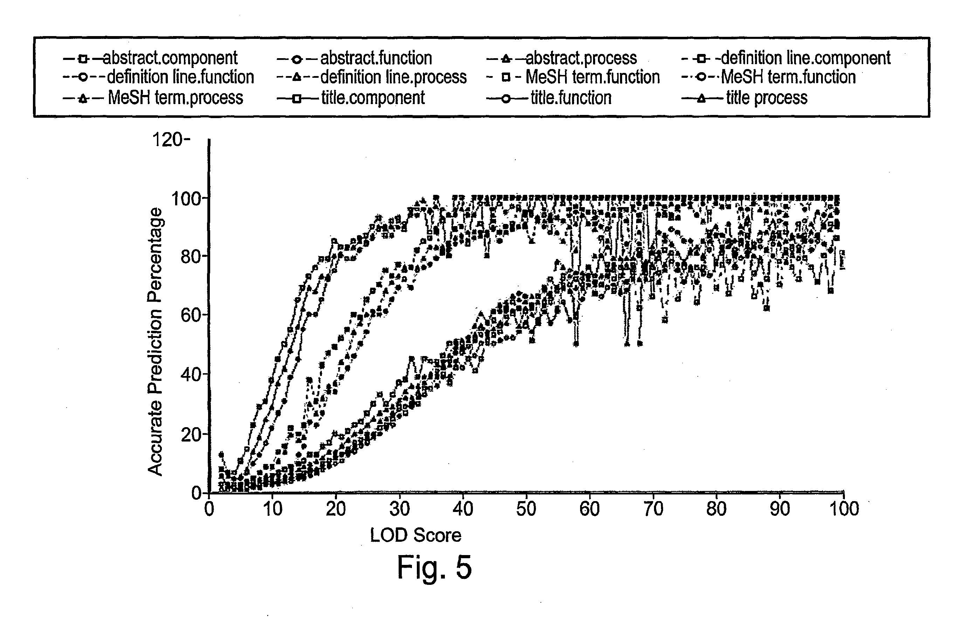

FIG. 5 is a graph illustrating a correlation between LOD scores of textual information analysis and accuracy of ontological annotation prediction. Results are based on self-validation studies. Only predictions made with LOD scores above 2 were evaluated and used for GO annotation process.

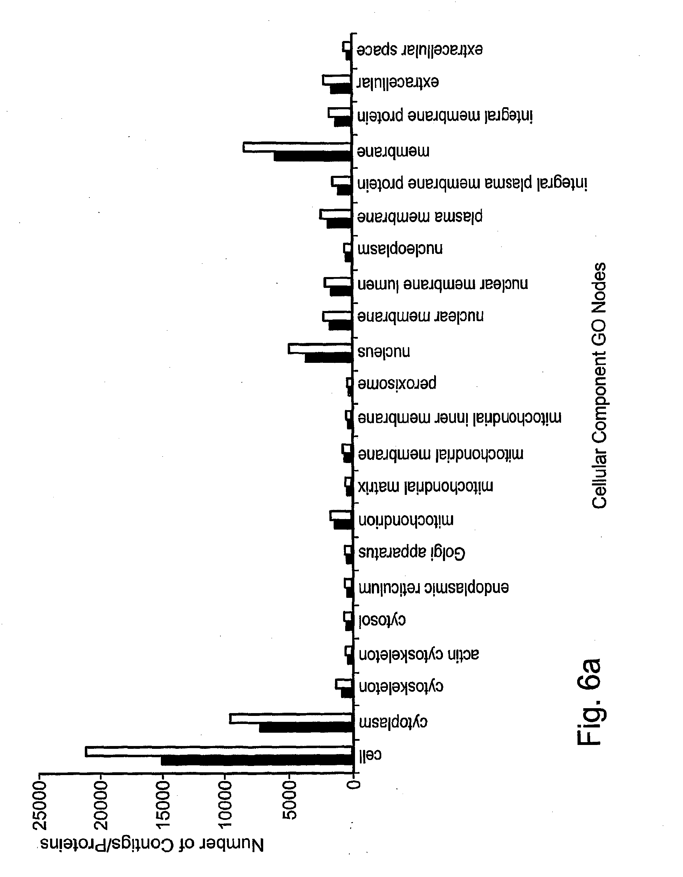

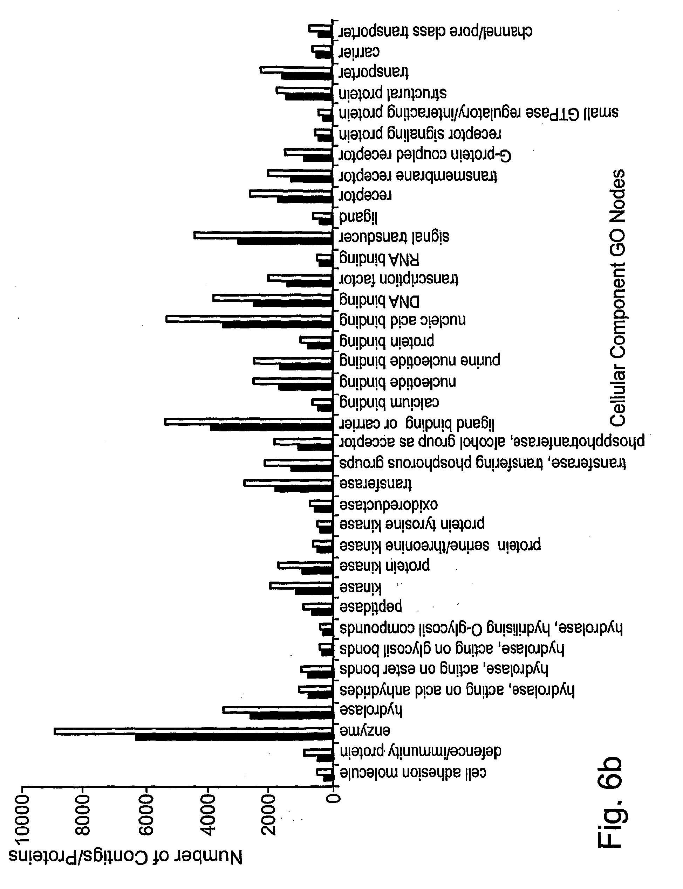

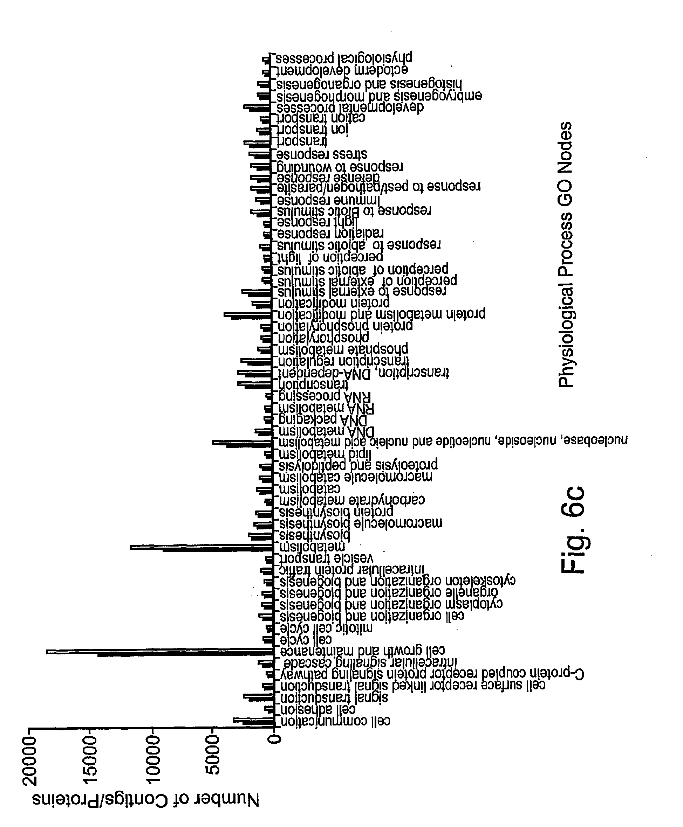

FIGs. 6a-c are histograms showing the distribution of proteins (closed squares) and contigs (opened squares) from Ensembl version 1.0.0 in the major nodes of three GO categories - cellular component (Figure 6a), molecular function (Figure 6b), and biological process (figure 6c).

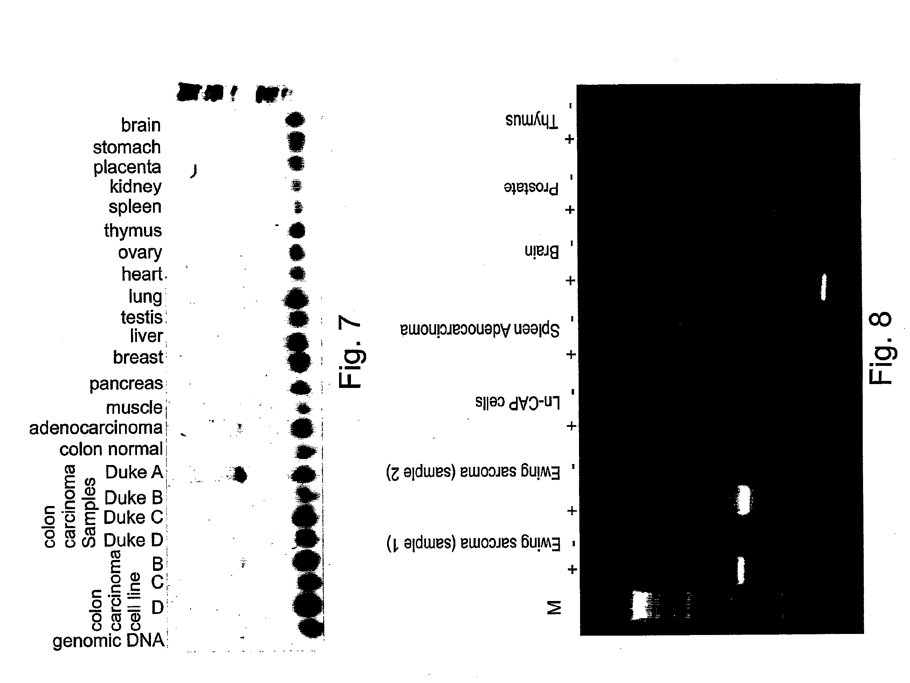

FIG. 7 illustrates results from RT-PCR analysis of the expression pattern of the AA535072 (SEQ ID NO: 39) colorectal cancer-specific transcript. The following cell and tissue samples were tested: B - colon carcinoma cell line SW480 (ATCC-228); C - colon carcinoma cell line SW620 (ATCC-227); D - colon carcinoma cell line colo-205 (ATCC-222). Colon normal tissue indicates a pool of 10 different samples, (Biochain, cat no A406029). The adenocarcinoma sample represents a pool of spleen, lung, stomach and kidney adenocarcinomas, obtained from patients. Each of the tissues (i.e., colon carcinoma samples Duke's A-D; and normal muscle, pancreas, breast, liver, testis, lung, heart, ovary, thymus, spleen kidney, placenta, stomach, brain) were obtained from 3-6 patients and pooled.

FIG. 8 illustrates results from RT-PCR analysis of the expression pattern of the AA513157 (SEQ II) NO: 7) Ewing sarcoma specific transcript. The (+) or (-) symbols, indicate presence or absence of reverse transcriptase in the reaction mixture. A molecular weight standard is indicated by M. Tissue samples (i.e., Ewing sarcoma samples, spleen adenocarcinoma, brain, prostate and thymus) were obtained from patients. The Ln-CAP human prostatic adenocarcinoma cell line was obtained from the ATCC (Manassas, VA).

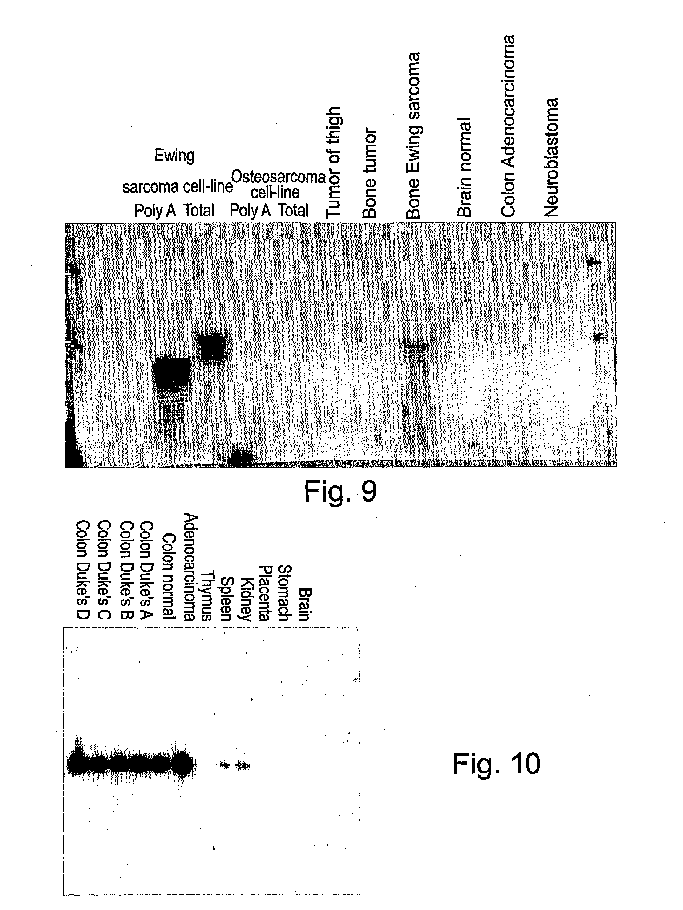

FIG. 9 is an autoradiogram of a northern blot analysis depicting tissue distribution and expression levels of AA513157 (SEQ ID NO: 7) Ewing sarcoma specific transcript. Arrows indicate the molecular weight of 28S and 18S ribosomal RNA subunits. The indicated tissue samples were obtained from patients and SK-ES-1 - Ewing sarcoma cell-line was obtained from the ATCC (CRL-1427).

FIG. 10 illustrates results from semi quantitative RT-PCR analysis of the expression pattern of the AA469088 (SEQ ID NO: 40) colorectal specific transcript Colon normal was obtained from Biochain, cat no: A406029. The adenocarcinoma sample represents a pool of spleen, lung, stomach and kidney adenocarcinomas, obtained from patients. Each of all other tissues (i.e., colon carcinoma samples Duke's A-D; and normal thymus, spleen, kidney, placenta, stomach, brain) were obtained from 3-6 patients and pooled.

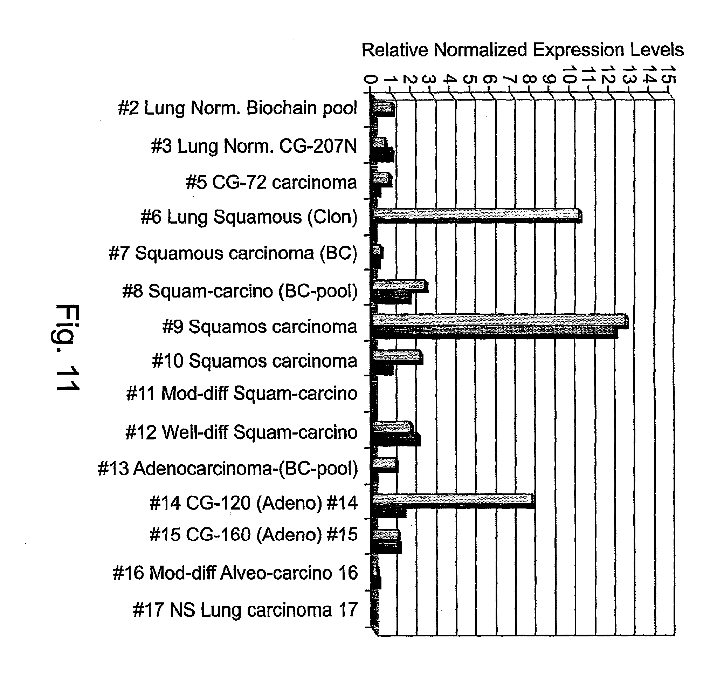

FIG. 11 is a histogram depicting Real-Time RT-PCR quantification of copy number, of a lung specific transcript, (SEQ ID NO: 15). Amplification products obtained from the following tissues were quantified; normal salivary gland from total RNA (Clontech, cat no:64110-1); lung normal from pooled adult total RNA (BioChain, cat no:A409363); lung tumor squamous cell carcinoma (Clontech, cat no:64013-1); lung tumor squamos cell carcinoma (BioChain, cat no:A409017); pooled lung tumor squamos cell carcinoma (BioChain, cat no: A411075); moderately differentiated squamos cell carcinoma (BioChain, cat no: A409091); well differentiated squamos cell carcinoma (BioChain, cat no: A408175); pooled adenocarcinoma (BioChain, cat no: A411076); moderately differentiated alveolus cell carcinoma (BioChain, cat no: A409089); non-small cell lung carcinoma cell line H1299; The following normal and tumor samples were obtained from patients: normal lung (internal number-CG-207N), lung carcinoma (internal number-CG-72), squamos cell carcinoma (internal number-CG-196), squamos cell carcinoma (internal number-CG-207), lung adenocarcinoma (internal number-CG-120), lung adenocarcinoma (internal number-CG-160). Copy number was normalized to the levels of expression of the housekeeping genes Proteasome 26S subunit (dark columns) and GADPH (bright columns).

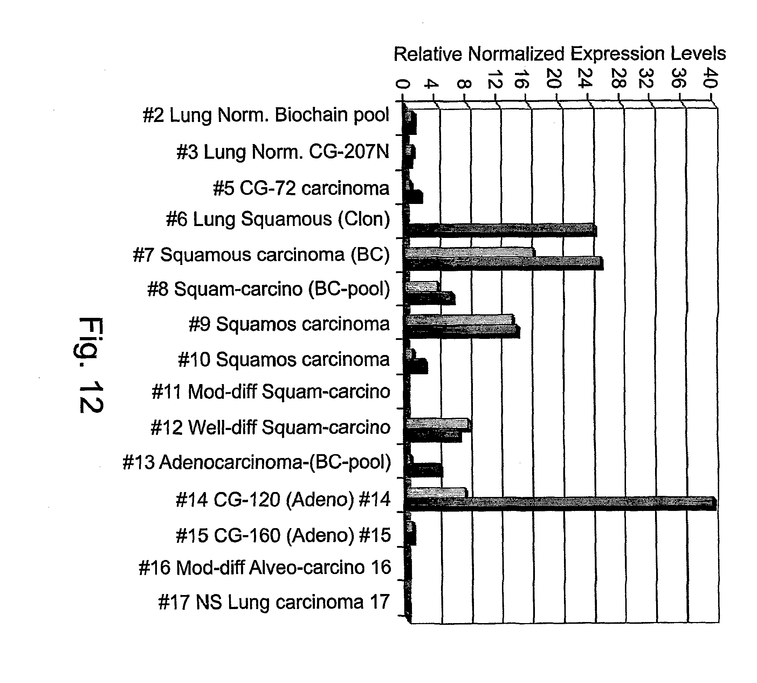

FIG. 12 is a histogram depicting Real-Time RT-PCR quantification of copy number, of the lung specific transcript (SEQ ID NO: 32). Amplification products obtained from the following tissues and cell-lines were quantified; lung normal from pooled adult total RNA (BioChain, cat no:A409363); lung tumor squamos cell carcinoma (Clontech, cat no:64013-1); lung tumor squamos cell carcinoma (BioChain, cat no:A409017); pooled lung tumor squamos cell carcinoma (BioChain, cat no: A411075); moderately differentiated squamos cell carcinoma (BioChain, cat no: A409091); well differentiated squamos cell carcinoma (BioChain, cat no: A408175); pooled adenocarcinoma (Bio-Chain, cat no: A411076); moderately differentiated alveolus cell carcinoma (BioChain, cat no: A409089); non-small cell lung carcinoma cell line H1299; The following normal and tumor samples were obtained from patients: normal lung (internal number-CG-207N), lung carcinoma (internal number-CG-72), squamos cell carcinoma (internal number-CG-196), squamos cell carcinoma (internal number-CG-207), lung adenocarcinoma (internal number-CG-120), lung adenocarcinoma (internal number-CG-160). Copy number was normalized to the levels of expression of the housekeeping genes Proteasome 26S subunit (dark columns) and GADPH (bright columns).

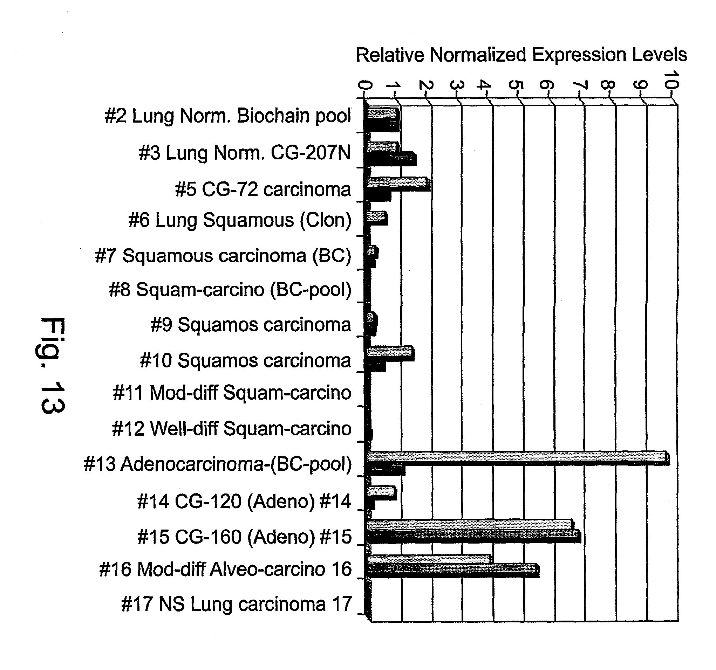

FIG. 13 is a histogram depicting Real-Time RT-PCR quantification of copy number, of the lung specific transcript (SEQ ID NO: 18). Amplification products obtained from the following tissues and cell-lines were quantified; lung normal from pooled adult total RNA (BioChain, cat no:A409363); lung tumor squamos cell carcinoma (Clontech, cat no:64013-1); lung tumor squamos cell carcinoma (BioChain, cat no:A409017); pooled lung tumor squamos cell carcinoma (BioChain, cat no: A411075); moderately differentiated squamos cell carcinoma (BioChain, cat no: A409091); well differentiated squamos cell carcinoma (BioChain, cat no: A408175); pooled adenocarcinoma (Bio-Chain, cat no: A411076); moderately differentiated alveolus cell carcinoma (BioChain, cat no: A409089); non-small cell lung carcinoma cell line H1299; The following normal and tumor samples were obtained from patients: normal lung (internal number-CG-207N), lung carcinoma (internal number-CG-72), squamos cell carcinoma (internal number-CG-196), squamos cell carcinoma (internal number-CG-207), lung adenocarcinoma (internal number-CG-120), lung adenocarcinoma (internal number-CG-160). Copy number was normalized to the levels of expression of the housekeeping genes Proteasome 26S subunit (dark columns) and GADPH (bright columns).

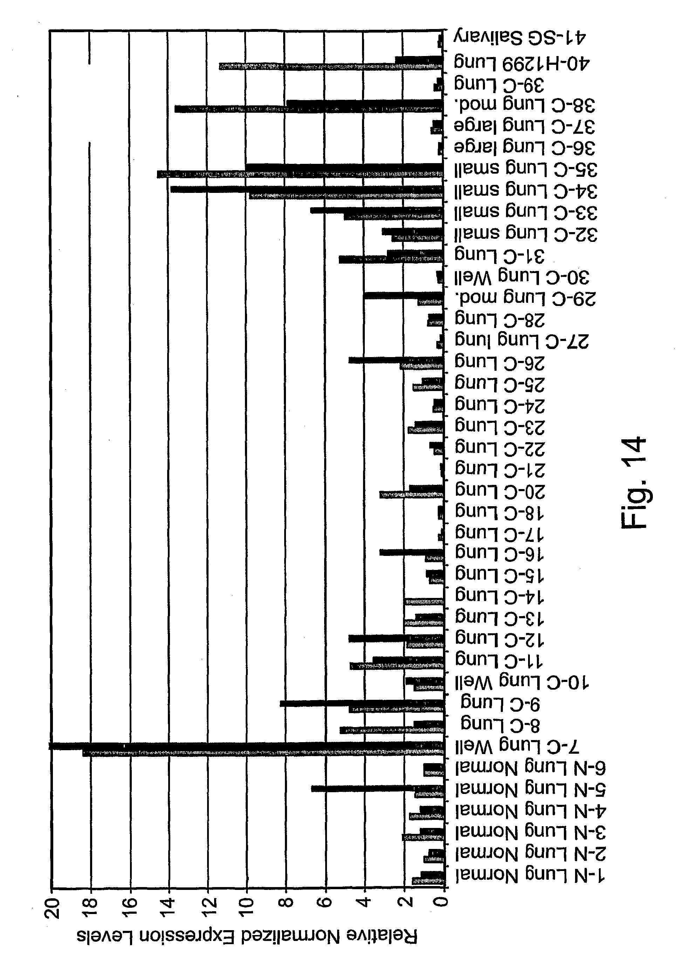

FIG. 14 is a histogram depicting Real-Time RT-PCR quantification of copy number, of a lung specific transcript (SEQ ID NO: 21). Amplification products obtained from the following tissues and cell-lines were quantified; Samples 1-6 are commercial normal lung samples (BioChain, CDP-061010; A503205, A503384, A503385, A503204, A503206, A409363). Sample 7 is lung well differentiated adenocarcinoma (BioChain, CDP-064004A; A504117). Sample 8 is lung moderately differentiated adenocarcinoma (BioChain, CDP-064004A; A504119). Sample 9 is lung moderately to poorly differentiated adenocarcinoma (BioChain, CDP-064004A; A504116). Sample 10 is lung well differentiated adenocarcinoma (BioChain, CDP-064004A; A504118). Samples11-16 are lung adenocarcinoma samples obtained from patients. Sample 17 is lung moderately differentiated squamous cell carcinoma (BioChain, CDP-064004B; A503187). Sample 18 is lung squamous cell carcinoma (BioChain, CDP-064004B; A503386). Samples 20-21 are lung moderately differentiated squamous cell carcinoma (BioChain, CDP-064004B; A503387, A503383). Sample 22 is lung squamous cell carcinoma pooled (BioChain, CDP-064004B; A411075). Samples 23-26 and sample 31 are lung squamous cell carcinoma obtained from patients. Sample 27 is lung squamous cell carcinoma (Clontech, 64013-1). Sample 28 is lung squamous cell carcinoma (BioChain, A409017). Sample 29 is lung moderately differentiated squamous cell carcinoma (BioChain, CDP-064004B; A409091). Sample 30 is lung well differentiated squamous cell carcinoma (BioChain, CDP-064004B; A408175). Samples 32-35 are lung small cell carcinoma (BioChain, CDP-064004D; A504115, A501390, A501389, A501391). Sample 36-37 are lung large cell carcinoma (BioChain, CDP-064004C; A504113, A504114). Sample 38 is lung moderately differentiated alveolus cell carcinoma (BioChain, A409089). Sample 39 is lung carcinoma obtained from patient Sample 40 is lung H1299 non-small cell carcinoma cell line. Sample 41 is normal salivary gland sample (Clontech, 64110-1). Copy number was normalized to the levels of expression of the housekeeping genes Proteasome 26S subunit (dark columns) and GADPH (bright columns).

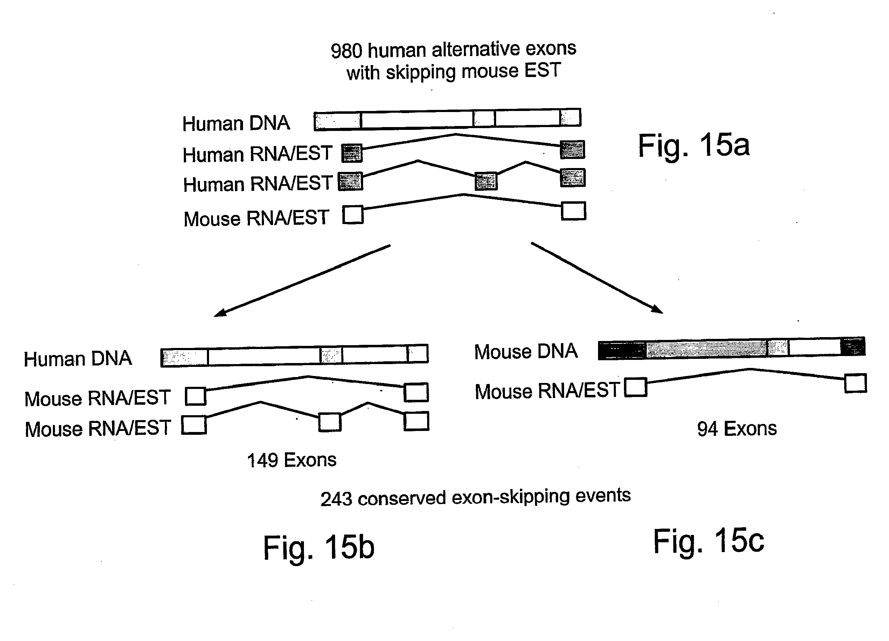

FIGs. 15a-c are schematic illustrations depicting the methodology undertaken for finding exon-skipping events which are conserved between human and mice genomes. 3,583 exon skipping events were found in the human genome using the methodology described in Sorek (2002) Genome Res. 12:1060-1067. Figure 15a - for 980 of these human exons, a mouse EST spanning the intron which represents the exon-skipping variant was found. Human ESTs are designated in purple. Mouse ESTs are denoted by light blue. Figures 15b-c depict two approaches for identifying exon conservation between mice and human. Figure 15b depicts the identification of mouse ESTs which contain the exon as well as the two flanking exons. Figure 15c illustrates a specific embodiment wherein the exon is absent in the mouse ESTs, in this case the human exon sequence is searched against the intron spanned by the skipping mouse EST on the mouse genome. If a significant conservation (i.e., above 80 %) was found and the alignment spanned the full length of the human exon, the exon was considered conserved.

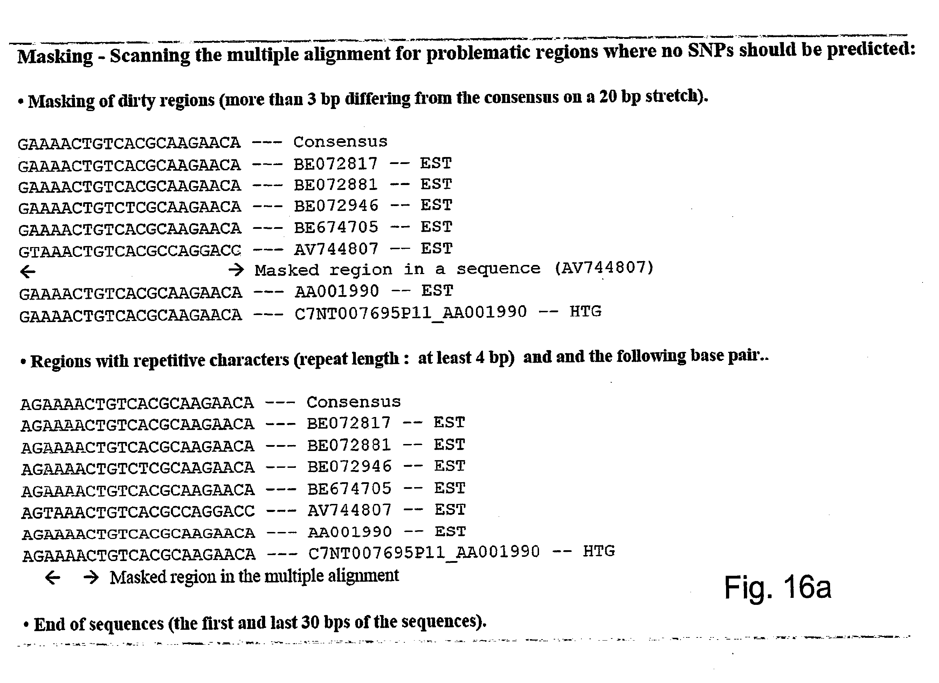

FIGs. 16a-d illustrate the stepwise methodology which is used to uncover true SNPs, as described in Example 22 of the Examples section.

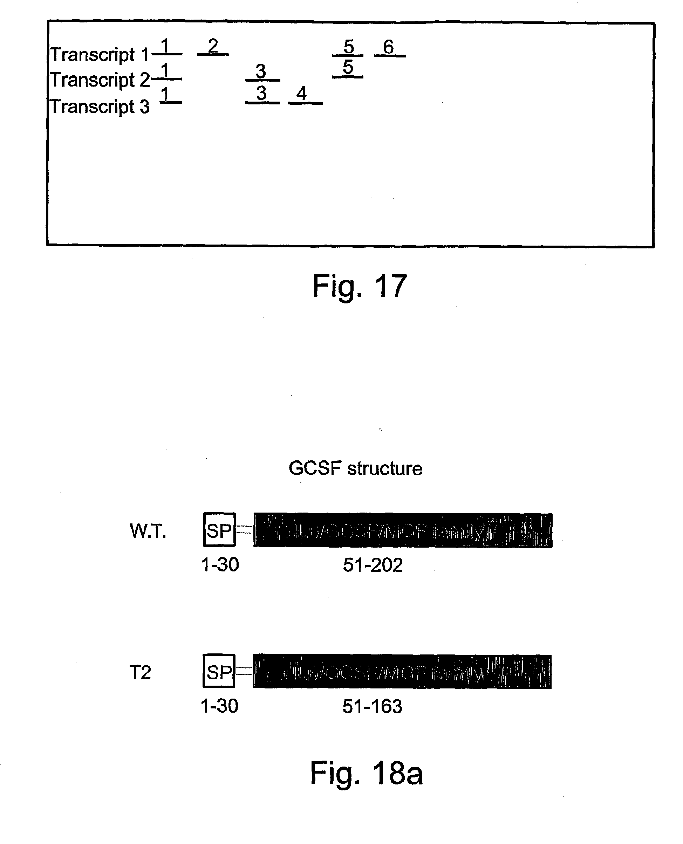

FIG. 17 is a schematic illustration, depicting grouping of transcripts of a given contig based on presence or absence of unique sequence regions. Region 1: common to all transcripts, thus it is not considered; Region 2: specific to T_1: T_1 unique regions (2+6) against T_2+3 unique regions (3+4); Region 3: specific to T_2+3: T_2+3 unique regions (3+4) against T1 unique regions (2+6); Region 4: specific to T_3: T_3 unique regions (4) against T1+2 unique regions (2+5+6); Region 5: specific to T_1+2: T_1+2 unique regions (2+5+6) against T3 unique regions (4); Region 6: specific to T_1: same as region 2.

FIG. 18a is a schematic illustration depicting the GCSF splice variant (SEQ ID NO: 68) as compared to the wild-type gene product.

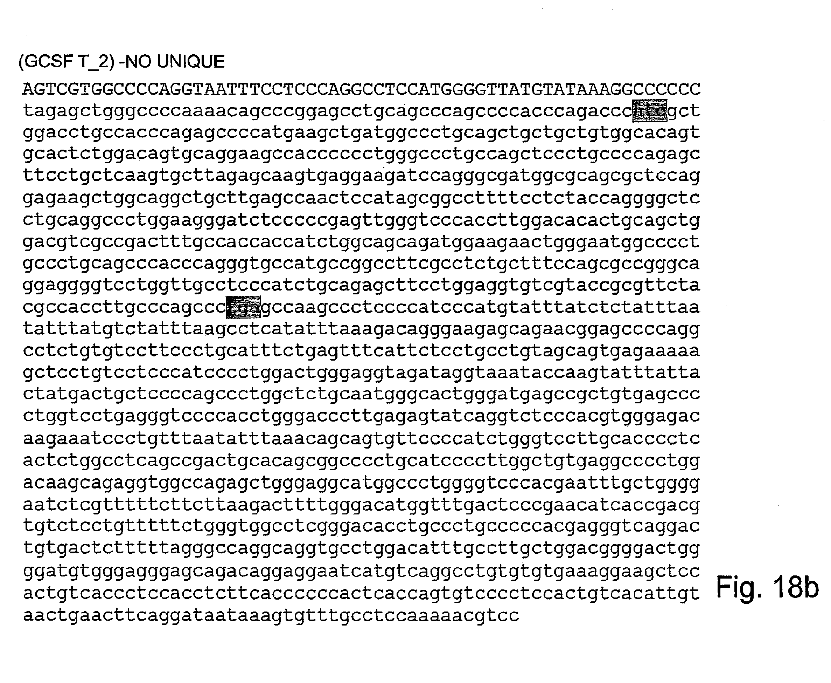

FIG. 18b present the nucleic acid sequence of the GCSF splice variant (SEQ ID NO: 71), which was uncovered using the teachings of the present invention. Start and stop codons are highlighted.

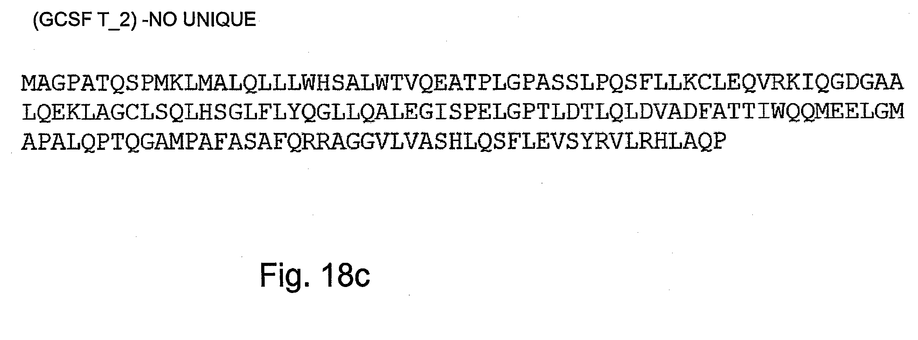

FIG. 18c present the amino acid sequence of the GCSF splice variant (SEQ ID NO: 68), which was uncovered using the teachings of the present invention.

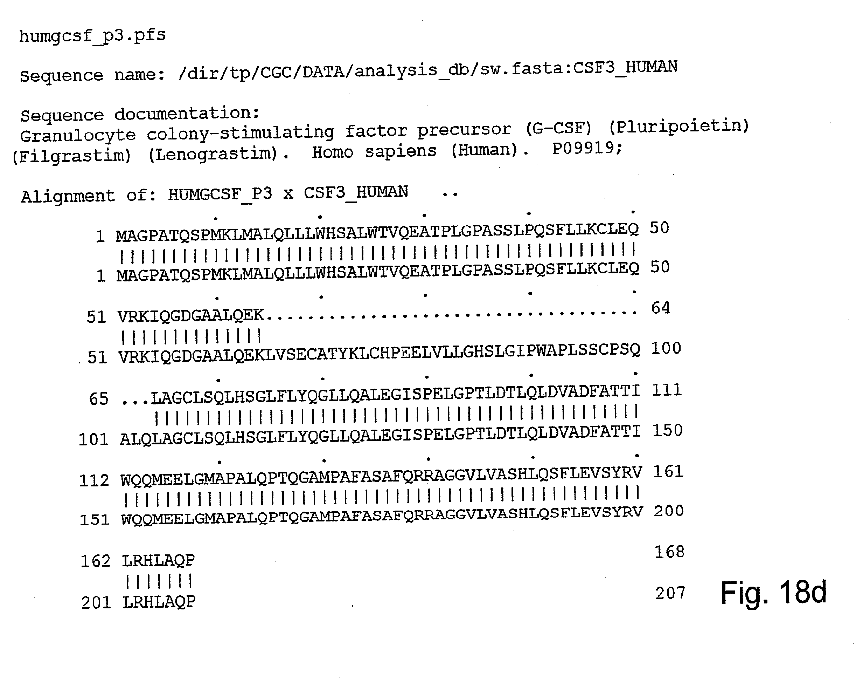

FIG. 18d is a sequence alignment depicting the protein product of a GCSF splice variant (SEQ ID NO: 68) as compared to the wild-type protein (Refsec Accession No. MN000759).

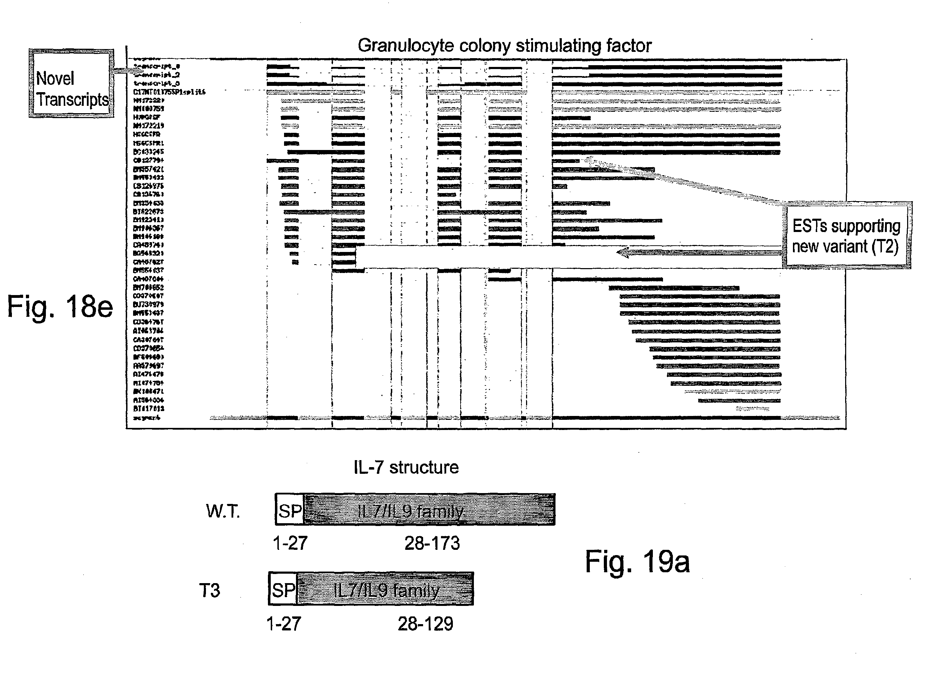

FIG. 18e is an illustration depicting a graphical viewer scheme presenting the a splice variant of GCSF (SEQ ID NO: 68) uncovered by the present invention as compared to the wild type mRNA of GCSF. ESTs supporting the variant are indicated. The transcript indicated as "0" represents known mRNA. The color code is as follows: red designates genomic DNA; pink designates Refseq mRNA; light blue designates known GenBank mRNAs; purple designates ESTs which are aligned in the same directionality as their annotation; gray designates ESTs without direction annotation; dark blue designates predicted transcripts; turquoise designates the predicted polypeptide.

FIG. 19a is a schematic illustration depicting the IL-7 splice variant (SEQ ID NO: 69) as compared to the wild-type gene product.

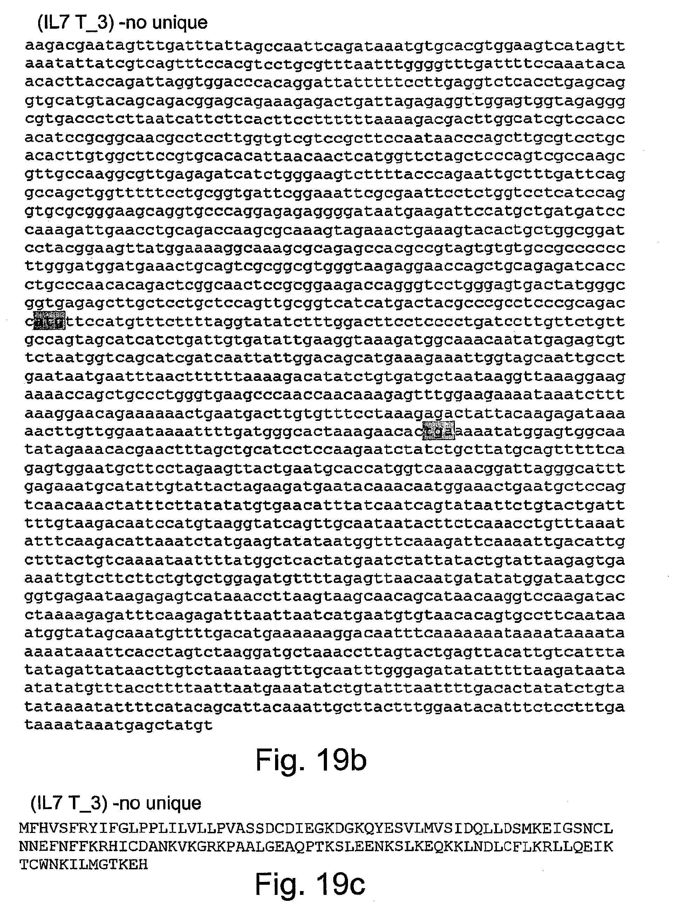

FIG. 19b present the nucleic acid sequence of the IL-7 splice variant (SEQ ID NO: 72), which was uncovered using the teachings of the present invention. Start and stop codons are highlighted.

FIG. 19c present the amino acid sequence of the IL-7 splice variant (SEQ ID NO: 69), which was uncovered using the teachings of the present invention.

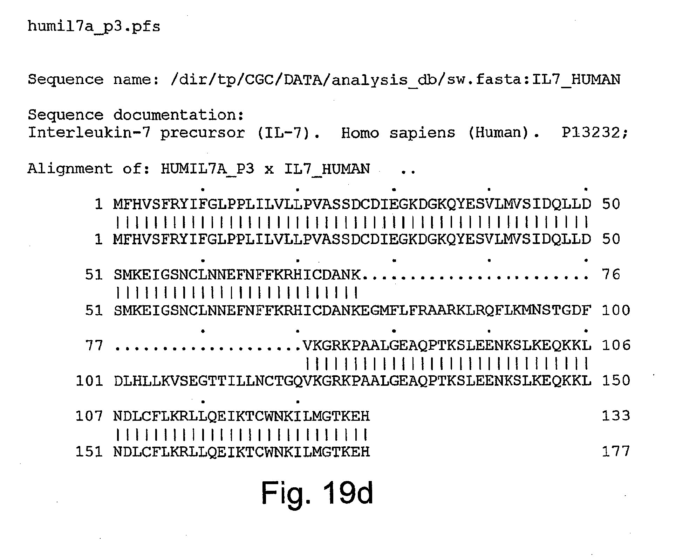

FIG. 19d is a sequence alignment depicting the protein product of an IL-7 splice variant (SEQ ID NO: 69) as compared to the wild-type protein (GenBank Accession No. IL7_HUMAN).

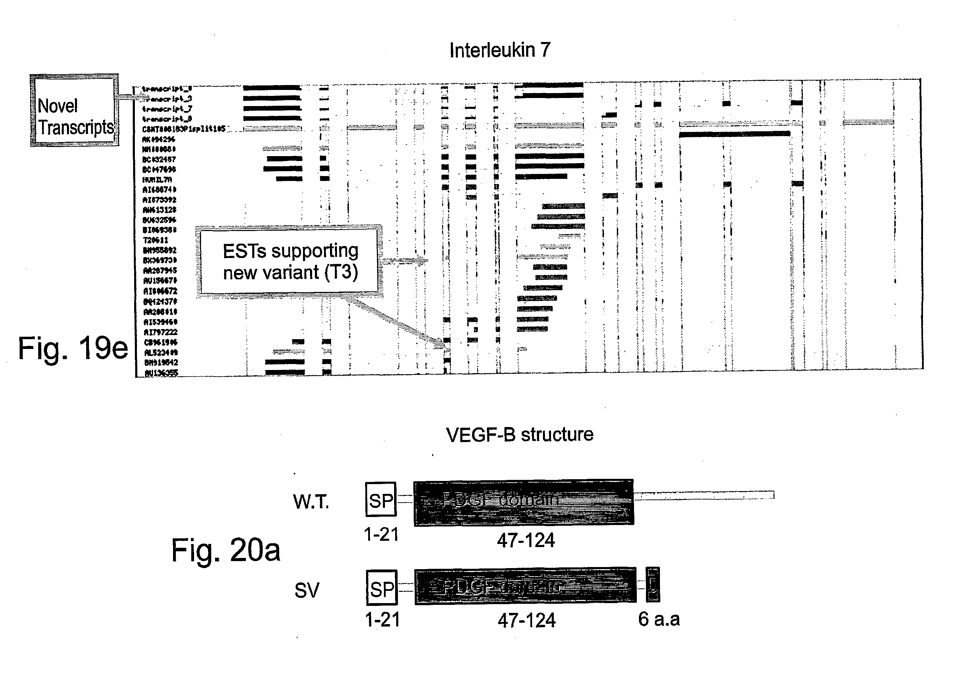

FIG. 19e is an illustration depicting a graphical viewer scheme presenting the a splice variant of IL-7 (SEQ ID NO: 69) uncovered by the present invention as compared to the wild type mRNA of IL-7. ESTs supporting the variant are indicated. The transcript indicated as "0" represents known mRNA. The color code is as follows: red designates genomic DNA; pink designates Refseq mRNA; light blue designates known GenBank mRNAs; purple designates ESTs which are aligned in the same directionality as their annotation; gray designates ESTs without direction annotation; dark blue designates predicted transcripts; turquoise designates the predicted polypeptide.

FIG. 20a is a schematic illustration depicting the VEGF-B splice variant (SEQ ID NO: 70) as compared to the wild-type gene product.

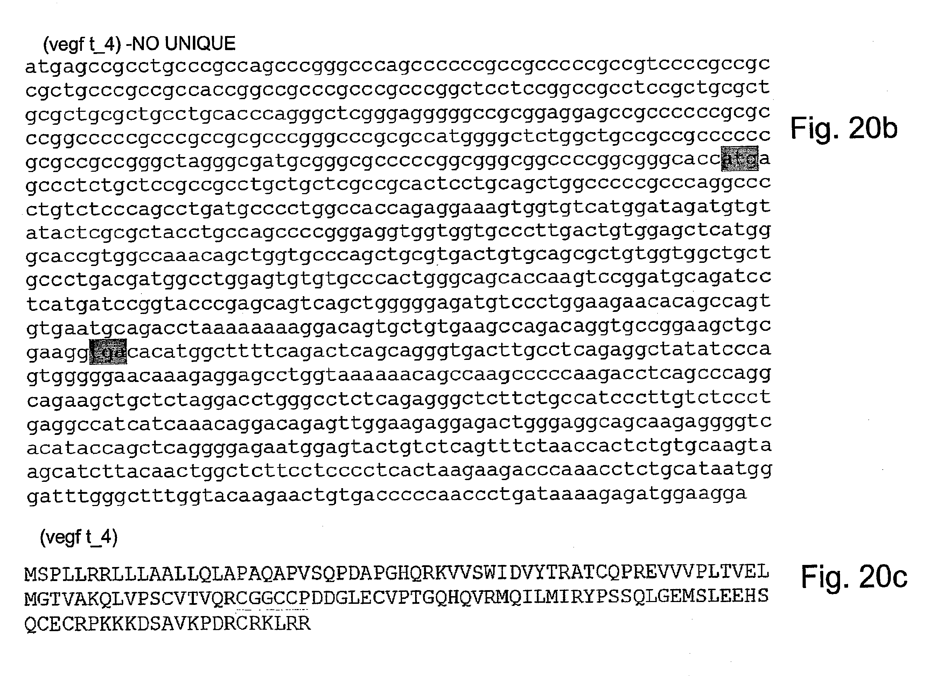

FIG. 20b present the nucleic acid sequence of the VEGF-B splice variant (SEQ ID NO: 73) which was uncovered using the teachings of the present invention. Start and stop codons are highlighted.

FIG. 20c present the amino acid sequence of the VEGF-B splice variant (SEQ ID NO: 70) which was uncovered using the teachings of the present invention.

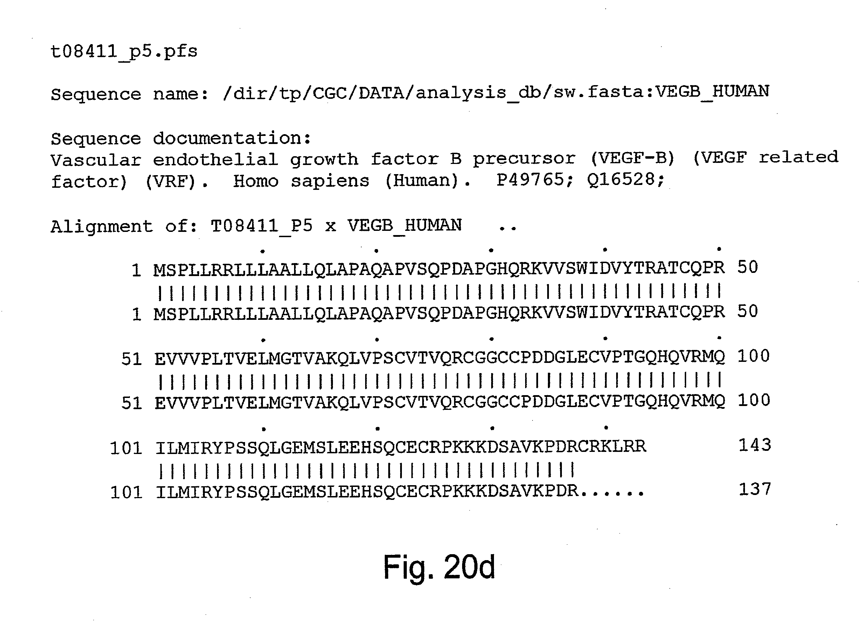

FIG. 20d is a sequence alignment depicting the protein product of a VEGF-B splice variant (SEQ ID NO: 70) as compared to the wild-type protein (GenBank accession No. VEGB_HUMAN).

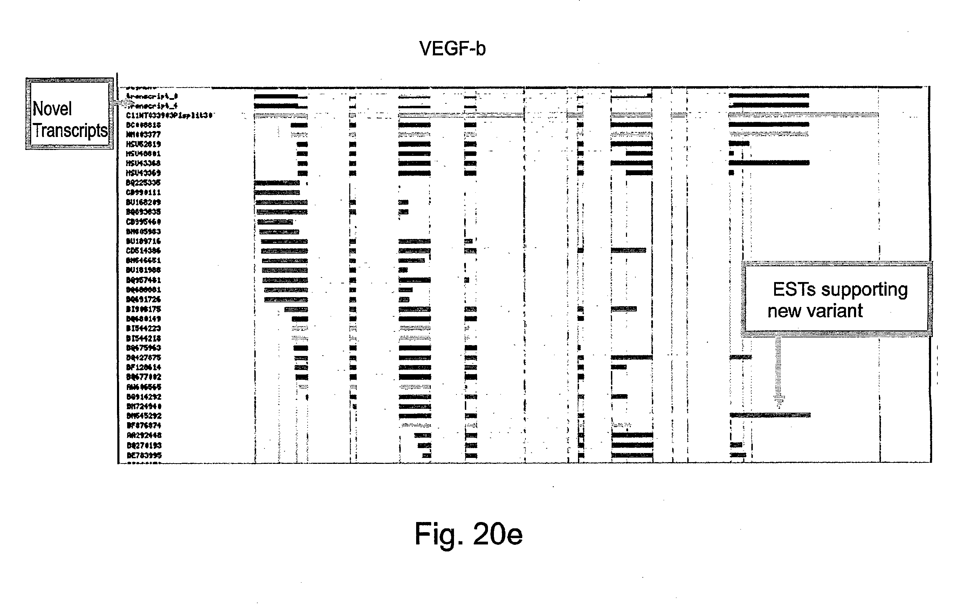

FIG. 20e is an illustration depicting a graphical viewer scheme presenting the a splice variant of VEGF-B (SEQ ID NO: 70) uncovered by the present invention as compared to the wild type mRNA of VEGF-B. ESTs supporting the variant are indicated. The transcript indicated as "0" represents known mRNA. The color code is as follows: red designates genomic DNA; pink designates Refseq mRNA; light blue designates known GenBank mRNAs; purple designates ESTs which are aligned in the same directionality as their annotation; black designates ESTs aligned in a direction opposite to the annotation; gray designates ESTs without direction annotation; dark blue designates predicted transcripts; turquoise designates the predicted polypeptide.

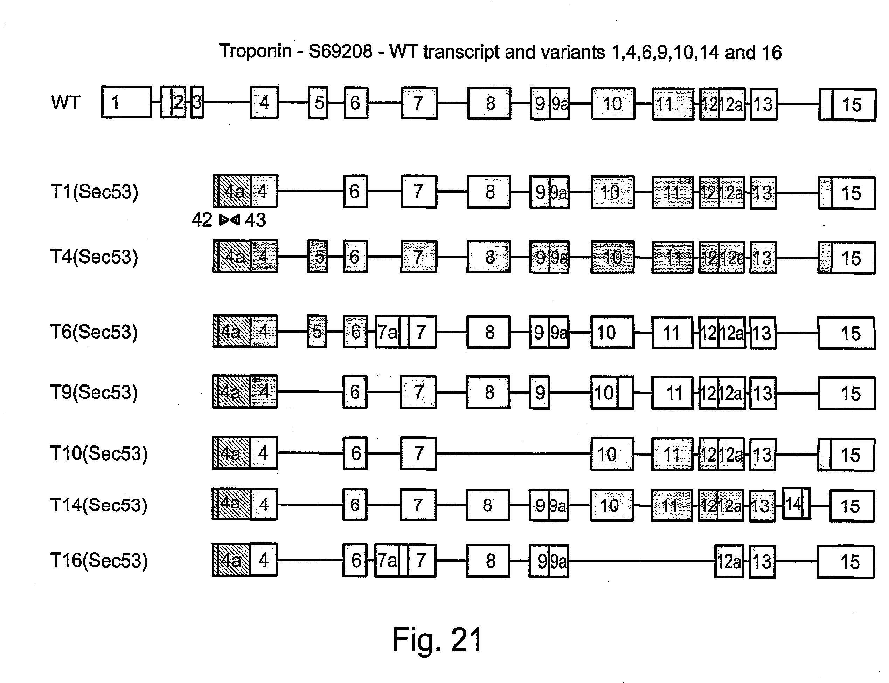

FIG. 21 is an illustration depicting schematic alignment of the nucleic acid sequences of wild type Troponin transcript (GenBank Accession No. NM_003283) and variants 1, 4, 6, 9, 10, 14 and 16 (SEQ ID NOs. 75, 77, 79, 81, 83, 66 and 67, respectively). Coding regions are marked by green. Sequence region 4a codes for the unique amino acid sequence and is marked by light green and diagonal stripes. Other regions marked in light green code for additional novel amino acids sequences. Red arrows indicate the location of the primers and SEQ ID NOs. thereof, which were used for real-time PCR validation.

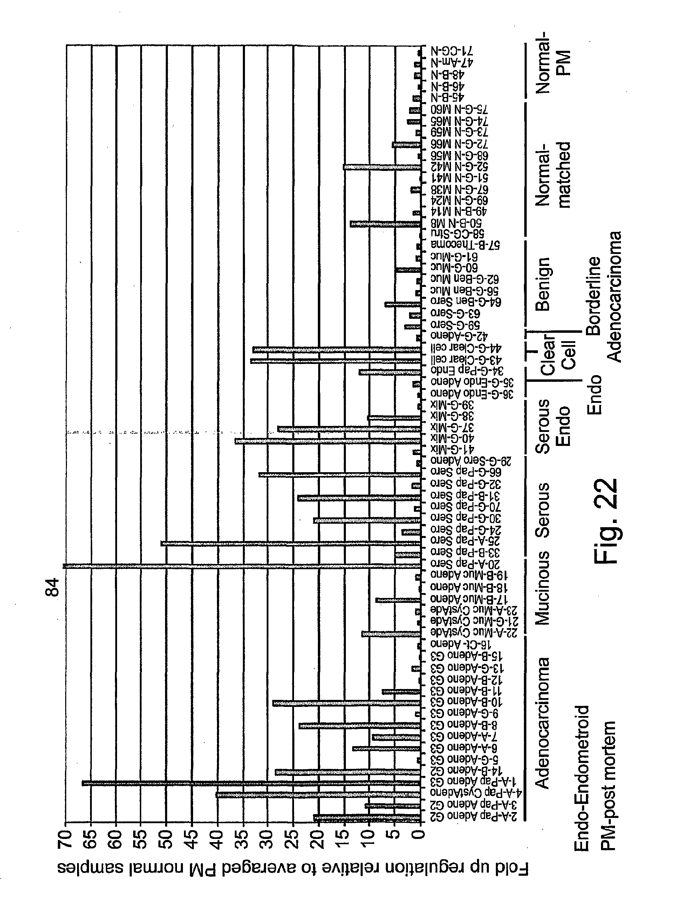

FIG. 22 is a histogram depicting the expression of troponin transcripts of the present invention in normal, benign and tumor derived ovarian samples as determined by real time PCR using a troponin-569208_unique_region derived fragment (SEQ ID NOs: 44 - amplicon). Expression was normalized to the averaged expression of four housekeeping genes PBGD, HPRT, GAPDH and SDHA.

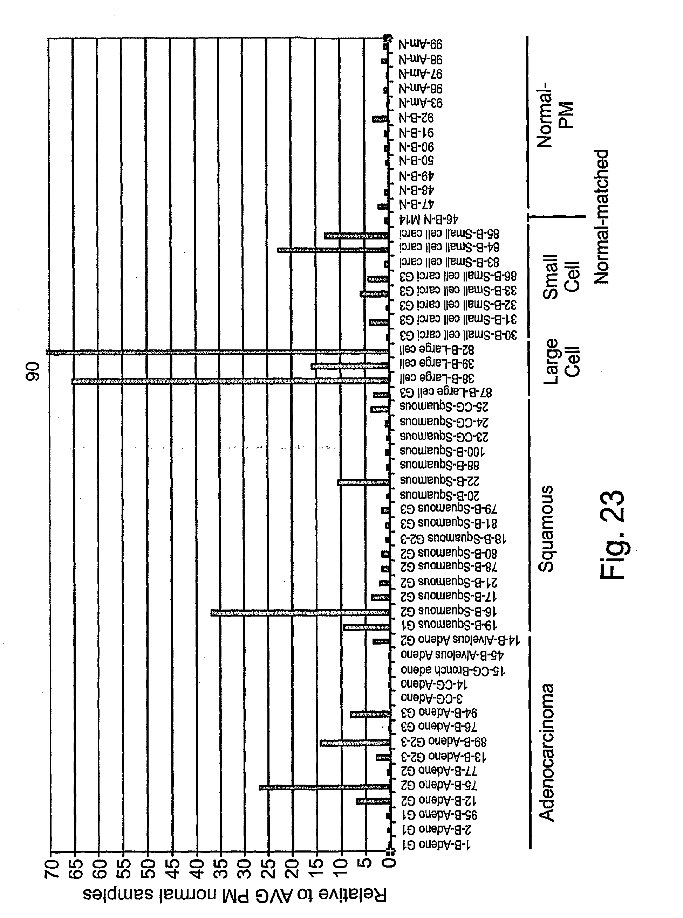

FIG. 23 is a histogram depicting the expression of troponin transcripts of the present invention in normal and tumor derived lung samples as determined by real time PCR using a troponin-S69208_unique_region derived fragment (SEQ ID NO: 44 - amplicon). Expression was normalized to the averaged expression of four housekeeping genes PBGD, HPRT, Ubiquitin and SDHA.

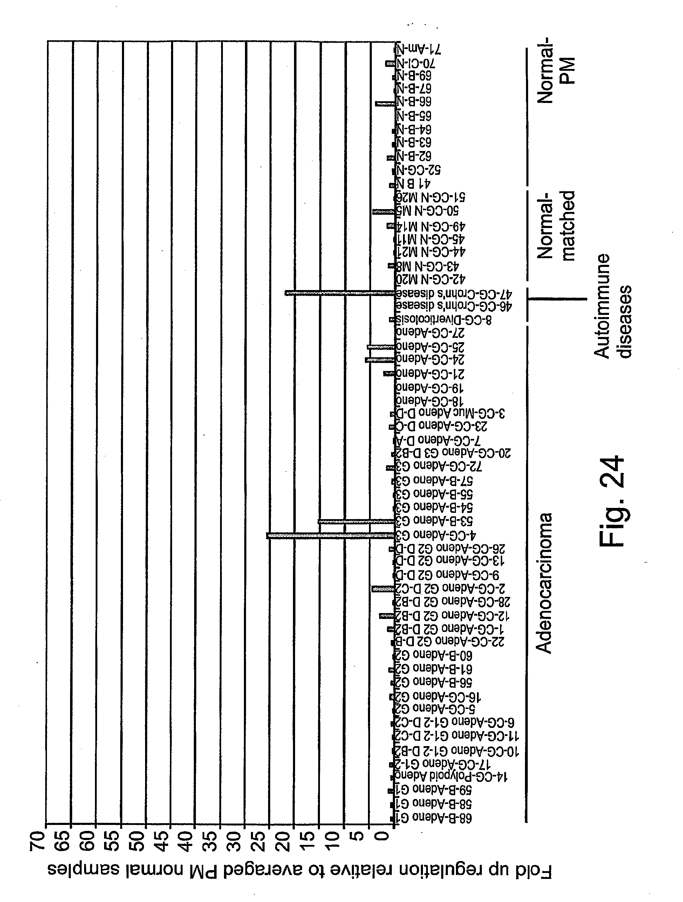

FIG. 24 is a histogram depicting the expression of troponin transcripts of the present invention in non-cancerous, and tumor derived colon samples as determined by real time PCR using a troponin-S69208_unique_region derived fragment (SEQ ID NOs: 44 - amplicon). Expression was normalized to the averaged expression of four housekeeping genes PBGD, HPRT, RPS27A and G6PD.

## DESCRIPTION OF THE PREFERRED EMBODIMENTS

The present invention is of methods and systems, which can be used for annotating biomolecular sequences. Specifically, the present invention can be used to identify and annotate differentially expressed biomolecular sequences, such as differentially expressed alternatively spliced sequences.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

### Terminology

As used herein, the term "oligonucleotide" refers to a single stranded or double stranded oligomer or polymer of ribonucleic acid (RNA) or deoxyribonucleic acid (DNA) or mimetics thereof. This term includes oligonucleotides composed of naturally-occurring bases, sugars and covalent internucleoside linkages (e.g., backbone) as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases.

The phrase "complementary DNA" (cDNA) refers to the double stranded or single stranded DNA molecule, which is synthesized from a messenger RNA template.

The term "contig" refers to a series of overlapping sequences with sufficient identity to create a longer contiguous sequence. A plurality of contigs may form a cluster. Clusters are generally formed based upon a specified degree of homology and overlap (e.g., a stringency), and/or based on prior knowledge of ESTs from different contigs derived from the same mRNA also known as clone mates. The different contigs in a cluster do not typically represent the entire sequence of the gene, rather the gene may comprise one or more unknown intervening sequences between the defined contigs.

The term "cluster" refers to a nucleic acid sequence cluster or a protein sequence cluster. The former refers to a group of nucleic acid sequences which share a requisite level of homology and or other similar traits according to a given clustering criterion; and the latter refers to a group of protein sequences which share a requisite level of homology and/or other similar traits according to a given clustering criterion.

A process and/or method to group nucleic acid or protein sequences as such is referred to as clustering, which is typically performed by a clustering (i.e., alignment) application program implementing a cluster algorithm.

As used herein the phrase "biomolecular sequences" refers to amino acid sequences (i.e., peptides, polypeptides) and nucleic acid sequences, which include but are not limited to genomic sequences, expressed sequence tags, contigs, complementary DNA (cDNA) sequences, pre-messenger RNA (mRNA) sequences, and mRNA sequences. Expressed sequences include also products of alternative splicing or RNA editing events which are well known for contributing to gene product diversity [Krevintseva Trends Genet. (2003) 19(3):124-8; Keegan (2001) Nat Rev. Genet. 2:869-78; Schaub (2002) Biobhimie 84:791-803; Adler (1994) Curr. Opin. Genet Dev. 4:316-22].

As used herein the phrase "functionally altered biomolecular sequences" refers to expressed sequences, (e.g., alternatively spliced sequences) which protein products exhibit gain of function or loss of function or modification of the original function.

As used herein the phrase "gain of function" refers to any gene product (e.g., product of alternative splicing, product of RNA editing), which exhibits increased functionality as compared to the wild type gene product. Such a gain of function may have a dominant effect on the wild-type gene product. An alternatively spliced variant of Max, a binding partner of the Myc oncogene, provides a typical example for a "gain of function" alteration. This variant is truncated at the COOH-terminus and while is still capable of binding to the CACGTG motif of c-Myc, it lacks the nuclear localization signal and the putative regulatory domain of Max. When tested in a myc-ras cotransformation assay in rat embryo fibroblasts, wild-type Max suppressed cellular transformation, whereas the above-described Max splice variant enhanced transformation [Makela TP, Koskinen PJ, Yastrik I, Alitalo K., Science. 1992 Apr 17;256(5055):373-7].

As used herein the phrase "loss of function" refers to any gene product (mRNA or protein), which exhibits total or partial reduction in function as compared to the wild type gene product. Loss of function can also manifest itself through a dominant negative effect.

As used herein the phrase "dominant negative" refers to the dominant negative effect of a gene product (e.g., product of alternative splicing, product of RNA editing) on the activity of wild type protein. For example, a protein product of an altered splice variant may bind a wild type target protein without enzymatically activating it (e.g., receptor dimers), thus blocking and preventing the active enzymes from binding and activating the target protein. This mode of action provides a mechanism to the dominant negative action of soluble receptors on wild-type membrane anchored receptors. Such soluble receptors may compete with wild-type receptors on ligand-binding and as such may be used as antagonists. For example, two splice variants of guanylyl cyclase-B receptor were recently described (GC-B1, Tamura N and Garbers DL, J. Biol. Chem. (2003) 278(49):48880-9). One form has a 25 amino acid deletion in the kinase homology domain. This variant binds the ligand but fails to activate the cyclase. A second variant includes only a portion of the extracellular domain. This form fails to bind the ligand. Both variants. When co-expressed with the wild-type receptor both act as dominant negative isoforms by virtue of blocking formation of active GC-B1 homodimers.

A dominant negative effect may also be exerted by miss-localization of the altered variant or by mutiple modes of action. For example, the splice variants of wild-type mytogen activated protein kinase 5a, ERK5b and mERK5c act as dominant negative inhibitors based on inhibition of mERK5a kinase activity and mERK5a-mediated MEF2C transactivation. The C-terminal tail, which contains a putative nuclear localization signal, is not required for activation and kinase activity but is responsible for the activation of nuclear transcription factor MEF2C due to nuclear targeting. In addition, the N-terminal domain spanning amino acids (aa) 1-77 is important for cytoplasmic targeting; the domain from aa 78 to 139 is required for association with the upstream kinase MEK5; and the domain from aa 140-406 is necessary for oligomerization [Yan et al. J Biol Chem. (2001) 276(14):10870-8].

The phrase "modification of the original function" may be exemplified by a changing a receptor function to a ligand function. For example, a soluble secreted receptor may exhibit change in functionality as compared to a membrane-anchored wild-type receptor by acting as a ligand, activating parallel signaling pathways by trans-signaling [e.g., the signaling reported for soluble IL-6R, Kallen Biochim Biophys Acta. (2002) Nov 11;1592(3):323-43], stabilizing ligand-receptor interactions or protecting the ligand or the wild-type receptor from degradation and/or prolonging their half-life.

In this case the soluble receptor will function as an agonist.

As used herein the term "modulator" refers to a molecule which inhibits (i.e., antagonist, inhibitor, suppressor) or activates (i.e., agonist, stimulant, activator) a downstream molecule to thereby modulate it's activity.

As used herein the phrase "functional domain" refers to a region of a biomolecular sequence, which displays a particular function. This function may give rise to a biological, chemical, or physiological consequence which may be reversible or irreversible and which may include protein-protein interactions (e.g., binding interactions) involving the functional domain, a change in the conformation or a transformation into a different chemical state of the functional domain or of molecules acted upon by the functional domain, the transduction of an intracellular or intercellular signal, the regulation of gene or protein expression, the regulation of cell growth or death, or the activation or inhibition of an immune response.

With the presentation of the human genome working draft, data analysis rather than data collection presents the biggest challenge to biologists. Efforts to ascribe biological meaning to genomic data, include the development of advanced wet laboratorial techniques as well as computerized algorithms. While the former are limited due to inaccuracy, time consumption, labor intensiveness and costs the latter are still unfeasible due to the poor organization of on hand sequence databases as well as the composite nature of biological data.

As is further described hereinbelow, the present inventors have developed a computer-based approach for the functional, spatial and temporal analysis of biological data. The present methodology generates comprehensive databases, which greatly facilitate the use of available genetic information in both research and commercial applications.

By applying the algorithms described hereinbelow and in the Examples section, which follows, the present inventors collected sequence information and corresponding sequence annotations as set forth in the files "Transcripts_nucleotide_seqs_part1" of CD_ROM1, "Transcripts_nucleotide_seqs_part2", "Transcripts_nucleotide_seqs_part3", "protein_seqs", "ProDG_seqs", "Transcripts_nucleotide_seqs_part4" of CD-ROM2, "summary_table" of CD-ROM3, "Annotations.gz", "Transcripts.gz", and "Proteins.gz" of enclosed CD_ROM4. This comprehensive database allows simple elucidation of yet unknown function of mass gene products and illustrates spatial and temporal patterns of gene expression in various types of tissues, such as healthy and diseased, which may contribute immensely to further understanding of disease mechanisms and allow use thereof in the configuration of therapeutic and diagnostic applications.

As is further described hereinunder, the present invention encompasses several novel approaches for annotating biomolecular sequences which can be individually applied or in combination.

"Annotating" refers to the act of discovering and/or assigning an annotation (i.e., critical or explanatory notes or comment) to a biomolecular sequence of the present invention.

The term "annotation" refers to a functional or structural description of a sequence, which may include identifying attributes such as locus name, keywords, Medline references, cloning data, single nucleotide polymorphism data, information of coding region, regulatory regions, catalytic regions, name of encoded protein, subcellular localization of the encoded protein, protein hydrophobicity, protein function, mechanism of protein function, information on metabolic pathways, regulatory pathways, protein-protein interactions, tissue expression profile, diseases and disorders (i.e., indications), therapies, pharmacological activities and diagnostic applications.

### The ontological annotation approach

An ontology refers to the body of knowledge in a specific knowledge domain or discipline such as molecular biology, microbiology, immunology, virology, plant sciences, pharmaceutical chemistry, medicine, neurology, endocrinology, genetics, ecology, genomics, proteomics, cheminformatics, pharmacogenomics, bioinformatics, computer sciences, statistics, mathematics, chemistry, physics and artificial intelligence.

An ontology includes domain-specific concepts - referred to herein as subontologies. A sub-ontology may be classified into smaller and narrower categories.

The ontological annotation approach of the present invention is effected as follows.

First, biomolecular sequences are computationally clustered according to a progressive homology range, thereby generating a plurality of clusters each being of a predetermined homology of the homology range.

Progressive homology according to this aspect of the present invention is used to identify meaningful homologies among biomolecular sequences and thereby assign new ontological annotations to sequences, which share requisite levels of homologies. Essentially, a biomolecular sequence is assigned to a specific cluster if displays a predetermined homology to at least one member of the cluster (i.e., single linkage). As used herein "progressive homology range" refers to a range of homology thresholds, which progress via predetermined increments from a low homology level (e.g. 35 %) to a high homology level (e.g. 99 %). Further description of a progressive homology range is provided in the Examples section which follows.

Following generation of clusters, one or more ontologies are assigned to each cluster. Ontologies are derived from an annotation preassociated with at least one biomolecular sequence of each cluster; and/or generated by analyzing (e.g., text-mining) at least one biomolecular sequence of each cluster thereby annotating biomolecular sequences.

Any annotational information identified and/or generated according to the teachings of the present invention can be stored in a database which can be generated by a suitable computing platform.

Thus, the method according to this aspect of the present invention provides a novel approach for annotating biomolecular sequences even on a scale of a genome, a transcriptom (i.e., the repertoire of all messenger RNA molecules transcribed from a genome) or a proteom (i.e., the repertoire of all proteins translated from messenger RNA molecules). This enables transcriptome-wise comparative analyses (e.g., analyzing chromosomal distribution of human genes) and cross-transcriptome comparative studies (e.g., comparing expressed data across species) both of which may involve various subontologies such as molecular function, biological process and cellular localization.

Biomolecular sequences which can be used as working material for the annotating process according to this aspect of the present invention can be obtained from a biomolecular sequence database. Such a database can include protein sequences and/or nucleic acid sequences derived from libraries of expressed messenger RNA [i.e., expressed sequence tags (EST)], cDNA clones, contigs, pre-mRNA, which are prepared from specific tissues or cell-lines or from whole organisms.

This database can be a pre-existing publicly available database [i.e., GenBank database maintained by the National Center for Biotechnology Information (NCBI), part of the National Library of Medicine, and the TIGR database maintained by The Institute for Genomic Research, Blocks database maintained by the Fred Hutchinson Cancer Research Center, Swiss-Prot site maintained by the University of Geneva and GenPept maintained by NCBI and including public protein-sequence database which contains all the protein databases from GenBank,] or private databases (i.e., the LifeSeq.™ and PathoSeq.™ databases available from Incyte Pharmaceuticals, Inc. of Palo Alto, CA). Optionally, biomolecular sequences of the present invention can be assembled from a number of pre-existing databases as described in Example 5 of the Examples section.

Alternatively, the database can be generated from sequence libraries including, but not limited to, cDNA libraries, EST libraries, mRNA libraries and the like.

Construction and sequencing of a cDNA library is one approach for generating a database of expressed mRNA sequences. cDNA library construction is typically effected by tissue or cell sample preparation, RNA isolation, cDNA sequence construction and sequencing.

It will be appreciated that such cDNA libraries can be constructed from RNA isolated from whole organisms, tissues, tissue sections, or cell populations. Libraries can also be constructed from a tissue reflecting a particular pathological or physiological state.

Once raw sequence data is obtained, biomolecular sequences are computationally clustered according to a progressive homology range using one or more clustering algorithms. To obtain progressive clusters, the biomolecular sequences are clustered through single linkage. Namely, a biomolecular sequence belongs to a cluster if this sequence shares a sequence homology above a certain threshold to one member of the cluster. The threshold increments from a high homology level to a low homology level with a predetermined resolution. Preferably the homology range is selected from 99 %-35 %.

Computational clustering can be effected using any commercially available alignment software including the local homology algorithm of Smith & Waterman, Adv. Appl. Math. 2:482 (1981), using the homology alignment algorithm of Needleman & Wunsch, J. Mol. Biol. 48:443 (1970), using the search for similarity method of Pearson & Lipman, Proc. Nat'l. Acad. Sci. USA 85:2444 (1988), or using computerized implementations of algorithms GAP, BESTFIT, FASTA, and TFASTA in the Wisconsin Genetics Software Package Release 7.0, Genetics Computer Group, 575 Science Dr., Madison, Wis.

Another example of an algorithm which is suitable for sequence alignment is the BLAST algorithm, which is described in Altschul et al., J. Mol. Biol. 215:403-410 (1990). Software for performing BLAST analyses is publicly available through the National Center for Biotechnology Information (http://www.ncbi.nlm.nih.gov/).

Since the present invention requires processing of large amounts of data, sequence alignment is preferably effected using assembly software.

A number of commonly used computer software fragment read assemblers capable of forming clusters of expressed sequences, and aligning members of the cluster (individually or as an assembled contig) with other sequences (e.g., genomic database) are now available. These packages include but are not limited to, The TIGR Assembler [Sutton G. et al. (1995) Genome Science and Technology 1:9-19], GAP [Bonfield JK. et al. (1995) Nucleic Acids Res. 23:4992-4999], CAP2 [Huang X. et al. (1996) Genomics 33:21-31], the Genome Construction Manager [Laurence CB. Et al. (1994) Genomics 23:192-201], Bio Image Sequence Assembly Manager, SeqMan [Swindell SR. and Plasterer JN. (1997) Methods Mol. Biol. 70:75-89], and LEADS and GenCarta (Compugen Ltd. Israel).

It will be appreciated that since applying sequence homology analysis on large number of sequences is computationally intensive, local alignment (i.e., the alignment of portions of protein sequences) is preferably effected prior to global alignment (alignment of protein sequences along their entire length), as described in Example 6 of the Examples section.

Once progressive clusters are formed, one or more ontological annotations (i.e., assigning an ontology) are assigned to each cluster.

Systematic and standardized ontological nomenclature is preferably used. Such nomenclature (i.e., keywords) can be obtained from several sources. For example, ontological annotations derived from three main ontologies: molecular function, biological process and cellular component are available from the Gene Ontology Consortium (www.geneontology.org).

Alternatively a list of homogenized ontological nomenclature can be obtained from AcroMed - a computer generated database of biomedical acronyms and the associated long forms extracted from the recent Medline abstracts (http://www.expasy.org/tools/).

Optionally, various conversion tables which link Enzyme Commission number, InterPro protein motifs and SwissProt keywords to gene ontology nodes are also available from www.geneontology.org and can be used with the present method.

Ontologies, sub ontologies, and their ontological relations (i.e., inherent relation - the sub-ontology "IS THE" ontology or composite relation - the ontology "HAS" the sub ontology) can be organized into various computer data structures such as a tree, a map, a graph, a stack or a list. These may also be presented in various data format such as, text, table, html, or extensible markup language (XML)

Ontologies and/or subontologies assigned to a specific biomolecular sequence can be derived from an annotation, which is preassociated with at least one biomolecular sequence in a cluster generated as described hereinabove.

For example, biomolecular sequences obtained from an annotated database are typically preassociated with an annotation. An "annotated database" refers to a database of biomolecular sequences, which are at least partially characterized with respect to functional or structural aspects of the sequence. Examples of annotated databases include but are not limited to: GenBank (www.ncbi.nlm.nih.gov/GenBank/), Swiss-Prot (www.expasy.ch/sprot/sprot-top.html), GDB (www.gdb.org/) , PIR (www.mips.biochem.mpg.de/proj/prostseqdb/), YDB (www.mips.biochem.mpg.de/proj/yeast/), MIPS (www.mips.biochem.mpg.de/proj/human) , HGI (www.tigr.org/tdb/hgi/), Celera Assembled Human Genome (www.celera.com/products/human_ann.cfm and LifeSeq Gold (https://lifeseqgold.incyte.com). Additional specialized annotated databases include annotative information on metabolic (http://www.genome.ad.jp/kegg/metabolism.html) and regulatory pathways (http://www.genome.ad.jp/kegg/regulation.html), and protein-protein interactions (http://dip.doe-mbi.ucla.edu/), etc.

Alternatively, ontologies can be generated from an analysis of at least one biomolecular sequence in each of the clusters of the present invention.

Preferably, analysis of the biomolecular sequence is effected by literature text mining. Since manual review of related-literature may be a daunting task, computational extraction of text information is preferably effected.

Thus, the method of the present invention can also process literature and other textual information and utilize processed textual data for generating additional ontological annotations. For example, text information contained in the sequence-related publications and definition lines in sequence records of sequence databases can be extracted and processed. Ontological annotations derived from processed text data are then assigned to the sequences in the corresponding clusters.

Ontological annotations can also be extracted from sequence associated Medical subject heading (MeSH) terms which are assigned to published papers.

Additional information on text mining is provided in Example 7 of the Examples section and is disclosed in "Mining Text Using Keyword Distributions," Ronen Feldnian, Ido Dagan, and Haym Hirsh, Proceedings of the 1995 Workshop on Knowledge Discovery in Databases, "Finding Associations in Collections of Text," Ronen Feldman and Haym Hirsh, Machine Learning and Data Mining: Methods and Applications, edited by R. S. Michalski, I. Bratko, and M Kubat, John Wiley & Sons, Ltd., 1997 "Technology Text Mining, Turning Information Into Knowledge: A White Paper from IBM," edited by Daniel Tkach, Feb. 17,1998, each of which is fully incorporated herein by reference.

It will be appreciated that text mining may be performed, in this and other embodiments of the present invention, for the text terms extracted from the definitions of gene or protein sequence records, retrievable from databases such as GenBank and Swiss-Prot and title line, abstract of scientific papers, retrievable from Medline database (e.g., http://www.ncbi.nlm.nih.gov/PubMed/).

Computer-dedicated software for biological text analysis is available from http://www.expasy.org/tools/. Examples include, but are not limited to, MedMiner - A software system which extracts and organizes relevant sentences in the literature based on a gene, gene-gene or gene-drug query; Protein Annotator's Assistant - A software system which assists protein annotators in the task of assigning functions to newly sequenced proteins; and XplorMed - A software system which explores a set of abstracts derived from a bibliographic search in MEDLINE.

Alternatively, assignment of ontological annotations may be effected by analyzing molecular, cellular and/or functional traits of the biomolecular sequences. Prediction of cellular localization may be done using any computer-dedicated software. For example prediction of cellular localization can be done using the ProLoc computational platform [Einat Hazkani-Covo, Erez Levanon, Galit Rotman, Dan Graur and Amit Novik; (2004) Evolution of multicellularity in metazoa: comparative analysis of the subcellular localization of proteins in Saccharomyces, Drosophila and Caenorhabditis. Cell Biology International (in press)], which predicts protein localization based on various parameters including, protein domains (e.g., prediction of trans-membranous regions and localization thereof within the protein), pI, protein length, amino acid composition, homology to pre-annotated proteins, recognition of sequence patterns which direct the protein to a certain organelle (such as, nuclear localization signal, NLS, mitochondria localization signal), signal peptide and anchor modeling and using unique domains from Pfam that are specific to a single compartment.

Other examples for cellular localization prediction software include PSORT - Prediction of protein sorting signals and localization sites and TargetP - Prediction of subcellular location, both available from http://www.expasy.org/tools/, see also Example 22.

Prediction of functional annotations may also be effected by motif analysis of the biomolecular sequences of the present invention. Thus for example, by implementing any motif analysis software, which is based on protein homology (see for example, http://motif.genome.ad.jp/ and http://www.accelrys.com/products/grailpro/index.html), it is possible to predict functional motifs of DNA sequences including repeats, promoter sequences and CpG islands and of encoded proteins such as zinc finger and leucine zipper. Such functional annotations may also be extracted from databases of protein families, domains and functional sites such as InterPro (http://www.ebi.ac.uk/interpro/).

Functional annotations may also be extracted by adopting annotations from ortholohgous species (i.e., from different species) such as, for example, from viral proteoms. Viral proteins have evolved to defy the host immune system and as such may provide functional annotations to ortholohgous proteins which exhibit sufficient level of homology in at least functional domains thereof. As such, such an annotation may be, for example, "immune system related". Detailed description of the method which is used to obtain such annotations is provided in U.S. Pat. Appl. No. 60/480,752.

Due to the progressive nature of the clusters of the present invention, ontology assignment starts at the highest level of homology. Any biomolecular sequence in the cluster, which shares identical level of homology compared to an ontologically annotated protein in the cluster is assigned the same ontological annotation. This procedure progresses from the highest level of homology to a lower threshold level with a predetermined increment resolution. Newly discovered homologies enable assignment of existing ontological annotations to biomolecular sequences sharing homologous sequences and being previously unannotated or partially annotated (see Examples 5-9 of the Examples section).

Once assignment of an annotation is effected, annotated clusters are disassembled resulting in annotation of each biomolecular sequence of the cluster.

Such annotated biomolecular sequences are then tested for false annotation. This is effected using the following scoring parameters:

(i) A degree of homology characterizing the progressive cluster - accuracy of the annotation directly correlates with the homology level used for the annotation process (see Examples 7-9 and 22 of the Examples section).
(ii) Relevance of annotation to information obtained from literature text mining - each assigned ontological annotation which results from literature text mining or functional or cellular prediction is assessed using scoring parameters such as LOD score (For further details see Example 7 of the Examples section).

The present invention also enables the use of the homologies identified according to the teachings of the present invention to annotate more sensitively and rapidly a query sequence. Essentially this involves building a sequence profile for each annotated cluster. A profile enables scoring of a biomolecular sequence according to functional domains along a sequence and generally makes searches more sensitive. Essentially, clustered sequences are also tested for relevance to the cluster based upon shared functional domains and other characteristic sequence features.

Ontologically annotated biomolecular sequences are stored in a database for further use. Additional information on generation and contents of such databases is provided hereinunder.

Such a database can be used to query functional domains and sequences comprising thereof. Alternatively, the database can be used to query a sequence, and retrieve the compatible annotations.

Although the present methodology can be effected using prior art systems modified for such purposes, due to the large amounts of data processed and the vast amounts of processing needed, the present methodology is preferably effected using a dedicated computational system.

Thus, according to another aspect of the present invention and as illustrated in Figures 1a-b, there is provided a system for generating a database of annotated biomolecular sequences.

System 10 includes a processing unit 12, which executes a software application designed and configured for annotating biomolecular sequences, as described hereinabove. System 10 further serves for storing biomolecular sequence information and annotations in a retrievable/searchable database 18. Database 18 further includes information pertaining to database generation.

System 10 may also include a user interface 14 (e.g., a keyboard and/or a mouse, monitor) for inputting database or database related information, and for providing database information to a user.

System 10 of the present invention may be any computing platform known in the art including but not limited to a personal computer, a work station, a mainframe and the like.

Preferably, database **18** is stored on a computer readable media such as a magnetic optico-magnetic or optical disk.

System **10** of the present invention may be used by a user to query the stored database of annotations and sequence information to retrieve biomolecular sequences stored therein according to inputted annotations or to retrieve annotations according to a biomolecular sequence query.

It will be appreciated that the connection between user interface **14** and processing unit **12** is bi-directional. Likewise, processing unit **12** and database **18** also share a two-way communication channel, wherein processing unit **12** may also take input from database **18** in performing annotations and iterative annotations. Further, user interface **14** is linked directly to database **18,** such a user may dispatch queries to database **18** and retrieve information stored therein. As such, user interface **14** allows a user to compile queries, send instructions, view querying results and performing specific analyses on the results as needed.

In performing ontological annotations, processing unit **12** may take input from one or more application modules **16.** Application module **16** performs a specific operation and produced a relevant annotative input for processing unit **12.** For example, application module 16 may perform cellular localization analysis on a biomolecular sequence query, thereby determining the cellular localization of the encoded protein. Such a functional annotation is then input to and used by processing unit **12.** Examples for application software for cellular localization prediction are provided hereinabove.

System **10** of the present invention may also be connected to one or more external databases **20.** External database **20** is linked to processing unit **12** in a bi-directional manner, similar to the connection between database **18** and processing unit **12.** External database **20** may include any background information and/or sequence information that pertains to the biomolecular sequence query. External database **20** may be a proprietary database or a publicly available database which is accessible through a public network such as the Internet. External database **20** may feed relevant information to processing unit **12** as it effects iterative ontological annotation. External database **20** may also receive and store ontological annotations generated by processing unit **12.** In this case external database **20** may interact with other components of system **10** like database **18.**

It will be appreciated that the databases and application modules of system **10** can be directly connected with processing unit **12** and/or user interface **14** as is illustrated in Figure 1a, or such a connection can be achieved via a network **22,** as is illustrated in Figure 1b.

Network **22** may be a private network (e.g., a local area network), a secured network, or a public network (such as the Internet), or a combination of public and private and/or secured networks.

Thus, the present invention provides a well-characterized approach for the systemic annotation of-biomolecular sequences. The use of text information analysis, annotation scoring system and robust sequence clustering procedure enables, for the first time, the creation of the best possible annotations and assignment thereof to a vast number of biomolecular sequences sharing homologous sequences. The availability of ontological annotations for a significant number of biomolecular sequences from different species can provide a comprehensive account of sequence, structural and functional information pertaining to the biomolecular sequences of interest.

*The hierarchical annotation approach*

"Hierarchical annotation" refers to any ontology and subontology, which can be hierarchically ordered. Examples include but are not limited to a tissue expression hierarchy, a developmental expression hierarchy, a pathological expression hierarchy, a cellular expression hierarchy, an intracellular expression hierarchy, a taxonomical hierarchy, a functional hierarchy and so forth.

According to another aspect of the present invention there is provided a method of annotating biomolecular sequences according to a hierarchy of interest. The method is effected as follows.

First, a dendrogram representing the hierarchy of interest is computationally constructed. As used herein a "dendrogram" refers to a branching diagram containing multiple nodes and representing a hierarchy of categories based on a degree of similarity or number of shared characteristics.

Each of the multiple nodes of the dendrogram is annotated by at least one keyword describing the node, and enabling literature and database text mining, as is further described hereinunder. A list of keywords can be obtained from the GO Consortium (www.geneontlogy.org); measures are taken to include as many keywords, and to include keywords which might be out of date. For example, for tissue annotation (see Figure 4), a hierarchy was built using all available tissue/libraries sources available in the GenBank, while considering the following parameters: ignoring GenBank synonyms, building anatomical hierarchies, enabling flexible distinction between tissue types (normal versus pathology) and tissue classification levels (organs, systems, cell types, etc.).

It will be appreciated that the dendrogram of the present invention can be illustrated as a graph, a list, a map or a matrix or any other graphic or textual organization, which can describe a dendrogram. An example of a dendrogram illustrating the gastrointestinal tissue hierarchy is provided in Figure 2.

In a second step, each of the biomolecular sequences is assigned to at least one specific node of the dendrogram.

The biomolecular sequences according to this aspect of the present invention can be annotated biomolecular sequences, unannotated biomolecular sequences or partially annotated biomolecular sequences.

Annotated biomolecular sequences can be retrieved from pre-existing annotated databases as described hereinabove.

For example, in GenBank, relevant annotational information is provided in the definition and keyword fields. In this case, classification of the annotated biomolecular sequences to the dendrogram nodes is directly effected. A search for suitable annotated biomolecular sequences is performed using a set of keywords which are designed to classify the biomolecular sequences to the hierarchy (i.e., same keywords that populate the dendrogram)

In cases where the biomolecular sequences are unannotated or partially annotated, extraction of additional annotational information is effected prior to classification to dendrogram nodes. This can be effected by sequence alignment, as described hereinabove. Alternatively, annotational information can be predicted from structural studies. Where needed, nucleic acid sequences can be transformed to amino acid sequences to thereby enable more accurate annotational prediction.

Finally, each of the assigned biomolecular sequences is recursively classified to nodes hierarchically higher than the specific nodes, such that the root node of the dendrogram encompasses the full biomolecular sequence set, which can be classified according to a certain hierarchy, while the offspring of any node represent a partitioning of the parent set.

For example, a biomolecular sequence found to be specifically expressed in "rhabdomyosarcoma", will be classified also to a higher hierarchy level, which is "sarcoma", and then to "Mesenchimal cell tumors" and finally to a highest hierarchy level "Tumor". In another example, a sequence found to be differentially expressed in endometrium cells, will be classified also to a higher hierarchy level, which is "uterus", and then to "women genital system" and to "genital system" and finally to a highest hierarchy level "genitourinary system". The retrieval can be performed according to each one of the requested levels.

Since annotation of publicly available databases is at times unreliable, newly annotated biomolecular sequences are confirmed using computational or laboratory approaches as is further described hereinbelow.

It will be appreciated that once temporal or spatial annotations of sequences are established using the teachings of the present invention, it is possible to identify those sequences, which are differentially expressed (i.e., exhibit spatial or temporal pattern of expression in'diverse cells or tissues). Such sequences are assigned to only a portion of the nodes, which constitute the hierarchical dendrogram.

Changes in gene expression are important determinants of normal cellular physiology, including cell cycle regulation, differentiation and development, and they directly contribute to abnormal cellular physiology, including developmental anomalies, aberrant programs of differentiation and cancer. Accordingly, the identification, cloning and characterization of differentially expressed genes can provide relevant and important insights into the molecular determinants of processes such as growth, development, aging, differentiation and cancer. Additionally, identification of such genes can be useful in development of new drugs and diagnostic methods for treating or preventing the occurrence of such diseases.

Newly annotated sequences identified according to the present invention are tested under physiological conditions (i.e., temperature, pH, ionic strength, viscosity, and like biochemical parameters which are compatible with a viable organism, and/or which typically exist intracellularly in a viable cultured yeast cell or mammalian cell). This can be effected using various laboratory approaches such as, for example, FISH analysis, PCR, RT-PCR, real-time PCR, southern blotting, northern blotting, electrophoresis and the like (see Examples 13-20 and 27 of the Examples section) or more elaborate approaches which are detailed in the Background section.

It will be appreciated that true involvement of differentially expressed genes in a biological process is better confirmed using an appropriate cell or animal model, as further described hereinunder.

The hierarchical annotation approach enables to assign an appropriate annotation level even in cases where expression is not restricted to a specific tissue type or cell type. For example, differentially expressed sequences of a single contig which are annotated as being expressed in several different tissue types of a single specific organ or a specific system, are also annotated by the present invention to a higher hierarchy level thus denoting association with the specific organ or system. In such cases using keywords alone would not efficiently identify differentially expressed sequences. Thus for example, a sequence found to be expressed in sarcoma, Ewing sarcoma tumors, pnet, rhabdomyosarcoma, liposarcoma and mesenchymal cell tumors, can not be assigned to specific sarcomas, but still can be annotated as mesenchymal cell tumor specific. Using this hierarchical annotation approach in combination with advanced sequence clustering and assembly algorithms, capable of predicting alternative splicing, may facilitate a simple and rapid identification of gene expression patterns.

Although the present methodology can be effected using prior art systems modified for such purposes, due to the large amounts of data processed and the vast amounts of processing needed, the present methodology is preferably effected using a dedicated computational system.

Such a system is described hereinabove. The system includes a processing unit which executes a software application designed and configured for hierarchically annotating biomolecular sequences as described hereinabove. The system further serves for storing biomolecular sequence information and annotations in a retrievable/searchable database.

***Annotation of differentially expressed alternatively spliced sequences***

Although numerous methods have been developed to identify differentially expressed genes, none of these addressed splice variants, which occur in over 50 % of human genes. Given the common sequence features of splice variants it is very difficult to identify splice variants which expression is differential, using prior art methodologies. Therefore assigning unique sequence features to differentially expressed splice variants may have an important impact to the understanding of disease development and may serve as valuable markers to various pathologies.

Thus, according to another aspect of the present invention there is provided a method of identifying sequence features unique to differentially expressed mRNA splice variants. The method is effected as follows.

First, unique sequence features are computationally identified in splice variants of alternatively spliced expressed sequences.

As used herein the phrase "splice variants" refers to naturally occurring nucleic acid sequences and proteins encoded therefrom which are products of alternative splicing. Alternative splicing refers to intron inclusion, exon exclusion, or any addition or deletion of terminal sequences, which results in sequence dissimilarities between the splice variant sequence and the wild-type sequence.

Although most alternatively spliced variants result from alternative exon usage, some result from the retention of introns not spliced-out in the intermediate stage of RNA transcript processing.

As used herein the phrase "unique sequence features" refers to donor/acceptor concatenations (i.e., exon-exon junctions), intron sequences, alternative exon sequences and alternative polyadenylation sequences.

Once a unique sequence feature is identified, the expression pattern of the splice variant is determined. If the splice variant is differentially expressed then the unique feature thereof is annotated accordingly.

Alternatively spliced expressed sequences of this aspect of the present invention, can be retrieved from numerous publicly available databases. Examples include but are not limited to ASDB - an alternative splicing database generated using GenBank and Swiss-Prot annotations (http://cbcg.nersc.gov/asdb, AsMamDB - a database of alternative splices in human, mouse and rat (http://166.111.30.65/ASMAMDB.html), Alternative splicing database - a database of alternative splices from literature (http://cgsigm.cshl.org/new_alt_exon_db2/), Yeast intron database - Database of intron in yeast (http://www.cse.ucsc.edu/research/compbio/yeast_introns.html), The Intronerator-alternative splicing in C. elegans based on analysis of EST data (http://www.cse.ucsc.edu/-kent/intronerator), ISIS - Intron Sequence Information System including a section of human alternative splices (http://isis.bit.uq.edu.au/), TAP-Transcript Assembly Program result of alternative splicing (http://stl.wustl.edu/- zkan/TAP/) and HASDB - database of alternative splices detected in human EST data.

Additionally, alternative splicing sequence data utilized by this aspect of the present invention can be obtained by any of the following bioinformatical approaches.

***(i) Genomically aligned ESTs -*** the method identifies ESTs which come from the same gene and looks for differences between them that are consistent with alternative splicing, such as large insertion or deletion in one EST. Each candidate splice variant can be further assessed by aligning the ESTs with respective genomic sequence. This reveals candidate exons (i.e., matches to the genomic sequence) separated by candidate splices (i.e., large gaps in the EST-genomic alignment). Since intronic sequences at splice junctions (i.e., donor/acceptor concatenations) are highly conserved (essentially 99.24 % of introns have a GT-AG at their 5' and 3' ends, respectively) sequence data can be used to verify candidate splices [Burset et al. (2000) Nucleic Acids Res. 28:4364-75 LEADS module [Shoshan, et al, Proceeding of SPIE (eds. M.L. Bittner, Y. Chen, A.N. Dorsel, E.D. Dougherty) Vol. 4266, pp. 86-95 (2001).;R. Sorek, G. Ast, D. Graur, Genome Res. In press; Compugen Ltd. US patent application 09/133,987].

***(ii) Identification based on intron information -*** The method creates a database of individual intron sequences annotated in GenBank and utilizes such sequences to search for EST sequences which include the intronic sequences [Croft et al. (2000) Nat. Genet 24:340-1].

***(iii) EST alignment to expressed sequences -*** looks for insertions and deletions in ESTs relative to a set of known mRNAs. Such a method enables to uncover alternatively spliced variants with having to align ESTs with genomic sequence [Brett et al. (2000) FEBS Lett. 474-83-86].

It will be appreciated that in order to avoid false positive identification of novel splice isoforms, a set of filters is applied. For example, sequences are filtered to exclude ESTs having sequence deviations, such as chimerism, random variation in which a given EST sequence or potential vector contamination at the ends of an EST.

Filtering can be effected by aligning ESTs with corresponding genomic sequences. Chimeric ESTs can be easily excluded by requiring that each EST aligns completely to a single genomic locus. Genomic location found by homology search and alignment can often be checked against radiation hybrid mapping data [Muneer et al (2002) Genomic 79:344-8]. Furthermore, since the genomic regions which align with an EST sequence correspond to exon sequences and alignment gaps correspond to introns, the putative splice sites at exon/intron boundaries can be confirmed. Because splice donor and acceptor sites primarily reside within the intron sequence, this methodology can provide validation which is independent of the EST evidence. Reverse transcriptase artifacts or other cDNA synthesis errors may also be filtered out using this approach. Improper inclusion of genomic sequence in ESTs can also be excluded by requiring pairs of mutually exclusive splices in different ESTs.

Additionally, it will be appreciated that observing a given splice variant in one EST but not in a second EST may be insufficient, as the latter can be an un-spliced EST rather than a biological significant intron inclusion. Therefore measures are taken to focus on mutually exclusive splice variants, two different splice variants observed in different ESTs, which overlap in a genomic sequence. A more stringent filtering may be applied by requiring two splice variants to share one splice site but differ in another. Another filter which can be used to identify true splicing events is sequence conservation. Essentially, exons and the borders of human introns which are identified in mice genome and/or supported by mouse ESTs are considered true splicing events (see Example 21 of the Examples section).

Once splice variants are identified, identification of unique sequence features therewithin can be effected computationally by identifying insertions, deletions and donor-acceptor concatenations in ESTs relative to mRNA and preferably genomic sequences.

As mentioned hereinabove, once alternatively spliced sequences (having unique sequence features) are identified, determination of their expression patterns is effected in order to assign an annotation to the unique sequence feature thereof.

Expression pattern identification may be effected by qualifying annotations which are preassociated with the alternatively spliced expressed sequences, as described hereinabove. This can be accomplished by scoring the annotations. For example scoring pathological expression annotations can be effected according to: (i) prevalence of the alternatively spliced expressed sequences in normal tissues; (ii) prevalence of the alternatively spliced expressed sequences in pathological tissues; (iii) prevalence of the alternatively spliced expressed sequence in total tissues; and (iv) number of tissues and/or tissue types expressing the alternatively spliced expressed sequences. Preferably expression pattern of alternatively spliced sequences is determined as described in the "Frequency-based annotative approach" section, which follows.

Alternatively, identifying the expression pattern of the alternatively spliced expressed sequences of the present invention, is accomplished by detecting the presence of the unique sequence feature in biological samples. This can be effected by any hybridization-based technique known in the art, such as northern blot, dot blot, RNase protection assay, RT-PCR and the like.

To this end oligonucleotides probes, which are substantially homologous to nucleic acid sequences that flank and/or extend across the unique sequence features of the alternatively spliced expressed sequences of the present invention are generated.

Preferably, oligonucleotides which are capable of hybridizing under stringent, moderate or mild conditions, as used in any polynucleotide hybridization assay are utilized. Further description of hybridization conditions is provided hereinunder.

Oligonucleotides generated by the teachings of the present invention may be used in any modification of nucleic acid hybridization based techniques, which are further detailed hereinunder. General features of oligonucleotide synthesis and modifications are also provided hereinunder.

Aside from being useful in identifying specific splice variants, oligonucleotides generated according to the teachings of the present invention may also be widely used as diagnostic, prognostic and therapeutic agents in a variety of disorders which are associated with the polynucleotides of the present invention (e.g., specific splice variants).

For example, regulation of splicing is involved in 15 % of genetic diseases [Krawzczak et aL (1992) Hum. Genet. 90:41-54] and may contribute for example to cancer mis-splicing of exon 18 in BRCA1, which is caused by a polymorphism in an exonic enhancer [Liu et al. (2001) Nature Genet. 27:55-58].

Thus, oligonucleotides generated according to the teachings of the present invention can be included in diagnostic kits. Such kits, may include oligonucleotides which are directed to the newly uncovered splice variant alone and also to previously uncovered splice variants or wild-type (w.t) sequences of the same gene which were previously associated with a disease of interest. For example, oligonucleotides sets pertaining to a specific disease associated with differential expression of an alternatively spliced transcript can be packaged in a one or more containers with appropriate buffers and preservatives along with suitable instructions for use and used for diagnosis or for directing therapeutic treatment. Additional information on such diagnostic kits is provided hereinunder.

It will be appreciated that an ability to identify alternatively spliced sequences, also facilitates identification of the various products of alternative splicing.

Recent studies indicate that most alternative splicing events result in an altered protein product [International human genome sequencing consortium (2001) Nature 409:860-921; Modrek et al. (2001) Nucleic Acids Res. 29:2850-2859]. The majority of these changes appear to have a functional relevance (i.e., up-regulating or down-regulating activity, "gain of function" or "loss of function", respectively. See terminology section), such as the replacement of the amino or carboxyl terminus, or in-frame addition and removal of a functional domain. For example, alternative splicing can lead to the use of a different site for translation initiation (i.e., alternative initiation), a different translation termination site due to a frameshift (i.e., truncation or extension), or the addition or removal of a stop codon in the alternative coding sequence (i.e., alternative termination). Additionally, alternative splicing can change an internal sequence region due to an in-frame insertion or deletion. One example of the latter is the new FC receptor $\beta$-like protein, whose C-terminal transmembrane domain and cytoplasmic tail, which is important for signal transduction in this class of receptors, is replaced with a new transmembrane domain and tail by alternative polyadenylation. Another example is the truncated Growth Hormone Receptor, which lacks most of its intracellular domain and has been shown to heterodimerize with the full-length receptor, thus causing inhibition of signaling by Growth Hormone [Ross, R.J.M., Growth hormone & IGF Research, 9:42-46, (1999)].

Thus, identifying splice variants having unique sequence features enables annotation and thus identification of functionally altered variants.

Identification of putative functionally altered splice variants, according to this aspect of the present invention, can be effected by identifying sequence deviations from functional domains of wild-type gene products.

Identification of functional domains can be effected by comparing a wild-type gene product with a series of profiles prepared by alignment of well characterized proteins from a number of different species. This generates a consensus profile, which can then be matched with the query sequence. Examples of programs suitable for such identification include, but are not limited to, InterPro Scan - Integrated search in PROSITE, Pfam, PRINTS and other family and domain databases; ScanProsite - Scans a sequence against PROSITE or a pattern against SWISS-PROT and TrEMBL; MotifScan - Scans a sequence against protein profile databases (including PROSITE); Frame-ProfileScan - Scans a short DNA sequence against protein profile databases (including PROSITE); Pfam HMM search - scans a sequence against the Pfam protein families database; FingerPRINTScan - Scans a protein sequence against the PRINTS Protein Fingerprint Database; FPAT - Regular expression searches in protein databases; PRATT - Interactively generates conserved patterns from a series of unaligned proteins; PPSEARCH - Scans a sequence against PROSITE (allows a graphical output); at EBI; PROSITE scan - Scans a sequence against PROSITE (allows mismatches); at PBIL; PATTINPROT - Scans a protein sequence or a protein database for one or several pattern(s); at PBIL; SMART - Simple Modular Architecture Research Tool; at EMBL; TEIRESIAS - Generate patterns from a collection of unaligned protein or DNA sequences; at IBM, all available from http://www.expasy.org/tools/.

It will be appreciated that functionally altered splice variants may also include a sequence alteration at a post-translation modification consensus site, such as, for example, a tyrosine sulfation site, a glycosylation site, etc. Examples of post-translational modification prediction programs include but are not limited to: SignalP - Prediction of signal peptide cleavage sites; ChloroP - Prediction of chloroplast transit peptides; MITOPROT - Prediction of mitochondrial targeting sequences; Predotar - Prediction of mitochondrial and plastid targeting sequences; NetOGlyc - Prediction of type O-glycosylation sites in mammalian proteins; DictyOGlyc - Prediction of GlcNAc O-glycosylation sites in Dictyostelium; YinOYang - O-beta-GlcNAc attachment sites in eukaryotic protein sequences; big-PI Predictor - GPI Modification Site Prediction; DGPI - Prediction of GPI-anchor and cleavage sites (Mirror site); NetPhos - Prediction of Serine, Threonine and Tyrosine phosphorylation sites in eukaryotic proteins; NetPicoRNA - Prediction of protease cleavage sites in picomaviral proteins; NMT - Prediction of N-terminal N-myristoylation; Sulfinator - Prediction of tyrosine sulfation sites all available from http://www.expasy.org/tools/.

Once putative functionally altered splice variants are identified, they are validated by experimental verification and functional studies, using methodologies well known in the art.

The Examples section which follows illustrates identification and annotation of splice variants. Identified and annotated sequences are contained within the enclosed CD-ROMs1-4. Some of these sequences represent (i.e., are transcribed from) entirely new splice variants, while others represent new splice variants of known sequences. In any case, the sequences contained in the enclosed CD-ROMs are novel in that they include previously undisclosed sequence regions in the context of a known gene or an entirely new sequence in the context of an unknown gene.

### Frequency-based annotative approach

The present invention also contemplates spatial and temporal gene annotations through comparing relative abundance in libraries of different origins.

Thus, according to still another aspect of the present invention there is provided a method of comparing an expression level of a gene of interest in at least two types of tissues.

As used herein the phrase "at least two types of tissues" refers to tissues of different developmental origin, different pathological origin or different cellular composition.

The method is effected by obtaining a contig assembled from a plurality of expressed sequences (e.g., ESTs, mRNAs) representing the gene of interest; and comparing the number of the plurality of expressed sequences corresponding to the contig, which are expressed in each of the at least two tissue types, to thereby compare the expression level of the gene of interest in the at least two tissue types.

Expressed sequences for generating the contig of this aspect of the present invention can be retrieved from pre-existing publicly available databases or generated as described in the "ontological annotation approach" section hereinabove.

A number of sequence assembly software are known in the art, which can be used to generate the contig of the gene of interest. Such software are described in the "ontological annotation approach" section hereinabove.

Alternatively, the contig of this aspect of the present invention can be obtained from pre-existing publicly available databases. Examples include, but are not limited to, the TIGR database (www.tigr.org), the SANBI database (http://www.za.embnet.org/), the SIB database which generates contig sequence information from Unigene clusters, the MIPS database (http://mips.gsf.de/) and the DoTS database (http://www.allgenes.org/).

It will be appreciated that the contig according to this aspect of the present invention can be composed of a plurality of expressed sequences, which present partial or complete exonal coverage of the gene of interest

Prior to, concomitant with or following contig assembly, expressed sequences are filtered to exclude sequences of poor quality (i.e., vector contaminants, low complexity sequences, sequences which originate from small libraries e.g., smaller than 1000 sequences), and to score true expression in the at least two types of tissues.

Expressed sequences, which originate from samples wherein clone frequency reflects mRNA abundance are highly scored. Thus expressed sequences from "non-normalized" expression libraries are highly scored, while expressed sequences from "normalized" libraries are poorly scored. Such scoring rules are described in details in Example 23 of the Examples section which follows.

Comparing the number of the plurality of expressed sequences corresponding to the contig which are expressed in each of the at least two tissue types is preferably effected by statistical pairing analysis. Examples of statistical tests which can be used in accordance with the present invention include, but are not limited to, chi square, Fisher's exact test, phi, Yule's Q, Lambda and Tau b. Preferably, to calculate an exact p-value for a two by two frequency table with a small number of expected frequencies, Fisher's exact test is used.

Genes exhibiting differential pattern of expression uncovered using the methodology of the present invention can be efficiently utilized as tissue markers and as putative drug targets.

As mentioned above, alternatively spliced transcripts may be extremely useful as cancer markers and draugs, since it appears likely that there may be striking contrasts in usage of alternatively spliced transcript variants between normal and tumor tissue in alterations in the general levels of gene expression [Caballero Dis Markers. (2001);17(2):67-75].

For example, members of the CD44 family of cell surface hyaluronate-binding proteins have been implicated in cell migration, cell-matrix interactions and tumor progression. Interestingly, normal spinal nerves and primary Schwann cell cultures express standard CD44 (CD44s) but not alternatively spliced variant isoforms. In contrast, Schwann cell tumors express both "wild-type" CD44 and a number of variants. Implicating a role for CD44 splice variants in cancer and as such in the development of potent diagnostic and therapeutic tools.

Thus, the present invention also envisages comparing an expression level of at least two splice variants of a gene of interest in a tissue. The method is effected by:

Obtaining a contig including exonal sequence presentation of the at least two splice variants of the gene of interest, the contig being assembled from a plurality of expressed sequences;

Identifying at least one contig sequence region unique to a portion (i.e., at least one and not all) of the at least two splice variants of the gene of interest Identification of such unique sequence region is effected using computer alignment software such as described hereinabove.

Comparing a number of the plurality of expressed sequences in the tissue having the at least one contig sequence region with a number of the plurality of expressed sequences not-having the at least one contig sequence region, to thereby compare the expression level of the at least two splice variants of the gene of interest in the tissue.

One configuration of the above-described methodology is described in details in Example 23 c of the Examples section which follows.

Biomolecular sequences (i.e., nucleic acid and polypeptide sequences) uncovered using the above-described methodology are annotated using the teachings of the present invention. Thus, for example, the hierarchical annotation approach can be used to assign a differentially expressed gene product to higher hierarchies. For example, gene products identified by the "Frequency-based annotative approach" engine as being overexpresed in prostate tumor, lung tumor, head and neck tumor, stomach tumor, colon tumor, mammary tumor, kidney tumor, ovary tumor, uterus/cervix tumor,

thyroid tumor, adrenal tumor, pancreas tumor, liver tumor and skin tumor might also be specific to other types of epithelial tumors. Gene products identified by the engine as being overexpressed in bone and muscle tumors might also be specific to other types of sarcomas. Gene products identified by the engine as being overexpressed in bone marrow tumor, blood cancer, T cell tumor and lymph nodes tumor may also be specific to other types of blood cancers. Sequence data uncovered by the above described methodologies and corresponding annotative data are stored in a database for future use (see, for example, files "Transcripts_nucleotide_seqs_part1", "Transcripts_nucleotide_seqs_part2", "Transcripts_nucleotide_seqs_part3", "Transcripts_nucleotide_seqs_part4", "protein_seqs", "ProDG_seqs", "Summary_table", "Annotations.gz", "Transcripts.gz", and "Proteins.gz" of the enclosed CD-ROMs 1-4).

As mentioned hereinabove, biomolecular sequences uncovered using the methodology of the present invention can be efficiently utilized as tissue or pathological markers and as putative drugs or drug targets for treating or preventing the disease.

Some examples are summarized infra:

For example, gene products (nucleic acid and/or protein products), which exhibit tumor specific expression (i.e., tumor associated antigens, TAAs) can be utilized for in-vitro generation of antibodies and/or for in-vivo immunization/cancer vaccination, essentially eliciting an immune response against such gene products and cells expressing same (see e.g., U.S. Pat No. 4,235,877, Vaccine preparation is generally described in, for example, M. F. Powell and M. J. Newman, eds., "Vaccine Design (the subunit and adjuvant approach)," Plenum Press (NY, 1995); Other references describing adjuvants, delivery vehicles and immunization in general include Rolland, Crit Rev. Therap. Drug Carrier Systems 15:143-198, 1998; Fisher-Hoch et al., Proc. Natl. Acad. Sci. USA 86:317-321, 1989; Flexner et al., Ann. N.Y Acad. Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993; Ulmer et al., Science 259:1745-1749, 1993; Cohen, Science 259:1691-1692, 1993; U.S. Pat Nos. 4,436,727; 4,877,611; 4,866,034 and 4,912,094; U.S. Pat. Nos. 6,008,200 and 5,856,462; Zitvogel et al., Nature Med. 4:594-600, 19980.

The tumor-specific gene products of the present invention, in particular membrane bound, can be utilized as targeting molecules for binding therapeutic toxins, antibodies and small molecules, to thereby specifically target the tumor cell. Alternatively, neoplastic properties of the tumor-specific tumor specific gene products (nucleic acid and/or protein products) of the present invention, may be beneficially used in the promotion of wound healing and neovascularization in ischemic conditions and diabetes.

Secreted splice variants of known autoantigens associated with a specific autoimmune syndrome, such as for example, those listed in Table 15, below, can be used to treat such syndromes. Typically, autoimmune disorders are characterized by a number of different autoimmune manifestations (e.g., multiple endocrine syndromes). For these reasons secreted variants may be used to treat any combination of autoimmune phenomena of a disease as detailed in Table 15, below. The therapeutic effect of these splice variants may be a result of (i) competing with autoantigens for binding with autoantibodies; (ii) antigen-specific immunotherapy, essentially suggesting that systemic administration of a protein antigen can inhibit the subsequent generation of the immune response to the same antigen (has been proved in mice models for Myasthenia Gravis and type I Diabetes).

In addition, any novel variant of autoantigens, not necessarily secreted, may be used for "specific immunoadsorption" - leading to a specific immunodepletion of antibodies when used in immunoadsorption columns.

It will be appreciated that splice variants of autoantigens may also have diagnostic value. The diagnosis of many autoimmune disorders is based on looking for specific autoantibodies to autoantigens known to be associated with a autoimmune condition. Most of the diagnostic techniques are based on having a recombinant form of the autoantigen and using it to look for serum autoantibodies. It is possible that what is considered an autoantigen is not the "true" autoantigen but rather a variant thereof. For example, TPO is a known autoantigen in thyroid autoimmunity. It has been shown that its variant TPOzanelli also take part in the autoimmune process and can bind the same antibodies as TPO [Biochemistry. 2001 Feb 27;40(8):2572-9.]. Antibodies formed against the true autoantigen may bind to other variants of the same gene due to sequence overlap but with reduced affinity. Novel splice variant of the genes in Table 15 may be revealed as true autoantigens, therefore their use for detection of autoantibodies is expected to result in a more sensitive and specific test.

Apart of clinical applications, the biomolecular sequences of the present invention can find other commercial uses such as in the food, agricultural, electro-mechanical, optical and cosmetic industries [http://www.physics.unc.edu/-rsuper/XYZweb/ XYZchipbiomotors.rs1.doc; http://www.bio.org/er/industrial.asp]. For example, newly uncovered gene products, which can disintegrate connective tissues, can be used as potent anti scarring agents for cosmetic purposes. Other applications include, but are not limited to, the making of gels, emulsions, foams and various specific products, including photographic films, tissue replacers and adhesives, food and animal feed, detergents, textiles, paper and pulp, and chemicals manufacturing (commodity and fine, e.g., bioplastics).

The nucleic acid sequences of the invention can be "isolated" or "purified." In the event the nucleic acid is genomic DNA, it is considered "isolated" when it does not include coding sequence(s) of a gene or genes immediately adjacent thereto in the naturally occurring genome of an organism; although some or all of the 5' or 3' non-coding sequence of an adjacent gene can be included. For example, an isolated nucleic acid (DNA or RNA) can include some or all of the 5' or 3' non-coding sequence that flanks the coding sequence (e.g., the DNA sequence that is transcribed into, or the RNA sequence that gives rise to, the promoter or an enhancer in the mRNA). For example, an isolated nucleic acid can contain less than about 5 kb (e.g., less than about 4kb, 3 kb, 2 kb, 1kb, 0.5kb, or 0.1 kb) of the 5' and/or 3' sequence that naturally flanks the nucleic acid molecule in a cell in which the nucleic acid naturally occurs. In the event the nucleic acid is RNA or mRNA, it is "isolated" or "purified" from a natural source (e.g., a tissue) or a cell culture when it is substantially free of the cellular components with which it naturally associates in the cell and, if the cell was cultured, the cellular components and medium in which the cell was cultured (e.g., when the RNA or mRNA is in a form that contains less than about 20 %, 10 %, 5 %, 1%, or less, of other cellular components or culture medium). When chemically synthesized, a nucleic acid (DNA or RNA) is "isolated" or "purified" when it is substantially free of the chemical precursors or other chemicals used in its synthesis (e.g., when the nucleic acid is in a form that contains less than about 20 %, 10 %, 5 %, 1%, or less, of the chemical precursors or other chemicals).

Variants, fragments, and other mutant nucleic acids are also envisaged by the present invention. As noted above, where a given biomolecular sequence represents a new gene (rather than a new splice variant of a known gene), the nucleic acids of the invention include the corresponding genomic DNA and RNA. Accordingly, where a given SEQ ID represents a new gene, variations or mutations can occur not only in that nucleic acid sequence, but in the coding regions, the non-coding regions, or both, of the genomic DNA or RNA from which it was made.

The nucleic acids of the invention can be double-stranded or single-stranded and can, therefore, either be a sense strand, an antisense strand, or a portion (i.e., a fragment) of either the sense or the antisense strand. The nucleic acids of the invention can be synthesized using standard nucleotides or nucleotide analogs or derivatives (e.g., inosine, phosphorothioate, or acridine substituted nucleotides), which can alter the nucleic acid's ability to pair with complementary sequences or to resist nucleases. Indeed, the stability or solubility of a nucleic acid can be altered (e.g., improved) by modifying the nucleic acid's base moiety, sugar moiety, or phosphate backbone. For example, the nucleic acids of the invention can be modified as taught by Toulmé [Nature Biotech. 19:17, (2001)] or Faria et al. [Nature Biotech. 19:40-44, (2001)], and the deoxyribose phosphate backbone of nucleic acids can be modified to generate peptide nucleic acids [PNAs; see Hyrup et al., (1996) Bioorganic & Medicinal Chemistry 4:5-23].

PNAs are nucleic acid "mimics"; the molecule's natural backbone is replaced by a pseudopeptide backbone and only the four nucleotide bases are retained. This allows specific hybridization to DNA and RNA under conditions of low ionic strength. PNAs can be synthesized using standard solid phase peptide synthesis protocols as described, for example by Hyrup et al. (supra) and Perry-O'Keefe et al. [Proc. Natl. Acad. Sci. USA (1996) 93:14670-675]. PNAs of the nucleic acids described herein can be used in therapeutic and diagnostic applications.

Moreover, the nucleic acids of the invention include not only protein-encoding nucleic acids per se (e.g., coding sequences produced by the polymerase chain reaction (PCR) or following treatment of DNA with an endonuclease), but also, for example, recombinant DNA that is: (a) incorporated into a vector (e.g., an autonomously replicating plasmid or virus), (b) incorporated into the genomic DNA of a prokaryote or eukaryote, or (c) part of a hybrid gene that encodes an additional polypeptide sequence (i.e., a sequence that is heterologous to the nucleic acid sequences of the present invention or fragments, other mutants, or variants thereof).

The present invention includes naturally occurring sequences of the nucleic acid sequences described above, allelic variants (same locus; functional or non-functional), homologs (different locus), and orthologs (different organism) as well as degenerate variants of those sequences and fragments thereof. The degeneracy of the genetic code is well known, and one of ordinary skill in the art will be able to make nucleotide sequences that differ from the nucleic acid sequences of the present invention but nevertheless encode the same proteins as those encoded by the nucleic acid sequences of the present invention. The variant sequences (e.g., degenerate variants) can be used in the same manner as naturally occurring sequences. For example, the variant DNA sequences of the invention can be incorporated into a vector, into the genomic DNA of a prokaryote or eukaryote, or made part of a hybrid gene. Moreover, variants (or, where appropriate, the proteins they encode) can be used in the diagnostic assays and therapeutic regimes described below.

The sequence of nucleic acids of the invention can also be varied to maximize expression in a particular expression system. For example, as few as one and as many as about 20 % of the codons in a given sequence can be altered to optimize expression in bacterial cells (e.g., E. coli), yeast, human, insect, or other cell types (e.g., CHO cells).

The nucleic acids of the invention can also be shorter or longer than those disclosed on CD-ROMs 1, 2 and 4. Where the nucleic acids of the invention encode proteins, the protein-encoding sequences can differ from those represented by specific sequences of file "Protein.seqs" in CD-ROM 2 and "Proteins.gz" in CD-ROM4. For example, the encoded proteins can be shorter or longer than those encoded by one of the nucleic acid sequences of the present invention. Nucleotides can be deleted from, or added to, either or both ends of the nucleic acid sequences of the present invention or the novel portions of the sequences that represent new splice variants. Alternatively, the nucleic acids can encode proteins in which one or more amino acid residues have been added to, or deleted from, one or more sequence positions within the nucleic acid sequences.

The nucleic acid fragments can be short (e.g., 15-30 nucleotides). For example, in cases where peptides are to be expressed therefrom such polynucleotides need only contain a sufficient number of nucleotides to encode novel antigenic epitopes. In cases where nucleic acid fragments serve as DNA or RNA probes or PCR primers, fragments are selected of a length sufficient for specific binding to one of the sequences representing a novel gene or a unique portion of a novel splice variant.

Nucleic acids used as probes or primers are often referred to as oligonucleotides, and they can hybridize with a sense or antisense strand of DNA or RNA. Nucleic acids that hybridize to a sense strand (i.e., a nucleic acid sequence that encodes protein, e.g., the coding strand of a double-stranded cDNA molecule) or to an mRNA sequence are referred to as antisense oligonucleotides. Oligonucleotides which specifically hybridize with the troponin variants of the present invention (SEQ ID NOs: 74, 76, 78, 80, 82, 84 and 66) and not with wild-type tropoinin are preferably directed at the unique nucleic acid sequence set forth in SEQ ID NO: 87. Alternatively, such oligonucleotides can be directed at a nucleic acid sequence which bridges the unique sequence with common upstream or downstream sequences (see Figure 21).

Antisense oligonucleotides can be used to specifically inhibit transcription of any of the nucleic acid sequences of the present invention.

Design of antisense molecules must be effected while considering two aspects important to the antisense approach. The first aspect is delivery of the oligonucleotide into the cytoplasm of the appropriate cells, while the second aspect is design of an oligonucleotide which specifically binds the designated mRNA within cells in a way which inhibits translation thereof.

The prior art teaches of a number of delivery strategies which can be used to efficiently deliver oligonucleotides into a wide variety of cell types [see, for example, Luft (1998) J Mol Med 76(2): 75-6; Kronenwett et al. (1998) Blood 91(3): 852-62; Rajur et al. (1997) Bioconjug Chem 8(6): 935-40; Lavigne et al. (1997) Biochem Biophys Res Commun 237(3): 566-71 and Aoki et al. (1997) Biochem Biophys Res Commun 231(3): 540-5].

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. (1999) Biotechnol Bioeng 65(1): 1-9].

Such algorithms have been successfully used to implement an antisense approach in cells. For example, the algorithm developed by Walton et al. enabled scientists to successfully design antisense oligonucleotides for rabbit beta-globin (RBG) and mouse tumor necrosis factor-alpha (TNF-$\alpha$) transcripts. The same research group has more recently reported that the antisense activity of rationally selected oligonucleotides against three model target mRNAs (human lactate dehydrogenase A and B and rat gp130) in cell culture as evaluated by a kinetic PCR technique proved effective in almost all cases, including tests against three different targets in two cell types with phosphodiester and phosphorothioate oligonucleotide chemistries.

In addition, several approaches for designing and predicting efficiency of specific oligonucleotides using an in vitro system were also published (Matveeva et al. (1998) Nature Biotechnology 16, 1374 -1375).

Several clinical trials have demonstrated safety, feasibility and activity of antisense oligonucleotides. For example, antisense oligonucleotides suitable for the treatment of cancer have been successfully used (Holmund et al. (1999) Curr Opin Mol Ther 1(3):372-85), while treatment of hematological malignances via antisense oligonucleotides targeting c-myb gene, p53 and Bcl-2 had entered clinical trials and had been shown to be tolerated by patients [Gerwitz (1999) Curr Opin Mol Ther 1(3):297-306].

More recently, antisense-mediated suppression of human heparanase gene expression has been reported to inhibit pleural dissemination of human cancer cells in a mouse model [Uno et al. (2001) Cancer Res 61(21):7855-60].

Thus, the current consensus is that recent developments in the field of antisense technology which, as described above, have led to the generation of highly accurate antisense design algorithms and a wide variety of oligonucleotide delivery systems, enable an ordinarily skilled artisan to design and implement antisense approaches suitable for down-regulating expression of known sequences without having to resort to undue trial and error experimentation.

Antisense oligonucleotides can also be $\alpha$-anomeric nucleic acids, which form specific double-stranded hybrids with complementary RNA in which, contrary to the usual b-units, the strands run parallel to each other [Gaultier et al., Nucleic Acids Res. 15:6625-6641, (1987)]. Alternatively, antisense nucleic acids can comprise a 2'-o-methylribonucleotide [Inoue et al., Nucleic Acids Res. 15:6131-6148, (1987)] or a chimeric RNA-DNA analogue [Inoue et al., FEBS Lett. 215:327-330, (1987)].

The nucleic acid sequences described above can also include ribozymes catalytic sequences. Such a ribozyme will have specificity for a protein encoded by the novel nucleic acids described herein (by virtue of having one or more sequences that are complementary to the cDNAs that represent novel genes or the novel portions (i.e., the portions not found in related splice variants) of the sequences that represent new splice variants. These ribozymes can include a catalytic sequence encoding a protein that cleaves mRNA [see U.S. Pat. No. 5,093,246 or Haselhoff and Gerlach, Nature 334:585-591, (1988)]. For example, a derivative of a tetrahymena L-19 IVS RNA can be constructed in which the nucleotide sequence of the active site is complementary to the nucleotide sequence to be cleaved in an mRNA of the invention (e.g., one of the nucleic acid sequences of the present invention; see, U.S. Patent Nos. 4,987,071 and 5,116,742). Alternatively, the mRNA sequences of the present invention can be used to select a catalytic RNA having a specific ribonuclease activity from a pool of RNA molecules [see, e.g., Bartel and Szostak, Science 261:1411-1418, (1993); see also Krol et al., Bio-Techniques 6:958-976, (1988)].

Alternatively, small interfering RNA oligonucleotides can be used to specifically inhibit transcription of any of the nucleic acid sequences of the present invention. RNA interference is a two step process the first step, which is termed as the initiation step, input dsRNA is digested into 21-23 nucleotide (nt) small interfering RNAs (siRNA), probably by the action of Dicer, a member of the RNase III family of dsRNA-specific ribonucleases, which processes (cleaves) dsRNA (introduced directly or via a transgene or a virus) in an ATP-dependent manner. Successive cleavage events degrade the RNA to 19-21 bp duplexes (siRNA), each with 2-nucleotide 3' overhangs [Hutvagner and Zamore Curr. Opin. Genetics and Development 12:225-232 (2002); and Bernstein Nature 409:363-366 (2001)].

In the effector step, the siRNA duplexes bind to a nuclease complex to from the RNA-induced silencing complex (RISC). An ATP-dependent unwinding of the siRNA duplex is required for activation of the RISC. The active RISC then targets the homologous transcript by base pairing interactions and cleaves the mRNA into 12 nucleotide fragments from the 3' terminus of the siRNA [Hutvagner and Zamore Curr. Opin. Genetics and Development 12:225-232 (2002); Hammond et al. (2001) Nat Rev. Gen. 2:110-119 (2001); and Sharp Genes. Dev. 15:485-90 (2001)]. Although the mechanism of cleavage is still to be elucidated, research indicates that each RISC contains a single siRNA and an RNase [Hutvagner and Zamore Curr. Opin. Genetics and Development 12:225-232 (2002)].

Because of the remarkable potency of RNAi, an amplification step within the RNAi pathway has been suggested. Amplification could occur by copying of the input dsRNAs which would generate more siRNAs, or by replication of the siRNAs formed. Alternatively or additionally, amplification could be effected by multiple turnover events of the RISC [Hammond et al. Nat. Rev. Gen. 2:110-119 (2001), Sharp Genes. Dev. 15:485-90 (2001); Hutvagner and Zamore Curr. Opin. Genetics and Development 12:225-232 (2002)]. For more information on RNAi see the following reviews Tuschl ChemBiochem. 2:239-245 (2001); Cullen Nat. Immunol. 3:597-599 (2002); and Brantl Biochem. Biophys. Act. 1575:15-25 (2002).

Synthesis of RNAi molecules suitable for use with the present invention can be effected as follows. First, the an mRNA sequence of interest is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex [Tuschl ChemBiochem. 2:239-245]. It will be appreciated though, that siRNAs directed at untranslated regions may also be effective, as demonstrated for GAPDH wherein siRNA directed at the 5' UTR mediated about 90 % decrease in cellular GAPDH mRNA and completely abolished protein level (www.ambion.com/techlib/tn/91/912.html).

Second, potential target sites are compared to an appropriate genomic database (e.g., human, mouse, rat etc.) using any sequence alignment software, such as the BLAST software available from the NCBI server (www.ncbi.nlm.nih.gov/BLAST/). Putative target sites which exhibit significant homology to other coding sequences are filtered out

Qualifying target sequences are selected as template for siRNA synthesis. Preferred sequences are those including low G/C content as these have proven to be more effective in mediating gene silencing as compared to those with G/C content higher than 55 %. Several target sites are preferably selected along the length of the target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

DNAzyme molecules can also be used to specifically inhibit transcription of any of the nucleic acid sequences of the present invention.

DNAzyme molecules are capable of specifically cleaving an mRNA transcript or DNA sequence of interest DNAzymes are single-stranded polynucleotides which are capable of cleaving both single and double stranded target sequences (Breaker, R.R. and Joyce, G. Chemistry and Biology 1995;2:655; Santoro, S.W. & Joyce, G.F. Proc. Natl, Acad. Sci. USA 1997;943:4262) A general model (the "10-23" model) for the DNAzyme has been proposed. "10-23" DNAzymes have a catalytic domain of 15 deoxyribonucleotides, flanked by two substrate-recognition domains of seven to nine deoxyribonucleotides each. This type of DNAzyme can effectively cleave its substrate RNA at purine:pyrimidine junctions (Santoro, S.W. & Joyce, G.F. Proc. Natl, Acad. Sci. USA 199; for rev of DNAzymes see Khachigian, LM [Curr Opin Mol Ther 4:119-21 (2002)].

Examples of construction and amplification of synthetic, engineered DNAzymes recognizing single and double-stranded target cleavage sites have been disclosed in U.S. Pat. No. 6,326,174 to Joyce et al. DNAzymes of similar design directed against the human Urokinase receptor were recently observed to inhibit Urokinase receptor expression, and successfully inhibit colon cancer cell metastasis in vivo (Itoh et al, 20002, Abstract 409, Ann Meeting Am Soc Gen Ther www.asgt.org). In another application, DNAzymes complementary to bcr-ab1 oncogenes were successful in inhibiting the oncogenes expression in leukemia cells, and lessening relapse rates in autologous bone marrow transplant in cases of CML and ALL.

Oligonucleotides having as few as 9-10 nucleotides (e.g., 12-14, 15-17, 18-20, 21-23, or 24-27 nucleotides) can be useful as probes or expression templates and are within the scope of the present invention. Indeed, fragments that contain about 15-20 nucleotides can be used in Southern blotting, Northern blotting, dot or slot blotting, PCR amplification methods (where naturally occurring or mutant nucleic acids are amplified; e.g., RT-PCR), colony hybridization methods, in situ hybridization, and the like.

The present invention also encompasses pairs of oligonucleotides (these can be used, for example, to amplify the new genes, or portions thereof, or the novel portions of the splice variant in, for example, potentially diseased tissue) and groups of oligonucleotides (e.g., groups that exhibit a certain degree of homology (e.g., nucleic acids that are 90 % identical to one another) or that share one or more functional attributes).

When used, for example, as probes, the nucleic acids of the invention can be labeled with a radioactive isotope (e.g., using polynucleotide kinase to add $^{32}$P-labeled ATP to the oligonucleotide used as the probe) or an enzyme. Other labels, such as chemiluminescent, fluorescent, or colorimetric, labels can be used.

As noted above, the invention features nucleic acids that are complementary to those represented by the nucleic acid sequences of the present invention or novel portions thereof (i.e., novel fragments) and as such are capable of hybridizing therewith. In many cases, nucleic acids that are used as probes or primers are absolutely or completely complementary to all, or a portion of, the target sequence. However, this is not always necessary. The sequence of a useful probe or primer can differ from that of a target sequence so long as it hybridizes with the target under the stringency conditions described herein (or the conditions routinely used to amplify sequences by PCR) to form a stable duplex.

Hybridization of a nucleic acid probe to sequences in a library or other sample of nucleic acids is typically performed under moderate to high stringency conditions. Nucleic acid duplex or hybrid stability is expressed as the melting temperature (Tm), which is the temperature at which a probe dissociates from a target DNA and, therefore, helps define the required stringency conditions. To identify sequences that are related or substantially identical to that of a probe, it is useful to first establish the lowest temperature at which only homologous hybridization occurs with a particular concentration of salt (e.g., SSC or SSPE). (The terms "identity" or "identical" as used herein are equated with the terms "homology" or "homologous"). Then, assuming a 1 % mismatch requires a 1 °C decrease in the Tm, the temperature of the wash (e.g., the final wash) following the hybridization reaction is reduced accordingly. For example, if sequences having at least 95 % identity with the probe are sought, the final wash temperature is decreased by 5 °C. In practice, the change in Tm can be between 0.5 °C and 1.5 °C per 1% mismatch

The hybridization conditions described here can be employed when the nucleic acids of the invention are used in, for example, diagnostic assays, or when it is desirable to identify, for example, the homologous genes that fall within the scope of the invention (as stated elsewhere, the invention encompasses allelic variants, homologues and orthologues of the sequences that represent new genes). Homologous genes will hybridize with the sequences that represent new genes under a stringency condition described herein.

The following is an example of "high stringency" hybridization conditions: 68°C in (a) 5X SSC/5X Denhardt's solution/1.0 % SDS, (b) 0.5 M NaHPO$_4$ (pH 7.2)/1 mM EDTA/7% SDS, or (c) 50 % formamide/0.25 M NaHPO$_4$ (pH 7.2)/0.25 M NaCl/1 mM EDTA/7% SDS, and washing is carried out with (a) 0.2X SSC/0.1% SDS at room temperature or at 42°C, (b) 0.1X SSC/0.1% SDS at 68°C, or (c) 40 mM NaHPO$_4$ (pH 7.2)/1 mM EDTA and either 1% or 5 % SDS at 50°C.

"Moderately stringent" hybridization conditions constitute, for example, the hybridization conditions described above and one or more washes in 3X SSC at 42°C. Of course, salt concentration and temperature can be varied to achieve the optimal level of identity between the probe and the target nucleic acid. This is well known in the art, and additional guidance is available in, for example, Sambrook et al., 1989, Molecular Cloning, A Laboratory Manual, Cold Spring Harbor Laboratory Press, Cold Spring Harbor, N.Y., and Ausubel et al. (eds.), 1995, Current Protocols in Molecular Biology, John Wiley & Sons, New York; N.Y.

As mentioned hereinabove, the nucleic acid sequences of the present invention can be modified to encode substitution mutants of the wild type forms. Substitution mutants can include amino acid residues that represent either a conservative or non-conservative change (or, where more than one residue is varied, possibly both). A "conservative" substitution is one in which one amino acid residue is replaced with another having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). The invention includes polypeptides that include one, two, three, five, or more conservative amino acid substitutions, where the resulting mutant polypeptide has at least one biological activity that is the same, or substantially the same, as a biological activity of the wild type polypeptide.

Fragments or other mutant nucleic acids can be made by mutagenesis techniques well known in the art, including those applied to polynucleotides, cells, or organisms (e.g., mutations can be introduced randomly along all or part of the nucleic acid sequences of the present invention by saturation mutagenesis). The resultant mutant proteins can be screened for biological activity to identify those that retain activity or exhibit altered activity.

In certain embodiments, nucleic acids of the invention differ from the nucleic acid sequences provided in files "Transcripts_nucleotide_seqs_part1", "Transcripts_nucleotide_seqs_part2", "Transcripts_nucleotide_seqs_part3.new", ""Transcripts_nucleotide_seqs_part4", "ProDG_seqs", and "Transcripts.gz" (provided in CD-ROM1, CD-ROM2 and CD-ROM4) by at least one, but less than 10, 20, 30, 40, 50, 100, or 200 nucleotides or, alternatively, at less than 1%, 5 %, 10 % or 20 % of the nucleotides in the subject nucleic acid (excluding, of course, splice variants known in the art). Similarly, in certain embodiments, proteins of the invention can differ from those encoded by those included in Files "Protein.seqs" and "Proteins.gz" (provided in CD-ROM2 and CD-ROM4) by at least one, but less than 10, 20, 30, 40, 50, 100, or 200 amino acid residues or, alternatively, at less than 1%, 5 %, 10 % or 20 % of the amino acid residues in a subject protein (excluding, of course, proteins encoded by splice variants known in the art (proteins of the invention are described in more detail below)). If necessary for this analysis (or any other test for homology or substantial identity described herein), the sequences should be aligned for maximum homology, as described elsewhere here.

The present invention also encompasses mutants [e.g., naturally-accurring or synthetic nucleic acids that exhibit an identity level of at least 50 %, at least 55 %, at least 60 %,, at least 65 %,, at least 70 %,, at least 75 %,, at least 80 %,, at least 85 %,, at least 90 %,, say 95-100 % to any of the nucleic acid sequences set forth in the files "Transcripts_nucleotide_seqs_part1", "Transcripts_nucleotide_seqs_part2", "Transcripts_nucleotide_seqs_part3", "Transcripts_nucleotide_seqs_part4", "TroDG_seqs", and "Transcripts.gz" of the enclosed CD-ROM1, CD-ROM2 and CD-ROM4, as determined using the BlastN software of the National Center of Biotechnology Information (NCBI) using default parameters], which encode proteins that retain substantially at least one, or preferably substantially all of the biological activities of the referenced protein (i.e., encoding a polypeptide having an amino acid sequence which exhibits a homology level of at least 50 %, at least 55 %, at least 60 %, at least 65 %, at least 70 %, at least 75 %, at least 80 %, at least 85 %, at least 90 %, say 95-100 % to any of the amino acid sequences set forth in the files "protein_seqs" and "Proteins.gz" of the enclosed CD-ROM2 and CD-ROM4 , as determined using the BlastP software of the National Center of Biotechnology Information (NCBI) using default parameters).. What constitutes "substantially all" may vary considerably. For example, in some instances, a variant or mutant protein may be about 5 % as effective as the protein from which it was derived. But if that level of activity is sufficient to achieve a biologically significant result (e.g., transport of a sufficient number of ions across a cell membrane), the variant or mutant protein is one that retains substantially all of at least one of the biological activities of the protein from which it was derived. A "biologically active" variant or mutant (e.g., fragment) of a protein can participate in an intra- or inter-molecular interaction that can be characterized by specific binding between molecules two or more identical molecules (in which case, homodimerization could occur) or two or more different molecules (in which case, heterodimerization could occur). Often, a biologically active fragment will be recognizable by virtue of a recognizable domain or motif, and one can confirm biological activity experimentally. More specifically, for example, one can make (by synthesis or recombinant techniques) a nucleic acid fragment that encodes a potentially biologically active portion of a protein of the present invention by inserting the active fragment into an expression vector, and expressing the protein (expression constructs and expression systems are described further below), and finally assessing the ability of the protein to function.

The present invention also encompasses chimeric nucleic acid sequences that encode fusion proteins. For example, a nucleic acid sequence of the invention can include a sequence that encodes a hexa-histidine tag (to facilitate purification of bacterially-expressed proteins) or a hemagglutinin tag (to facilitate purification of proteins expressed in eukaryotic cells).

The fused heterologous sequence can also encode a portion of an immunoglobulin (e.g., the constant region (Fc) of an IgG molecule), a detectable marker, or a signal sequence (e.g., a sequence that is recognized and cleaved by a signal peptidase in the host cell in which the fusion protein is expressed). Fusion proteins containing an Fc region can be purified using a protein A column, and they have increased stability (e.g., a greater circulating half-life) in vivo.

Detectable markers are well known in the art and can be used in the context of the present invention. For example, the expression vector pUR278 (Ruther et al., EMBO J., 2:1791, 1983) can be used to fuse a nucleic acid of the invention to the lacZ gene (which encodes β-galactosidase).

A nucleic acid sequence of the invention can also be fused to a sequence that, when expressed, improves the quantity or quality (e.g., solubility) of the fusion protein. For example, pGEX vectors can be used to express the proteins of the invention fused to glutathione S-transferase (GST). In general, such fusion proteins are soluble and can be easily purified from lysed cells by adsorption to glutathione-agarose beads followed by elution in the presence of free glutathione. The pGEX vectors (Pharmacia Biotech Inc; Smith and Johnson, Gene 67:31-40, 1988) are designed to include thrombin or factor Xa protease cleavage sites so that the cloned target gene product can be released from the GST moiety. Other useful vectors include pMAL (New England Biolabs, Beverly, MA) and pRIT5 (Pharmacia, Piscataway, NJ), which fuse maltose E binding protein and protein A, respectively, to a protein of the invention.

A signal sequence, when present, can facilitate secretion of the fusion protein from a cell, and can be cleaved off by the host cell. The nucleic acid sequences of the present invention can also be fused to "inactivating" sequences, which render the fusion protein encoded, as a whole, inactive. Such proteins can be referred to as "preproteins," and they can be converted into an active form of the protein by removal of the inactivating sequence.

The present invention also encompasses expression constructs (e.g., plasmids, cosmids, and other vectors that transport nucleic acids) that include a nucleic acid of the invention in a sense or antisense orientation. The nucleic acids can be operably linked to a regulatory sequence (e.g., a promoter, enhancer, or other expression control sequence, such as a polyadenylation signal) that facilitates expression of the nucleic acid. The vector can replicate autonomously or integrate into a host genome, and can be a viral vector, such as a replication defective retrovirus, an adenovirus, or an adeno-associated virus.

When present, the regulatory sequence can direct constitutive or tissue-specific expression pf the nucleic acid. Tissue-specific promoters include, for example, the liver-specific albumin promoter (Pinkert et al., Genes Dev. 1:268-277, 1987), lymphoid-specific promoters (Calame and Eaton, Adv. Immunol. 43:235-275, 1988), such as those of T cell receptors (Winoto and Baltimore, EMBO J. 8:729-733, 1989) and immunoglobulins (Banerji et al., Cell 33:729-740, 1982; Queen and Baltimore, Cell 33:741-748, 1983), the neuron-specific neurofilament promoter (Byrne and Ruddle, Proc. Natl. Acad. Sci. USA 86:5473-5477, 1989), pancreas-specific promoters (Edlund et al., Science 230:912-916, 1985), and mammary gland-specific promoters (e.g., milk whey promoter; see U.S. Patent No. 4,873,31.6 and European Application Publication No. 264,166). Developmentally-regulated promoters can also be used. Examples of such promoters include the murine hox promoters (Kessel and Gruss, Science 249:374-379, 1990) and the fetoprotein promoter (Campes and Tilghman, Genes Dev. 3:537-546, 1989). Moreover, the promoter can be an inducible promoter. For example, the promoter can be regulated by a steroid hormone, a polypeptide hormone, or some other polypeptide (e.g., that used in the tetracycline-inducible system, "Tet-On" and "Tet-Off"; see, e.g., Clontech Inc. (Palo Alto, CA), Gossen and Bujard Proc.-Natl. Acad. Sci. USA 89:5547, 1992, and Paillard, Human Gene Therapy 9:983, 1989).

The expression vector will be selected or designed depending on, for example, the type of host cell to be transformed and the level of protein expression desired. For example, when the host cells are mammalian cells, the expression vector can include viral regulatory elements, such as promoters derived from polyoma, Adenovirus 2, cytomegalovirus and Simian Virus 40. The nucleic acid inserted (i.e., the sequence to be expressed) can also be modified to encode residues that are preferentially utilized in E. coli (Wada et al., Nucleic Acids Res. 20:2111-2118, 1992). These modifications can be achieved by standard DNA synthesis techniques.

Expression vectors can be used to produce the proteins encoded by the nucleic acid sequences of the invention ex vivo (e.g., the expressed proteins can be purified from expression systems such as those described herein) or in vivo (in, for example, whole organisms). Proteins can be expressed in vivo in a way that restores expression to within normal limits and/or restores the temporal or spatial patterns of expression normally observed. Alternatively, proteins can be aberrantly expressed in vivo (i.e., at a time or place, or to an extent, that does not normally occur in vivo). For example, proteins can be over expressed or under expressed with respect to expression in a wild-type state; expressed at a different developmental stage; expressed at a different time during the cell cycle; or expressed in a tissue or cell type where expression does not normally occur.

The present invention also encompasses various engineered cells, including cells that have been engineered to express or over-express a nucleic acid sequence described herein. Accordingly, the cells can be transformed with a expression construct, such as those described above. A "transformed" cell is a cell into which (or into an ancestor of which) one has introduced a nucleic acid that encodes a protein of the invention. The nucleic acid can be introduced by any of the art-recognized techniques for introducing nucleic acids into a host cell (e.g., calcium phosphate or calcium chloride co-precipitation, DEAE-dextran-mediated transfection, lipofection, or electroporation).

The phrases "transformed cell" or "host cell" refer not only to the particular subject cell, but also to the progeny or potential progeny of such cells. Mutations or environmental influences may modify the cells in succeeding generations and, even though such progeny may not be identical to the parent cell, they are nevertheless within the scope of the invention. The cells of the invention can be "isolated" cells or "purified preparations" of cells (e.g., an in vitro preparation of cells), either of which can be obtained from multicellular organisms such as plants and animals (in which case the purified preparation would constitute a subset of the cells from the organism). In the case of unicellular microorganisms (e.g., microbial cells), the preparation is purified when at least 10 % (e.g., 25 %, 50 %, 75 %, 80 %, 90 %, 95 % or more) of the cells within it are the cells of interest (e.g., the cells that express a protein of the invention).

The expression vectors of the invention can be designed to express proteins in prokaryotic or eukaryotic cells. For example, polypeptides of the invention can be expressed in bacterial cells (e.g., E. coli), fungi, yeast, or insect cells (e.g., using baculovirus expression vectors). For example, a baculovirus such as Autographa californica nuclear polyhedrosis virus (AcNPV), which grows in Spodoptera frugiperda cells, can be used as a vector to express foreign genes.

A nucleic acid of the invention can be cloned into a non-essential region (for example the polyhedrin gene) of the viral genome and placed under control of a promoter (e.g., the polyhedrin promoter). Successful insertion of the nucleic acid results in inactivation of the polyhedrin gene and production of non-occluded recombinant virus (i.e., virus lacking the proteinaceous coat encoded by the polyhedrin gene). These recombinant viruses are then typically used to infect insect cells (e.g., Spodoptera frugiperda cells) in which the inserted gene is expressed (see, e.g., Smith et al., J. Virol. 46:584, 1983 and U.S. Patent No. 4,215,051). If desired, mammalian cells can be used in lieu of insect cells, provided the virus is engineered so that the nucleic acid is placed under the control of a promoter that is active in mammalian cells.

Useful mammalian cells include rodent cells, such as Chinese hamster ovary cells (CHO) or COS cells, primate cells, such as African green monkey kidney cells, rabbit cells, or pig cells). The mammalian cells can also be human cells (e.g., a hematopoietic cell, a fibroblast, or a tumor cell). For example, HeLa cells, 293 cells, 3T3 cells, and WI38 cells are useful. Other suitable host cells are known to those skilled in the art and are discussed further in Goeddel [Gene Expression Technology: Methods in Enzymology 185, Academic Press, San Diego, CA, (1990)].

Proteins can also be produced in plant cells, if desired. For plant cells, viral expression vectors (e.g., cauliflower mosaic virus and tobacco mosaic virus) and plasmid expression vectors (e.g., Ti plasmid) are suitable. These cells and other types are available from a wide range of sources [e.g., the American Type Culture Collection, Manassas, VA; see also, e.g., Ausubel et al., Current Protocols in Molecular Biology, John Wiley & Sons, New York, (1994)]. The optimal methods of transformation (by, for example, transfection) and, as noted above, the choice of expression vehicle will depend on the host system selected. Transformation and transfection methods are described in, for example, Ausubel et al., supra; expression vehicles can be chosen from those provided in, for example, Pouwels et al., Cloning Vectors: A Laboratory Manual, (1985), Supp. (1987). The host cells harboring the expression vehicle can be cultured in conventional nutrient media, adapted as needed for activation of a chosen nucleic acid, repression of a chosen nucleic acid, selection of transformants, or amplification of a chosen nucleic acid.

Expression systems can be selected based on their ability to produce proteins that are modified (e.g., by phosphorylation, glycosylation, or cleavage) in substantially the same way they would be in a cell in which they are naturally expressed. Alternatively, the system can be one in which naturally occurring modifications do not occur, or occur in a different position, or to a different extent, than they otherwise would.

If desired, the host cells can be those of a stably-transfected cell line. Vectors suitable for stable transfection of mammalian cells are available to the public (see, e.g., Pouwels et al. (supra) as are methods for constructing them (see, e.g., Ausubel et al. (supra). In one example, a nucleic acid of the invention is cloned into an expression vector that includes the dihydrofolate reductase (DHFR) gene. Integration of the plasmid and, therefore, the nucleic acid it contains, into the host cell chromosome is selected for by including 0.01-300 mM methotrexate in the cell culture medium (as described in Ausubel et al., supra). This dominant selection can be accomplished in most cell types.

Moreover, recombinant protein expression can be increased by DHFR-mediated amplification of the transfected gene. Methods for selecting cell lines bearing gene amplifications are described in Ausubel et al. (supra) and generally involve extended culture in medium containing gradually increasing levels of methotrexate. DHFR-containing expression vectors commonly used for this purpose include pCVSEII-DHFR and pAdD26SV(A) (which are also described in Ausubel et al., supra).

A number of other selection systems can be used. These include those based on herpes simplex virus thymidine kinase, hypoxanthine-guanine phosphoribosyl-transferase, and adenine phosphoribosyltransferase genes, which can be employed in tk, hgprt, or aprt cells, respectively. In addition, gpt, which confers resistance to mycophenolic acid (Mulligan et al., Proc. Natl. Acad. Sci. USA, 78:2072, 1981); neo, which confers resistance to the aminoglycoside G-418 (Colberre-Garapin et al., J. Mol. Biol. 150:1, 1981); and hygro, which confers resistance to hygromycin (Santerre et al., Gene 30:147, 1981), can be used.

In view of the foregoing, it is clear that one can synthesize proteins encoded by the nucleic acid sequences of the present invention (i.e., recombinant proteins). Methods of generating and recombinant proteins are well known in the art. Recombinant protein purification can be effected by affinity. Where a protein of the invention has been fused to a heterologous protein (e.g., a maltose binding protein, a $\beta$-galactosidase protein, or a trpE protein), antibodies or other agents that specifically bind to the latter can facilitate purification. The recombinant protein can, if desired, be further purified (e.g., by high performance liquid chromatography or other standard techniques [see, Fisher, Laboratory Techniques In Biochemistry And Molecular Biology, Eds., Work and Burdon, Elsevier, (1980)].

Other purification schemes are known as well. For example, non-denatured fusion proteins can be purified from human cell lines as described by Janknecht et al. (Proc. Natl. Acad. Sci. USA, 88:8972, 1981). In this system, a nucleic acid is subcloned into a vaccinia recombination plasmid such that it is translated, in frame, with a sequence encoding an N-terminal tag consisting of six histidine residues. Extracts of cells infected with the recombinant vaccinia virus are loaded onto $Ni^{2+}$ nitriloacetic acid-agarose columns, and histidine-tagged proteins are selectively eluted with imidazole-containing buffers.

Alternatively, Chemical synthesis can also be utilized to generate the proteins of the present invention [e.g., proteins can be synthesized by the methods described in Solid Phase Peptide Synthesis, 2nd Ed., The Pierce Chemical Co., Rockford, IL, (1984)].

The invention also features expression vectors that can be transcribed and translated in vitro using, for example, a T7 promoter and T7 polymerase. Thus, the invention encompasses methods of making the proteins described herein in vitro.

Sufficiently purified proteins can be used as described herein. For example, one can administer the protein to a patient, use it in diagnostic or screening assays, or use it to generate antibodies (these methods are described further below).

The cells per se can also be administered to patients in the context of replacement therapies. For example, a nucleic acid of the present invention can be operably linked to an inducible promoter (e.g., a steroid hormone receptor-regulated promoter) and introduced into a human or nonhuman (e.g., porcine) cell and then into a patient. Optionally, the cell can be cultivated for a time or encapsulated in a biocompatible material, such as poly-lysine alginate. See, e.g., Lanza, Nature Biotechnol. 14:1107, (1996); Joki et al. Nature Biotechnol. 19:35, 2001; and U.S. Patent No. 5,876,742] When a steroid hormone receptor-regulated promoter is used, protein production can be regulated in the subject by administering a steroid hormone to the subject. Implanted recombinant cells can also express and secrete an antibody that specifically binds to one of the proteins encoded by the nucleic acid sequences of the present invention. The antibody can be any antibody or any antibody derivative described herein. An antibody "specifically binds" to a particular antigen when it binds to that antigen but not, to a detectable level, to other molecules in a sample (e.g., a tissue or cell culture) that naturally includes the antigen.

While the host cells described above express recombinant proteins, the invention also encompasses cells in which gene expression is disrupted (e.g., cells in which a gene has been knocked out). These cells can serve as models of disorders that are related to mutated or mis-expressed alleles and are also useful in drug screening.

Protein expression can also be regulated in cells without using the expression constructs described above. Instead, one can modify the expression of an endogenous gene within a cell (e.g., a cell line or microorganism) by inserting a heterologous DNA regulatory element into the genome of the cell such that the element is operably linked to the endogenous gene. For example, an endogenous gene that is "transcriptionally silent," (i.e., not expressed at detectable levels) can be activated by inserting a regulatory element that promotes the expression of a normally expressed gene product in that cell. Techniques such as targeted homologous recombination can be used to insert the heterologous DNA (see, e.g., U.S. Patent No. 5,272,071 and WO 91/06667).

The polypeptides of the present invention include the protein sequences contained in the Files "Protein.seqs" of CD-ROM2 and "Proteins.gz" of the enclosed CD-ROM4 and those encoded by the nucleic acids described herein (so long as those nucleic acids contain coding sequence and are not wholly limited to an untranslated region of a nucleic acid sequence), regardless of whether they are recombinantly produced (e.g., produced in and isolated from cultured cells), otherwise manufactured (by, for example, chemical synthesis), or isolated from a natural biological source (e.g., a cell or tissue) using standard protein purification techniques.

The terms "peptide," "polypeptide," and "protein" are used herein interchangeably to refer to a chain of amino acid residues, regardless of length or post-translational modification (e.g., glycosylation or phosphorylation). Proteins (including antibodies that specifically bind to the products of those nucleic acid sequences that encode protein or fragments thereof) and other compounds can be "isolated" or "purified." The proteins and compounds of the present invention are "isolated" or "purified" when they exist as a composition that is at least 60 % (e.g., 70 %, 75 %, 80 %, 85 %, 90 %, 95 %, or 99% or more) by weight the protein or compound of interest. Thus, the proteins of the invention are substantially free from the cellular material (or other biological or cell culture material) with which they may have, at one time, been associated (naturally or otherwise). Purity can be measured by any appropriate standard method (e.g., column chromatography, polyacrylamide gel electrophoresis, or HPLC analysis

The proteins of the present invention also include those encoded by novel fragments or other mutants (i.e., naturally-accurring or synthetic) or variants of the protein-encoding sequences of the present invention. Thus, the present invention envisages polypeptide sequences having amino acid sequences which exhibit a homology level of at least 50 %, at least 55 %, at least 60 %, at least 65 %, at least 70 %, at least 75 %, at least 80 %, at least 85 %, at least 90 %, say 95-100 % to any of the polypeptide sequences set forth in the files "protein_seqs", and "Proteins.gz" of the enclosed CD-ROM2 and CD-ROM4, as determined using the BlastP software of the National Center of Biotechnology Information (NCBI) using default parameters. These proteins can retain substantially all (e.g., 70 %, 80 %, 90 %, 95 %, or 99%) of the biological activity of the full-length protein from which they were derived and can, therefore, be used as agonists or mimetics of the proteins from which they were derived. The manner in which biological activity can be determined is described generally herein, and specific assays (e.g., assays of enzymatic activity or ligand-binding ability) are known to those of ordinary skill in the art. In some instances, retention of biological activity is not necessary or desirable. For example, fragments that retain little, if any, of the biological activity of a full-length protein can be used as immunogens, which, in turn, can be used as therapeutic agents (e.g., to generate an immune response in a patient), diagnostic agents (e.g., to detect the presence of antibodies or other proteins in a tissue sample obtained from a patient), or to generate or test antibodies that specifically bind the proteins of the invention.

In other instances, the proteins encoded by nucleic acids of the invention can be modified (e.g., fragmented or otherwise mutated) so their activities oppose those of the naturally occurring protein (i.e., the invention encompasses variants of the proteins encoded by nucleic acids of the invention that are antagonistic to a biological process). One of ordinary skill in the art will recognize that the more extensive the mutation, the more likely it is to affect the biological activity of the protein (this is not to say that minor modifications cannot do so as well). Thus, it is likely that mutant proteins that are agonists of those encoded by wild type proteins will differ from those wild type proteins only at non-essential residues or will contain only conservative substitutions. Conversely, antagonists are likely to differ at an essential residue or to contain non-conservative substitutions. Moreover, those of ordinary skill in the art can engineer proteins so that they retain desirable traits (i.e., those that make them efficacious in a particular therapeutic, diagnostic, or screening regime) and lose undesirable traits (i.e., those that produce side effects, or produce false-positive results through non-specific binding).

In the event a protein of the invention is encoded by a new gene, the invention encompasses proteins that arise following alternative transcription, RNA splicing, translational- or post-translational events (e.g., the invention encompasses splice variants of the new genes). In the event a protein of the invention is encoded by a novel splice variant, the invention encompasses proteins that arise following alternative translational- or post-translational events (i.e., the invention does not encompass proteins encoded by known splice variants, but does encompass other variants of the novel splice variant). Post-translational modifications are discussed above in the context of expression systems.

The fragmented or otherwise mutant proteins of the invention can differ from those encoded by the nucleic acids of the invention to a limited extent (e.g., by at least one but less than 5, 10 or 15 amino acid residues). As with other, more extensive mutations, the differences can be introduced by adding, deleting, and/or substituting one or more amino acid residues. Alternatively, the mutant proteins can differ from the wild type proteins from which they were derived by at least one residue but less than 5 %, 10 %, 15 % or 20 % of the residues when analyzed as described herein. If the mutant and wild type proteins are different lengths, they can be aligned and analyzed using the algorithms described above.

Useful variants, fragments, and other mutants of the proteins encoded by the nucleic acids of the invention can be identified by screening combinatorial libraries of these variants, fragments, and other mutants for agonist or antagonist activity. For example, libraries of fragments (e.g., N-terminal, C-terminal, or internal fragments) of one or more of the proteins of the invention can be used to generate populations of fragments that can be screened and, once identified, isolated. The proteins can include those in which one or more cysteine residues are added or deleted, or in which a glycosylated residue is added or deleted. Methods for screening libraries (e.g., combinatorial libraries of proteins made from point mutants or cDNA libraries) for proteins or genes having a particular property are known in the art. These methods can be adapted for rapid screening. Recursive ensemble mutagenesis (REM), a new technique that enhances the frequency of functional mutants in libraries, can be used in combination with screening assays to identify useful variants of the proteins of the present invention [Arkin and Yourvan, Proc. Natl. Acad. Sci. USA 89:7811-7815, (1992); Delgrave et al., Protein Engineering 6:327-331, (1993)].

Cell-based assays can be exploited to analyze variegated libraries constructed from one or more of the proteins of the invention. For example, cells in a cell line (e.g., a cell line that ordinarily responds to the protein(s) of interest in a substrate-dependent manner) can be transfected with a library of expression vectors. The transfected cells are then contacted with the protein and the effect of the expression of the mutant on signaling by the protein (substrate) can be detected (e.g., by measuring redox activity or protein folding). Plasmid DNA can then be recovered from the cells that score for inhibition, or alternatively, potentiation of signaling by the protein (substrate). Individual clones are then further characterized.

The invention also contemplates antibodies (i.e., immunoglobulin molecules) that specifically bind (see the definition above) to the proteins described herein and antibody fragments (e.g., antigen-binding fragments or other immunologically active portions of the antibody). For example, an antibody which specifically binds the troponin variants of the present invention is preferably directed to the unique amino acid sequence region which is not shared by wild-type troponin (see Figure 21, SEQ ID NO: 87). Alternatively, such an antibody can be directed to an amino acid sequence which bridges the unqiue sequence region and common sequence regions. Antibodies are proteins, and those of the invention can have at least one or two heavy chain variable regions (VH), and at least one or two light chain variable regions (VL). The VH and VL regions can be further subdivided into regions of hypervariability, termed "complementarity determining regions" (CDR), which are interspersed with more highly conserved "framework regions" (FR). These regions have been precisely defined [see, Kabat et al., Sequences of Proteins of Immunological Interest, Fifth Edition, U.S. Department of Health and Human Services, NIH Publication No. 91-3242, (1991) and Chothia.et al., J. Mol. Biol. 196:901-917, (1987)], and antibodies or antibody fragments containing one or more of them are within the scope of the invention.

The antibodies of the invention can also include a heavy and/or light chain constant region [constant regions typically mediate binding between the antibody and host tissues or factors, including effector cells of the immune system and the first component (C1q) of the classical complement system], and can therefore form heavy and light immunoglobulin chains, respectively. For example, the antibody can be a tetramer (two heavy and two light immunoglobulin chains, which can be connected by, for example, disulfide bonds). The heavy chain constant region contains three domains (CH1, CH2 and CH3), whereas the light chain constant region has one (CL).

An antigen-binding fragment of the invention can be: (i) a Fab fragment (i.e., a monovalent fragment consisting of the VL, VH, CL and CH1 domains); (ii) a F(ab')$_2$ fragment (i.e., a bivalent fragment containing two Fab fragments linked by a disulfide bond at the hinge region); (iii) a Fd fragment consisting of the VH and CH1 domains; (iv) a Fv fragment consisting of the VL and VH domains of a single arm of an antibody, (v) a dAb fragment [Ward et al., Nature 341:544-546, (1989)], which consists of a VH domain; and (vi) an isolated complementarity determining region (CDR).

F(ab')$_2$ fragments can be produced by pepsin digestion of the antibody molecule, and Fab fragments can be generated by reducing the disulfide bridges of F(ab')$_2$ fragments. Alternatively, Fab expression libraries can be constructed [Huse et al., Science 246:1275, (1989)] to allow rapid and easy identification of monoclonal Fab fragments with the desired specificity. Methods of making other antibodies and antibody fragments are known in the art. For example, although the two domains of the Fv fragment, VL and VH, are coded for by separate genes, they can be joined, using recombinant methods or a synthetic linker that enables them to be made as a single protein chain in which the VL and VH regions pair to form monovalent molecules [known as single chain Fv (scFv); see e.g., Bird et al., Science 242:423-426, (1988); Huston et al., Proc. Natl. Acad. Sci. USA 85:5879-5883, (1988); Colcher et al., Ann. NY Acad. Sci. 880:263-80, (1999); and Reiter, Clin. Cancer Res. 2:245-52, (1996)]. Techniques for producing single chain antibodies are also described in U.S. Patent Nos. 4,946,778 and 4,704,692. Such single chain antibodies are encompassed within the term "antigen-binding fragment" of an antibody. These antibody fragments are obtained using conventional techniques known to those of ordinary skill in the art, and the fragments are screened for utility in the same manner that intact antibodies are screened. Moreover, a single chain antibody can form dimers or multimers and, thereby, become a multivalent antibody having specificities for different epitopes of the same target protein.

The antibody can be a polyclonal (i.e., part of a heterogeneous population of antibody molecules derived from the sera of the immunized animals) or a monoclonal antibody (i.e., part of a homogeneous population of antibodies to a particular antigen), either of which can be recombinantly produced (e.g., produced by phage display or by combinatorial methods, as described in, e.g., U.S. Patent No. 5,223,409; WO 92/18619; WO 91/17271; WO 92/20791; WO 92/15679; WO 93/01288; WO 92/01047; WO 92/09690; WO 90/02809; Fuchs et al., Bio/Technology 9:1370-1372, (1991); Hay et al. Human Antibody Hybridomas 3:81-85, (1992); Huse et al. Science 246:1275-1281, (1989); Griffths et al. EMBO J 12:725-734, (1993); Hawkins et al., J. Mol Biol 226:889-896, (1992); Clackson et al. Nature 352:624-628, (1991); Gram et al., Proc. Natl. Acad. Sci. USA 89:3576-3580, (1992); Garrad et al., Bio/Technology 9:1373-1377, (1991); Hoogenboom et al. Nucl. Acids Res. 19:4133-4137, (1991); and Barbas et al., Proc. Natl. Acad. Sci. USA 88:7978-7982, (1991). In one embodiment, an antibody is made by immunizing an animal with a protein encoded by a nucleic acid of the invention (one, of course, that contains coding sequence) or a mutant or fragment (e.g., an antigenic peptide fragment) thereof. Alternatively, an animal can be immunized with a tissue sample (e.g., a crude tissue preparation, a whole cell (living, lysed, or fractionated) or a membrane fraction). Thus, antibodies of the invention can specifically bind to a purified antigen or a tissue (e.g., a tissue section, a whole cell (living, lysed, or fractionated) or a membrane fraction).

In the event an antigenic peptide is used, it can include at least eight (e.g., 10, 15, 20, or 30) consecutive amino acid residues found in a protein of the invention. The antibodies generated can specifically bind to one of the proteins in their native form (thus, antibodies with linear or conformational epitopes are within the invention), in a denatured or otherwise non-native form, or both. Conformational epitopes can sometimes be identified by identifying antibodies that bind to a protein in its native form, but not in a denatured form.

The host animal (e.g., a rabbit, mouse, guinea pig, or rat) can be immunized with the antigen, optionally linked to a carrier (i.e., a substance that stabilizes or otherwise improves the immunogenicity of an associated molecule), and optionally administered with an adjuvant (see, e.g., Ausubel et al., supra). An exemplary carrier is keyhole limpet hemocyanin (KLH) and exemplary adjuvants, which will be selected in view of the host animal's species, include Freund's adjuvant (complete or incomplete), adjuvant mineral gels (e.g., aluminum hydroxide), surface active substances such as lysolecithin, pluronic polyols, polyanions, peptides, oil emulsions, dinitrophenol, BCG (bacille Calmette-Guerin), and Corynebacterium parvum. KLH is also sometimes referred to as an adjuvant The antibodies generated in the host can be purified by, for example, affinity chromatography methods in which the polypeptide antigen is immobilized on a resin.

Epitopes encompassed by an antigenic peptide may be located on the surface of the protein (e.g., in hydrophilic regions), or in regions that are highly antigenic (such regions can be selected, initially, by virtue of containing many charged residues). An Emini surface probability analysis of human protein sequences can be used to indicate the regions that have a particularly high probability of being localized to the surface of the protein.

The antibody can be a fully human antibody (e.g., an antibody made in a mouse that has been genetically engineered to produce an antibody from a human immunoglobulin sequence, such as that of a human immunoglobulin gene (the kappa, lambda, alpha (IgA1 and IgA2), gamma (IgG1, IgG2, IgG3, IgG4), delta, epsilon and mu constant region genes or the myriad immunoglobulin variable region genes). Alternatively, the antibody can be a non-human antibody (e.g., a rodent (e.g., a mouse or rat), goat, or non-human primate (e.g., monkey) antibody).

Methods of producing antibodies are well known in the art. For example, as noted above, human monoclonal antibodies can be generated in transgenic mice carrying the human immunoglobulin genes rather than those of the mouse. Splenocytes obtained from these mice (after immunization with an antigen of interest) can be used to produce hybridomas that secrete human mAbs with specific affinities for epitopes from a human protein (see, e.g., WO 91/00906, WO 91/10741; WO 92/03918; WO 92/03917; Lonberg et al., Nature 368:856-859, 1994; Green et al., Nature Genet. 7:13-21, 1994; Morrison et al. Proc. Natl. Acad. Sci. USA 81:6851-6855, 1994; Bruggeman et al., Immunol. 7:33-40, 1993; Tuaillon et al., Proc. Natl. Acad. Sci. USA 90:3720-3724, 1993; and Bruggeman et al., Eur. J. Immunol 21:1323-1326, 1991).

The antibody can also be one in which the variable region, or a portion thereof (e.g., a CDR), is generated in a non-human organism (e.g., a rat or mouse). Thus, the invention encompases chimeric, CDR-grafted, and humanized antibodies and antibodies that are generated in a non-human organism and then modified (in, e.g., the variable framework or constant region) to decrease antigenicity in a human. Chimeric antibodies (i.e., antibodies in which different portions are derived from different animal species (e.g., the variable region of a murine mAb and the constant region of a human immunoglobulin) can be produced by recombinant techniques known in the art. For example, a gene encoding the Fc constant region of a murine (or other species) monoclonal antibody molecule can be digested with restriction enzymes to remove the region encoding the murine Fc, and the equivalent portion of a gene encoding a human Fc constant region can be substituted therefore [see European Patent Application Nos. 125,023; 184,187; 171,496; and 173,494; see also WO 86/01533; U.S. Patent No. 4,816,567; Better et al., Science 240:1041-1043, (1988); Liu et al., Proc. Natl. Acad. Sci. USA 84:3439-3443, (1987); Liu et al., J. Immunol. 139:3521-3526, (1987); Sun et al., Proc. Natl. Acad. Sci. USA 84:214-218, (1987); Nishimura et al., Cancer Res. 47:999-1005, (1987); Wood et al., Nature 314:446-449, (1985); Shaw et al., J. Natl. Cancer Inst. 80:1553-1559, (1988); Morrison et al., Proc. Natl. Acad. Sci. USA 81:6851, (1984); Neuberger et al., Nature 312:604, (1984); and Takeda et al., Nature 314:452, (1984)].

In a humanized or CDR-grafted antibody, at least one or two, but generally all three of the recipient CDRs (of heavy and or light immuoglobulin chains) will be replaced with a donor CDR. One need only replace the number of CDRs required for binding of the humanized antibody to a protein described herein or a fragment thereof. The donor can be a rodent antibody, and the recipient can be a human framework or a human consensus framework. Typically, the immunoglobulin providing the CDRs is called the "donor" (and is often that of a rodent) and the immunoglobulin providing the framework is called the "acceptor." The acceptor framework can be a naturally occurring (e,g., a human) fratnework, a consensus framework or sequence, or a sequence that is at least 85 % (e.g., 90 %, 95 %, 99%) identical thereto. A "consensus sequence" is one formed from the most frequently occurring amino acids (or nucleotides) in a family of related sequences (see, e.g., Winnaker, From Genes to Clones, Verlagsgesellschaft, Weinheim, Germany, 1987). Each position in the consensus sequence is occupied by the amino acid residue that occurs most frequently at that position in the family (where two occur equally frequently, either can be included). A "consensus framework" refers to the framework region in the consensus immunoglobulin sequence.

An antibody can be humanized by methods known in the art. For example, humanized antibodies can be generated by replacing sequences of the Fv variable region that are not directly involved in antigen binding with equivalent sequences from human Fv variable regions. General methods for generating humanized antibodies are provided by Morrison [Science 229:1202-1207, (1985)], Oi et al. [BioTechniques 4:214, (1986)], and Queen et al. (US Patent Nos. 5,585,089; 5,693,761 and 5,693,762). Those nucleic acid sequences required by these methods can be obtained from a hybridoma producing an antibody the polypeptides of the present invention, or fragments thereof. The recombinant DNA encoding the humanized antibody, or fragment thereof, can then be cloned into an appropriate expression vector.

Humanized or CDR-grafted antibodies can be produced such that one, two, or all CDRs of an immunoglobulin chain can be replaced [see, e.g., U.S. Patent No. 5,225,539; Jones et al., Nature 321:552-525, (1986); Verhoeyan et al., Science 239:1534, (1988); and Beidler et al., J. Immunol. 141:4053-4060, (1988)]. Thus, the invention features humanized antibodies in which specific amino acid residues have been substituted, deleted or added (in, e.g., in the framework region to improve antigen binding). For example, a humanized antibody will have framework residues identical to those of the donor or to amino acid residues other than those of the recipient framework residue. To generate such antibodies, a selected, small number of acceptor framework residues of the humanized immunoglobulin chain are replaced by the corresponding donor amino acids. The substitutions can occur adjacent to the CDR or in regions that interact with a CDR (U.S. Patent No. 5,585,089, see especially columns 12-16). Other techniques for humanizing antibodies are described in EP 519596 A1.

In certain embodiments, the antibody has an effector function and can fix complement, while in others it can neither recruit effector cells nor fix complement. The antibody can also have little or no ability to bind an Fc receptor. For example, it can be an isotype or subtype, or a fragment or other mutant that cannot bind to an Fc receptor (e.g., the antibody can have a. mutant (e.g., a deleted) Fc receptor binding region). The antibody may or may not alter (e.g., increase or decrease) the activity of a protein to which it binds.

In other embodiments, the antibody can be coupled to a heterologous substance, such as a toxin (e.g., ricin, diphtheria toxin, or active fragments thereof), another type of therapeutic agent (e.g., an antibiotic), or a detectable label.

A detectable label can include an enzyme (e.g., horseradish peroxidase, alkaline phosphatase, $\beta$-galactosidase, or acetylcholinesterase), a prosthetic group (e.g., streptavidin/biotin and avidin/biotin), or a fluorescent, luminescent, bioluminescent, or radioactive material.(e.g., umbelliferone, fluorescein, fluorescein isothiocyanate, rhodamine, dichlorotriazinylamine fluorescein, dansyl chloride or phycoerythrin (which are fluorescent), luminol (which is luminescent), luciferase, luciferin, and aequorin (which are bioluminescent), and $^{125}I$, $^{131}I$, $^{31}S$ or $^{3}H$ (which are radioactive)).

The antibodies of the invention (e.g., a monoclonal antibody) can be used to isolate the proteins of the invention (by, for example, affinity chromatography or immunoprecipitation) or to detect them in, for example, a cell lysate or supernatant (by Western blotting, ELISAs, radioimmune assays, and the like) or a histological section. One can therefore determine the abundance and pattern of expression of a particular protein. This information can be useful in making a diagnosis or in evaluating the efficacy of a clinical test.

The invention also includes the nucleic acids that encode the antibodies described above and vectors and cells (e.g., mammalian cells such as CHO cells or lymphatic cells) that contain them. Similarly, the invention includes cell lines (e.g., hybridomas) that make the antibodies of the invention and methods of making those cell lines.

Non-human transgenic animals are also within the scope of the invention. These animals can be used to study the function or activity of proteins of the invention and to identify or evaluate agents that modulate their activity. A "transgenic animal" can be a mammal (e.g., a mouse, rat, dog, pig, cow, sheep, goat, or non-human primate), an avian (e.g., a chicken), or an amphibian (e.g. a frog) having one or more cells that include a transgene (e.g., an exogenous DNA molecule or a rearrangement (e.g., deletion of) endogenous chromosomal DNA). The transgene can be integrated into or can occur within the genome of the cells of the animal, and it can direct the expression of an encoded gene product in one or more types of cells or tissues. Alternatively, a transgene can "knock out" or reduce gene expression. This can occur when an endogenous gene has been altered by homologous recombination, which occurs between it and an exogenous DNA molecule that was introduced into a cell of the animal (e.g., an embryonic cell) at a very early stage in the animal's development.

Intronic sequences and polyadenylation signals can be included in the transgene and, when present, can increase expression. One or more tissue-specific regulatory sequences can also be operably linked to a transgene of the invention to direct expression of protein to particular cells (exemplary regulatory sequences are described above, and many others are known to those of ordinary skill in the art).

A "founder" animal is one that carries a transgene of the invention in its genome or expresses mRNA from the transgene in its cells or tissues. Founders can be bred to produce a line of transgenic animals carrying the founder's transgene or bred with founders carrying other transgenes (in which case the progeny would bear the transgenes borne by both founders). Accordingly, the invention features founder animals, their progeny, cells or populations of cells obtained therefrom, and proteins obtained therefrom. For example, a nucleic acid of the invention can be placed under the control of a promoter that directs expression of the encoded protein in the milk or eggs of the transgenic animal. The protein can then be purified or recovered from the animal's milk or eggs. Animals suitable for such purpose include pigs, cows, goats, sheep, and chickens.

Biomolecular sequences of the present invention can be classified to functional groups based on known activity of homologous sequences. This functional group classification, allows the identification of diseases and conditions, which may be diagnosed and treated based on the novel sequence information and annotations as described in the present invention.

This functional group classification includes the following groups:

### Proteins involved in Drug-Drug interactions:

The phrase "proteins involved in drug-drug interactions" refers to proteins involved in a biological process which mediates the interaction between at least two consumed drugs.

Pharmaceutical compositions including such proteins or protein encoding sequences, antibodies directed against such proteins or polynucleotides capable of altering expression of such proteins, may be used to modulate drug-drug interactions. Antibodies and polynucleotides such as PCR primers and molecular probes designed to identify such proteins or protein encoding sequences may be used for diagnosis of such drug-drug interactions.

Examples of these conditions include, but are not limited to the cytochrom P450 protein family, which is involved in the metabolism of many drugs.

Examples of proteins involved in drug-drug interactions are listed in Table 16, below.

### Proteins involved in the metabolism of a pro-drug to a drug:

The phrase "proteins involved in the metabolism of a pro-drug to a drug" refers to proteins that activate an inactive pro-drug by chemically chaining it into a biologically active compound. Preferably, the metabolizing enzyme is expressed in the target tissue thus reducing systemic side effects.

Pharmaceutical compositions including such proteins or protein encoding sequences, antibodies directed against such proteins or polynucleotides capable of altering expression of such proteins, may be used to modulate the metabolism of a pro-drug into drug. Antibodies and polynucleotides such as PCR primers and molecular probes designed to identify such proteins or protein encoding sequences may be used for diagnosis of such conditions.

Examples of these proteins include, but are not limited to esterases hydrolyzing the cholesterol lowering drug simvastatin into its hydroxy acid active form.

*MDR proteins:*

The phrase "MDR proteins" refers to Multi Drug Resistance proteins that are responsible for the resistance of a cell to a range of drugs, usually by exporting these drugs outside the cell. Preferably, the MDR proteins are ABC binding cassette proteins. Preferably, drug resistance is associated with resistance to chemotherapy.

Pharmaceutical compositions including such proteins or protein encoding sequences, antibodies directed against such proteins or polynucleotides capable of altering expression of such proteins, may be used to treat diseases in which the transport of molecules and macromolecules such as neurotransmitters, hormones, sugar etc. is abnormal leading to various pathologies. Antibodies and polynucleotides such as PCR primers and molecular probes designed to identify such proteins or protein encoding sequences may be used for diagnosis of such diseases.

Examples of MDR proteins include, but are not limited to the multi-drug resistant transporter MDRI/P-glycoprotein, which is the gene product of MDR1, belonging to the ATP-binding cassette (ABC) superfamily of membrane transporters. This protein was shown to increase the resistance of malignant cells to therapy by exporting the therapeutic agent out of the cell.

*Hydrolases acting on amino acids:*

The phrase "hydrolases acting on amino acids" refers to hydrolases acting on a pair of amino acids.

Pharmaceutical compositions including such proteins or protein encoding sequences, antibodies directed against such proteins or polynucleotides capable of altering expression of such proteins, may be used to treat diseases in which the transfer of a glycosyl chemical group from one molecule to another is abnormal thus, a beneficial effect may be achieved by modulation of such reaction. Antibodies and polynucleotides such as PCR primers and molecular probes designed to identify such proteins or protein encoding sequences may be used for diagnosis of such diseases.

Examples of such diseases include, but are not limited to reperfusion of clotted blood vessels by TPA (Tissue Plasminogen Activator) which converts the abundant, but inactive, zymogen plasminogen to plasmin by hydrolyzing a single ARG-VAL bond in plasminogen.

*Transaminases:*

The term "transaminases" refers to enzymes transferring an amine group from one compound to another.

Pharmaceutical compositions including such proteins or protein encoding sequences, antibodies directed against such proteins or polynucleotides capable of altering expression of such proteins, may be used to treat diseases in which the transfer of an amine group from one molecule to another is abnormal thus, a beneficial effect may be achieved by modulation of such reaction. Antibodies and polynucleotides such as PCR primers and molecular probes designed to identify such proteins or protein encoding sequences may be used for diagnosis of such diseases.

Examples of such transaminases include, but are not limited to two liver enzymes, frequently used as markers for liver function - SGOT (Serum Glutamic-Oxalocetic Transaminase - AST) and SGPT (Serum Glutamic-Pyruvic Transaminase - ALT).

*Immunoglobulins:*

The term "immunoglobulins" refers to proteins that are involved in the immune and complement systems such as antigens and autoantigens, immunoglobulins, MHC and HLA proteins and their associated proteins.

Pharmaceutical compositions including such proteins or protein encoding sequences, antibodies directed against such proteins or polynucleotides capable of altering expression of such proteins, may be used to treat diseases involving the immune system such as inflammation, autoimmune diseases, infectious diseases, and cancerous processes. Antibodies and polynucleotides such as PCR primers and molecular probes designed to identify such proteins or protein encoding sequences may be used for diagnosis of such diseases.

Examples of such diseases and molecules that may be target for diagnostics include, but are not limited to members of the complement family such as C3 and C4 that their blood level is used for evaluation of autoimmune diseases and allergy state and C1 inhibitor that its absence is associated with angioedema. Thus, new variants of these genes are expected to be markers for similar events. Mutation in variants of the complement family may be associated with other immunological syndromes, such as increased bacterial infection that is associated with mutation in C3. C1 inhibitor was shown to provide safe and effective inhibition of complement activation after reperfused acute myocardial infarction and may reduce myocardial injury [Eur. Heart J. 2002, 23(21):1670-7], thus, its variant may have the same or improved effect.

*Transcription factor binding:*

The phrase "transcription factor binding" refers to proteins involved in transcription process by binding to nucleic acids, such as transcription factors, RNA and DNA binding proteins, zinc fingers, helicase, isomerase, histones, and nucleases.

Pharmaceutical compositions including such proteins or protein encoding sequences, antibodies directed against such proteins or polynucleotides capable of altering expression of such proteins may be used to treat diseases involving transcription factors binding proteins. Such treatment may be based on transcription factor that can be used to for modulation of gene expression associated with the disease. Antibodies and polynucleotides such as PCR primers and molecular probes designed to identify such proteins or protein encoding sequences may be used for diagnosis of such diseases.

Examples of such diseases include, but are not limited to breast cancer associated with ErbB-2 expression that was shown to be successfully modulated by a transcription factor [Proc. Natl. Acad. Sci. USA. 2000, 97(4):1495-500]. Examples of novel transcription factors used for therapeutic protein production include, but are not limited to those described for Erythropoietin production [J. Biol. Chem. 2000, 275(43):33850-60; J. Biol. Chem. 2000, 275(43):33 850-60] and zinc fingers protein transcription factors (ZFP-TF) variants [J. Biol. Chem. 2000, 275(43):33850-60].

*Small GTPase regulatory/interacting proteins:*

The phrase "Small GTPase regulatory/interacting proteins" refers to proteins capable of regulating or interacting with GTPase such as RAB escort protein, guanyl-nucleotide exchange factor, guanyl-nucleotide exchange factor adaptor, GDP-dissociation inhibitor, GTPase inhibitor, GTPase activator, guanyl-nucleotide releasing factor, GDP-dissociation stimulator, regulator of G-protein signaling, RAS interactor, RHO interactor, RAB interactor, and RAL interactor.

Pharmaceutical compositions including such proteins or protein encoding sequences, antibodies directed against such proteins or polynucleotides capable of altering expression of such proteins, may be used to treat diseases in which G-proteases mediated signal-transduction is abnormal, either as a cause, or as a result of the disease. Antibodies and polynucleotides such as PCR primers and molecular probes designed to identify such proteins or protein encoding sequences may be used for diagnosis of such diseases.

Examples of such diseases include, but are not limited to diseases related to prenylation. Modulation of prenylation was shown to affect therapy of diseases such as osteoporosis, ischemic heart disease, and inflammatory processes. Small GTPases regulatory/interacting proteins are major component in the prenylation post translation modification, and are required to the normal activity of prenylated proteins. Thus, their variants maybe used for therapy of prenylation associated diseases.

*Calcium binding proteins:*

The phrase "calcium binding proteins" refers to proteins involve in calcium binding, preferably, calcium binding proteins, ligand binding or carriers, such as diacylglycerol kinase, Calpain, calcium-dependent protein serine/threonine phosphatase, calcium sensing proteins, calcium storage proteins.

Pharmaceutical compositions including such proteins or protein encoding sequences, antibodies directed against such proteins or polynucleotides capable of altering expression of such proteins, may be used to treat calcium involved diseases. Antibodies and polynucleotides such as PCR primers and molecular probes designed to identify such proteins or protein encoding sequences may be used for diagnosis of such diseases.

Examples of such diseases include, but are not limited to diseases related to hypercalcemia, hypertension, cardiovascular disease, muscle diseases, gastro-intestinal diseases, uterus relaxing, and uterus. An example for therapy use of calcium binding proteins variant may be treatment of emergency cases of hypercalcemia, with secreted variants of calcium storage proteins.

*Oxidoreductase:*

The term "oxidoreductase" refers to enzymes that catalyze the removal of hydrogen atoms and electrons from the compounds on which they act. Preferably, oxidoreductases acting on the following groups of donors: CH-OH, CH-CH, CH-NH2, CH-NH; oxidoreductases acting on NADH or NADPH, nitrogenous compounds, sulfur group of donors, heme group, hydrogen group, diphenols and related substances as donors; oxidoreductases acting on peroxide as acceptor, superoxide radicals as acceptor, oxidizing metal ions, CH2 groups; oxidoreductases acting on reduced ferredoxin as donor; oxidoreductases acting on reduced flavodoxin as donor; and oxidoreductases acting on the aldehyde or oxo group of donors.

Pharmaceutical compositions including such proteins or protein encoding sequences, antibodies directed against such proteins or polynucleotides capable of altering expression of such, proteins, may be used to treat diseases caused by abnormal activity of oxidoreductases. Antibodies and polynucleotides such as PCR primers and molecular probes designed to identify such proteins or protein encoding sequences may be used for diagnosis of such diseases.

Examples of such diseases include, but are not limited to malignant and autoimmune diseases in which the enzyme DHFR (DiHydroFolateReductase) that participates in folate metabolism and essential for *de novo* glycine and purine synthesis is the target for the widely used drug Methotrexate (MTX).

*Receptors:*

The term "receptors" refers to protein-binding sites on a cell's surface or interior, that recognize and binds to specific messenger molecule leading to a biological response, such as signal transducers, complement receptors, ligand-dependent nuclear receptors, transmembrane receptors, GPI-anchored membrane-bound receptors, various coreceptors, internalization receptors, receptors to neurotransmitters, hormones and various other effectors and ligands.

Pharmaceutical compositions including such proteins or protein encoding sequences, antibodies directed against such proteins or polynucleotides capable of altering expression of such proteins, may be used to treat diseases caused by abnormal activity of receptors, preferably, receptors to neurotransmitters, hormones and various other effectors and ligands. Antibodies and polynucleotides such as PCR primers and molecular probes designed to identify such proteins or protein encoding sequences may be used for diagnosis of such diseases.

Examples of such diseases include, but are not limited to, chronic myelomonocytic leukemia caused by growth factor $\beta$ receptor deficiency [Rao D. S., et al., (2001) Mol. Cell Biol., 21(22):7796-806], thrombosis associated with protease-activated receptor deficiency [Sambrano G. R., et al., (2001) Nature, 413(6851):26-7], hypercholesterolemia associated with low density lipoprotein receptor deficiency [Koivisto U. M., et al., (2001) Cell, 105(5):575-85], familial Hibernian fever associated with tumor necrosis factor receptor deficiency [Simon A., et al., (2001) Ned Tijdschr Geneeskd, 145(2):77-8], colitis associated with immunoglobulin E receptor expression [Dombrowicz D., et al., (2001) J. Exp. Med., 193(1):25-34], and alagille syndrome associated with Jagged1 [Stankiewicz P. et al., (2001) Am. J. Med. Genet., 103(2):166-71], breast cancer associated with mutated BRCA2 and androgen, hypertension associated with $\beta$ and $\alpha$ adrenergic receptors, diabetes associated with the insulin receptor. Therapeutic applications of nuclear receptors variants may be based on secreted version of receptors such as the thyroid nuclear receptor that by binding plasma free thyroid hormone to reduce its levels may have a therapeutic effect in cases of thyrotoxicosis. A secreted version of glucocorticoid nuclear receptor, by binding plasma free cortisol, thus, reducing, may have a therapeutic effect in cases of Cushing's disease (a disease associated with high cortisole levels in the plasma).

Secreted soluble TNF receptor is an example for a molecule, which can be used to treat conditions in which downregulation of TNF levels or activity is benefitial, including, but not limited to, Rheumatoid Arthritis, Juvenile Rheumatoid Arthritis, Psoriatic Arthritis and Ankylosing Spondylitis.

*Protein serine/threonine kinases*:

The phrase protein serine/threonine kinases" refers to proteins which phosphorylate serine/threonine residues, mainly involved in signal transduction, such as transmembrane receptor protein serine/threonine kinase, 3-phosphoinositide-dependent protein kinase, DNA-dependent protein kinase, G-protein-coupled receptor phosphorylating protein kinase, SNF1A/AMP-activated protein kinase, casein kinase, calmodulin regulated protein kinase, cyclic-nucleotide dependent protein kinase, cyclin-dependent protein kinase, eukaryotic translation initiation factor $2\alpha$ kinase, galactosyltransferase-associated kinase, glycogen synthase kinase 3, protein kinase C, receptor signaling protein serine/threonine kinase, ribosomal protein S6 kinase, and IkB kinase.

Pharmaceutical compositions including such proteins or protein encoding sequences, antibodies directed against such proteins or polynucleotides capable of altering expression of such proteins, may be used to treat diseases ameliorated by a modulating kinase activity. Antibodies and polynucleotides such as PCR primers and molecular probes designed to identify such proteins or protein encoding sequences may be used for diagnosis of such diseases.

Examples of such diseases include, but are not limited to schizophrenia. 5-HT(2A) serotonin receptor is the principal molecular target for LSD-like hallucinogens and atypical antipsychotic drugs. It has been shown that a major mechanism for the attenuation of this receptor signaling following agonist activation typically involves the phosphorylation of serine and/or threonine residues by various kinases. Therefore, serine/threonine kinases specific for the 5-HT(2A) serotonin receptor may serve as drug targets for a disease such as schizophrenia. Other diseases that may be treated through serine/thereonine kinases modulation are Peutz-Jeghers syndrome (PJS, a rare autosomal-dominant disorder characterized by hamartomatous polyposis of the gastrointestinal tract and melanin pigmentation of the skin and mucous membranes [Hum. Mutat 2000, 16(1):23-30], breast cancer [Oncogene. 1999, 18(35):4968-73], Type 2 diabetes insulin resistance [Am. J. Cardiol. 2002, 90(5A):11G-18G], and fanconi anemia [Blood. 2001, 98(13):3650-7].

***Channel/pore class transporters:***

The phrase "Channel/pore class transporters" refers to proteins that mediate the transport of molecules and macromolecules across membranes, such as $\alpha$-type channels, porins, and pore-forming toxins.

Pharmaceutical compositions including such proteins or protein encoding sequences, antibodies directed against such proteins or polynucleotides capable of altering expression of such proteins, may be used to treat diseases in which the transport of molecules and macromolecules are abnormal, therefore leading to various pathologies. Antibodies and polynucleotides such as PCR primers and molecular probes designed to identify such proteins or protein encoding sequences may be used for diagnosis of such diseases.

Examples of such diseases include, but are not limited to diseases of the nerves system such as Parkinson, diseases of the hormonal system, diabetes and infectious diseases such as bacterial and fungal infections. One specific example is the $\alpha$-hemolysin, which is produced by S. aureus creating ion conductive pores in the cell membrane, thereby deminishing its integrity.

***Hydrolases, acting on acid anhydrides:***

The phrase "hydrolases, acting on acid anhydrides" refers to hydrolytic enzymes that are acting on acid anhydrides, such as hydrolases acting on acid anhydrides in phosphorus-containing anhydrides or in sulfonyl-containing anhydrides, hydrolases catalyzing transmembrane movement of substances, and involved in cellular and subcellular movement.

Pharmaceutical compositions including such proteins or protein encoding sequences, antibodies directed against such proteins or polynucleotides capable of altering expression of such proteins may be used to treat diseases in which the hydrolase-related activities are abnormal. Antibodies and polynucleotides such as PCR primers and molecular probes designed to identify such proteins or protein encoding sequences may be used for diagnosis of such diseases.

Examples of such diseases include, but are not limited to glaucoma treated with carbonic anhydrase inhibitors (e.g. Dorzolamide), peptic ulcer disease treated with $H(^+)K(^+)ATPase$ inhibitors that were shown to affect disease by blocking gastric carbonic anhydrase (e.g. Omeprazole).

***Transferases, transferring phosphorus-containing groups:***

The phrase "transferases, transferring phosphorus-containing groups" refers to enzymes that catalyze the transfer of phosphate from one molecule to another, such as phosphotransferases using the following groups as acceptors: alcohol group, carboxyl group, nitrogenous group, phosphate; phosphotransferases with regeneration of donors catalyzing intramolecular transfers; diphosphotransferases; nucleotidyltransferase; and phosphotransferases for other substituted phosphate groups.

Pharmaceutical compositions including such proteins or protein encoding sequences, antibodies directed against such proteins or polynucleotides capable of altering expression of such proteins may be used to treat diseases in which the transfer of a phosphorous containing functional group to a modulated moiety is abnormal. Antibodies and polynucleotides such as PCR primers and molecular probes designed to identify such proteins or protein encoding sequences may be used for diagnosis of such diseases.

Examples of such diseases include, but are not limited to acute MI [Ann. Emerg. Med. 2003, 42(3):343-50], Cancer [Oral. Dis. 2003, 9(3):119-28; J. Surg. Res. 2003, 113(1):102-8] and Alzheimer's disease [Am. J. Pathol. 2003, 163(3):845-58]. Examples for possible utilities of such transferases for drug improvement include, but are not limited to aminoglycosides treatment (antibiotics) to which resistance is mediated by aminoglycoside phosphotransferases [Front. Biosci. 1999, 1;4:D9-21]. Using aminoglycoside phosphotransferases variants or inhibiting these enzymes may reduce aminoglycosides resistance. Since aminoglycosides can be toxic to some patients, proving the expression of aminoglycoside phosphotransferases in a patient can deter from treating him with aminoglycosides and risking the patient in vain.

***Phosphoric monoester hydrolases:***

The phrase "phosphoric monoester hydrolases" refers to hydrolytic enzymes that are acting on ester bonds, such as nuclease, sulfuric ester hydrolase, carboxylic ester hydrolase, thiolester hydrolase, phosphoric monoester hydrolase, phosphoric diester hydrolase, triphosphoric monoester hydrolase, diphosphoric monoester hydrolase, and phosphoric triester hydrolase,

Pharmaceutical compositions including such proteins or protein encoding sequences, antibodies directed against such proteins or polynucleotides capable of altering expression of such proteins, may be used to treat diseases in which the hydrolytic cleavage of a covalent bond with accompanying addition of water (-H being added to one product of the cleavage and -OH to the other), is abnormal. Antibodies and polynucleotides such as PCR primers and molecular probes designed to identify such proteins or protein encoding sequences maybe used for diagnosis of such diseases.

Examples of such diseases include, but are not limited to diabetes and CNS diseases such as Parkinson and cancer.

*Enzyme inhibitors:*

The term "enzyme inhibitors" refers to inhibitors and suppressors of other proteins and enzymes, such as inhibitors of: kinases, phosphatases, chaperones, guanylate cyclase, DNA gyrase, ribonuclease, proteasome inhibitors, diazepam-binding inhibitor, ornithine decarboxylase inhibitor, GTPase inhibitors, dUTP pyrophosphatase inhibitor, phospholipase inhibitor, proteinase inhibitor, protein biosynthesis inhibitors, and $\alpha$-amylase, inhibitors.

Pharmaceutical compositions including such proteins or protein encoding sequences, antibodies directed against such proteins or polynucleotides capable of altering expression of such proteins, may be used to treat diseases in which beneficial effect may be achieved by modulating the activity of inhibitors and suppressors of proteins and enzymes. Antibodies and polynucleotides such as PCR primers and molecular probes designed to identify such proteins or protein encoding sequences may be used for diagnosis of such diseases.

Examples of such diseases include, but are not limited to $\alpha$-1 antitrypsin (a natural serine proteases, which protects the lung and liver from proteolysis) deficiency associated with emphysema, COPD and liver chirosis. $\alpha$-1 antitrypsin is also used for diagnostics in cases of unexplained liver and lung disease. A variant of this enzyme may act as protease inhibitor or a diagnostic target for related diseases.

*Electron transporters:*

The term "Electron transporters" refers to ligand binding or carrier proteins involved in electron transport such as flavin-containing electron transporter, cytochromes, electron donors, electron acceptors, electron carriers, and cytochrome-c oxidases.

Pharmaceutical compositions including such proteins or protein encoding sequences, antibodies directed against such proteins or polynucleotides capable of altering expression of such proteins, may be used to treat diseases in which beneficial effect may be achieved by modulating the activity of electron transporters. Antibodies and polynucleotides such as PCR primers and molecular probes designed to identify such proteins or protein encoding sequences may be used for diagnosis of such diseases.

Examples of such diseases include, but are not limited to cyanide toxicity, resulting from cyanide binding to ubiquitous metalloenzymes rendering them inactive, and interfering with the electron transport. Novel electron transporters to which cyanide can bind may serve as drug targets for new cyanide antidotes.

*Transferases, transferring glycosyl groups:*

The phrase "transferases, transferring glycosyl groups" refers to enzymes that catalyze the transfer of a glycosyl chemical group from one molecule to another such as murein lytic endotransglycosylase E, and sialyltransferase.

Pharmaceutical compositions including such proteins or protein encoding sequences, antibodies directed against such proteins or polynucleotides capable of altering expression of such proteins, may be used to treat diseases in which the transfer of a glycosyl chemical group is abnormal. Antibodies and polynucleotides such as PCR primers and molecular probes designed to identify such proteins or protein encoding sequences may be used for diagnosis of such diseases.

*Ligases, forming carbon-oxygen bonds:*

The phrase "ligases, forming carbon-oxygen bonds" refers to enzymes that catalyze the linkage between carbon and oxygen such as ligase forming aminoacyl-tRNA and related compounds.

Pharmaceutical compositions including such proteins or protein encoding sequences, antibodies directed against such proteins or polynucleotides capable of altering expression of such proteins, may be used to treat diseases in which the linkage between carbon and oxygen in an energy dependent process is abnormal. Antibodies and polynucleotides such as PCR primers and molecular probes designed to identify such proteins or protein encoding sequences may be used for diagnosis of such diseases.

*Ligases:*

The term "ligases" refers to enzymes that catalyze the linkage of two molecules, generally utilizing ATP as the energy donor, also called synthetase. Examples for ligases are enzymes such as $\beta$-alanyl-dopamine hydrolase, carbon-oxygen bonds forming ligase, carbon-sulfur bonds forming ligase, carbon-nitrogen bonds forming ligase, carbon-carbon bonds forming ligase, and phosphoric ester bonds forming ligase.

Pharmaceutical compositions including such proteins or protein encoding sequences, antibodies directed against such proteins or polynucleotides capable of altering expression of such proteins, may be used to treat diseases in which the joining together of two molecules in an energy dependent process is abnormal. Antibodies and polynucleotides such as PCR primers and molecular probes designed to identify such proteins or protein encoding sequences may be used for diagnosis of such diseases.

Examples of such diseases include, but are not limited to neurological disorders such as Parkinson's disease [Science. 2003, 302(5646):819-22; J. Neurol. 2003, 250 Suppl. 3:III25-III29] or epilepsy [Nat. Genet. 2003, 35(2):125-7], cancerous diseases [Cancer Res. 2003, 63(17):5428-37; Lab. Invest. 2003, 83(9):1255-65], renal diseases [Am. J. Pathol. 2003, 163(4):1645-52], infectious diseases [Arch. Virol. 2003, 148(9):1851-62] and fanconi anemia [Nat. Genet. 2003, 35(2):165-70].

*Hydrolases, acting on glycosyl bonds:*

The phrase "hydrolases, acting on glycosyl bonds" refers to hydrolytic enzymes that are acting on glycosyl bonds such as hydrolases hydrolyzing N-glycosyl compounds, S-glycosyl compounds, and O-glycosyl compounds.

Pharmaceutical compositions including such proteins or protein encoding sequences, antibodies directed against such proteins or polynucleotides capable of altering expression of such proteins, may be used to treat diseases in which the hydrolase-related activities are abnormal. Antibodies and polynucleotides such as PCR primers and molecular probes designed to identify such proteins or protein encoding sequences may be used for diagnosis of such diseases.

Examples of such diseases include cancerous diseases [J. Natl. Cancer Inst. 2003, 95(17):1263-5; Carcinogenesis. 2003, 24(7):1281-2; author reply 1283] vascular diseases [J. Thorac. Cardiovasc. Surg. 2003, 126(2):344-57], gastrointestinal diseases such as colitis [J. Immunol. 2003, 171(3):1556-63] or liver fibrosis [World J. Gastroenterol. 2002, 8(5):901-7].

*Kinase:*

The term "kinases" refers to enzymes which phosphorylate serine/threonine or tyrosine residues, mainly involved in signal transduction. Examples for kinases include enzymes such as 2-amino-4-hydroxy-6-hydroxymethyldihydropteridine pyrophosphokinase, NAD($^+$) kinase, acetylglutamate kinase, adenosine kinase, adenylate kinase, adenylsulfate kinase, arginine kinase, aspartate kinase, choline kinase, creatine kinase, cytidylate kinase, deoxyadenosine kinase, deoxycytidine kinase, deoxyguanosine kinase, dephospho-CoA kinase, diacylglycerol kinase, dolichol kinase, ethanolamine kinase, galactokinase, glucokinase, glutamate 5-kinase, glycerol kinase, glycerone kinase, guanylate kinase, hexokinase, homoserine kinase, hydroxyethylthiazole kinase, inositol/phosphatidylinositol kinase, ketohexokinase, mevalonate kinase, nucleoside-diphosphate kinase, pantothenate kinase, phosphoenolpyruvate carboxykinase, phosphoglycerate kinase, phosphomevalonate kinase, protein kinase, pyruvate dehydrogenase (lipoamide) kinase, pyruvate kinase, ribokinase, ribose-phosphate pyrophosphokinase, selenide, water dikinase, shikimate kinase, thiamine pyrophosphokinase, thymidine kinase, thymidylate kinase, uridine kinase, xylulokinase, 1D-myo-inositol-trisphosphate 3-kinase, phosphofructokinase, pyridoxal kinase, sphinganine kinase, riboflavin kinase, 2-dehydro-3-deoxygalactonokinase, 2-dehydro-3-deoxygluconokinase, 4-diphosphocytidyl-2C-methyl-D-erythritol kinase, GTP pyrophosphokinase, L-fuculokinase, L-ribulokinase, L-xylulokinase, isocitrate dehydrogenase (NADP$^+$) kinase, acetate kinase, allose kinase, carbamate kinase, cobinamide kinase, diphosphate-purine nucleoside kinase, fructokinase, glycerate kinase, hydroxymethylpyrimidine kinase, hygromycin-B kinase, inosine kinase, kanamycin kinase, phosphomethylpyrimidine kinase, phosphoribulokinase, polyphosphate kinase, propionate kinase, pyruvate,water dikinase, rhamnulokinase, tagatose-6-phosphate kinase, tetraacyldisaccharide 4'-kinase, thiamine-phosphate kinase, undecaprenol kinase, uridylate kinase, N-acylmannosamine kinase, D-erythro-sphingosine kinase.

Pharmaceutical compositions including such proteins or protein encoding sequences, antibodies directed against such proteins or polynucleotides capable of altering expression of such proteins, may be used to treat diseases which may be ameliorated by a modulating kinase activity. Antibodies and polynucleotides such as PCR primers and molecular probes designed to identify such proteins or protein encoding sequences may be used for diagnosis of such diseases.

Examples of such diseases include, but are not limited to, acute lymphoblastic leukemia associated with spleen tyrosine kinase deficiency [Goodman P.A., et al., (2001) Oncogene, 20(30):3969-78], ataxia telangiectasia associated with ATM kinase deficiency [Boultwood J., (2001) J. Clin. Pathol., 54(7):512-6], congenital haemolytic anaemia associated with erythrocyte pyruvate kinase deficiency [Zanella A., et al., (2001) Br. J. Haematol., 113(1):43-8], mevalonic aciduria caused by mevalonate kinase deficiency [Houten S. M., et al., (2001) Eur. J. Hum. Genet., 9(4):253-9], and acute myelogenous leukemia associated with over-expressed death-associated protein kinase [Guzman M. L., et al., (2001) Blood, 97(7):2177-9].

### Nucleotide binding:

The term "nucleotide binding" refers to ligand binding or carrier proteins, involved in physical interaction with a nucleotide, preferably, any compound consisting of a nucleoside that is esterified with [ortho]phosphate or an oligophosphate at any hydroxyl group on the glycose moiety, such as purine nucleotide binding proteins.

Pharmaceutical compositions including such proteins or protein encoding sequences, antibodies directed against such proteins or polynucleotides capable of altering expression of such proteins, may be used to treat diseases that are associated with abnormal nucleotide binding. Antibodies and polynucleotides such as PCR primers and molecular probes designed to identify such proteins or protein encoding sequences may be used for diagnosis of such diseases.

Examples of such diseases include, but are not limited to Gout (a syndrome characterized by high urate level in the blood). Since urate is a breakdown metabolite of purines, reducing purines serum levels could have a therapeutic effect in Gout disease.

### Tubulin binding:

The term "tubulin binding" refers to binding proteins that bind tubulin such as microtubule binding proteins.

Pharmaceutical compositions including such proteins or protein encoding sequences, antibodies directed against such proteins or polynucleotides capable of altering expression of such proteins, may be used to treat diseases which are associated with abnormal tubulin activity or structure. Binding the products of the genes of this family, or antibodies reactive therewith, can modulate a plurality of tubulin activities as well as change microtubulin structure. Antibodies and polynucleotides such as PCR primers and molecular probes designed to identify such proteins or protein encoding sequences may be used for diagnosis of such diseases.

Examples of such diseases include, but are not limited to, Alzheimer's disease associated with t-complex polypeptide 1 deficiency [Schuller E., et al., (2001) Life Sci., 69(3):263-70], neurodegeneration associated with *apo*E deficiency [Masliah E., et al., (1995) Exp. Neurol., 136(2):107-22], progressive axonopathy associated with disfluctional neurofilaments [Griffiths I. R., et al., (1989) Neuropathol. Appl. Neurobiol., 15(1):63-74], familial frontotemporal dementia associated with tau deficiency [astor P., et al., (2001) Ann. Neurol., 49(2):263-7], and colon cancer suppressed by APC [White R. L., (1997) Pathol. Biol. (Paris), 45(3):240-4]. En example for a drug whose target is tubulin is the anticancer drug - Taxol. Drugs having similar mechanism of action (interfering with tubulin polymerization) may be developed based on tubulin binding proteins.

### Receptor signaling proteins:

The phrase "receptor signaling proteins" refers to receptor proteins involved in signal transduction such as receptor signaling protein serine/threonine kinase, receptor signaling protein tyrosine kinase, receptor signaling protein tyrosine phosphatase, aryl hydrocarbon receptor nuclear translocator, hematopoeitin/interferon-class (D200-domain) cytokine receptor signal transducer, transmembrane receptor protein tyrosine kinase signaling protein, transmembrane receptor protein serine/threonine kinase signaling protein, receptor signaling protein serine/threonine kinase signaling protein, receptor signaling protein serine/threonine phosphatase signaling protein, small GTPase regulatory/interacting protein, receptor signaling protein tyrosine kinase signaling protein, and receptor signaling protein serine/threonine phosphatase.

Pharmaceutical compositions including such proteins or protein encoding sequences, antibodies directed against such proteins or polynucleotides capable of altering expression of such proteins, may be used to treat diseases in which the signal-transduction is abnormal, either as a cause, or as a result of the disease. Antibodies and polynucleotides such as PCR primers and molecular probes designed to identify such proteins or protein encoding sequences may be used for diagnosis of such diseases.

Examples of such diseases include, but are not limited to, complete hypogonadotropic hypogonadism associated with GnRH receptor deficiency [Kottler M. L., et a., (2000) J. Clin. Endocrinol. Metab., 85(9):3002-8], severe combined immunodeficiency disease associated with IL-7 receptor deficiency [Puel A. and Leonard W. J., (2000) Curr. Opin. Immunol., 12(4):468-7], schizophrenia associated N-methyl-D-aspartate receptor deficiency [Mohn A.R, et al., (1999) Cell, 98(4):427-36], Yesinia-associated arthritis associated with tumor necrosis factor receptor p55 deficiency [Zhao Y. X., et al., (1999) Arthritis Rheum., 42(8):1662-72], and Dwarfism of Sindh caused by growth hormone-releasing hormone receptor deficiency [aheshwari H. G., et al., (1998) J. Clin. Endocrinol. Metab., 83(11):4065-74].

**Molecular function unknown:**

The phrase "molecular function unknown" refers to various proteins with unknown molecular function, such as cell surface antigens.

Pharmaceutical compositions including such proteins or protein encoding sequences, antibodies directed against such proteins or polynucleotides capable of altering expression of such proteins, may be used to treat diseases in which regulation of the recognition, or participation or bind of cell surface antigens to other moieties may have therapeutic effect Antibodies and polynucleotides such as PCR primers and molecular probes designed to identify such proteins or protein encoding sequences may be used for diagnosis of such diseases.

Examples of such diseases include, but are not limited to, autoimmune diseases, various infectious diseases, cancer diseases which involve non cell surface antigens recognition and activity.

**Enzyme activators:**

The term "enzyme activators" refers to enzyme regulators such as activators of: kinases, phosphatases, sphingolipids, chaperones, guanylate cyclase, tryptophan hydroxylase, proteases, phospholipases, caspases, proprotein convertase 2 activator, cyclin-dependent protein kinase 5 activator, superoxide-generating NADPH oxidase activator, sphingomyelin phosphodiesterase activator, monophenol monooxygenase activator, proteasome activator, and GTPase activator.

Pharmaceutical compositions including such proteins or protein encoding sequences, antibodies directed against such proteins or polynucleotides capable of altering expression of such proteins, may be used to treat diseases in which beneficial effect may be achieved by modulating the activity of activators of proteins and enzymes. Antibodies and polynucleotides such as PCR primers and molecular probes designed to identify such proteins or protein encoding sequences maybe used for diagnosis of such diseases.

Examples of such diseases include, but are not limited to all complement related diseases, as most complement proteins activate by cleavage other complement proteins.

**Transferases, transferring one-carbon groups:**

The phrase "transferases, transferring one-carbon groups" refers enzymes that catalyze the transfer of a one-carbon chemical group from one molecule to another such as methyltransferase, amidinotransferase, hydroxymethyl-, formyl- and related transferase, carboxyl- and carbamoyltransferase.

Pharmaceutical compositions including such proteins or protein encoding sequences, antibodies directed against such proteins or polynucleotides capable of altering expression of such proteins, may be used to treat diseases in which the transfer of a one-carbon chemical group from one molecule to another is abnormal so that a beneficial effect may be achieved by modulation of such reaction. Antibodies and polynucleotides such as PCR primers and molecular probes designed to identify such proteins or protein encoding sequences may be used for diagnosis of such diseases.

**Transferases:**

The term "transferases" refers to enzymes that catalyze the transfer of a chemical group, preferably, a phosphate or amine from one molecule to another. It includes enzymes such as transferases, transferring one-carbon groups, aldehyde or ketonic groups, acyl groups, glycosyl groups, alkyl or aryl (other than methyl) groups, nitrogenous, phosphorus-containing groups, sulfur-containing groups, lipoyltransferase, deoxycytidyl transferases.

Pharmaceutical compositions including such proteins or protein encoding sequences, antibodies directed against such proteins or polynucleotides capable of altering expression of such proteins, may be used to treat diseases in which the transfer of a chemical group from one molecule to another is abnormal. Antibodies and polynucleotides such as PCR primers and molecular probes designed to identify such proteins or protein encoding sequences may be used for diagnosis of such diseases.

Examples of such diseases include, but are not limited to cancerous diseases such as prostate cancer [Urology. 2003, 62(5 Suppl 1):55-62] or lung cancer [Invest. New Drugs. 2003, 21(4):435-43; JAMA. 2003, 22;290(16):2149-58], psychiatric disorders [Am. J. Med. Genet. 2003, 15;123B(1):64-9], colorectal disease such as Crohn's disease [Dis.

Colon Rectum. 2003, 46(11):1498-507] or celiac diseases [N Engl. J. Med. 2003, 349(17):1673-4; author reply 1673-4], neurological diseases such as Prkinson's disease [J. Chem Neuroanat. 2003, 26(2):143-51], Alzheimer disease [Hum. Mol. Genet. 2003 21] or Charcot-Marie-Tooth Disease [Mol. Biol. Evol. 2003 31].

*Chaperones:*

The term "chaperones" refers to functional classes of unrelated families of proteins that assist the correct non-covalent assembly of other polypeptide-containing structures *in vivo,* but are not components of these assembled structures when they a performing their normal biological function. The group of chaperones include proteins such as ribosomal chaperone, peptidylprolyl isomerase, lectin-binding chaperone, nucleosome assembly chaperone, chaperonin ATPase, cochaperone, heat shock protein, HSP70/HSP90 organizing protein, fimbrial chaperone, metallochaperone, tubulin folding, and HSC70-interacting protein.

Pharmaceutical compositions including such proteins or protein encoding sequences, antibodies directed against such proteins or polynucleotides capable of altering expression of such proteins, may be used to treat diseases which are associated with abnormal protein activity, structure, degradation or accumulation of proteins. Antibodies and polynucleotides such as PCR primers and molecular probes designed to identify such proteins or protein encoding sequences maybe used for diagnosis of such diseases.

Examples of such diseases include, but are not limited to neurological syndromes [J. Neuropathol. Exp. Neurol. 2003, 62(7):751-64; Antioxid Redox Signal. 2003, 5(3):337-48; J. Neurochem. 2003, 86(2):394-404], neurological diseases such as Parkinson's disease [Hum. Genet. 2003, 6; Neurol Sci. 2003, 24(3):159-60; J. Neurol. 2003, 250 Suppl. 3:III25-III29] ataxia [J. Hum. Genet 2003;48(8):415-9] or Alzheimer diseases [J. Mol. Neurosci. 2003, 20(3):283-6; J. Alzheimers Dis. 2003, 5(3):171-7], cancerous diseases [Semin. Oncol. 2003, 30(5):709-16], prostate cancer [Semin. Oncol. 2003, 30(5):709-16] metabolic diseases [J Neurochem. 2003, 87(1):248-56], infectious diseases, such as prion infection [EMBO J. 2003,22(20);5435-5445]. Chaperones may be also used for manipulating therapeutic proteins binding to their receptors therefore, improving their therapeutic effect.

*Cell adhesion molecule:*

The phrase "cell adhesion molecule" refers to proteins that serve as adhesion molecules between adjoining cells such as membrane-associated protein with guanylate kinase activity, cell adhesion receptor, neuroligin, calcium-dependent cell adhesion molecule, selectin, calcium-independent cell adhesion molecule, and extracellular matrix protein.

Pharmaceutical compositions including such proteins or protein encoding sequences, antibodies directed against such proteins or polynucleotides capable of altering expression of such proteins, may be used to treat diseases in which adhesion between adjoining cells is involved, typically conditions in which the adhesion is abnormal. Antibodies and polynucleotides such as PCR primers and molecular probes designed to identify such proteins or protein encoding sequences may be used for diagnosis of such diseases.

Examples of such diseases include, but are not limited to cancer in which abnormal adhesion may cause and enhance the process of metastasis and abnormal growth and development of various tissues in which modulation adhesion among adjoining cells can improve the condition. Leucocyte-endothlial interactions characterized by adhesion molecules involved in interactions between cells lead to a tissue injury and ischemia reperfusion disorders in which activated signals generated during ischemia may trigger an exuberant inflammatory response during reperfusion, provoking greater tissue damage than initial ischemic insult [Crit Care Med. 2002, 30(5 Suppl):S214-9]. The blockade of leucocyte-endothelial adhesive interactions has the potential to reduce vascular and tissue injury. This blockade may be achieved using a soluble variant of the adhesion molecule.

States of septic shock and ARDS involve large recruitment of neutrophil cells to the damaged tissues. Neutrophil cells bind to the endothelial cells in the target tissues through adhesion molecules. Neutrophils possess multiple effector mechanisms that can produce endothelial and lung tissue injury, and interfere with pulmonary gas transfer by disruption of surfactant activity [Eur. J. Surg. 2002, 168(4):204-14]. In such cases, the use of soluble variant of the adhesion molecule may decrease the adhesion of neutrophils to the damaged tissues.

Examples of such diseases include, but are not limited to, Wiskott-Aldrich syndrome associated with WAS deficiency [Westerberg L., et al., (2001) Blood, 98(4):1086-94], asthma associated with intercellular adhesion molecule-1 deficiency [Tang M. L. and Fiscus L. C., (2001) Pulm. Pharmacol. Ther., 14(3):203-10], intra-atrial thrombogenesis associated with increased von Willebrand factor activity [Fukuchi M., et al., (2001) J. Am. Coll. Cardiol., 37(5):1436-42], junctional epidermolysis bullosa associated with laminin 5-$\beta$-3 deficiency [Robbins P. B., et al., (2001) Proc. Natl. Acad. Sci., 98(9):5193-8], and hydrocephalus caused by neural adhesion molecule L1 deficiency [Rolf B., et aL, (2001) Brain Res., 891(1-2):247-52].

*Motor proteins:*

The term "motor proteins" refers to proteins that generate force or energy by the hydrolysis of ATP and that function in the production of intracellular movement or transportation. Examples of such proteins include microfilament motor, axonemal motor, microtubule motor, and kinetochore motor (dynein, kinesin, or myosin).

Pharmaceutical compositions including such proteins or protein encoding sequences, antibodies directed against such proteins or polynucleotides capable of altering expression of such proteins, may be used to treat diseases in which force or energy generation is impaired. Antibodies and polynucleotides such as PCR primers and molecular probes designed to identify such proteins or protein encoding sequences may be used for diagnosis of such diseases.

Examples of such diseases include, but are not limited to, malignant diseases where microtubules are drug targets for a family of anticancer drugs such as myodystrophies and myopathies [Trends Cell Biol. 2002,12(12):585-91], neurological disorders [Neuron. 2003, 25;40(1):25-40; Trends Biochem. Sci. 2003, 28(10):558-65; Med. Genet. 2003, 40(9):671-5], and hearing impairment [Trends Biochem. Sci. 2003, 28(10):558-65].

*Defense/immunity proteins:*

The term "defense/immunity proteins" refers to proteins that are involved in the immune and complement systems such as acute-phase response proteins, antimicrobial peptides, antiviral response proteins, blood coagulation factors, complement components, immunoglobulins, major histocompatibility complex antigens and opsonins.

Pharmaceutical compositions including such proteins or protein encoding sequences, antibodies directed against such proteins or polynucleotides capable of altering expression of such proteins, may be used to treat diseases involving the immunological system including inflammation, autoimmune diseases, infectious diseases, as well as cancerous processes or diseases which are manifested by abnormal coagulation processes, which may include abnormal bleeding or excessive coagulation. Antibodies and polynucleotides such as PCR primers and molecular probes designed to identify such proteins or protein encoding sequences maybe used for diagnosis of such diseases.

Examples of such diseases include, but are not limited to, late (C5-9) complement component deficiency associated with opsonin receptor allotypes [Fijen C. A., et al., (2000) Clin. Exp. Immunol., 120(2):338-45], combined immunodeficiency associated with defective expression of MHC class II genes [Griscelli C., et al., (1989) Immunodefic. Rev. 1(2):135-53], loss of antiviral activity of CD4 T cells caused by neutralization of endogenous TNF$\alpha$ [Pavic I., et al., (1993) J. Gen. Virol., 74 (Pt 10):2215-23], autoimmune diseases associated with natural resistance-associated macrophage protein deficiency [Evans C. A., et al., (2001) Neurogenetics, 3(2):69-78], Epstein-Barr virus-associated lymphoproliferative disease inhibited by combined GM-CSF and IL-2 therapy [Baiocchi R. A., et al., (2001) J. Clin. Invest., 108(6):887-94], multiple sclerosis in which recombinant proteins from the interferons family are the treatment of choice and sepsis in which activated protein C is a therapeutic protein itself.

*Intracellular transporters:*

The term "intracellular transporters" refers to proteins that mediate the transport of molecules and macromolecules inside the cell, such as intracellular nucleoside transporter, vacuolar assembly proteins, vesicle transporters, vesicle fusion proteins, type II protein secretors.

Pharmaceutical compositions including such proteins or protein encoding sequences, antibodies directed against such proteins or polynucleotides capable of altering expression of such proteins, may be used to treat diseases in which the transport of molecules and macromolecules is abnormal leading to various pathologies. Antibodies and polynucleotides such as PCR primers and molecular probes designed to identify such proteins or protein encoding sequences may be used for diagnosis of such diseases.

*Transporters:*

The term "transporters" refers to proteins that mediate the transport of molecules and macromolecules, such as channels, exchangers, and pumps. Transporters include proteins such as: amine/polyamine transporter, lipid transporter, neurotransmitter transporter, organic acid transporter, oxygen transporter, water transporter, carriers, intracellular transports, protein transporters, ion transporters, carbohydrate transporter, polyol transporter, amino acid transporters, vitamin/cofactor transporters, siderophore transporter, drug transporter, channel/pore class transporter, group translocator, auxiliary transport proteins, permeases, murein transporter, organic alcohol transporter, nucleobase, nucleoside, and nucleotide and nucleic acid transporters.

Pharmaceutical compositions including such proteins or protein encoding sequences, antibodies directed against such proteins or polynucleotides capable of altering expression of such proteins, may be used to treat diseases in which the transport of molecules and macromolecules such as neurotransmitters, hormones, sugar etc. is impaired leading to various pathologies. Antibodies and polynucleotides such as PCR primers and molecular probes designed to identify such proteins or protein encoding sequences may be used for diagnosis of such diseases.

Examples of such diseases include, but are not limited to, glycogen storage disease caused by glucose-6-phosphate transporter deficiency [Hiraiwa H., and Chou J. Y. (2001) DNA Cell Biol., 20(8):447-53], tangier disease associated with ATP-binding cassette transporter-1 deficiency [McNeish J., et al., (2000) Proc. Natl. Acad. Sci., 97(8):4245-50], systemic primary carnitine deficiency associated with organic cation transporter deficiency [Tang N. L., et al., (1999) Hum. Mol. Genet., 8(4):655-60], Wilson disease associated with copper-transporting ATPases deficiency [Payne A. S., et al., (1998) Proc. Natl. Acad. Sci. 95(18):10854-9], and atelosteogenesis associated with diastrophic dysplasia sulphate transporter deficiency [Newbury-Ecob R., (1998) J. Med. Genet., 35(1):49-53], Central Nervous system diseases treated by inhibiting neurotransmitter transporter (e.g. Depression, treated with serotonin transporters inhibitors - Prozac), and Cystic fibrosis mediated by the chloride channel CFTR. Other transporter related diseases are cancer [Oncogene. 2003, 22(38):6005-12] and especially cancer resistant to treatment [Oncologist. 2003, 8(5):411-24; J. Med. Invest. 2003,50(3-4):126-35], infectious diseases, especially fungal infections [Annu. Rev. Phytopathol. 2003, 41:641-67], neurological diseases, such as Parkinson [FASEB J. 2003, Sep 4 [Epub ahead of print]], diabetes where ATP-sensitive potassium channel in beta cells is the target for insulin secretagogues, hypertension where calcium channels are the target for calcium blockers, and cardiovascular diseases, including hypercholesterolemia [Am. J. Cardiol. 2003, 92(4B):10K-16K].

There are about 30 membrane transporter genes linked to a known genetic clinical syndrome. Secreted versions of splice variants of transporters may be therapeutic as the case with soluble receptors. These transporters may have the capability to bind the compound in the serum they would normally bind on the membrane. For example, a secreted form ATP7B, a transporter involved in Wilson's disease, is expected to bind plasma Copper, therefore have a desired therapeutic effect in Wilson's disease.

*Lyases:*

The term "lyases" refers to enzymes that catalyze the formation of double bonds by removing chemical groups from a substrate without hydrolysis or catalyze the addition of chemical groups to double bonds. It includes enzymes such as carbon-carbon lyase, carbon-oxygen lyase, carbon-nitrogen lyase, carbon-sulfur lyase, carbon-halide lyase, and phosphorus-oxygen lyase.

Pharmaceutical compositions including such proteins or protein encoding sequences, antibodies directed against such proteins or polynucleotides capable of altering expression of such proteins, may be used to treat diseases in which the double bonds formation catalyzed by these enzymes is impaired. Antibodies and polynucleotides such as PCR primers and molecular probes designed to identify such proteins or protein encoding sequences may be used for diagnosis of such diseases.

Examples of such diseases include, but are not limited to, autoimmune diseases [JAMA. 2003, 290(13):1721-8; JAMA. 2003, 290(13):1713-20], diabetes [Diabetes. 2003, 52(9):2274-8], neurological disorders such as epilepsy [J. Neurosci. 2003, 23(24):8471-9], Parkinson [J. Neurosci. 2003, 23(23):8302-9; Lancet. 2003, 362(9385):712] or Creutzfeldt-Jakob disease [Clin. Neurophysiol. 2003, 114(9):1724-8], and cancerous diseases [J. Pathol. 2003, 201(1):37-45; J. Pathol. 2003, 201(1):37-45; Cancer Res. 2003, 63(16):4952-9; Eur. J. Cancer. 2003, 39(13):1899-903].

*Actin binding proteins:*

The phrase "actin binding proteins" refers to proteins binding actin as actin crosslinking, actin bundling, F-actin capping, actin monomer binding, actin lateral binding, actin depolymerizing, actin monomer sequestering, actin filament severing, actin modulating, membrane associated actin binding, actin thin filament length regulation, and actin polymerizing proteins.

Pharmaceutical compositions including such proteins or protein encoding sequences, antibodies directed against such proteins or polynucleotides capable of altering expression of such proteins, maybe used to treat diseases in which actin binding is impaired. Antibodies and polynucleotides such as PCR primers and molecular probes designed to identify such proteins or protein encoding sequences may be used for diagnosis of such diseases.

Examples of such diseases include, but are not limited to, neuromuscular diseases such as muscular dystrophy [Neurology. 2003, 61(3):404-6], Cancerous diseases [Urology. 2003, 61(4):845-50; J. Cutan. Pathol. 2002, 29(7):430; Cancer. 2002, 94(6):1777-86; Clin. Cancer Res. 2001, 7(8):2415-24; Breast Cancer Res. Treat 2001, 65(1):11-21], renal diseases such as glomerulonephritis [J. Am. Soc. Nephrol. 2002, 13(2):322-31; Eur. J. Immunol. 2001, 31(4):1221-7], and gastrointestinal diseases such as Crohn's disease [J. Cell Physiol. 2000, 182(2):303-9].

*Protein binding proteins:*

The phrase "protein binding proteins" refers to proteins involved in diverse biological functions through binding other proteins. Examples of such biological function include intermediate filament binding, LIM-domain binding, LLR-domain binding, clathrin binding, ARF binding, vinculin binding, KU70 binding, troponin C binding PDZ-domain binding, SH3-domain binding, fibroblast growth factor binding, membrane-associated protein with guanylate kinase activity interacting, Wnt-protein binding , DEAD/H-box RNA helicase binding, $\beta$-amyloid binding, myosin binding, TATA-binding protein binding DNA topoisomerase I binding, polypeptide hormone binding, RHO binding, FH-domain binding, syntaxin-1 binding, HSC70-interacting, transcription factor binding, metarhodopsin binding, tubulin binding, JUN kinase binding, RAN protein binding, protein signal sequence binding, importin $\alpha$ export receptor, poly-glutamine tract binding, protein carrier, $\beta$-catenin binding, protein C-terminus binding, lipoprotein binding, cytoskeletal protein binding protein, nuclear localization sequence binding, protein phosphatase 1 binding, adenylate cyclase binding, eukaryotic initiation factor 4E binding, calmodulin binding, collagen binding, insulin-like growth factor binding, lamin binding, profilin binding, tropomyosin binding, actin binding, peroxisome targeting sequence binding, SNARE binding, and cyclin binding.

Pharmaceutical compositions including such proteins or protein encoding sequences, antibodies directed against such proteins or polynucleotides capable of altering expression of such proteins, may be used to treat diseases which are associated with impaired protein binding. Antibodies and polynucleotides such as PCR primers and molecular probes designed to identify such proteins or protein encoding sequences may be used for diagnosis of such diseases.

Examples of such diseases include, but are not limited to, neurological and psychiatric diseases [J. Neurosci. 2003, 23(25):8788-99; Neurobiol. Dis. 2003, 14(1):146-56; J. Neurosci. 2003, 23(17):6956-64; Am. J. PathoL 2003, 163(2):609-19], and cancerous diseases [Cancer Res. 2003, 63(15):4299-304; Semin. Thromb. Hemost 2003, 29(3):247-58; Proc. Natl. Acad. Sci. U S A. 2003, 100(16):9506-11].

*Ligand binding or carrier proteins:*

The phrase "ligand binding or carrier proteins" refers to proteins involved in diverse biological functions such as: pyridoxal phosphate binding, carbohydrate binding, magnesium binding, amino acid binding, cyclosporin A binding, nickel binding, chlorophyll binding, biotin binding, penicillin binding, selenium binding, tocopherol binding, lipid binding, drug binding, oxygen transporter, electron transporter, steroid binding, juvenile hormone binding, retinoid binding, heavy metal binding, calcium binding, protein binding, glycosaminoglycan binding, folate binding, odorant binding, lipopolysaccharide binding and nucleotide binding.

Pharmaceutical compositions including such proteins or protein encoding sequences, antibodies directed against such proteins or polynucleotides capable of altering expression of such proteins, may be used to treat diseases which are associated with impaired function of these proteins. Antibodies and polynucleotides such as PCR primers and molecular probes designed to identify such proteins or protein encoding sequences may be used for diagnosis of such diseases.

Examples of such diseases include, but are not limited to, neurological disorders [J. Med. Genet. 2003, 40(10):733-40; J. Neuropathol. Exp. Neurol. 2003, 62(9):968-75; J. Neurochem. 2003, 87(2):427-36], autoimmune diseases (N. Engl. J. Med. 2003, 349(16):1526-33; JAMA. 2003, 290(13):1721-8]; gastroesophageal reflux disease [Dig. Dis. Sci. 2003, 48(9):1832-8], cardiovascular diseases [J. Vasc. Surg. 2003, 38(4):827-32], cancerous diseases [Oncogene. 2003,22(43):6699-703; Br. J, Haematol. 2003, 123(2):288-96], respiratory diseases [Circulation. 2003, 108(15):1839-44], and ophtalmic diseases [Ophthalmology. 2003,110(10):2040-4; Am. J. Ophthalmol. 2003,136(4):729-32].

*ATPases:*

The term "ATPases" refers to enzymes that catalyze the hydrolysis of ATP to ADP, releasing energy that is used in the cell. This group include enzymes such as plasma membrane cation-transporting ATPase, ATP-binding cassette (ABC) transporter, magnesium-ATPase, hydrogen-/sodium-translocating ATPase or ATPase translocating any other elements, arsenite-transporting ATPase, protein-transporting ATPase, DNA translocase, P-type ATPase, and hydrolase, acting on acid anhydrides involved in cellular and subcellular movement.

Pharmaceutical compositions including such proteins or protein encoding sequences, antibodies directed against such proteins or polynucleotides capable of altering expression of such proteins, may be used to treat diseases which are associated with impaired conversion of the hydrolysis of ATP to ADP or resulting energy use. Antibodies and polynucleotides such as PCR primers and molecular probes designed to identify such proteins or protein encoding sequences may be used for diagnosis of such diseases.

Examples of such diseases include, but are not limited to, infectious diseases such as helicobacter pylori ulcers [BMC Gastroenterology 2003, 3:31 (published 6 November 2003)], Neurological, muscular and psychiatric diseases [Int. J. Neurosci. 2003, 13(12):1705-1717; Int. J. Neurosci. 2003, 113(11):1579-1591; Ann. Neurol. 2003, 54(4):494-509], Amyotrophic Lateral Sclerosis [Other Motor Neuron Disord. 2003 4(2):96-9], cardiovascular diseases [J. Nippon. Med. Sch. 2003, 70(5):384-92; Endocrinology. 2003, 144(10):4478-83], metabolic diseases [Mol. Pathol. 2003, 56(5):302-4; Neurosci. Lett. 2003, 350(2):105-8], and peptic ulcer disease treated with inhibitors of the gastric $H^+$-$K^+$ ATPase (e.g. Omeprazole) responsible for acid secretion in the gastric mucosa.

### Carboxylic ester hydrolases:

The phrase carboxylic ester hydrolases" refers to hydrolytic enzymes acting on carboxylic ester bonds such as N-acetylglucosaminylphosphatidylinositol deacetylase, 2-acetyl-1-alkylglycerophosphocholine esterase, aminoacyl-tRNA hydrolase, arylesterase, carboxylesterase, cholinesterase, gluconolactonase, sterol esterase, acetylesterase, carboxymethylenebutenolidase, protein-glutamate methylesterase, lipase, and 6-phosphogluconolactonase.

Pharmaceutical compositions including such proteins or protein encoding sequences, antibodies directed against such proteins or polynucleotides capable of altering expression of such proteins, may be used to treat diseases in which the hydrolytic cleavage of a covalent bond with accompanying addition of water (-H being added to one product of the cleavage and -OH to the other) is abnormal so that a beneficial effect may be achieved by modulation of such reaction. Antibodies and polynucleotides such as PCR primers and molecular probes designed to identify such proteins or protein encoding sequences may be used for diagnosis of such diseases.

Examples of such diseases include, but are not limited to, autoimmune neuromuscular disease Myasthenia Gravis, treated with cholinesterase inhibitors.

### Hydrolase, acting on ester bonds:

The phrase "hydrolase, acting on ester bonds" refers to hydrolytic enzymes acting on ester bonds such as nucleases, sulfuric ester hydrolase, carboxylic ester hydrolases, thiolester hydrolase, phosphoric monoester hydrolase, phosphoric diester hydrolase, triphosphoric monoester hydrolase, diphosphoric monoester hydrolase, and phosphoric triester hydrolase.

Pharmaceutical compositions including such proteins or protein encoding sequences, antibodies directed against such proteins or polynucleotides capable of altering expression of such proteins, may be used to treat diseases in which the hydrolytic cleavage of a covalent bond with accompanying addition of water (-H being added to one product of the cleavage and -OH to the other), is abnormal. Antibodies and polynucleotides such as PCR primers and molecular probes designed to identify such proteins or protein encoding sequences may be used for diagnosis of such diseases.

### Hydrolases:

The term "hydrolases" refers to hydrolytic enzymes such as GPI-anchor transamidase, peptidases, hydrolases, acting on ester bonds, glycosyl bonds, ether bonds, carbon-nitrogen (but not peptide) bonds, acid anhydrides, acid carbon-carbon bonds, acid halide bonds, acid phosphorus-nitrogen bonds, acid sulfur-nitrogen bonds, acid carbon-phosphorus bonds, acid sulfur-sulfur bonds.

Pharmaceutical compositions including such proteins or protein encoding sequences, antibodies directed against such proteins or polynucleotides capable of altering expression of such proteins, may be used to treat diseases in which the hydrolytic cleavage of a covalent bond with accompanying addition of water (-H being added to one product of the cleavage and -OH to the other) is abnormal. Antibodies and polynucleotides such as PCR primers and molecular probes designed to identify such proteins or protein encoding sequences may be used for diagnosis of such diseases.

Examples of such diseases include, but are not limited to, cancerous diseases [Cancer. 2003, 98(9):1842-8; Cancer. 2003, 98(9):1822-9], neurological diseases such as Parkinson diseases [J. Neurol. 2003, 250 Suppl 3:III15-III24; J. NeuroL 2003, 250 Suppl 3:III2-III10], endocrinological diseases such as pancreatitis [Pancreas. 2003, 27(4):291-6] or childhood genetic diseases [Eur. J. Pediatr. 1997, 156(12):935-8], coagulation diseases [BMJ. 2003, 327(7421):974-71, cardiovascular diseases [Ann. Intern. Med. 2003, Oct 139(8):670-82], autoimmunity diseases [J. Med. Genet. 2003, 40(10):761-6], and metabolic diseases [Am. J. Hum. Genet. 2001, 69(5):1002-12].

### Enzymes:

The term "enzymes' refers to naturally occurring or synthetic macromolecular substance composed mostly of protein, that catalyzes, to various degree of specificity, at least one (bio)chemical reactions at relatively low temperatures. The action of RNA that has catalytic activity (ribozyme) is often also regarded as enzymatic. Nevertheless, enzymes are mainly proteinaceous and are often easily inactivated by heating or by protein-denaturing agents. The substances upon which they act are known as substrates, for which the enzyme possesses a specific binding or active site.

The group of enzymes include various proteins possessing enzymatic activities such as mannosylphosphate transferase, para-hydroxybenzoate:polyprenyltransferase, rieske iron-sulfur protein, imidazoleglycerol-phosphate synthase, sphingosine hydroxylase, tRNA 2'-phosphotransferase, sterol C-24(28) reductase, C-8 sterol isomerase, C-22 sterol desaturase, C-14 sterol reductase, C-3 sterol dehydrogenase (C-4 sterol decarboxylase), 3-keto sterol reductase, C-4 methyl sterol oxidase, dihydronicotinamide riboside quinone reductase, glutamate phosphate reductase, DNA repair enzyme, telomerase, $\alpha$-ketoacid dehydrogenase, $\beta$-alanyl-dopamine synthase, RNA editase, aldo-keto reductase, alkylbase DNA glycosidase, glycogen debranching enzyme, dihydropterin deaminase, dihydropterin oxidase, dimethylnitrosamine demethylase, ecdysteroid UDP-glucosyl/UDP glucuronosyl transferase, glycine cleavage system, helicase, histone deacetylase, mevaldate reductase, monooxygenase, poly(ADP-ribose) glycohydrolase, pyruvate dehydrogenase, serine esterase, sterol carrier protein X-related thiolase, transposase, tyramine-$\beta$ hydroxylase, para-aminobenzoic acid (PABA) synthase, glu-tRNA(gln) amidotransferase, molybdopterin cofactor sulfurase, lanosterol 14-$\alpha$-demethylase, aromatase, 4-hydroxybenzoate octaprenyltransferase, 7,8-dihydro-8-oxoguanine-triphosphatase, CDP-alcohol phosphotransferase, 2,5-diamino-6-(ribosylamino)-4(3H)-pyrimidonone 5'-phosphate deaminase, diphosphoinositol polyphosphate phosphohydrolase, $\gamma$-glutamyl carboxylase, small protein conjugating enzyme, small protein activating enzyme, 1-deoxyxylulose-5-phosphate synthase, 2'-phosphotransferase, 2-octoprenyl-3-methyl-6-methoxy-1,4-benzoquinone hydroxylase, 2C-methyl-D-erythritol 2,4-cyclodiphosphate synthase, 3,4 dihydroxy-2-butanone-4-phosphate synthase, 4-amino-4-deoxychorismate lyase, 4-diphosphocytidyl-2C-methyl-D-erythritol synthase, ADP-L-glycero-D-manno-heptose synthase, D-erythro-7,8-dihydroneopterin triphosphate 2'-epimerase, N-ethylmaleimide reductase, O-antigen ligase, O-antigen polymerase, UDP-2,3-diacylglucosamine hydrolase, arsenate reductase, carnitine racemase, cobalamin [5'-phosphate] synthase, cobinamide phosphate guanylyltransferase, enterobactin synthetase, enterochelin esterase, enterochelin synthetase, glycolate oxidase, integrase, lauroyl transferase, peptidoglycan synthetase, phosphopantetheinyltransferase, phosphoglucosamine mutase, phosphoheptose isomerase, quinolinate synthase, siroheme synthase, N-acylmannosamine-6-phosphate 2-epimerase, N-acetyl-anhydromuramoyl-L-alanine amidase, carbon-phosphorous lyase, heme-copper terminal oxidase, disulfide oxidoreductase, phthalate dioxygenase reductase, sphingosine-1-phosphate lyase, molybdopterin oxidoreductase, dehydrogenase, NADPH oxidase, naringenin-chalcone synthase, N-ethylammeline chlorohydrolase, polyketide synthase, aldolase, kinase, phosphatase, CoA-ligase, oxidoreductase, transferase, hydrolase, lyase, isomerase, ligase, ATPase, sulfhydryl oxidase, lipoate-protein ligase, $\delta$-1-pyrroline-5-carboxyate synthetase, lipoic acid synthase, and tRNA dihydrouridine synthase.

Pharmaceutical compositions including such proteins or protein encoding sequences, antibodies directed against such proteins or polynucleotides capable of altering expression of such proteins, may be used to treat diseases which can be ameliorated by modulating the activity of various enzymes which are involved both in enzymatic processes inside cells as well as in cell signaling. Antibodies and polynucleotides such as PCR primers and molecular probes designed to identify such proteins or protein encoding sequences may be used for diagnosis of such diseases.

Examples of such diseases include, but are not limited to diabetes where alpha-glucosidase is the target for drugs which delay glucose absorption, Osteoporosis where farnsesyl diphosphate synthase is the target for bisphosphonates, thyroid autoimmune disease associated with thyroid peroxidase, MUCOPOLYSACCHARIDOSES associated with defects in lysosomal enzymes, Tay-Sachs Disease associated with defects in b-hexosaminidase and hypertension where Angiotensin Converting Enzyme is the target for the common hypertension drugs - ACE inhibitors.

*Cytoskeletal proteins:*

The term "cytoskeletal proteins" refers to proteins involved in the structure formation of the cytoskeleton.

Pharmaceutical compositions including such proteins or protein encoding sequences, antibodies directed against such proteins or polynucleotides capable of altering expression of such proteins, may be used to treat diseases which are caused or due to abnormalities in cytoskeleton, including cancerous cells, and diseased cells such as cells that do not propagate, grow or function normally. Antibodies and polynucleotides such as PCR primers and molecular probes designed to identify such proteins or protein encoding sequences may be used for diagnosis of such diseases.

Examples of such diseases include, but are not limited to, liver diseases such as cholestatic diseases [Lancet. 2003, 362(9390):1112-9], vascular diseases [J. Cell Biol. 2003, 162(6):1111-22], endocrinological diseases [Cancer Res. 2003, 63(16):4836-41], neuromuscular disorders such as muscular dystrophy [Neuromuscul. Disord. 2003, 13(7-8):579-88], or myopathy [Neuromuscul. Disord. 2003, 13(6):456-67] neurological disorders such as Alzheimer's disease [J. Alzheimers Dis. 2003, 5(3):209-28], cardiac disorders [J. Am. Coll. Cardiol. 2003,42(2):319-27], skin disorders [J. Am. Coll. Cardiol. 2003, 42(2):319-27], and cancer [Proteomics. 2003, 3(6):979-90].

*Structural proteins:*

The term "structural proteins" refers to proteins involved in the structure formation of the cell, such as structural proteins of ribosome, cell wall structural proteins, structural proteins of cytoskeleton, extracellular matrix structural proteins, extracellular matrix glycoproteins, amyloid proteins, plasma proteins, structural proteins of eye lens, structural protein of chorion (sensu Insecta), structural protein of cuticle (sensu Insecta), puparial glue protein (sensu Diptera), structural proteins of bone, yolk proteins, structural proteins of muscle, structural protein of vitelline membrane (sensu Insecta), structural proteins of peritrophic membrane (sensu Insecta), and structural proteins of nuclear pores.

Pharmaceutical compositions including such proteins or protein encoding sequences, antibodies directed against such proteins or polynucleotides capable of altering expression of such proteins, may be used to treat diseases which are caused by abnormalities in cytoskeleton, including cancerous cells, and diseased cells such as cells that do not propagate, grow or function normally. Antibodies and polynucleotides such as PCR primers and molecular probes designed to identify such proteins or protein encoding sequences may be used for diagnosis of such diseases.

Examples of such diseases include, but are not limited to, blood vessels diseases such as aneurysms [Cardiovasc. Res. 2003, 60(1):205-13], joint diseases [Rheum. Dis. Clin. North Am. 2003,29(3):631-45], muscular diseases such as muscular dystrophies [Curr. Opin. Clin. Nutr. Metab. Care. 2003, 6(4):435-9], neuronal diseases such as encephalitis [Neurovirol. 2003, 9(2):274-83], retinitis pigmentosa [Dev. Ophthalmol. 2003, 37:109-25], and infectious diseases [J. Virol. Methods. 2003, 109(1):75-83; FEMS Immunol. Med. Microbiol. 2003, 35(2):125-30; J. Exp. Med. 2003, 197(5):633-42].

*Ligands:*

The term "ligands" refers to proteins that bind to another chemical entity to form a larger complex, involved in various biological processes, such as signal transduction, metabolism, growth and differentiation, etc. This group of proteins includes opioid peptides, baboon receptor ligand, branchless receptor ligand, breathless receptor ligand, ephrin, frizzled receptor ligand, frizzled-2 receptor ligand, heartless receptor ligand, Notch receptor ligand, patched receptor ligand, punt receptor ligand, Ror receptor ligand, saxophone receptor ligand, SE20 receptor ligand, sevenless receptor ligand, smooth receptor ligand, thickveins receptor ligand, Toll receptor ligand, Torso receptor ligand, death receptor ligand, scavenger receptor ligand, neuroligin, integrin ligand, hormones, pheromones, growth factors, and sulfonylurea receptor ligand.

Pharmaceutical compositions including such proteins or protein encoding sequences, antibodies directed against such proteins or polynucleotides capable of altering expression of such proteins, may be used to treat diseases involved in impaired hormone function or diseases which involve abnormal secretion of proteins which may be due to abnormal presence, absence or impaired normal response to normal levels of secreted proteins. Those secreted proteins include hormones, neurotransmitters, and various other proteins secreted by cells to the extracellular environment. Antibodies and polynucleotides such as PCR primers and molecular probes designed to identify such proteins or protein encoding sequences may be used for diagnosis of such diseases.

Examples of such diseases include, but are not limited to, analgesia inhibited by orphanin FQ/nociceptin [Shane R., et al., (2001) Brain Res., 907(1-2):109-16], stroke protected by estrogen [Alkayed N. J., et al., (2001) J. Neurosci., 21(19):7543-50], atherosclerosis associated with growth hormone deficiency [Elhadd T .A., et al., (2001) J. Clin. Endocrinol. Metab., 86(9):4223-32], diabetes inhibited by $\alpha$-galactosylceramide [Hong S., et al., (2001) Nat. Med., 7(9):1052-6], and Huntington's disease [Rao D. S., et al., (2001) Mol. Cell Biol., 21(22):7796-806].

*Signal transducer:*

The term "signal transducers" refers to proteins such as activin inhibitors, receptor-associated proteins, $\alpha$-2 macroglobulin receptors, morphogens, quorum sensing signal generators, quorum sensing response regulators, receptor signaling proteins, ligands, receptors, two-component sensor molecules, and two-component response regulators.

Pharmaceutical compositions including such proteins or protein encoding sequences, antibodies directed against such proteins or polynucleotides capable of altering expression of such proteins, may be used to treat diseases in which the signal-transduction is impaired, either as a cause, or as a result of the disease. Antibodies and polynucleotides such as PCR primers and molecular probes designed to identify such proteins or protein encoding sequences maybe used for diagnosis of such diseases.

Examples of such diseases include, but are not limited to, altered sexual dimorphism associated with signal transducer and activator of transcription 5b [Udy G. B., et al., (1997) Proc. Natl. Acad. Sci. USA, 94(14):7239-44], multiple sclerosis associated with sgp130 deficiency [Padberg F., et al., (1999) J. Neuroimmunol., 99(2):218-23], intestinal inflammation associated with elevated signal transducer and activator of transcription 3 activity [Suzuki A., et al., (2001) J Exp Med, 193(4):471-81], carcinoid tumor inhibited by increased signal transducer and activators of transcription 1 and 2 [Zhou Y., et al., (2001) Oncology, 60(4):330-8], and esophageal cancer associated with loss of EGF-STAT1 pathway [Watanabe G., et al., (2001) Cancer J., 7(2):132-9].

*RNA polymerase II transcription factors:*

The phrase "RNA polymerase II transcription factors" refers to proteins such as specific and non-specific RNA polymerase II transcription factors, enhancer binding, ligand-regulated transcription factor, and general RNA polymerase II transcription factors.

Pharmaceutical compositions including such proteins or protein encoding sequences, antibodies directed against such proteins or polynucleotides capable of altering expression of such proteins, may be used to treat diseases involving impaired function of RNA polymerase II transcription factors. Antibodies and polynucleotides such as PCR primers and molecular probes designed to identify such proteins or protein encoding sequences may be used for diagnosis of such diseases.

Examples of such diseases include, but are not limited to, cardiac diseases [Cell Cycle. 2003, 2(2):99-104], xeroderma pigmentosum [Bioessays. 2001, 23(8):671-3; Biochim. Biophys. Acta. 1997, 1354(3):241-51], muscular atrophy [J. Cell Biol. 2001, 152(1):75-85], neurological diseases such as Alzheimer's disease [Front Biosci. 2000, 5:D244-57], cancerous diseases such as breast cancer [Biol. Chem. 1999, 380(2):117-28], and autoimmune disorders [Clin. Exp. Immunol. 1997,109(3):488-94].

*RNA binding proteins:*

The phrase "RNA binding proteins" refers to RNA binding proteins involved in splicing and translation regulation such as tRNA binding proteins, RNA helicases, double-stranded RNA and single-stranded RNA binding proteins, mRNA binding proteins, snRNA cap binding proteins, 5S RNA and 7S RNA binding proteins, poly-pyrimidine tract binding proteins, snRNA binding proteins, and AU-specific RNA binding proteins.

Pharmaceutical compositions including such proteins or protein encoding sequences, antibodies directed against such proteins or polynucleotides capable of altering expression of such proteins, may be used to treat diseases involving transcription and translation factors such as helicases, isomerases, histones and nucleases, diseases where there is impaired transcription, splicing, post-transcriptional processing, translation or stability of the RNA. Antibodies and polynucleotides such as PCR primers and molecular probes designed to identify such proteins or protein encoding sequences may be used for diagnosis of such diseases.

Examples of such diseases include, but are not limited to, cancerous diseases such as lymphomas [Tumori. 2003, 89(3):278-84], prostate cancer [Prostate. 2003, 57(1):80-92] or lung cancer [J. Pathol. 2003, 200(5):640-6], blood diseases, such as fanconi anemia [Curr. Hematol. Rep. 2003, 2(4):335-40], cardiovascular diseases such as atherosclerosis [J. Thromb. Haemost. 2003, 1(7):1381-90] muscle diseases [Trends Cardiovasc. Med. 2003, 13(5):188-95] and brain and neuronal diseases [Trends Cardiovasc. Med. 2003, 13(5):188-95; Neurosci. Lett. 2003, 342(1-2):41-4].

*Nucleic acid binding proteins:*

The phrase "nucleic acid binding proteins" refers to proteins involved in RNA and DNA synthesis and expression regulation such as transcription factors, RNA and DNA binding proteins, zinc fingers, helicase, isomerase, histones, nucleases, ribonucleoproteins, and transcription and translation factors.

Pharmaceutical compositions including such proteins or protein encoding sequences, antibodies directed against such proteins or polynucleotides capable of altering expression of such proteins, may be used to treat diseases involving DNA or RNA binding proteins such as: helicases, isomerases, histones and nucleases, for example diseases where there is abnormal replication or transcription of DNA and RNA respectively. Antibodies and polynucleotides such as PCR primers and molecular probes designed to identify such proteins or protein encoding sequences maybe used for diagnosis of such diseases.

Examples of such diseases include, but are not limited to, neurological diseases such as renitis pigmentoas [Am. J. Ophthalmol. 2003, 136(4):678-87] parkinsonism [Proc. Natl. Acad. Sci. USA. 2003, 100(18):10347-52], Alzheimer [J. Neurosci. 2003, 23(17):6914-27] and canavan diseases [Brain Res Bull. 2003, 61(4):427-35], cancerous diseases such as leukemia [Anticancer Res. 2003, 23(4):3419-26] or lung cancer [J. Pathol. 2003, 200(5):640-6], miopathy [Neuromuscul Disord. 2003, 13(7-8):559-67] and liver diseases [J. Pathol. 2003, 200(5):553-60].

*Proteins involved in Metabolism:*

The phrase "proteins involved in metabolism" refers to proteins involved in the totality of the chemical reactions and physical changes that occur in living organisms, comprising anabolism and catabolism; may be qualified to mean the chemical reactions and physical processes undergone by a particular substance, or class of substances, in a living organism. This group includes proteins involved in the reactions of cell growth and maintenance such as: metabolism resulting in cell growth, carbohydrate metabolism, energy pathways, electron transport, nucleobase, nucleoside, nucleotide and nucleic acid metabolism, protein metabolism and modification, amino acid and derivative metabolism, protein targeting, lipid metabolism, aromatic compound metabolism, one-carbon compound metabolism, coenzymes and prosthetic group metabolism, sulfur metabolism, phosphorus metabolism, phosphate metabolism, oxygen and radical metabolism, xenobiotic metabolism, nitrogen metabolism, fat body metabolism (sensu Insecta), protein localization, catabolism, biosynthesis, toxin metabolism , methylglyoxal metabolism, cyanate metabolism, glycolate metabolism, carbon utilization and antibiotic metabolism.

Pharmaceutical compositions including such proteins or protein encoding sequences, antibodies directed against such proteins or polynucleotides capable of altering expression of such proteins, may be used to treat diseases involving cell metabolism. Antibodies and polynucleotides such as PCR primers and molecular probes designed to identify such proteins or protein encoding sequences may be used for diagnosis of such diseases.

Examples of such metabolism-related diseases include, but are not limited to, multisystem mitochondrial disorder caused by mitochondrial DNA cytochrome C oxidase II deficiency [Campos Y., et al., (2001) Ann. Neurol. 50(3):409-13], conduction defects and ventricular dysfunction in the heart associated with heterogeneous connexin43 expression [Gutstein D. E., et al., (2001) Circulation, 104(10):1194-9], atherosclerosis associated with growth suppressor p27 deficiency [Diez-Juan A., and Andres V. (2001) FASEB J., 15(11):1989-95], colitis associated with glutathione peroxidase deficiency [Esworthy R. S., et al., (2001) Am. J. Physiol. Gastrointest. Liver Physiol., 281(3):G848-55], systemic lupus erythematosus associated with deoxyribonuclease I deficiency [Yasutomo K., et al., (2001) Nat. Genet., 28(4):313-4], alcoholic pancreatitis [Pancreas. 2003, 27(4):281-5], amyloidosis and diseases that are related to amyloid metabolism, such as FMF, atherosclerosis, diabetes, and especially diabetes long term consequences, neurological diseases such as Creutzfeldt-Jakob disease, and Parkinson or Rasmussen's encephalitis.

*Cell growth and/or maintenance proteins:*

The phrase "Cell growth and/or maintenance proteins" refers to proteins involved in any biological process required for cell survival, growth and maintenance, including proteins involved in biological processes such as cell organization and biogenesis, cell growth, cell proliferation, metabolism, cell cycle, budding, cell shape and cell size control, sporulation (sensu Saccharomyces), transport, ion homeostasis, autophagy, cell motility, chemi-mechanical coupling, membrane fusion, cell-cell fusion, and stress response.

Pharmaceutical compositions including such proteins or protein encoding sequences, antibodies directed against such proteins or polynucleotides capable of altering expression of such proteins, may be used to treat or prevent diseases such as cancer, degenerative diseases, for example neurodegenerative diseases or conditions associated with aging, or alternatively, diseases wherein apoptosis which should have taken place, does not take place. Antibodies and polynucleotides such as PCR primers and molecular probes designed to identify such proteins or protein encoding sequences may be used for diagnosis of such diseases, detection of pre-disposition to a disease, and determination of the stage of a disease.

Examples of such diseases include, but are not limited to, ataxia-telangiectasia associated with ataxia-telangiectasia mutated deficiency [Hande et al., (2001) Hum. Mol. Genet., 10(5):519-28], osteoporosis associated with osteonectin deficiency [Delany et al., (2000) J. Clin. Invest., 105(7):915-23], arthritis caused by membrane-bound matrix metalloproteinase deficiency [Holmbeck et al., (1999) Cell, 99(1):81-92], defective stratum corneum and early neonatal death associated with transglutaminase 1 deficiency [Matsuki et al., (1998) Proc. Natl. Acad. Sci. USA, 95(3):1044-9], and Alzheimer's disease associated with estrogen [Simpkins et al., (1997) Am. J. Med., 103(3A):19S-25S].

*Variants of proteins which accumulate an element/compound*

Variant proteins which their wild type version naturally binds a certain compound or element inside the cell, such as for storage, may have therapeutic effect as secreted variants. For example, Ferritin, accumulates iron inside the cells. A secreted variant of this protein is expected to bind plasma iron, reduce its levels to thereby have therapeutic effects in hemodisorders which are characterized by high levels of free-iron in the blood.

*Autoantigens*

Autoantigens refer to "self" proteins which evoke autoimmune response. Examples of autoantigens are listed in Table 15, below. Secreted splice variants of such autoantigens can be used to treat such autoimmune disorders. Since autoimmune disorders are occasionally accompanied by different autoimmune manifestations (including but not limited to multiple endocrine syndromes, i.e., syndrome), the secreted variants of the present invention may treat these multiple symptoms. Therapeutic mechanisms of such variants may include: (i) sequestration of auto-antibodies to thereby reduce their circulating levels; (ii) antigen specific immunotherapy - based on the observation that prior systemic administration of a protein antigen could inhibit the subsequent generation of the immune response to the same antigen (has been proved in mice models for Myasthenia Gravis and type I Diabetes).

In addition, any novel variant of autoantigens (not necessarily secreted) may be used for "specific immunoadsorption" - leading to a specific immunodepletion of an antibody when used in immunoadsorption columns.

Variants of autoantigens are also of a diagnostic value. The diagnosis of many autoimmune disorders is based on looking for specific autoantibodies to autoantigens known to be associated with an autoimmune condition. Most of the diagnostic techniques are based on having a recombinant form of the autoantigen and using it to screen for serum autoantibodies. However these antibodies may bind the variants of the present invention with a similar or augmented affinity. For example, TPO is a known autoantigen in thyroid autoimmunity. It has been shown that its variant TPOzanelli also take part in the autoimmune process and can bind the same antibodies as TPO [Biochemistry. 2001 Feb 27; 40(8):2572-9.].

The nucleic acid sequences of the present invention, the proteins encoded thereby and the cells and antibodies described hereinabove can be used in screening assays, therapeutic or prophylactic methods of treatment, or predictive medicine (e.g., diagnostic and prognostic assays, including those used to monitor clinical trials, and pharmacogenetics).

More specifically, the nucleic acids of the present invention can be used to: (i) express a protein of the invention in a host cell in culture or in an intact multicellular organism following, e.g., gene therapy; (ii) detect an mRNA; or (iii) detect an alteration in a gene to which a nucleic acid of the invention specifically binds; or to modulate such a gene's activity.

The nucleic acids and proteins of the present invention can also be used to treat disorders characterized by either insufficient or excessive production of those nucleic acids or proteins, a failure in a biochemical pathway in which they normally participate in a cell, or other aberrant or unwanted activity relative to the wild type protein (e.g., inappropriate enzymatic activity or unproductive protein folding). The proteins of the invention are useful in screening for naturally occurring protein substrates or other compounds (e.g., drugs) that modulate protein activity. The antibodies of the invention can also be used to detect and isolate the proteins of the invention, to regulate their bioavailability, or otherwise modulate their activity. Examplary uses, and the methods by which they can be achieved, are described in detail below.

### Possible utilities for variants of drug targets

Finding a variant of a known drug target can be advantageous in cases where the known drug has a major side effect, the therapeutic efficacy of the known drug is medium, a known drug has failed clinical trials due to one of the above. A drug which is specific to a new protein variant of the target or to the target only (without affecting the novel variant) is likely to have lower side effects as compared to the original drug, higher therapeutic efficacy, and broader or different range of activities.

For example, COX3, which is a variant of COX1, is known to bind COX inhibitors in different affinity than COX1. This molecule is also associated with different physiological processes than COX1. Therefore, a compound specific to COX1 or compounds specific to COX3 would have lower side effects (by not affecting the other variants), and higher therapeutic efficacy to larger populations.

### Diseases that may be treated/diagnosed using the teaching of the present invention

### Inflammatory diseases

Examples of inflammatory diseases include, but are not limited to, chronic inflammatory diseases and acute inflammatory diseases.

### Inflammatory diseases associated with hypersensitivity

Examples of hypersensitivity include, but are not limited to, Types I-IV hypersensitivity, immediate hypersensitivity, antibody mediated hypersensitivity, immune complex mediated hypersensitivity, T lymphocyte mediated hypersensitivity and DTH. An example of type I or immediate hypersensitivity is asthma. Examples of type II hypersensitivity include, but are not limited to, rheumatoid diseases, rheumatoid autoimmune diseases, rheumatoid arthritis [Krenn V. et al., Histol Histopathol 2000 Jul;15 (3):791], spondylitis, ankylosing spondylitis [Jan Voswinkel et al., Arthritis Res 2001; 3 (3): 189], systemic diseases, systemic autoimmune diseases, systemic lupus erythematosus [Erikson J. et al., Immunol Res 1998;17 (1-2):49], sclerosis, systemic sclerosis [Renaudineau Y. et al., Clin Diagn Lab Immunol. 1999 Mar;6 (2):156; Chan OT. et al., Immunol Rev 1999 Jun;169:107], glandular diseases, glandular autoimmune diseases, pancreatic autoimmune diseases, diabetes, Type I diabetes [Zimmet P. Diabetes Res Clin Pract 1996 Oct;34 Suppl:S125], thyroid diseases, autoimmune thyroid diseases, Graves' disease [Orgiazzi J. Endocrinol Metab Clin North Am 2000 Jun;29 (2):339], thyroiditis, spontaneous autoimmune thyroiditis [Braley-Mullen H. and Yu S, J Immunol 2000 Dec 15;165 (12):7262], Hashimoto's thyroiditis [ToyodaN. et al., Nippon Rinsho 1999 Aug;57 (8):1810], myxedema, idiopathic myxedema [Mitsuma T. Nippon Rinsho. 1999 Aug;57 (8):1759], autoimmune reproductive diseases, ovarian diseases, ovarian autoimmunity [Garza KM. et al., J Reprod Immunol 1998 Feb;37 (2):87], autoimmune anti-sperm infertility [Diekman AB. et al., Am J Reprod Immunol. 2000 Mar;43 (3):134], repeated fetal loss [Tincani A. et al., Lupus 1998;7 Suppl 2:S107-9], neurodegenerative diseases, neurological diseases, neurological autoimmune diseases, multiple sclerosis [Cross AH. et al., J Neuroimmunol 2001 Jan 1;112 (1-2):1], Alzheimer's disease [Oron L. et al., J Neural Transm Suppl. 1997;49:77], myasthenia gravis [Infante AJ. And Kraig E, Int Rev Immunol 1999;18 (1-2):83], motor neuropathies [Kornberg AJ. J Clin Neurosci. 2000 May;7 (3):191], Guillain-Barre syndrome, neuropathies and autoimmune neuropathies [Kusunoki S. Am J Med Sci. 2000 Apr;319 (4):234], myasthenic diseases, Lambert-Eaton myasthenic syndrome [Takamori M. Am J Med Sci. 2000 Apr;319 (4):204], paraneoplastic neurological diseases, cerebellar atrophy, paraneoplastic cerebellar atrophy, non-paraneoplastic stiff man syndrome, cerebellar atrophies, progressive cerebellar atrophies, encephalitis, Rasmussen's encephalitis, amyotrophic lateral sclerosis, Sydeham chorea, Gilles de la Tourette syndrome, polyendocrinopathies, autoimmune polyendocrinopathies [Antoine JC. and Honnorat J. Rev Neurol (Paris) 2000 Jan;156 (1):23], neuropathies, dysimmune neuropathies [Nobile-Orazio E. et al., Electroencephalogr Clin Neurophysiol Suppl 1999;50:419], neuromyotonia, acquired neuromyotonia, arthrogryposis multiplex congenita [Vincent A. et al., Ann N Y Acad Sci. 1998 May 13;841:482], cardiovascular diseases, cardiovascular autoimmune diseases, atherosclerosis [Matsuura E. et al., Lupus. 1998;7 Suppl 2:S13S], myocardial infarction [Vaarala O. Lupus. 1998;7 Suppl 2:S132], thrombosis [Tincani A. et al., Lupus 1998;7 Suppl 2:S107-9], granulomatosis, Wegener's granulomatosis, arteritis, Takayasu's arteritis and Kawasaki syndrome [Praprotnik S. et al., Wien Klin Wochenschr 2000 Aug 25;112 (15-16):660], anti-factor VIII autoimmune disease [Lacroix-Desmazes S. et al., Semin Thromb Hemost.2000;26 (2):157], vasculitises, necrotizing small vessel vasculitises, microscopic polyangiitis, Churg and Strauss syndrome, glomerulonephritis, pauci-immune focal necrotizing glomerulonephritis, crescentic glomerulonephritis [Noel LH. Ann Med Interne (Paris). 2000 May;151 (3):178], antiphospholipid syndrome [Flamholz R. et al., J Clin Apheresis 1999;14 (4):171], heart failure, agonist-like $\beta$-adrenoceptor antibodies in heart failure [Wallukat G. et al., Am J Cardiol. 1999 Jun 17;83 (12A):75H], thrombocytopenic purpura [Moccia F. Ann Ital Med Int. 1999 Apr-Jun;14 (2):114], hemolytic anemia, autoimmune hemolytic anemia [Efremov DG. et al., Leuk Lymphoma 1998 Jan;28 (3-4):285], gastrointestinal diseases, autoimmune diseases of the gastrointestinal tract, intestinal diseases, chronic inflammatory intestinal disease [Garcia Herola A. et al., Gastroenterol Hepatol. 2000 Jan;23 (1):16], celiac disease [Landau YE. and Shoenfeld Y. Harefuah 2000 Jan 16;138 (2):122], autoimmune diseases of the musculature, myositis, autoimmune myositis, Sjogren's syndrome [Feist E. et al., Int Arch Allergy Immunol 2000 Sep;123 (1):92], smooth muscle autoimmune disease [Zauli D. et al., Biomed Pharmacother 1999 Jun;53 (5-6):234], hepatic diseases, hepatic autoimmune diseases, autoimmune hepatitis [Manns MP. J Hepatol 2000 Aug;33 (2):326] and primary biliary cirrhosis [Strassburg CP. et al., Eur J Gastroenterol Hepatol. 1999 Jun; 11 (6):595].

Examples of type IV or T cell mediated hypersensitivity, include, but are not limited to, rheumatoid diseases, rheumatoid arthritis [Tisch R, McDevitt HO. Proc Natl Acad Sci U S A 1994 Jan 18;91 (2):437], systemic diseases, systemic autoimmune diseases, systemic lupus erythematosus [Datta SK., Lupus 1998;7 (9):591], glandular diseases, glandular autoimmune diseases, pancreatic diseases, pancreatic autoimmune diseases, Type 1 diabetes [Castano L. and Eisenbarth GS. Ann. Rev. Immunol. 8:647], thyroid diseases, autoimmune thyroid diseases, Graves' disease [Sakata S. et al., Mol Cell Endocrinol 1993 Mar;92 (1):77], ovarian diseases [Garza KM. et al., J Reprod Immunol 1998 Feb;37 (2):87], prostatitis, autoimmune prostatitis [Alexander RB. et al., Urology 1997 Dec;50 (6):893], polyglandular syndrome, autoimmune polyglandular syndrome, Type I autoimmune polyglandular syndrome [Hara T. et al., Blood. 1991 Mar 1;77 (5):1127], neurological diseases, autoimmune neurological diseases, multiple sclerosis, neuritis, optic neuritis [Soderstrom M. et al., J Neurol Neurosurg Psychiatry 1994 May;57 (5):544], myasthenia gravis [Oshima M. et al., Eur J Immunol 1990 Dec;20 (12):2563], stiff-man syndrome [Hiemstra HS. et al., Proc Natl Acad Sci U S A 2001 Mar 27;98 (7):3988], cardiovascular diseases, cardiac autoimmunity in Chagas' disease [Cunha-Neto E. et al., J Clin Invest 1996 Oct 15;98 (8):1709], autoimmune thrombocytopenic purpura [Semple JW. et al., Blood 1996 May 15;87 (10):4245], anti-helper T lymphocyte autoimmunity [Caporossi AP. et al., Viral Immunol 1998;11 (1):9], hemolytic anemia [Sallah S. et al., Ann Hematol 1997 Mar;74 (3):139], hepatic diseases, hepatic autoimmune diseases, hepatitis, chronic active hepatitis [Franco A. et al., Clin Immunol Immunopathol 1990 Mar;54 (3):382], biliary cirrhosis, primary biliary cirrhosis [Jones DE. Clin Sci (Colch) 1996 Nov;91 (5):551], nephric diseases, nephric autoimmune diseases, nephritis, interstitial nephritis [Kelly CJ. J Am Soc Nephrol 1990 Aug;1 (2):140], connective tissue diseases, ear diseases, autoimmune connective tissue diseases, autoimmune ear disease [Yoo TJ. et al., Cell Immunol 1994 Aug;157 (1):249], disease of the inner ear [Gloddek B. et al., Ann N Y Acad Sci 1997 Dec 29;830:266], skin diseases, cutaneous diseases, dermal diseases, bullous skin diseases, pemphigus vulgaris, bullous pemphigoid and pemphigus foliaceus.

Examples of delayed type hypersensitivity include, but are not limited to, contact dermatitis and drug eruption.

### Autoimmune diseases

Examples of autoimmune diseases include, but are not limited to, cardiovascular diseases, rheumatoid diseases, glandular diseases, gastrointestinal diseases, cutaneous diseases, hepatic diseases, neurological diseases, muscular diseases, nephric diseases, diseases related to reproduction, connective tissue diseases and systemic diseases.

Examples of autoimmune cardiovascular and blood diseases include, but are not limited to atherosclerosis [Matsuura E. et al., Lupus. 1998;7 Suppl 2:S135], myocardial infarction [Vaarala O. Lupus. 1998;7 Suppl 2:S132], thrombosis [Tincani A. et al., Lupus 1998;7 Suppl 2:S107-9], Wegener's granulomatosis, Takayasu's arteritis, Kawasaki syndrome [Praprotnik S. et al., Wien Klin Wochenschr 2000 Aug 25;112 (15-16):660], anti-factor VIII autoimmune disease [Lacroix-Desmazes S. et al., Semin Thromb Hemost.2000;26 (2):157], necrotizing small vessel vasculitis, microscopic polyangiitis, Churg and Strauss syndrome, pauci-immune focal necrotizing and crescentic glomerulonephritis [Noel LH. Ann Med Interne (Paris). 2000 May;151 (3):178], antiphospholipid syndrome [Flamholz R. et al., J Clin Apheresis 1999;14 (4):171], antibody-induced heart failure [Wallukat G. et al., Am J Cardiol. 1999 Jun 17;83 (12A):75H], thrombocytopenic purpura [Moccia F. Ann Ital Med Int. 1999 Apr-Jun;14 (2):114; Semple JW. et al., Blood 1996 May 15;87 (10):4245], autoimmune hemolytic anemia [Efremov DG. et al., Leuk Lymphoma 1998 Jan;28 (3-4):285; Sallah S. et al., Ann Hematol 1997 Mar;74 (3):139], cardiac autoimmunity in Chagas' disease [Cunha-Neto E. et al., J Clin Invest 1996 Oct 15;98 (8):1709) and anti-helper T lymphocyte autoimmunity [Caporossi AP. et al., Viral Immunol 1998;11 (1):9].

Examples of autoimmune rheumatoid diseases include, but are not limited to rheumatoid arthritis [Krenn V. et al., Histol Histopathol 2000 Jul;15 (3):791; Tisch R, McDevitt HO. Proc Natl Acad Sci units S A 1994 Jan 18;91 (2):437) and ankylosing spondylitis [Jan Voswinkel et al., Arthritis Res 2001; 3 (3): 189].

Examples of autoimmune glandular diseases include, but are not limited to, autoimmune diseases of the pancreas, Type 1 diabetes [Castano L. and Eisenbarth GS. Ann. Rev. Immunol.,8:647; Zimmet P. Diabetes Res Clin Pract 1996 Oct;34 Suppl:S125], autoimmune thyroid diseases, Graves' disease [Orgiazzi J. Endocrinol Metab Clin North Am 2000 Jun;29 (2):339; Sakata S. et al., Mol Cell Endocrinol 1993 Mar;92 (1):77], spontaneous autoimmune thyroiditis [Braley-Mullen H. and Yu S, J Immunol 2000 Dec 15;165 (12):7262], Hashimoto's thyroiditis [Toyoda N. et al., Nippon Rinsho 1999 Aug;57 (8):1810], idiopathic myxedema [Mitsuma T. Nippon Rinsho. 1999 Aug;57 (8):1759], ovarian autoimmunity [Garza KM. et al., J Reprod Immunol 1998 Feb;37 (2):87], autoimmune anti-sperm infertility, autoimmune prostatitis and Type I autoimmune polyglandular syndrome.

Examples of autoimmune gastrointestinal diseases include, but are not limited to, chronic inflammatory intestinal diseases [Garcia Herola A. et al., Gastroenterol Hepatol. 2000 Jan;23 (1):16], celiac disease [Landau YE. and Shoenfeld Y. Harefuah 2000 Jan 16;138 (2):122], colitis, ileitis and Crohn's disease and ulcerative colitis.

Examples of autoimmune cutaneous diseases include, but are not limited to, autoimmune bullous skin diseases, such as, but are not limited to, pemphigus vulgaris, bullous pemphigoid and pemphigus foliaceus.

Examples of autoimmune hepatic diseases include, but are not limited to, hepatitis, autoimmune chronic active hepatitis [Franco A. et al., Clin Immunol Immunopathol 1990 Mar;54 (3):382], primary biliary cirrhosis [Jones DE. Clin Sci (Colch) 1996 Nov;91 (5):551; Strassburg CP. et al., Eur J Gastroenterol Hepatol. 1999 Jun;11 (6):595) and autoimmune hepatitis [Manns MP. J Hepatol 2000 Aug;33 (2):326].

Examples of autoimmune neurological diseases include, but are not limited to, multiple sclerosis [Cross AH. et al., J Neuroimmunol 2001 Jan 1;112 (1-2):1], Alzheimer's disease [Oron L. et al., J Neural Transm Suppl. 1997;49:77], myasthenia gravis [Infante AJ. And Kraig E, Int Rev Immunol 1999;18 (1-2):83; Oshima M. et al., Eur J Immunol 1990 Dec;20 (12):2563], neuropathies, motor neuropathies [Kornberg AJ. J Clin Neurosci. 2000 May;7 (3):191], Guillain-Barre syndrome and autoimmune neuropathies [Kusunoki S. Am J Med Sci. 2000 Apr;319 (4):234], myasthenia, Lambert-Eaton myasthenic syndrome [Takamori M. Am J Med Sci. 2000 Apr;319 (4):204], paraneoplastic neurological diseases, cerebellar atrophy, paraneoplastic cerebellar atrophy and stiff-man syndrome [Hiemstra HS. et al., Proc Natl Acad Sci units S A 2001 Mar 27;98 (7):3988], non-paraneoplastic stiff man syndrome, progressive cerebellar atrophies, encephalitis, Rasmussen's encephalitis, amyotrophic lateral sclerosis, Sydeham chorea, Gilles de la Tourette syndrome and autoimmune polyendocrinopathies [Antoine JC. and Honnorat J. Rev Neurol (Paris) 2000 Jan;156 (1):23], dysimmune neuropathies [Nobile-Orazio E. et al., Electroencephalogr Clin Neurophysiol Suppl 1999;50:419], acquired neuromyotonia, arthrogryposis multiplex congenita [Vincent A. et al., Ann N Y Acad Sci. 1998 May 13;841:482], neuritis, optic neuritis [Soderstrom M. et al., J Neurol Neurosurg Psychiatry 1994 May;57 (5):544) multiple sclerosis and neurodegenerative diseases.

Examples of autoimmune muscular diseases include, but are not limited to, myositis, autoimmune myositis and primary Sjogren's syndrome [Feist E. et al., Int Arch Allergy Immunol 2000 Sep;123 (1):92) and smooth muscle autoimmune disease [Zauli D. et al., Biomed Pharmacother 1999 Jun;53 (5-6):234].

Examples of autoimmune nephric diseases include, but are not limited to, nephritis and autoimmune interstitial nephritis [Kelly CJ. J Am Soc Nephrol 1990 Aug;1 (2):140], glommerular nephritis.

Examples of autoimmune diseases related to reproduction include, but are not limited to, repeated fetal loss [Tincani A. et al., Lupus 1998;7 Suppl 2:S107-9].

Examples of autoimmune connective tissue diseases include, but are not limited to, ear diseases, autoimmune ear diseases [Yoo TJ. et al., Cell Immunol 1994 Aug;157 (1):249) and autoimmune diseases of the inner ear [Gloddek B. et al., Ann N Y Acad Sci 1997 Dec 29;830:266].

Examples of autoimmune systemic diseases include, but are not limited to, systemic lupus erythematosus [Erikson J. et al., Immunol Res 1998;17 (1-2):49) and systemic sclerosis [Renaudineau Y. et al., Clin Diagn Lab Immunol. 1999 Mar;6 (2):156; Chan OT. et al., Immunol Rev 1999 Jun;169:107].

*Infectious diseases*

Examples of infectious diseases include, but are not limited to, chronic infectious diseases, subacute infectious diseases, acute infectious diseases, viral diseases, bacterial diseases, protozoan diseases, parasitic diseases, fungal diseases, mycoplasma diseases, and prion diseases.

*Graft rejection diseases*

Examples of diseases associated with transplantation of a graft include, but are not limited to, graft rejection, chronic graft rejection, subacute graft rejection, hyperacute graft rejection, acute graft rejection, and graft versus host disease.

*Allergic diseases*

Examples of allergic diseases include, but are not limited to, asthma, hives, urticaria, pollen allergy, dust mite allergy, venom allergy, cosmetics allergy, latex allergy, chemical allergy, drug allergy, insect bite allergy, animal dander allergy, stinging plant allergy, poison ivy allergy and food allergy.

*Cancerous diseases*

Examples of cancer include but are not limited to carcinoma, lymphoma, blastoma, sarcoma, and leukemia. Particular examples of cancerous diseases but are not limited to: Myeloid leukemia such as Chronic myelogenous leukemia. Acute myelogenous leukemia with maturation. Acute promyelocytic leukemia, Acute nonlymphocytic leukemia with increased basophils, Acute monocytic leukemia. Acute myelomonocytic leukemia with eosinophilia; malignant lymphoma, such as Birkitt's Non-Hodgkin's; Lymphoctyic leukemia, such as acute lumphoblastic leukemia. Chronic lymphocytic leukemia; Myeloproliferative diseases, such as Solid tumors Benign Meningioma, Mixed tumors of salivary gland, Colonic adenomas; Adenocarcinomas, such as Small cell lung cancer, Kidney, Uterus, Prostate, Bladder, Ovary, Colon, Sarcomas, Liposarcoma, myxoid, Synovial sarcoma, Rhabdomyosarcoma (alveolar), Extraskeletel myxoid chondrosarcoma, Ewing's tumor; other include Testicular and ovarian dysgerminoma, Retinoblastoma, Wilms' tumor, Neuroblastoma, Malignant melanoma, Mesothelioma, breast, skin, prostate, and ovarian.

Thus, the nucleic acid sequences of the present invention and the proteins encoded thereby and the cells and antibodies described hereinabove can be used in, for example, screening assays, therapeutic or prophylactic methods of treatment, or predictive medicine (e.g., diagnostic and prognostic assays, including those used to monitor clinical trials, and pharmacogenetics).

More specifically, the nucleic acids of the invention can be used to: (i) express a protein of the invention in a host cell (in culture or in an intact multicellular organism following, e.g., gene therapy, given, of course, that the transcript in question contains more than untranslated sequence); (ii) detect an mRNA; or (iii) detect an alteration in a gene to which a nucleic acid of the invention specifically binds; or to modulate such a gene's activity.

The nucleic acids and proteins of the invention can also be used to treat disorders characterized by either insufficient or excessive production of those nucleic acids or proteins, a failure in a biochemical pathway in which they normally participate in a cell, or other aberrant or unwanted activity relative to the wild type protein (e.g., inappropriate enzymatic activity or unproductive protein folding). The proteins of the invention are especially useful in screening for naturally occurring protein substrates or other compounds (e.g., drugs) that modulate protein activity. The antibodies of the invention can also be used to detect and isolate the proteins of the invention, to regulate their bioavailability, or otherwise modulate their activity. These uses, and the methods by which they can be achieved, are described in detail below.

*Screening Assays*

The present invention provides methods (or "screening assays") for identifying agents (or "test compounds" that bind to or otherwise modulate (i.e., stimulate or inhibit) the expression or activity of a nucleic acid of the present invention or the protein it encodes. An agent may be, for example, a small molecule such as a peptide, peptidomimetic (e.g., a peptoid), an amino acid or an analog thereof, a polynucleotide or an analog thereof, a nucleotide or an analog thereof, or an organic or inorganic compound (e.g., a heteroorganic or organometallic compound) having a molecular weight less than about 10,000 (e.g., about 5,000, 1,000, or 500) grams per mole and salts, esters, and other pharmaceutically acceptable forms of such compounds.

Agents identified in the screening assays can be used, for example, to modulate the expression or activity of the nucleic acids or proteins of the invention in a therapeutic protocol, or to discover more about the biological functions of the proteins.

The assays can be constructed to screen for agents that modulate the expression or activity of a protein of the invention or another cellular component with which it interacts. For example, where the protein of the invention is an enzyme, the screening assay can be constructed to detect agents that modulate either the enzyme's expression or activity or that of its substrate. The agents tested can be those obtained from combinatorial libraries. Methods known in the art allow the production and screening of: biological libraries; peptoid libraries [i.e., libraries of molecules that function as peptides even though they have a non-peptide backbone that confers resistance to enzymatic degradation; see, e.g., Zuckermann et al., J. Med. Chem. 37:2678-85, (1994)]; spatially addressable parallel solid phase or solution phase libraries; synthetic libraries requiring deconvolution; "one-bead one-compound" libraries; and synthetic libraries. The biological and peptoid libraries can be used to test only peptides, but the other four are applicable to testing peptides, non-peptide oligomers or libraries of small molecules [Lam, Anticancer Drug Des. 12:145, (1997)]. Molecular libraries can be synthesized as described by DeWitt et al. [Proc. Natl. Acad. Sci. USA 90:6909, (1993)] Erb et al. [Proc. Natl. Acad. Sci. USA 91:11422, (1994)] Zuckermann et al. [J. Med. Chem. 37:2678, (1994)] Cho et al. [Science 261:1303, (1993)] and Gallop et al. [J. Med. Chem. 37:1233, (1994)].

Libraries of compounds may be presented in solution [see, e.g., Houghten, Biotechniques 13:412-421, (1992)], or on beads [Lam, Nature 354:82-84, (1991)], chips [Fodor, Nature 364:555-556, (1993)], bacteria or spores (U.S. Patent No. 5,223,409), plasmids [Cull et al., Proc Natl Acad Sci USA 89:1865-1869, (1992)] or on phage [Scott and Smith, Science 249:386-390, (1990); Devlin, Science 249:404-406, (1990); Cwirla et al., Proc. Natl. Acad. Sci. USA 87:6378-6382, (1990); Felici, J. Mol. Biol. 222:301-310, (1991); and U.S. Patent No. 5,223,409].

The screening assay can be a cell-based assay, in which case the screening method includes contacting a cell that expresses a protein of the invention with a test compound and determining the ability of the test compound to modulate the protein's activity. The cell used can be a mammalian cell, including a cell obtained from a human or from a human cell line.

Alternatively, or in addition to examining the ability of an agent to modulate expression or activity generally, one can examine the ability of an agent to interact with, for example, to specifically bind to, a nucleic acid or protein of the invention. For example, one can couple an agent (e.g., a substrate) to a label (those described above, including radioactive or enzymatically active substances, are suitable), contact the nucleic acid or protein of the invention with the labeled agent, and determine whether they bind one another (by detecting, for example, a complex containing the nucleic acid or protein and the labeled agent). Labels are not, however, always required. For example, one can use a microphysiometer to detect interaction between an agent and a protein of the invention, neither of which were previously labeled [McConnell et al., Science 257:1906-1912, (1992). A microphysiometer (also known as a cytosensor) is an analytical instrument that measures the rate at which a cell acidifies its environment. The instrument uses a light-addressable potentiometric sensor (LAPS), and changes in the acidification rate indicate interaction between an agent and a protein of the invention. Molecular interactions can also be detected using fluorescence energy transfer (FET; see, e.g., U.S. Patent Nos. 5,631,169 and 4,868,103). An FET binding event can be conveniently measured through fluorometric detection means well known in the art (e.g., by means of a fluorimeter). Where analysis in real time is desirable, one can examine the interaction (e.g., binding) between an agent and a protein of the invention with Biomolecular Interaction Analysis [BIA; see, e.g., Sjolander and Urbaniczky Anal. Chem. 63:2338-2345, (1991) and Szabo et al., Curr. Opin. Struct. Biol. 5:699-705, (1995)]. BIA allows one to detect biospecific interactions in real time without labeling any of the interactants (e.g., BIAcore).

The screening assays can also be cell-free assays (i.e., soluble or membrane-bound forms of the proteins of the invention, including the variants, mutants, and other fragments described above, can be used to identify agents that bind those proteins or otherwise modulate their expression or activity). The basic protocol is the same as that for a cell-based assay in that, in either case, one must contact the protein of the invention with an agent of interest [for a sufficient time and under appropriate (e.g., physiological) conditions] to allow any potential interaction to occur and then determine whether the agent binds the protein or otherwise modulates its expression or activity.

Those of ordinary skill in the art will, however, appreciate that there are differences between cell-based and cell-free assays. For example, when membrane-bound forms of the protein are used, it may be desirable to utilize a solubilizing agent (e.g., non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)$_n$, 3-[(3-cholamidopropyl)dimethylamminio]-1-propane sulfonate (CHAPS), 3-[(3-cholamidopropyl)dimethyl-lamminio]-2-hydroxy-1-propane sulfonate (CHAPSO), or N-dodecyl=N,N-dimethyl-3-ammonio-1-propane sulfonate).

In the assays of the invention, any of the proteins described herein or the agents being tested can be anchored to a solid phase or otherwise immobilized (assays in which one of two substances that interact with one another are anchored to a solid phase are sometimes referred to as "heterogeneous" assays). For example, a protein of the present invention can be anchored to a microtiter plate, a test tube, a microcentrifuge tube, a column, or the like before it is exposed to an agent. Any complex that forms on the solid phase is detected at the end of the period of exposure. For example, a protein of the present invention can be anchored to a solid surface, and the test compound (which is not anchored and can be labeled, directly or indirectly) is added to the surface bearing the anchored protein. Un-reacted (e.g., unbound) components can be removed (by, e.g., washing) under conditions that allow any complexes formed to remain immobilized on the solid surface, where they can be detected (e.g., by virtue of a label attached to the protein or the agent or with a labeled antibody that specifically binds an immobilized component and may, itself, be directly or indirectly labeled).

One can immobilize either a protein of the present invention or an antibody to which it specifically binds to facilitate separation of complexed (or bound) protein from uncomplexed (or unbound) protein. Such immobilization can also make it easier to automate the assay, and fusing the proteins of the invention to heterologous proteins can facilitate their immobilization. For example, proteins fused to glutathione-S-transferase can be adsorbed onto glutathione sepharose beads (Sigma Chemical Co., St. Louis, MO) or glutathione derivatized microtiter plates, then combined with the agent and incubated under conditions conducive to complex formation (e.g., conditions in which the salt and pH levels are within physiological levels). Following incubation, the solid phase is washed to remove any unbound components (where the solid phase includes beads, the matrix can be immobilized), the presence or absence of a complex is determined. Alternatively, complexes can be dissociated from a matrix, and the level of protein binding or activity can be determined using standard techniques.

Immobilization can be achieved with methods known in the art. For example, biotinylated protein can be prepared from biotin-NHS (N-hydroxy-succinimide) using techniques known in the art (e.g., the biotinylation kit from Pierce Chemicals, Rockford, IL) and immobilized in the wells of streptavidin-coated tissue culture plates (also from Pierce Chemical).

The screening assays of the invention can employ antibodies that react with the proteins of the invention but do not interfere with their activity. These antibodies can be derivatized to a solid surface, where they will trap a protein of the invention. Any interaction between a protein of the invention and an agent can then be detected using a second antibody that specifically binds the complex formed between the protein of the invention and the agent to which it is bound.

Cell-free assays can also be conducted in a liquid phase, in which case any reaction product can be separated (and thereby detected) by, for example: differential centrifugation (Rivas and Minton, Trends Biochem Sci 18:284-7, 1993); chromatography (e.g., gel filtration or ion-exchange chromatography); electrophoresis [see, e.g., Ausubel et al., Eds., Current Protocols in Molecular Biology, J. Wiley & Sons, New York, N.Y., (1999)]; or immunoprecipitation [see, e.g., Ausubel et al. (supra); see also Heegaard, J. Mol. Recognit. 11:141-148, (1998) and Hage and Tweed, J. Chromatogr. Biomed. Sci. Appl. 699:499-525, (1997)]. Fluorescence energy transfer (see above) can also be used, and is convenient because binding can be detected without purifying the complex from solution. Assays in which the entire reaction of interest is carried out in a liquid phase are sometimes referred to as homogeneous assays.

The screening methods of the invention can also be designed as competition assays in which an agent and a substance that is known to bind a protein of the present invention compete to bind that protein. Depending upon the order of addition of reaction components and the reaction conditions (e.g., whether the reaction is allowed to reach equilibrium), agents that inhibit complex formation can be distinguished from those that disrupt preformed complexes.

In either approach, the order in which reactants are added can be varied to obtain different information about the agents being tested. For example, agents that interfere with the interaction between a gene product and one or more of its binding partners (by, e.g., competing with the binding partner), can be identified by adding the binding partner and the agent to the reaction at about the same time. Agents that disrupt preformed complexes (by, e.g., displacing one of the components from the complex), can be added after a complex containing the gene product and its binding partner has formed.

The proteins of the invention can also be used as "bait proteins" in a two- or three-hybrid assay [see, e.g., U.S. Patent No. 5,283,317; Zervos et al., Cell 72:223-232, (1993); Madura et al., J. Biol. Chem. 268:12046-12054, (1993); Bartel et al. Biotechniques 14:920-924, (1993); Iwabuchi et al., Oncogene 8:1693-1696, (1993); and WO 94/10300] to identify other proteins that bind to (e.g., specifically bind to) or otherwise interact with a protein of the invention. Such binding proteins can activate or inhibit the proteins of the invention (and thereby influence the biochemical pathways and events in which those proteins are active).

As noted above, the screening assays of the invention can be used to identify an agent that inhibits the expression of a protein of the invention by, for example, inhibiting the transcription or translation of a nucleic acid that encodes it. In these assays, one can contact a cell or cell free mixture with the agent and then evaluate mRNA or protein expression relative to the levels that are observed in the absence of the agent (a statistically significant increase in expression indicating that the agent stimulates mRNA or protein expression and a decrease (again, one that is statistically significant) indicating tat the agent inhibits mRNA or protein expression). Methods for determining levels of mRNA or protein expression are known in the art and, here, would employ the nucleic acids, proteins, and antibodies of the present invention.

It should be noted that if desired, two or more of the methods described herein can be practiced together. For example, one can evaluate an agent that was first identified in a cell-based assay in a cell free assay. Similarly, and the ability of the agent to modulate the activity of a protein of the invention can be confirmed in vivo (e.g., in a transgenic animal).

The screening methods of the present invention can also be used to identify proteins (in the event transcripts of the present invention encode proteins) that are associated (e.g., causally) with drug resistance. One can then block the activity of these proteins (with, e.g., an antibody of the invention) and thereby improve the ability of a therapeutic agent to exert a desirable effect on a cell or tissue in a subject (e.g., a human patient).

Monitoring the influence of therapeutic agents (e.g., drugs) or other events (e.g., radiation therapy) on the expression or activity of a biomolecular sequence of the present invention can be useful in clinical trials (a desired extension of the screening assays described above). For example, agents that exert an effect by, in part, altering the expression or activity of a protein of the invention ex vivo can be tested for their ability to do so as the treatment progresses in a subject. Moreover, in animal or clinical trials, the expression or activity of a nucleic acid can be used, optionally in conjunction with that of other genes, as a "read out" or marker of the phenotype of a particular cell.

### *Detection Assays*

The nucleic acid sequences of the invention can serve as polynucleotide reagents that are useful in detecting a specific nucleic acid sequence. For example, one can use the nucleic acid sequences of the present invention to map the corresponding genes on a chromosome (and thereby discover which proteins of the invention are associated with genetic disease) or to identify an individual from a biological sample (i.e., to carry out tissue typing, which is useful in criminal investigations and forensic science). The novel transcripts of the present invention can be used to identify those tissues or cells affected by a disease (e.g., the nucleic acids of the invention can be used as markers to identify cells, tissues, and specific pathologies, such as cancer), and to identify individuals who may have or be at risk for a particular cancer. Specific methods of detection are described herein and are known to those of ordinary skill in the art.

The nucleic acids of the present invention can be used to determine whether a particular individual is the source of a biological sample (e.g., a blood sample). This is presently achieved by examining restriction fragment length polymorphisms (RFLPs; U.S. Patent No. 5,272,057), and the sequences disclosed here are useful as additional DNA markers for RFLP. For example, one can digest a sample of an individual's genomic DNA, separate the fragments (e.g. by Southern blotting), and expose the fragments to probes generated from the nucleic acids of the present invention (methods employing restriction endonucleases are discussed further below). If the pattern of binding matches that obtained from a tissue of an unknown source, then the individual is the source of the tissue.

The nucleic acids of the present invention can also be used to determine the sequence of selected portions of an individual's genome. For example, the sequences that represent new genes can be used to prepare primers that can be used to amplify an individual's DNA and subsequently sequence it Panels of DNA sequences (each amplified with a different set of primers) can uniquely identify individuals (as every person will have unique sequences due to allelic differences).

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences disclosed herein can provide positive individual identification with a panel of perhaps 10 to 1,000 primers which each yield a noncoding amplified sequence of 100 bases. If predicted coding sequences are used, a more appropriate number of primers for positive individual identification would be 500-2,000.

If a panel of reagents from the nucleic acids described herein is used to generate a unique identification database for an individual, those same reagents can later be used to identify tissue from that individual. Using the database, the individual, whether still living or dead, can subsequently be linked to even very small tissue samples.

DNA-based identification techniques, including those in which small samples of DNA are amplified (e.g, by PCR) can also be used in forensic biology. Sequences amplified from tissues (such as hair or skin) or body fluids (such as blood, saliva, or semen) found at a crime scene can be compared to a standard (e.g., sequences obtained and amplified from a suspect), thereby allowing one to determine whether the suspect is the source of the tissue or bodily fluid.

The nucleic acids of the invention, when used as probes or primers, can target specific loci in the human genome. This will improve the reliability of DNA-based forensic identifications because the more identifying markers examined, the less likely it is that one individual will be mistaken for another. Moreover, tests that rely on obtaining actual genomic sequence (which is possible here) are more accurate than those in which identification is based on the patterns formed by restriction enzyme generated fragments.

The nucleic acids of the invention can also be used to study the expression of the mRNAs in histological sections (i.e., they can be used in in situ hybridization). This approach can be useful when forensic pathologists are presented with tissues of unknown origin or when the purity of a population of cells (e.g., a cell line) is in question. The nucleic acids can also be used in diagnosing a particular condition and in monitoring a treatment regime.

*Predictive Medicine*

The nucleic acids, proteins, antibodies, and cells described hereinabove are generally useful in the field of predictive medicine and, more specifically, are useful in diagnostic and prognostic assays and in monitoring clinical trials. For example, one can determine whether a subject is at risk of developing a disorder associated with a lesion in, or the misexpression of, a nucleic acid of the invention (e.g., a cancer such as pancreatic cancer, breast cancer, or a cancer within the urinary system). In addition, the nucleic acids expressed in tumor tissues and not in normal tissues are markers that can be used to determine whether a subject has or is likely to develop a particular type of cancer.

The "subject" referred to in the context of any of the methods of the present invention, is a vertebrate animal (e.g., a mammal such as an animal commonly used in experimental studies (e.g. rats, mice, rabbits and guinea pigs); a domesticated animal (e.g., a dog or cat); an animal kept as livestock (e.g., a pig, cow, sheep, goat, or horse); a non-human primate (e.g. an ape, monkey, or chimpanzee); a human primate; an avian (e.g., a chicken); an amphibian (e.g., a frog); or a reptile. The animal can be an unborn animal (accordingly, the methods of the invention can be used to carry out genetic screening or to make prenatal diagnoses). The subject can also be a human.

The methods related to predictive medicine can also be carried out by using a nucleic acid of the invention to, for example detect, in a tissue of a subject: (i) the presence or absence of a mutation that affects the expression of the corresponding gene (e.g., a mutation in the 5' regulatory region of the gene); (ii) the presence or absence of a mutation that alters the structure of the corresponding gene; (iii) an altered level (i.e., a non-wild type level) of mRNA of the corresponding gene (the proteins of the invention can be similarly used to detect an altered level of protein expression); (iv) a deletion or addition of one or more nucleotides from the nucleic acid sequences of the present invention; (v) a substitution of one or more nucleotides in the nucleic acid sequences of the present invention (e.g., a point mutation); (vi) a gross chromosomal rearrangement (e.g., a translocation, inversion, or deletion); or (vii) aberrant modification of a gene corresponding to the nucleic acid sequences of the present invention (e.g., modification of the methylation pattern of the genomic DNA). Similarly, one can test for inappropriate post-translational modification of any protein encoded. Abnormal expression or abnormal gene or protein structures indicate that the subject is at risk for the associated disorder.

A genetic lesion can be detected by, for example, providing an oligonucleotide probe or primer having a sequence that hybridizes to a sense or antisense strand of a nucleic acid sequence of the present invention, a naturally occurring mutant thereof, or the 5' or 3' sequences that are naturally associated with the corresponding gene, and exposing the probe or primer to a nucleic acid within a tissue of interest (e.g., a tumor). One can detect hybridization between the probe or primer and the nucleic acid of the tissue by standard methods (e.g., in situ hybridization) and thereby detect the presence or absence of the genetic lesion. Where the probe or primer specifically hybridizes with a new splice variant, the probe or primer can be used to detect a non-wild type splicing pattern of the mRNA. The antibodies of the invention can be similarly used to detect the presence or absence of a protein encoded by a mutant, mis-expressed, or otherwise deficient gene. Diagnostic and prognostic assays are described further below.

Qualitative or quantitative analyses (which reveal the presence or absence of a substance or its level of expression or activity, respectively) can be carried out for any one of the nucleic acid sequences of the present invention, or (where the nucleic acid encodes a protein) the proteins they encode, by obtaining a biological sample from a subject and contacting the sample with an agent capable of specifically binding a nucleic acid represented by the nucleic acid sequences of the present invention or a protein those nucleic acids encode. The conditions in which contacting is performed should allow for specific binding. Suitable conditions are known to those of ordinary skill in the art. The biological sample can be a tissue, a cell, or a bodily fluid (e.g., blood or serum), which may or may not be extracted from the subject (i.e., expression can be monitored in vivo).

More specifically, the expression of a nucleic acid sequence can be examined by, for example, Southern or Northern analyses, polymerase chain reaction analyses, or with probe arrays. For example, one can diagnose a condition associated with expression or mis-expression of a gene by isolating mRNA from a cell and contacting the mRNA with a nucleic acid probe with which it can hybridize under stringent conditions (the characteristics of useful probes are known to those of ordinary skill in the art and are discussed elsewhere herein). The mRNA can be immobilized on a surface (e.g., a membrane, such as nitrocellulose or other commercially available membrane) following gel electrophoresis.

Alternatively, one or more nucleic acids (the target sequence or the probe) can be distributed on a two-dimensional array (e.g., a gene chip). Arrays are useful in detecting mutations because a probe positioned on the array can have one or more mismatches to a nucleic acid of the invention (e.g., a destabilizing mismatch). For example, genetic mutations in any of nucleic acid sequences of the present invention can be identified in two-dimensional arrays containing light-generated DNA probes [Cronin et al., Human Mutation 7:244-255, (1996)]. Briefly, when a light-generated DNA probe is used, a first array of probes is used to scan through long stretches of DNA in a sample and a control to identify base changes between the sequences by making linear arrays of sequential overlapping probes. This step allows the identification of point mutations, and it can be followed by use of a second array that allows the characterization of specific mutations by using smaller, specialized probe arrays complementary to all variants or mutations detected. Each mutation array is composed of parallel probe sets, one complementary to the wild-type gene and the other complementary to the mutant gene. Arrays are discussed further below; see also; Kozal et al. [Nature Medicine 2:753-759, (1996)].

The level of an mRNA in a sample can also be evaluated with a nucleic acid amplification technique e.g., RT-PCR (U.S. Patent No. 4,683,202), ligase chain reaction [LCR; Barany, Proc. Natl. Acad. Sci. USA 88:189-193, (1991)]; LCR can be particularly useful for detecting point mutations), self sustained sequence replication [Guatelli et al., Proc. Natl. Acad. Sci. USA 87:1874-1878, (1990)], transcriptional amplification system [Kwoh et al., Proc. Natl. Acad. Sci. USA 86:1173-1177, (1989)], Q-Beta Replicase [Lizardi et al., Bio/Technology 6:1197, (1988)], or rolling circle replication (U.S. Patent No. 5,854,033). Following amplification, the nucleic acid can be detected using techniques known in the art. Amplification primers are a pair of nucleic acids that anneal to 5' or 3' regions of a gene (plus and minus strands, respectively, or vice-versa) at some distance (possibly a short distance) from one another. For example, each primer can consist of about 10 to 30 nucleotides and bind to sequences that are about 50 to 200 nucleotides apart. Serial analysis of gene expression can be used to detect transcript levels (U.S. Patent No. 5,695,937). Other useful amplification techniques (useful in, for example, detecting an alteration in a gene) include anchor PCR, real-time PCR or RACE PCR.

Mutations in the gene sequences of the invention can also be identified by examining alterations in restriction enzyme cleavage patterns. For example, one can isolate DNA from a sample cell or tissue and a control, amplify it (if necessary), digest it with one or more restriction endonucleases, and determine the length(s) of the fragment(s) produced (e.g., by gel electrophoresis). If the size of the fragment obtained from the sample is different from the size of the fragment obtained from the control, there is a mutation in the DNA in the sample tissue. Sequence specific ribozymes (see, for example, U.S. Patent No. 5,498,531) can be used to detect specific mutations by development or loss of a ribozyme cleavage site.

Any sequencing reaction known in the art (including those that are automated) can also be used to determine whether there is a mutation, and, if so, how the mutant differs from the wild type sequence. Mutations can also be identified by using cleavage agents to detect mismatched bases in RNA/RNA or RNA/DNA duplexes [Myers et al., Science 230:1242, (1985); Cotton et al., Proc. Natl. Acad. Sci. USA 85:4397, (1988); Saleeba et al., Methods Enzymol. 217:286-295, (1992)]. Mismatch cleavage reactions employ one or more proteins that recognize mismatched base pairs in double-stranded DNA (so called "DNA mismatch repair" enzymes; e.g., the mutY enzyme of E. coli cleaves A at G/A mismatches and the thymidine DNA glycosylase from HeLa cells cleaves T at G/T mismatches [see Hsu et al., Carcinogenesis 15:1657-1662, (1994) and U.S. Patent No. 5,459,039].

Alterations in electrophoretic mobility can also be used to identify mutations. For example, single strand conformation polymorphism (SSCP) can be used to detect differences in electrophoretic mobility between mutant and wild type nucleic acids [Orita et al., Proc. Natl. Acad. Sci. USA 86:2766, (1989); see also Cotton Mutat. Res. 285:125-144, (1993); and Hayashi, Genet. Anal. Tech. Appl. 9:73-79, (1992)]. Single-stranded DNA fragments of sample and control nucleic acids are denatured and allowed to renature. The secondary structure of single-stranded nucleic acids varies according to sequence, and the resulting alteration in electrophoretic mobility enables the detection of even a single base change. The sensitivity of the assay is enhanced when RNA (rather than DNA) is used because RNA's secondary structure is more sensitive to a change in sequence. See also Keen et al., Trends Genet. 7:5, (1991). The movement of mutant or wild-type fragments through gels containing a gradient of denaturant is also informative.

When denaturing gradient gel electrophoresis [DGGE; Myers et al., Nature 313:495, (1985)] is used, DNA can be modified so it will not completely denature (this can be done by, for example by adding a GC clamp of approximately 40 bp of high-melting GC-rich DNA by PCR). A temperature gradient can be used in place of a denaturing gradient to identify differences in the mobility of control and sample DNA [Rosenbaum and Reissner, Biophys. Chem. 265:12753, (1987)].

Point mutations can also be detected by selective oligonucleotide hybridization, selective amplification, or selective primer extension [Point et al., Nature 324:163, (1986); Saiki et al., Proc. Natl. Acad. Sci. USA 86:6230, (1989)] or by chemical ligation of oligonucleotides as described in Xu et al., Nature Biotechnol. 19:148, (2001). Allele specific amplification technology can also be used [see, e.g., Gibbs et al., Nucleic Acids Res. 17:2437-2448, (1989); Prossner, Tibtech. 11:238, (1993); and Barany, Proc. Natl. Acad. Sci. USA 88:189, (1991)].

When analysis of a gene or protein is carried out in a cell or tissue sample, the cell or tissue can be immobilized on a support, typically a glass slide, and then contacted with a probe that can hybridize to the nucleic acid or protein of interest.

The detection methods of the invention can be carried out with appropriate controls (e.g., analyses can be conducted in parallel with a sample known to contain the target sequence and a target known to lack it).

Various approaches can be used to determine protein expression or activity. For example, one can evaluate the amount of protein in a sample by exposing the sample to an antibody that specifically binds the protein of interest. The antibodies described above (e.g., monoclonal antibodies, detectably labeled antibodies, intact antibodies and fragments thereof) can be used. The methods can be carried out in-vitro (e.g., one can perform an enzyme linked immunosorbent assay (ELISA), an immunoprecipitation, an immunofluorescence analysis, an enzyme immunoassay (EIA), a radioimmunoassay (RIA), or a Western blot analysis) or in vivo (e.g., one can introduce a labelled antibody that specifically binds to a protein of the present invention into a subject and then detect it by a standard imaging technique). Alternatively, the sample can be labeled and then contacted with an antibody. For example, one can biotinylate the sample, contact it with an antibody (e.g., an antibody positioned on an antibody array) and then detect the bound sample (e.g., with avidin coupled to a fluorescent label). As with methods to detect nucleic acids, appropriate control studies can be performed in parallel with those designed to detect protein expression.

The diagnostic molecules disclosed herein can be assembled as kits. Accordingly, the invention features kits for detecting the presence of the biomolecular sequences of the present invention in a biological sample. The kit can include a probe (e.g., a nucleic acid sequence or an antibody), a standard and, optionally, instructions for use. More specifically, antibody-based kits can include a first antibody (e.g., in solution or attached to a solid support) that specifically binds a protein of the present invention and, optionally, a second, different antibody that specifically binds to the first antibody and is conjugated to a detectable agent. Oligonucleotide-based kits can include an oligonucleotide (e.g., a labeled oligonucleotide) that hybridizes with one of the nucleic acids of the present invention under stringent conditions or a pair of oligonucleotides that can be used to amplify a nucleic acid sequence of the present invention. The kits can also include a buffering agent, a preservative, a protein-stabilizing agent, or a component necessary for detecting any included label (e.g., an enzyme or substrate). The kits can also contain a control sample or a series of control samples that can be assayed and compared to the test sample contained. Each component of the kit can be enclosed within an individual container, and all of the various containers can be within a single package. It will be appreciated that the diagnostic kits of the present invention may also include additional diagnostic reagents, such as diagnostic reagents for detecting the wild-type gene product or known variants thereof. This combination of diagnostic markers is likely to establish a more accurate diagnosis.

The detection methods described herein can identify a subject who has, or is at risk of developing; a disease, disorder, condition, or syndrome (the term "disease" is used to encompass all deviations from a normal state) associated with aberrant or unwanted expression or activity of a biomolecular sequence of the present invention. The detection methods also have prognostic value (e.g., they can be used to determine whether or not it is likely that a subject will respond positively (i.e., be effectively treated with) to an agent (e.g., a nucleic acid, protein, small molecule or other drug)). Samples can also be obtained from a subject during the course of treatment to monitor the treatment's efficacy at a cellular level.

The present invention also features methods of evaluating a sample by creating a gene expression profile for the sample that includes the level of expression of one or more of biomolecular sequences of the present invention. The sample's profile can be compared with that of a reference profile (such as the profile of a wild-type gene product), either of which can be obtained by the methods described herein (e.g., by obtaining a nucleic acid from the sample and contacting the nucleic acid with those on an array). As with other detection methods, profile-based assays can be performed prior to the onset of symptoms (in which case they can be diagnostic), prior to treatment (in which case they can be predictive) or during the course of treatment (in which case they serve as monitors) [see, e.g., Golub et al., Science 286:531, (1999)].

As described hereinabove, the screening methods of the invention can be used to identify candidate therapeutic agents, and those agents can be evaluated further by examining their ability to alter the expression of one or more of the proteins of the invention. For example, one can obtain a cell from a subject, contact the cell with the agent, and subsequently examine the cell's expression profile with respect to a reference profile (which can be, for example, the profile of a normal cell or that of a cell in a physiologically acceptable condition). The agent is evaluated favorably if the expression profile in the subject's cell is, following exposure to the agent, more similar to that of a normal cell or a cell in a physiologically acceptable condition. A control assay can be performed with, for example, a cell that is not exposed to the agent.

Expression profiles (obtained by evaluating either nucleic acid or protein expression) are also useful in evaluating subjects. One can obtain a sample from a subject (either directly or indirectly from a caregiver), create an expression profile, and, optionally, compare the subject's expression profile to one or more reference profiles and/or select a reference profile most similar to that of the subject. A variety of routine statistical measures can be used to compare two reference profiles. One possible metric is the length of the distance vector that is the difference between the two profiles. Each of the subject and reference profile is represented as a multi-dimensional vector, wherein each dimension is a value in the profile.

The result, which can be communicated to the subject, a caregiver, or another interested party, can be the subject's expression profile per se, a result of a comparison of the subject's expression profile with another profile, a most similar reference profile, or a descriptor of any of these. Communication can be mediated by a computer network (e.g., in the form of a computer transmission such as a computer data signal embedded in a carrier wave).

Accordingly, the invention also features a computer medium having executable code for effecting the following steps: receive a subject expression profile; access a database of reference expression profiles; and either i) select a matching reference profile most similar to the subject expression profile, or ii) determine at least one comparison score for the similarity of the subject expression profile to at least one reference profile. The subject expression profile and the reference expression profile each include a value representing the level of expression of one or more of the biomolecular sequences of the present invention.

### *Arrays and uses thereof*

The present invention also encompasses arrays that include a substrate having a plurality of addresses, at least one of which includes a capture probe that specifically binds or hybridizes to a nucleic acid represented by any one of the biomolecular sequences of the present invention. The array can have a density of at least 10, 50, 100, 200, 500, 1,000, 2,000, or 10,000 or more addresses/$cm^2$, or densities between these. In some embodiments, the plurality of addresses includes at least 10, 100, 500, 1,000, 5,000, 10,000, or 50,000 addresses, while in other embodiments, the plurality of addresses can be equal to, or less than, those numbers.

Regardless of whether the array contains nucleic acids (as probes or targets) or proteins (as probes or targets), the substrate can be two-dimensional (formed, e.g., by a glass slide, a wafer (e.g., silica or plastic), or a mass spectroscopy plate) or three-dimensional (formed, e.g., by a gel or pad). Addresses in addition to the addresses of the plurality can be disposed on the array.

At least one address of the plurality can include a nucleic acid capture probe that hybridizes specifically to one or more of the nucleic acid sequences of the present invention. In certain embodiments, a subset of addresses of the plurality will be occupied by a nucleic acid capture probe for one of the nucleic acid sequences of the present invention; each address in the subset can bear a capture probe that hybridizes to a different region of a selected nucleic acid. In other embodiments, the probe at each address is unique, overlapping, and complementary to a different variant of a selected nucleic acid (e.g., an allelic variant, or all possible hypothetical variants). If desired, the array can be used to sequence the selected nucleic acid by hybridization (see, e.g., U.S. Patent No. 5,695,940). Alternatively, the capture probe can be a protein that specifically binds to a protein of the present invention or a fragment thereof (e.g., a naturally-occurring interaction partners of a protein of the invention or an antibody described herein). In some instances (e.g., in the event of an autoimmune disease), it is significant that a subject produces antibodies, and the arrays described herein can be used to detect those antibodies. More generally, an array that contains some or all of the proteins of the present invention can be used to detect any substance to which one or more those proteins bind (e.g., a natural binding partner, an antibody, or a synthetic molecule).

An array can be generated by methods known to those of ordinary skill in the art. For example, an array can be generated by photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145). Methods of producing protein-based arrays are described in, for example, De Wildt et al. [Nature Biotech. 18:89-994, (2000)], Lueking et al. [Anal. Biochem. 270:103-111, (1999)], Ge [Nucleic Acids Res. 28:e3, I-VII, (2000)], MacBeath and Schreiber [Science 289:1760-1763, (2000)], and WO 99/51773A1. Addresses in addition to the address of the plurality can be disposed on the array.

The arrays described above can be used to analyze the expression of any of the biomolecular sequences of the present invention. For example, one can contact an array with a sample and detect binding between a component of the sample and a component of the array. In the event nucleic acids are analyzed, one can amplify the nucleic acids obtained from a sample prior to their application to the array. The array can also be used to examine tissue-specific gene expression. For example, the nucleic acids or proteins of the invention (all or a subset thereof) can be distributed on an array that is then exposed to nucleic acids or proteins obtained from a particular tissue, tumor, or cell type. If a sufficient number of diverse samples are analyzed, clustering (e.g., hierarchical clustering, k-means clustering, Bayesian clustering and the like) can be used to identify other genes that are co-regulated with those of the invention. The array can be used not only to determine tissue specific expression, but also to ascertain the level of expression of a battery of genes.

Array analysis of the nucleic acids or proteins of the invention can be used to study the effect of cell-cell interactions or therapeutic agents on the expression of those nucleic acids or proteins. For example, nucleic acid or protein that has been obtained from a cell that has been placed in the vicinity of a tissue that has been perturbed in some way can be obtained and exposed to the probes of an array. Thus, one can use the methods of the invention to determine the effect of one cell type on another (i.e., the response (e.g., a change in the type or quantity of nucleic acids or proteins expressed) to a biological stimulus can be determined). Similarly, nucleic acid or protein that has been obtained from a cell that has been treated with an agent can be obtained and exposed to the probes of an array. In this scenario, one can determine how the therapeutic agent affects the expression of any of the biomolecular sequences of the present invention. Appropriate controls (e.g., assays using cells that have not received a biological stimulus or a potentially therapeutic treatment) can be performed in parallel. Moreover, desirable and undesirable responses can be detected. If an event (e.g., exposure to a biological stimulus or therapeutic compound) has an undesirable effect on a cell, one can either avoid the event (by, e.g., prescribing an alternative therapy) or take steps to counteract or neutralize it.

In more straightforward assays, the arrays described here can be used to monitor the expression of one or more of the biomolecular sequences of the present invention, with respect to time. Such analyses allow one to characterize a disease process associated with the examined sequence.

The arrays are also useful for ascertaining the effect of the expression of a gene on the expression of other genes in the same cell or in different cells (e.g., ascertaining the effect of the expression of any one of the biomolecular sequences of the present invention on the expression of other genes). If altering the expression of one gene has a deleterious effect on the cell (due to its effect on the expression of other genes) one can, again, avoid that effect (by, e.g., selecting an alternate molecular target or counteracting or neutralizing the effect).

*Markers*

The molecules of the present invention are also useful as markers of: (i) a cell or tissue type; (ii) disease; (iii) a pre-disease state; (iv) drug activity, and (v) predisposition for disease.

Using the methods described herein, the presence or amount of the biomolecular sequences of the present invention, can be detected and correlated with one or more biological states (e.g., a disease state or a developmental state). When used in this way, the compositions of the invention serve as surrogate markers; they provide an objective indicia of the presence or extent of a disease (e.g., cancer). Surrogate markers are particularly useful when a disease is difficult to assess with standard methods (e.g., when a subject has a small tumor or when pre-cancerous cells are present). It follows that surrogate markers can be used to assess a disease before a potentially dangerous clinical endpoint is reached. Other examples of surrogate markers are known in the art (see, e.g., Koomen et al., J. Mass Spectrom. 35:258-264, 2000, and James, AIDS Treatment News Archive 209, 1994). As mentioned hereinabove, the biomolecular sequences of the present invention may be used as markers alone or with other markers to establish an earlier and more accurate diagnosis of the disease.

The biomolecular sequences of the present invention, can also serve as pharmacodynamic markers, which provide an indicia of a therapeutic result As pharmacodynamic markers are not directly related to the disease for which the drug is being administered, their presence (or levels of expression) indicates the presence or activity of a drug in a subject (i.e., the pharmacodynamic marker may indicate the concentration of a drug in a biological tissue, as the gene or protein serving as the marker is either expressed or transcribed (or not) in the body in relationship to the level or activity of the drug). One can also monitor the distribution of a drug with a pharmacodynamic marker (e.g., these markers can be used to determine whether a drug is taken up by a particular cell type). The presence or amount of pharmacodynamic markers can be related to the drug per se or to a metabolite produced from the drug. Thus, these markers can indicate the rate at which a drug is broken down in vivo. Pharmacodynamic markers can be particularly sensitive (e.g., even a small amount of a drug may activate substantial transcription or translation of a marker), and they are therefore useful in assessing drugs that are administered at low doses. Examples regarding the use of pharmacodynamic markers are known in the art and include: U.S. Patent No. 6,033,862; Hattis et al. Env. Health Perspect. 90: 229-238, (1991); Schentag, Am. J. Health-Syst. Pharm. 56 Suppl. 3:S21-S24, (1999); and Nicolau, Am. J. Health-Syst. Pharm. 56 Suppl. 3: S16-S20, (1991).

The biomolecular sequences of the present invention, are also useful as pharmacogenomic markers, which can provide an objective correlate to a specific clinical drug response or susceptibility in a particular subject or class of subjects [see, e.g.., McLeod et al., Eur. J. Cancer 35:1650-1652, (1999)]. The presence or amount of the pharmacogenomic marker is related to the predicted response of a subject to a specific drug (or type of drug) prior to administration of the drug. By assessing one or more pharmacogenomic markers in a subject, the drug therapy that is most appropriate for the subject, or which is predicted to have a greater likelihood of success, can be selected. For example, based on the presence or amount of RNA or protein associated with a specific tumor marker in a subject, an optimal drug or treatment regime can be prescribed for the subject

More generally, pharmacogenomics addresses the relationship between an individual's genotype and that individual's response to a foreign compound or drug. Differences in the way individual subjects metabolize therapeutics can lead to severe toxicity or therapeutic failure because metabolism alters the relation between dose and blood concentration of the pharmacologically active drug. Thus, a physician would consider the results of pharmacogenomic studies when determining whether to administer a composition of the present invention and how to tailor a therapeutic regimen for the subject.

Pharmacogenomics deals with clinically significant hereditary variations in the response to drugs due to altered drug disposition and abnormal action in affected persons. See, e.g., Eichelbaum et al., Clin. Exp. Pharmacol. Physiol. 23:983-985, (1996), and Linder et al., Clin. Chem. 43:254-266, (1997). In general, two types of pharmacogenetic conditions can be differentiated. Genetic conditions transmitted as a single factor can: (i) alter the way drugs act on the body (altered drug action) or (ii) the way the body acts on drugs (altered drug metabolism). These pharmacogenetic conditions can occur either as rare genetic defects or as naturally-occurring polymorphisms.

One approach that can be used to identify genes that predict drug response, known as "a genome-wide association," relies primarily on a high-resolution map of the human genome consisting of already known gene-related markers (e.g., a "bi-allelic" gene marker map that consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, a high resolution map can be generated from a combination of known and newly uncovered single nucleotide polymorphisms (SNPs; a common alteration that occurs in a single nucleotide base in a stretch of DNA, see Example 22) in the human genome. For example, a SNP may occur once per every 1000 bases of DNA. While a SNP may be involved in a disease process, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Two alternative methods, the "candidate gene approach" and "gene expression profiling," can be used to identify pharmacogenomic markers. According to the first method, if a gene that encodes a drug's target is known, all common variants of that gene can be fairly easily identified in the population, and one can determine whether having one version of the gene versus another is associated with a particular drug response. In the second approach, the gene expression of an animal dosed with a drug (e.g., a composition of the invention) can reveal whether gene pathways related to toxicity have been activated.

Information generated using one or more of the approaches described above can be used in designing therapeutic or prophylactic treatments that are less likely to fail or to produce adverse side effects when a subject is treated with a therapeutic composition.

### *Informatics*

The biomolecular sequences of the present invention can be provided in a variety of media to facilitate their use. For example, one or more of the sequences (e.g., subsets of the sequences expressed in a defined tissue type) can be provided as a manufacture (e.g., a computer-readable storage medium such as a magnetic, optical, optico-magnetic, chemical or mechanical information storage device). The manufacture can provide a nucleic acid or amino acid sequence in a form that will allow examination of the manufacture in ways that are not applicable to a sequence that exists in nature or in purified form. The sequence information can include full-length sequences, fragments thereof, polymorphic sequences including single nucleotide polymorphisms (SNPs), epitope sequence, and the like.

The computer readable storage medium further includes sequence annotations (as described in Examples 10 and 22 of the Examples section).

The computer readable storage medium can further include information pertaining to generation of the data and/or potential uses thereof.

As used herein, a "computer-readable medium" refers to any medium that can be read and accessed directly by a machine [e.g., a digital or analog computer; e.g., a desktop PC, laptop, mainframe, server (e.g., a web server, network server, or server farm), a handheld digital assistant, pager, mobile telephone, or the like]. Computer-readablemedia include: magnetic storage media, such as floppy discs, hard disc storage medium, and magnetic tape; optical storage media such as CD-ROM; electrical storage media such as RAM, ROM, EPROM, EEPROM, flash memory, and the like; and hybrids of these categories such as magnetic/optical storage media.

A variety of data storage structures are available to those of ordinary skill in the art and can be used to create a computer-readablemedium that has recorded one or more (or all) of the nucleic acids and/or amino acid sequences of the present invention. The data storage structure will generally depend on the means chosen to access the stored information. In addition, a variety of data processor programs and formats can be used to store the sequence information of the present invention on machine or computer-readable medium. The sequence information can be represented in a word processing text file, formatted in commercially-available software such as WordPerfect and Microsoft Word, or represented in a file using a form of encoding of chartacters such as ASCII or EBCDIC, stored in a database application, such as DB2, Sybase, Oracle, or the like. One of ordinary skill in the art can readily adapt any number of data processor structuring formats (e.g., text file or database) to obtain machine or computer-readable medium having recorded thereon the sequence information of the present invention.

The sequence information and annotations are stored in a relational database (such as Sybase or Oracle) that can have a first table for storing sequence (nucleic acid and/or amino acid sequence) information. The sequence information can be stored in one field (e.g., a first column) of a table row and an identifier for the sequence can be stored in another field (e.g., a second column) of the table row. The database can have a second table (to, for example, store annotations). The second table can have a field for the sequence identifier, a field for a descriptor or annotation text (e.g., the descriptor can refer to a functionality of the sequence), a field for the initial position in the sequence to which the annotation refers, and a field for the ultimate position in the sequence to which the annotation refers. Examples for annotation to nucleic acid sequences and amino acid sequences are provided in Examples 10 and 14-20 of the Examples section.

### *Pharmaceutical Compositions*

The nucleic acids, fragments thereof, hybrid sequences of which they are a part, and gene constructs containing them; proteins, fragments thereof, chimeras, and antibodies that specifically bind thereto; and cells, including those that are engineered to express the nucleic acids or proteins of the invention) can be incorporated into pharmaceutical compositions. These compositions typically also include a solvent, a dispersion medium, a coating, an antimicrobial (e.g., an antibacterial or antifungal) agent, an absorption delaying agent (when desired, such as aluminum monostearate and gelatin), or the like, compatible with pharmaceutical administration (see below). Active compounds, in addition to those of the present invention, can also be included in the composition and may enhance or supplement the activity of the present agents.

The composition will be formulated in accordance with their intended route of administration. Acceptable routes include oral or parenteral routes (e.g., intravenous, intradermal, transdermal (e.g., subcutaneous or topical), or transmucosal (i.e., across a membrane that lines the respiratory or anogenital tract). The compositions can be formulated as a solution or suspension and, thus, can include a sterile diluent (e.g., water, saline solution, a fixed oil, polyethylene glycol, glycerine, propylene glycol or another synthetic solvent); an antimicrobial agent (e.g., benzyl alcohol or methyl parabens; chlorobutanol, phenol, ascorbic acid, thimerosal, and the like); an antioxidant (e.g., ascorbic acid or sodium bisulfite); a chelating agent (e.g., ethylenediaminetetraacetic acid); or a buffer (e.g., an acetate-, citrate-, or phosphate-based buffer): When necessary, the pH of the solution or suspension can be adjusted with an acid (e.g., hydrochloric acid) or a base (e.g., sodium hydroxide). Proper fluidity (which can ease passage through a needle) can be maintained by a coating such as lecithin, by maintaining the required particle size (in the case of a dispersion), or by the use of surfactants.

The compositions of the invention can be prepared as sterile powders (by, e.g., vacuum drying or freeze-drying), which can contain the active ingrediaent plus any additional desired ingredient from a previously sterile-filtered solution.

Oral compositions generally include an inert diluent or an edible carrier. For example, the active compound can be incorporated with excipients and used in the form of tablets, troches, or capsules (e.g., gelatin capsules). Oral compositions can be prepared using fluid carries and used as mouthwashes. The tablets etc. can also contain a binder (e.g., microcrystalline cellulose, gum tragacanth, or gelatin); an excipient (e.g., starch or lactose), a disintegrating agent (e.g., alginic acid, Primogel, or corn starch); a lubricang (e.g., magnesium stearate or Sterotes); a glidant (e.g., colloidal silicon dioxide); a sweetening agent (e.g., sucrose or saccharine); or a flavoring agent (e.g., peppermint, methyl salicylate, or orange flavoring.

For administration by way of the respiratory system, the compositions can be formulated as aerosol sprays (e.g., from a pressured container or dispenser that contains a suitable propellant (e.g., a gas such as carbon dioxide), or a nebulizer. The ability of a composition to cross a biological barrier can be enhanced by agents known in the art. For example, detergents, bile salts, and fusidic acid derivatives can facilitate transport across the mucosa (and therefore, be included in nasal sprays or suppositories).

For topical administration, the active compounds are formulated into ointments, salves, gels, or creams according to methods known in the art.

Controlled release can also be achieved by using implants and microencapsulated delivery systems (see, e.g., the materials commercially available from Alza Corporation and Nova Pharmaceuticals, Inc.; see also U.S. Patent No. 4,522,811 for the use of liposome-based suspensions).

The pharmaceutical compositions of the invention can be formulated in dosage units (i.e., physically discrete units containing a predetermined quantity of the active compound) for uniformity and ease of administration.

The toxicity and therapeutic efficacy of any given compound can be determined by standard pharmaceutical procedures carried out in cell culture or in experimental animals. For example, one of ordinary skill in the art can routinely determine the LD50 (the dose lethal to 50 % of the population) and the ED50 (the dose therapeutically effective in 50 % of the population). The dose ratio between toxic and therapeutic effects is the therapeutic index. Compounds that exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies described hereinabove can be used to formulate a range of dosage for use in humans (prefarably a dosage within a range of circulating concentrations that include the ED50 with little or no toxicity). The dosage may vary within this range depending upon the formulation and the route of administration. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

A therapeutically effective amount of a protein of the present invention can range from about 0.001 to 30 mg/kg body weight (e.g., about 0.01 to 25 mg/kg, about 0.1 to 20 mg/kg, or about 1 to 10 (e.g., 2-9, 3-8, 4-7, or 5-6) mg/kg). The protein can be administered one time per week for between about 1 to 10 weeks (e.g., 2 to 8 weeks, 3 to 7 weeks, or about 4, 5, or 6 weeks). However, a single administration can also be efficacious. Certain factors can influence the dosage and timing required to effectively treat a subject. These factors include the severity of the disease, previous treatments, and the general health or age of the subject

When the active ingredient is an antibody, the dosage can be about 0.1 mg/kg of body weight (generally 10-20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration are often possible with these types of antibodies. Modifications such as lipidation can be used to stabilize antibodies and to enhance uptake and tissue penetration [e.g., into the brain; see Cruikshank et al., J. Acquired Immune Deficiency Syndromes and Human Retrovirology 14:193, (1997)].

As noted above, the present invention encompasses agents (e.g., small molecules) that modulate expression or activity of a nucleic acid represented by any of biomolecular sequences of the present invention. Examplery doses of these agents include milligram or microgram amounts of the small molecule per kilogram of subject or sample weight (e.g., about 1-500 mg/kg; about 100 mg/kg; about 5 mg/kg; about 1 mg/kg; or about 50 $\mu$g/kg). Appropriate doses of a small molecule depend upon the potency of the small molecule with respect to the expression or activity to be modulated. When one or more of these small molecules is to be administered to an animal (e.g., a human) to modulate expression or activity of nucleic acid or protein of the invention, a physician, veterinarian, or researcher may prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

Pharmaceutical compositions of the present invention may also include a therapeutic moiety such as a cytotoxin (i.e., an agent that is detrimental to a cell), a therapeutic agent, or a radioactive ion can be conjugated to the biomolecular sequences of the present invention or related compositions, described hereinabove (e.g., antibodies, antisense molecules, ribozymes etc.). The cytotoxin can be, for example, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids (e.g., maytansinol; see U.S. Patent No. 5,208,020), CC-1065 (see U.S. Patent Nos. 5,475,092, 5,585,499, and 5,846,545) and analogs or homologs thereof. Therapeutic agents include antimetabolites (e.g., methotrexate, 6-mercaptopurine, 6-thioguanine, cytarabine, 5-fluorouracil decarbazine), alkylating agents (e.g., mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine, vinblastine, taxol and maytansinoids). Radioactive ions include, but are not limited to iodine, yttrium and praseodymium.

Other therapeutic moieties include, but are not limited to, toxins such as abrin, ricin A, pseudomonas exotoxin, or diphtheria toxin; a protein such as tumor necrosis factor, $\gamma$-interferon, $\beta$-interferon, nerve growth factor, platelet derived growth factor, tissue plasminogen activator; or, biological response modifiers such as, for example, lymphokines, interleukin-1 (IL-1), interleukin-2 (IL-2), interleukin-6 (IL-6), granulocyte macrophase colony stimulating factor (GM-CSF), granulocyte colony stimulating factor (G-CSF), or other growth factors.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen et al., Proc. Natl. Acad. Sci. USA 91:3054-3057, 1994). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells (e.g. retroviral vectors), the pharmaceutical preparation can include one or more cells which produce the gene delivery system. The pharmaceutical compositions of the invention can be included in a container, pack, or dispenser together with instructions for administration.

### Methods of Treatment

The present invention provides for both prophylactic and therapeutic methods of treating a subject at risk of (or susceptible to) a disorder or having a disorder associated with aberrant or unwanted expression or activity of a nucleic acid or protein of the invention. "Treatment" encompasses the application or administration of a therapeutic agent to a patient, or to an isolated tissue or cell line (e.g., one obtained from the patient to be treated), with the purpose of curing or lessening the severity of the disease or a symptom associated with the disease.

Whether carried out prophylactically or therapeutically, the methods of the invention can be specifically tailored or modified, based on knowledge obtained from the field of pharmacogenomics (see above).

Thus, the invention provides a method for preventing in a subject, a disease which onset or progression depends on the expression and/or activity of the biomolecular sequences of the present invention or variants or homologs thereof. Such diseases include cellular proliferative and/or differentiative disorders, disorders associated with bone metabolism, immune disorders, cardiovascular disorders, liver disorders, viral diseases, pain or metabolic disorders.

Examples of cellular proliferative and/or differentiative disorders include cancer (e.g., carcinoma, sarcoma, metastatic disorders or hematopoietic neoplastic disorders such as leukemias and lymphomas). A metastatic tumor can arise from a multitude of primary tumor types, including but not limited to those of prostate, colon, lung, breast or liver.

The terms "cancer," "hyperproliferative," and "neoplastic," are used in reference to cells that have exhibited a capacity for autonomous growth (i.e., an abnormal state or condition characterized by rapid cellular proliferation). Hyperproliferative and neoplastic disease states can be categorized as pathologic (i.e., characterizing or constituting a disease state), or can be categorized as non-pathologic (i.e., deviating from normal but not associated with a disease state). The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genitourinary tract, as well as adenocarcinomas, which include malignancies such as most colon cancers, renal-cell carcinoma, prostate cancer and/or testicular tumors, non-small cell carcinoma of the lung, cancer of the small intestine and cancer of the esophagus.

The term "carcinoma" refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term also includes carcinosarcomas (e.g., which include malignant tumors composed of carcinomatous and sarcomatous tissues). An "adenocarcinoma" refers to a carcinoma derived from glandular tissue or in which the tumor cells form recognizable glandular structures. The term "sarcoma" is art recognized and refers to malignant tumors of mesenchymal derivation. As used herein, the term "hematopoietic neoplastic disorder(s)" includes diseases involving hyperplastic/neoplastic cells of hematopoietic origin. A hematopoietic neoplastic disorder can arise from myeloid, lymphoid or erythroid lineages, or precursor cells thereof. Preferably, the diseases arise from poorly differentiated acute leukemias (e.g., erythroblastic leukemia and acute megakaryoblastic leukemia). Additional exemplary myeloid disorders include, but are not limited to, acute promyeloid leukemia (APML), acute myelogenous leukemia (AML) and chronic myelogenous leukemia (CML) (see Vaickus, Crit Rev. in Oncol./Hemotol. 11:267-97, 1991); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

The leukemias, including B-lymphoid leukemias, T-lymphoid leukemias, undifferentiated leukemias, erythroleukemia, megakaryoblastic leukemia, and monocytic leukemias are encompassed with and without differentiation; chronic and acute lymphoblastic leukemia, chronic and acute lymphocytic leukemia, chronic and acute myelogenous leukemia, lymphoma, myelo dysplastic syndrome, chronic and acute myeloid leukemia, myelomonocytic leukemia; chronic and acute myeloblastic leukemia, chronic and acute myelogenous leukemia, chronic and acute promyelocytic leukemia, chronic and acute myelocytic leukemia, hematologic malignances of monocyte-macrophage lineage, such as juvenile chronic myelogenous leukemia; secondary AML, antecedent hematological disorder; refractory anemia; aplastic anemia; reactive cutaneous angioendotheliomatosis; fibrosing disorders involving altered expression in dendritic cells, disorders including systemic sclerosis, E-M syndrome, epidemic toxic oil syndrome, eosinophilic fasciitis localized forms of scleroderma, keloid, and fibrosing colonopathy; angiomatoid malignant fibrous histiocytoma; carcinoma, including primary head and neck squamous cell carcinoma; sarcoma, including kaposi's sarcoma; fibroadanoma and phyllodes tumors, including mammary fibroadenoma; stromal tumors; phyllodes tumors, including histiocytoma; erythroblastosis; and neurofibromatosis.

Examples of disorders involving the heart or "cardiovascular disorders" include, but are not limited to, a disease, disorder, or state involving the cardiovascular system, e.g., the heart, the blood vessels, and/or the blood. A cardiovascular disorder can be caused by an imbalance in arterial pressure, a malfunction of the heart, or an occlusion of a blood vessel, e.g., by a thrombus. Examples of such disorders include hypertension, atherosclerosis, coronary artery spasm, congestive heart failure, coronary artery disease, valvular disease, arrhythmias, and cardiomyopathies.

As discussed, diseases associated (e.g., causally associated) with increased expression or activity of a protein of the present invention (as determined, for example, by the in vivo or ex vivo analyses described above), can be treated with techniques in which one inhibits the expression or activity of the nucleic acid or its gene products. For example, a compound (e.g., an agent identified using an assay described above) that exhibits negative modulatory activity with respect to a nucleic acid of the invention (the expression or over expression of which is causally associated with a disease) can be used to prevent and/or ameliorate that disease or one or more of the symptoms associated with it. The compound can be a peptide, phosphopeptide, small organic or inorganic molecule, or antibody (e.g., a polyclonal, monoclonal, humanized, anti-idiotypic, chimeric or single chain antibodies, and Fab, F(ab')2 and Fab expression library fragments, scFV molecules, and epitope-binding fragments thereof).

Further, antisense, ribozyme, and triple-helix molecules (see above) that inhibit expression of the target gene (e.g., a gene of the invention) can also be used to reduce the level of target gene expression, thus effectively reducing the level of target gene activity. If necessary, to achieve a desirable level of gene expression, molecules that inhibit gene expression can be administered with nucleic acid molecules that encode and express target gene polypeptides exhibiting normal target gene activity. Of course, where the assays of the invention indicate that expression or over expression is desirable, the nucleic acid can be introduced into cells via gene therapy methods with little or no treatment with inhibitory agents (this can be done to combat not only under expression, but over secretion of a gene product).

Aptamer molecules (nucleic acid molecules having a tertiary structure that permits them to specifically bind to protein ligands; [see, e.g., Osborne et al., Curr. Opin. Chem. Biol. 1: 5-9, (1997) and Patel Curr. Opin. Chem. Biol. 1:32-46, (1997)] are also useful therapeutics. Since nucleic acid molecules can usually be more conveniently introduced into target cells than therapeutic proteins may be, aptamers offer a method by which protein activity can be specifically decreased without the introduction of drugs or other molecules that may have pluripotent effects.

As noted above, the nucleic acids of the invention and the proteins they encode can be used as immunotherapeutic agents (to, e.g., elicit an immune response against a protein of interest). However, in some circumstances, undesirable effects occur when a subject is injected with a protein or an epitope that stimulate antibody production. In those circumstances, one can instead generate an immune response with an anti-idiotypic antibody [see, e.g., Herlyn, Ann. Med. 31:66-78, 1991 and Bhattacharya-Chatterjee and Foon, Cancer Treat. Res. 94:51-68, (1998)]. Effective anti-idiotypic antibodies stimulate the production of anti-anti-idiotypic antibodies, which specifically bind the protein in question. Vaccines directed to a disease characterized by expression of the nucleic acids of the present invention can also be generated in this fashion. In other circumstances, the target antigen is intracellular. In these circumstances, antibodies (including fragments, single chain antibodies, or other types of antibodies described above) can be internalized within a cell by delivering them with, for example, a lipid-based delivery system (e.g., Lipofectin™ or liposomes). Single chain antibodies can also be administered by delivering nucleotide sequences that encode them to the target cell population (see, e.g., Marasco et al., Proc. Natl. Acad. Sci. USA 90:7889-7893, 1993).

Alternatively, treatment of diseases associated with over expression or activity of a wild-type variant of the biomolecular sequences of the present invention can be effected by upregulating expression or activity of the polypeptides of the present invention in cases where they have an activity which antagonizes that of the wild-type protein (e.g., soluble receptor which antagonizes the activity of the wild type receptor as described hereinabove). Upregulating expression of the polypeptides of the present invention in a subject may be effected via the administration of at least one of the exogenous polynucleotide sequences of the present invention ligated into a nucleic acid expression construct designed for expression of coding sequences in eukaryotic cells (e.g., mammalian cells). Accordingly, the exogenous polynucleotide sequence may be a DNA or RNA sequence encoding the polypeptides of the present invention or active portions thereof.

It will be appreciated that the nucleic acid construct can be administered to the individual employing any suitable mode of administration, described hereinbelow (i.e., in-vivo gene therapy). Alternatively, the nucleic acid construct is introduced into a suitable cell via an appropriate gene delivery vehicle/method (transfection, transduction, homologous recombination, etc.) and an expression system as needed and then the modified cells are expanded in culture and returned to the individual (i.e., ex-vivo gene therapy).

Preferably, the promoter utilized by the nucleic acid construct of the present invention is active in the specific cell population transformed. Examples of cell type-specific and/or tissue-specific promoters include promoters, such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byme et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166).

Examples of suitable constructs include, but are not limited to, pcDNA3, pcDNA3.1 (+/-), pGL3, PzeoSV2 (+/-), pDisplay, pEF/myc/cyto, pCMV/myc/cyto each of which is commercially available from Invitrogen Co. (www.invitrogen.com). Examples of retroviral vector and packaging systems are those sold by Clontech, San Diego, Calif., including Retro-X vectors pLNCX and pLXSN, which permit cloning into multiple cloning sites and the trasgene is transcribed from CMV promoter. Vectors derived from Mo-MuLV are also included such as pBabe, where the transgene will be transcribed from the 5'LTR promoter.

Currently preferred in vivo nucleic acid transfer techniques include transfection with viral or non-viral constructs, such as adenovirus, lentivirus, Herpes simplex I virus, or adeno-associated virus (AAV) and lipid-based systems. Useful lipids for lipid-mediated transfer of the gene are, for example, DOTMA, DOPE, and DC-Chol [Tonkinson et al., Cancer Investigation, 14(1): 54-65 (1996)]. The most preferred constructs for use in gene therapy are viruses, most preferably adenoviruses, AAV, lentiviruses, or retroviruses. A viral construct such as a retroviral construct includes at least one transcriptional promoter/enhancer or locus-defining element(s), or other elements that control gene expression by other means such as alternate splicing, nuclear RNA export, or post-translational modification of messenger. Such vector constructs also include a packaging signal, long terminal repeats (LTRs) or portions thereof, and positive and negative strand primer binding sites appropriate to the virus used, unless it is already present in the viral construct. In addition, such a construct typically includes a signal sequence for secretion of the peptide from a host cell in which it is placed. Preferably the signal sequence for this purpose is a mammalian signal sequence or the signal sequence of the polypeptide variants of the present invention. Optionally, the construct may also include a signal that directs polyadenylation, as well as one or more restriction sites and a translation termination sequence. By way of example, such constructs will typically include a 5' LTR, a tRNA binding site, a packaging signal, an origin of second-strand DNA synthesis, and a 3' LTR or a portion thereof. Other vectors can be used that are non-viral, such as cationic lipids, polylysine, and dendrimers.

It will be appreciated that the present methodology may also be effected by specifically upregulating the expression of the splice variants of the present invention endogenously in the subject. Agents for upregulating endogenous expression of specific splice variants of a given gene include antisense oligonucleotides, which are directed at splice sites of interest, thereby altering the splicing pattern of the gene. This approach has been successfully used for shifting the balance of expression of the two isoforms of Bcl-x [Taylor (1999) Nat. Biotechnol. 17:1097-1100; and Mercatante (2001) J. Biol. Chem. 276:16411-16417]; IL-5R [Karras (2000) Mol. Pharmacol. 58:380-387]; and c-myc [Giles (1999) Antisense Acid Drug Dev. 9:213-220].

For example, interleukin 5 and its receptor play a critical role as regulators of hematopoiesis and as mediators in some inflammatory diseases such as allergy and asthma. Two alternatively spliced isoforms are generated from the IL-5R gene, which include (i.e., long form) or exclude (i.e., short form) exon 9. The long form encodes for the intact membrane-bound receptor, while the shorter form encodes for a secreted soluble non-functional receptor: Using 2'-O-MOE-oligonucleotides specific to regions of exon 9, Karras and co-workers (supra) were able to significantly decrease the expression of the wild type receptor and increase the expression of the shorter isoforms. Design and synthesis of oligonucleotides which can be used according to the present invention are described hereinbelow and by Sazani and Kole (2003) Moleclular and Subcellular Biology 31:217-239.

Alternatively or additionally, upregulation may be effected by administering to the subject at least one of the polypeptides of the present invention (e.g., recombinant or synthetic) or an active portion thereof, as described hereinabove. However, since the bioavailability of large polypeptides is relatively small due to high degradation rate and low penetration rate, administration of polypeptides is preferably confined to small peptide fragments (e.g., about 100 amino acids).

It will be appreciated that the treatment methods of the present invention may be combined with other therapeutic modalities (e.g., radiotherapy, chemotherapy) to increase therapeutic efficacy.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document The procedures therein are believed to be well known in the art and are provided for the convenience of the reader. All the information contained therein is incorporated herein by reference.

## EXAMPLE 1

### Identification of alternatively spliced expressed sequences - Background

The etiology of many kinds of cancers, especially those involving multiple genes or sporadic mutations, is yet to be elucidated. Accumulative EST information coming from heterogeneous tissues and cell-types, can be used as a considerable source to understanding some of the events inherent to carcinogenesis.

Although a large number of current bioinformatics tools are used to predict tissue specific genes in general and cancer specific genes in particular, all fail to consider alternatively spliced variants [Boguski and Schuler (1995) Nat. Genet. 10:369-71, Audic and Claverie (1997) Genome Res. 7:986-995; Huminiecki and Bicknell (2000) Genome Res. 10:1796-1806; Kawamoto et al. (2000) Genome Res. 10:1817-1827]. Alternative splicing is also overlooked by wet laboratory methods such as SAGE and microarray experiments which have been widely used to study gene expression, however remain to be linked to alternative splicing modeling [see Background section and Valculescu et al. (1995) Science 270:484-487; Caron et al. (2001) Science 291:1289-1292 and Schena et al. (1995) Science 270:467-470].

A computational-based approach was developed to identify alternatively spliced transcripts, which are expressed in a temporal and/or spatial pattern. Examples 1-4 below describe the identification of cancer specific alternatively spliced isoforms, which were identified according to the teachings of the present invention.

### Experimental procedures and reagents

*DATA and LEADS alternative splicing modeling -* GenBank version 125 with genomic build # 25 from National Center for Biotechnology Information (NCBI) was used as an input to the LEADS platform as described [Shoshan et al. (2001) Proc. SPIE Microarrays: Optical Technologies and Informatics 4266:86-95; Matloubian (2000) Nat. Immunol. 1:298-304; David et al. (2002) J. Biol. Chem. 277:18084-18090; Sorek et al. (2002) Genome Res. 12:1060-7]. UniGene Build #146 and libraryQuest.txt were obtained from NCBI and Cancer Genome Anatomy Project (CGAP) in National Cancer Institute (NCI), respectively.

*EST tissue information -* EST information was available in web form from Library Browser or Library Finder in NCBI or in the flat file libraryQuest.txt. The file listed 53 tissue sources, 5 histological states (cancer, multiple histology, normal, pre-cancer, and uncharacterized histology), 6 types of tissue preparations (bulk, cell line, flow-sorted, microdissected, multiple preparation, and uncharacterized), and brief descriptions on each library. 5318 libraries were from bulk tissue preparation {including 5000 ORESTES libraries [Camargo et al. (2001) Proc. Natl. Acad. Sci. USA 98:12103-12108]}, 329 from cell lines, 37 flow-sorted, 66 microdissected, 5 multiple preparation, and 1121 were from uncharacterized preparations. Excluding ORESTES libraries, 507 libraries were designated as 'non-normalized' and 100 were designated 'normalized' or 'subtracted' indicating the pretreatment of mRNA before cDNA library construction. A small number of libraries were derived from the same original sample. These were not considered separately. Library counts of ESTs rather than direct EST counts were used to provide semiquantitative measurements of expression level, since EST counts in some cases reflect the prevalence of ESTs in one or a few particular libraries, and library counts provide better indications across different tissue types when both normalized and non-normalized libraries were analyzed. Such tissue information analyses are limited to those tissues with a sufficient number of libraries. The inclusion of normalized cDNA libraries allowed the examination of genes expressed at low levels.

The ESTs from 'pooled tissue' or 'uncharacterized tissue' were considered as non-conforming in order to maintain the robustness of the results. In addition, 139,243 ESTs that had no library information were considered non-conforming in investigating tissue- or cancer-specific alternative splicing events.

*Results -* Human EST and mRNA sequences aligned against genomic sequences and clustered through Compugen's LEADS platform were used to identify intron boundaries and alternative splicing sites [Shoshan et al. (2001) Proc. SPIE Microarrays: Optical Technologies and Informatics 4266:86-95; Matloubian (2000) Nat. Immunol. 1:298-304; David et al. (2002) J. Biol. Chem. 277:18084-18090; Sorek et al. (2002) Genome Res. 12:1060-7].

20,301 clusters with 2.0 million ESTs contained at least one mRNA sequence, in general agreement with UniGene build #148 with 20,876 such clusters. The remaining EST sequences, which were clustered to unknown regions of known genes or to unknown genes were not analyzed. Table 1 below provides some statistics about EST and mRNA clustering. 125,115 introns, and 213,483 exons were aligned either with an mRNA, or with ESTs from at least two libraries if there was no RNA aligned to the gene segment. This was effected to exclude possible genomic contamination in expressed sequences, or other EST technology associated faults.

*Table 1*

| EST | Cluster | RNA | Cluster |
|---|---|---|---|
| 1 | 963 | 1 | 6527 |
| 2 - 3 | 1457 | 2-3 | 6372 |
| 4 - 7 | 1532 | 4-7 | 6204 |
| 8 - 15 | 1655 | 8-15 | 1915 |
| 16 - 31 | 1879 | 16-31 | 226 |
| 32 - 63 | 2500 | 32-63 | 40 |
| 64 - 127 | 3481 | 64 and above | 17 |
| 128 - 255 | 3240 | Total | 20301 |
| 256 - 511 | 1406 | | |
| 512 - 1023 | 422 | | |
| 1024 - above | 1766 | | |
| Total | 20301 | | |

## EXAMPLE 2

### *Cluster distribution of alternatively spliced donor and acceptor sites*

Alternative splice events include exon skipping, alternative 5' or 3' splicing, and intron retention, which can be described by the following simplification: a single exon connects to at least two other exons in either the 3' end (donor site) or the 5' end (acceptor site), as shown in Figure 3. Table 2 below lists some statistics of alternative splicing events based on this simplification.

*Table 2*

| Alternative donor site | Cluster | Alternative acceptor site | Cluster |
|---|---|---|---|
| 1 | 3690 | 1 | 3751 |
| 2 | 2269 | 2 | 2388 |
| 3 | 1348 | 3 | 1511 |
| 4 | 760 | 4 | 799 |
| 5 | 435 | 5 | 508 |
| 6 and above | 566 | 6 and above | 710 |
| Total | 9068 | Total | 9667 |

*Distribution analysis -* As described hereinabove a valid donor-acceptor concatenation must be supported by at least one mRNA or by ESTs from at least two different libraries. 8254 clusters were found to have both alternatively spliced donor and acceptor sites. When the lower bound on the number of EST libraries supporting each donor-acceptor concatenation was increased to three, 13,402 alternatively spliced donor sites were shown to be included in 6892 clusters and 15,015 alternatively spliced acceptor sites where shown to be included in 7570 clusters, while 6111 clusters comprised both alternatively spliced donor and acceptor sites.

## EXAMPLE 3

### Tissue distribution of ESTs and libraries following LEADS alternative splicing modeling

Cluster analysis performed to identify alternatively spliced ESTs (see Example 2) was further used for tissue information extraction. Table 3 below lists ten tissue types with the largest numbers of ESTs along with those from pooled or uncharacterized tissues.

**Table 3**

| Tissue | Number of ESTs | | | Number of Libraries | | |
|---|---|---|---|---|---|---|
| | Normal | Cancer | Total | Normal | Cancer | Total |
| Brain | 93024 | 87803 | 180827 | 30 | | 55 |
| Lung | 35455 | 85596 | 121051 | 92 | 156 | 248 |
| Placenta | 86571 | 27291 | 113862 | 259 | 3 | 262 |
| Uterus | 30052 | 71521 | 101573 | 99 | 107 | 206 |
| Colon | 23796 | 74998 | 98794 | 274 | 445 | 719 |
| Kidney | 42628 | 46811 | 89439 | 9 | 54 | 63 |
| Skin | 32436 | 43085 | 75521 | 8 | 10 | 18 |
| Prostate | 40312 | 27963 | 68275 | 131 | 135 | 266 |
| Mammary gland | 26509 | 36638 | 63147 | 305 | 665 | 970 |
| Head and neck | 12354 | 50167 | 62521 | 62 | 800 | 862 |
| Pooled | 178618 | 992 | 179610 | 15 | 1 | 16 |
| Uncharacterized | 76193 | 9721 | 85914 | 778 | 106 | |

Evidently, ESTs derived from lung, uterus, colon, kidney, mammary gland, head and neck were obtained aminly from cancerous libraries. The distribution of ESTs in normal and cancer libraries in each case was taken into a consideration and used as a parameter for scoring the differential expression annotation.

## EXAMPLE 4

### Identification of putative cancer specific alternatively spliced transcripts

Alternative splicing events restricted to cancer tissues were identified by looking for any donor-acceptor concatenation exclusively supported by ESTs from cancer tissues. Table 4 below lists six examples for such. An interesting example is the NONO gene (GenBank Accession No: BC003129), represented by 1496 ESTs. The NONO gene has been previously suggested to code for a possible splicing factor [Dong B, Horowitz DS, Kobayashi R, Krainer AR. Nucleic Acids Res (1993) 25;21(17):4085-92]. It's newly discovered restricted expression to cancer tissues suggests that alternative splicing of multiple genes maybe regulated during carcinogenesis.

**Table 4**

| mRNA/ EST | Uni Gene ID | Pos. | Total | | Type | Specific | | Non-specific | | | Possible function |
|---|---|---|---|---|---|---|---|---|---|---|---|
| | | | *E* | *R* | | *E* | *R* | *E* | | *R* | |
| | | | | | | | | *C* | *N* | | |

(continued)

| mRNA/ EST | Uni Gene ID | Pos. | Total | | Type | Specific | | Non-specific | | | Possible function |
|---|---|---|---|---|---|---|---|---|---|---|---|
| | | | E | R | | E | R | E | | R | |
| BC003129 | 172207 | 123, 237 | 1496 | 8 | d+ | 15 | 1 | 46 | 20 | 3 | Splicing factor candidate |
| NM_018035 | 279851 | 220, 301 | 584 | 2 | d- | 7 | 0 | 21 | 9 | 2 | No known function |
| AL519365 | 21938 | 474, 513 | 162 | 3 | a- | 8 | 3 | 6 | 1 | 0 | Oxysterol blinding |
| BF341144 | 155596 | 507, 542 | 148 | 1 | a+ | 6 | 0 | 7 | 4 | 1 | BCL2/adeno vir-us E1B interacting |
| AB009357 | 7510 | 1372, 1452 | 205 | 6 | a+ | 7 | 4 | 2 | 4 | 2 | MAPKKK 7 |
| NM_002382 | 42712 | 57, 84 | 165 | 7 | a- | 8 | 1 | 7 | 3 | 6 | MAX protein |

One mRNA/EST containing both splicing junctions identifies the cluster. 'Type'-indicates the type of transcript, which was shown to be cancer specific. The following symbols were used, (d) donor site; (a) acceptor site; ('+) proximal exon; ('-') distal exon. 'Total' - indicates the number of ESTs or mRNAs which were used for analysis. 'Specific/non-specific' - indicates total library number which was used for analysis. All mRNA sequences under 'specific' were from cancer tissues. 'Position' - identifies splicing boundaries on the sequence. E- EST; R-RNA; C- Cancer; N- Normal.

## EXAMPLE 5

### Ontological annotation of proteins - data Collection Background

Recent progress in genomic sequencing, computational biology and ontology development has presented an opportunity to investigate broad biological systems

A gene ontology system was developed and specifically used to annotate human proteins. Examples 5-9 below describe the development of an ontology engine, a computational platform for annotation and resultant annotations of human proteins.

Gene Ontology (GO) and gene association files were obtained from the Gene Ontology Consortium http://www.geneontology.org/. InterPro scan from http://www.ebi.ac:uk/interpro/, and enzyme database from http://expasy.proteome.org.au/enzyme/. The following databases and versions were used, GenBank release 122.0, SwissProt release 39.0, Enzyme database Release 26.0, InterPro database as of April 6, 2001, NCBI LocusLink data as of March 6, 2001, MEDLINE databases as of April 6, 2001, and the following files from Gene Ontology Consortium: gene_association.fb (version 1.26, 2001/02/19), gene_association.mgi (version 1.19, 2001/03/01), gene_association.sgd (version 1.251,2001/03/13), gene_association.pombase (version 1.2, 2000/07/22), ec2go (version 1.2, 2000/10/23), and swp2go (version 1.4, 2000/11/15). 58118 SWISS-Prot proteins have been assigned with at least one GO node by the following sources: 15534 proteins were assigned with at least a functional GO node by conversion of EC (enzyme nomenclature) to GO node. MGI has assigned 5984 SwissProt proteins with GO nodes (http://www.mgi.org). 31869 SwissProt proteins were assigned a GO node using SwissProt keyword correspondence and 33048 SwissProt proteins were assigned GO node by InterPro scanning (http://www.ebi.ac.uk/interpro/). The nonredundant protein database was constructed from GenPep file from NCBI, along with proteins collected from the Saccharomyces genome database (SGD) [Dwight et al. (2002) Nucleic Acids Res. 30:69-72] and the Drosophila genome database (Flybase) [The Flybase consortium 2002 Nucleic Acids Res. 30:106-108], with a total number of 670130.

## EXAMPLE 6

### Generation of progressive sequence clusters

A two-stage strategy was used to build a detailed homology map between all proteins in the comprehensive protein database (Example 5). In a first stage, all protein pairs with an E score lower than 0.01 using Blastp with default parameters were cataloged. Table 5 lists the distribution of Blastp results.

*Table 5*

| E escore | Percentage |
|---|---|
| $10^{-10}$ - $10^{-2}$ | 17.58 |
| $10^{-20}$ - $10^{-10}$ | 13.81 |
| $10^{-30}$ - $10^{-20}$ | 11.02 |
| $10^{-40}$ - $10^{-30}$ | 12.91 |
| $10^{-50}$ - $10^{-40}$ | 10.24 |
| $10^{-60}$ - $10^{-50}$ | 5.81 |
| $10^{-70}$ - $10^{-60}$ | 3.64 |
| $10^{-80}$ - $10^{-70}$ | 2.65 |
| $10^{-90}$ - $10^{-80}$ | 2.86 |
| $10^{-100}$ - $10^{-90}$ | 2.53 |
| $10^{-110}$ - $10^{-100}$ | 2.18 |
| $10^{-120}$ - $10^{-110}$ | 1.58 |
| $10^{-130}$ - $10^{-120}$ | 1.50 |
| $10^{-140}$ - $10^{-130}$ | 1.13 |
| $10^{-150}$ - $10^{-140}$ | 1.01 |
| $10^{-160}$ - $10^{-150}$ | 1.01 |
| $10^{-170}$ - $10^{-160}$ | 0.92 |
| $10^{-178}$ - $10^{-170}$ | 0.90 |
| 0.00 | 6.72 |

In the second stage, all homologous protein pairs were aligned through Needlman-Wunsch algorithm with a global alignment to obtain the percentage of identical amino acids between the two proteins.. BLOSUM62 was used as the substitution matrix. The percentage of identity was defined as the number of amino acids aligned with nonnegative scores divided by the number of amino acids in both aligned and unaligned length of two proteins in the global alignment. Table 6 shows a percent identity distribution of protein pairs following global alignment. Evidently, the majority of protein pairs (i.e., 68.5 %) exhibited identity levels in the range of 10-50 %.

*Table 6*

| Identity Level | Percentage |
|---|---|
| 0 - 10% | 5.67 |
| 10 - 20% | 24.66 |
| 20 - 30% | 19.94 |
| 30 - 40% | 10.94 |
| 40 - 50% | 7.31 |
| 50 - 60% | 7.09 |
| 60 - 70% | 7.24 |
| 70 - 80% | 6.70 |
| 80 - 90% | 5.98 |

(continued)

| Identity Level | Percentage |
|---|---|
| 90 - 100% | 4.47 |

**EXAMPLE 7**

**Text mining**

Correlations between presence of specific MeSH terms, or specific English words in available text information and Gene Ontology assignments in the training data were obtained. The correlations were then used to predict Gene Ontology for unassigned genes.

*Method -* Non-characters in titles and abstracts, and in definition line of gene records were eliminated and words were stemmed through the Lingua::stem module from www.cpan.org. Due to the standardized and curated nature of MeSH terms, MeSH terms were not parsed or stemmed. The frequency of each word in all the available text information was calculated. Words that occurred at least 5 times over the whole text information space were retained for further studies. This cutoff threshold was used to eliminate rare words, wrong spellings, and sometimes even the base pair sequence present in either the definition lines or abstracts. In addition, an upper limit of word frequency (common words such as 'and', 'gene', 'protein') and a lower limit of word frequency were defined through repeated training process and manual review. The words within the upper and the lower limits were considered as predictive. Since the correlation between the GO nodes and specific words is positive by nature, negative sentences with words such as 'not' and its variants, such as 'unlikely' or 'unresponsive' were excluded from consideration.

Genes with GO annotation from other sources such as GO consortium, InterPro scanning or keyword mappings were used as training data to obtain the correlation between specific words and specific GO nodes.

The following formula was used. $S = \log(P(m,g)/P(m)P(g))$, wherein S is the LOD score for word m - GO g combination, wherein $P(m,g)$ is the frequency of term m and GO node g co-occurrence among all word and GO combinations, $P(m)$ is the frequency of occurrence of term m among all word occurrences, and $P(g)$ is the frequency of occurrence of GO node g among all GO occurrences.

In order to predict GO node for any specific gene which is linked to one to a few dozen words, the sums of LOD scores from all these words for each possible GO were calculated and sorted, and used for further GO annotation. Multiple MeSH terms - GO correlations were tested and were found to be no more informative than the single MeSH term - GO correlation, and therefore they were not used.

*Results -* Table 7 below, lists general statistics of text information from publicly available sequence databases.

*Table 7*

| | MeSH term | Title | Abstract | Definition line |
|---|---|---|---|---|
| Number of proteins | 110608 | 106190 | 113073 | 516952 |
| Number of articles | 71703 | 77314 | 82654 | n/a |
| Number of unique words* | 40011 | 18175 | 26630 | 25915 |
| Average number of words per article or per definition line | 19.05 | 2.70 | 11.65 | 6.56 |

A predictive probabilistic model was then applied to create possible GO annotations based on the associated text information. Definition lines of sequence records, MeSH term annotations, titles and abstracts from sequence related publications were modeled separately.

The frequency of association of a specific term with a specific GO node in the training data was examined. Parameters such as boundaries of the frequency of MeSH terms and other words were optimized through the training process, using self-validation and cross validation methods. LOD (logarithm of odds) scores, defined as the logarithm of the ratio between the association frequency of any term-GO pair and the calculated frequency of the random combination of this pair, were used to indicate the relatedness of certain terms with certain GO node. These LOD scores were found to be correlative with the accuracy of GO prediction, as shown in Figure 5. Text information from titles of MEDLINE records appeared to have more predictive power, in particular at lower LOD scores, than text information from other categories. This suggests that the title tended to summarize the gist of an article in a straightforward manner. MeSH terms had similar predictive capabilities as the abstracts, possibly because the MeSH terms were derived from the abstracts, and thus had similar information contents.

Based on text information, a significant number of proteins were predicted to be associated with one or more GO nodes. Table 8 below, lists the number of proteins with predicted GO node from four types of text information in the three categories of GO. These predicted GO annotations were incorporated in GO process to increase the accuracy of homology-based GO annotation and to generate de novo annotations.

**Table 8**

|  | MeSH term | Title | Abstract | Definition Line | Total |
|---|---|---|---|---|---|
| Cellular Component | 57845 | 52094 | 57597 | 514191 | 521396 |
| Molecular Function | 57845 | 54152 | 57632 | 516319 | 523384 |
| Biological Process | 57845 | 53970 | 57631 | 516402 | 523385 |

To further enhance the accuracy and coverage of GO annotation process, a computational platform for predicting cellular localization, ProLoc (Einat Hazkani-Covo, Erez Levanon, Galit Rotman, Dan Graur and Amit Novik, Supra) was used to predict the cellular localization of individual proteins based on their inherent features such as specific localization signatures, protein domains, amino acid composition, pI, and protein length. Only protein sequences that begin with methionine underwent ProLoc analysis. Thus, 88997 out of 93110 proteins in SwissProt version 39 were analyzed, and 78111 proteins have one to three GO predictions in cellular component category.

## EXAMPLE 8

### Gene ontology assignment

Progressive single-linkage clusters with 1 % resolution were generated to assign GO annotations (i.e., nodes) to proteins (see Example 6). Protein clustering and annotation assignment were effected at each level of homology. The resolution was 1 % for global alignment identity (i.e., clustering was first effected at 98 %, then at 97 % and so forth). The resolution was 10 fold for the E score of a BlastP homology pair. For example, clustering was performed at $10^{-50}$, then at $10^{-40}$ and so forth.

To examine clustering efficiency and homology transitivity, all homology pairs clustered with at least 90 % identity were examined. At this level, there were a total of 57,004 clusters containing 263,259 protein members. Among these clusters, 23,231 clusters contained at least three protein members (see Figures 6a-c). The lowest homology pairs had an identity of 46 % while being clustered at 90 % or higher identity levels.

Clusters containing proteins with preassociated or predicted ontological annotations were analyzed and best annotations for individual proteins in the clusters were selected through an error weight calculation. Table 9 below, provides statistics on the number of input gene ontology annotations and the number of output annotations following processing.

**Table 9**

|  | Input | | Output |
|---|---|---|---|
|  | GO Consortium annotation, Enzyme conversion, InterPro mapping, etc. | Text mining ProLoc |  |
| Cellular Component | 44702 | 522179 | 574607 |
| Molecular Function | 85626 | 526083 | 580767 |
| Biological Process | 69726 | 525842 | 578636 |

Over 85 % of proteins were annotated with one or more GO nodes in each of three GO categories. Table 10 below, analyses the number of proteins annotated at different homology levels, showing that GO annotations were achieved throughout the homology spectra.

**Table 10**

|  | Cellular Component | Molecular Function | Biological Process |
|---|---|---|---|
| Text | 32257 | 34137 | 30149 |

(continued)

| | Cellular Component | Molecular Function | Biological Process |
|---|---|---|---|
| $10^{-2}$ - $10^{-10}$ | 87967 | 71717 | 74277 |
| $10^{-10}$ - $10^{-50}$ | 122992 | 70088 | 79318 |
| $10^{-50}$ - 0.0 | 98059 | 55132 | 59051 |
| 35 % - 75 % | 111130 | 97209 | 108334 |
| 75 % - 90 % | 38509 | 68282 | 67429 |
| 90 % - 99% | 38991 | 98576 | 90352 |
| Input GO | 44702 | 85626 | 69726 |

## EXAMPLE 9

### Statistical validation of ontological annotations

Gene ontology annotations, which were assigned according to the teachings of the present invention, were assessed by automatic cross-validation. One fifth of input of input GO annotations were withheld during the GO annotation process and the resultant annotations were compared with the withheld GO nodes. For each protein, the GO node with the lowest error score was examined. Table 11 below, lists the coverage and accuracy of such representative test.

**Table 11**

| | Total | Predicted GO | Accurate GO |
|---|---|---|---|
| Cellular Component | 7431 | 7186 | 4642 |
| Molecular Function | 12999 | 12864 | 10138 |
| Biological Process | 10811 | 10690 | 8080 |

Evidently, sample coverage ranged from 96 % to 99 % and the reproducibility was between 65 % and 80 %. The lower reproducibility of GO annotations in the "cellular component" category, as compared with that in the other two GO categories was consistent with the notion that a short amino acid segment such as a signal peptide affects significantly protein localization. The presence or absence of such small amino acid segments could not be completely captured in sequence similarity comparisons. Detailed analysis of the validation of data indicated that the accuracy of the annotations correlated with the homology levels (data not shown). Manual validation of assigned annotations was performed on a total of 500 annotations and about 85 % - 93 % of annotations were found to be correct. The higher percentage of accuracy in the manual examination over the automatic cross-validation resulted from the incomplete annotation of input GO.

## EXAMPLE 10

### Description of data

Example 10a-e below describe the data table in "Summary_table" file, on the attached CD-ROM3. The data table shows a collection of annotations of biomolecular sequences, which were identified according to the teachings of the present invention.

Each feature in the data table is identified by "#".

Each transcript in the data table is identified by:

(i) A Serial number, e.g. *"251470"* in Example 10a, *"445259"- "445262"* in Example 10b. I
(ii) An internal arbitrary transcript accession number, e.g. "N62228_4" in Example 10a, *"BE674469_0",*
*"BE674469_0_124", "BE674469_1", "BE674469_1_124"* in Example 10b.

The first number of the internal transcript accession number is shared by all transcripts which belong to the same contig, and represent alternatively spliced variants of each other, e.g. *"BE674469"* in *"BE674469_0",*

*"BE674469_0_124", "BE674469_1", "BE674469_1_124"* in Example 10b.

The second number of the internal transcript accession number is an internal serial transcript number of a specific contig, e.g. *"_0"* or *"_1" "* in *"BE674469_0", "BE674469_0_124", "BE674469_1", "BE674469_1_124"* in Example 10b.

The third number of the internal transcript accession number is optional, and represents the GenBank database version used for clustering , assembly and annotation processes. Unless otherwise mentioned, GenBank database version 126 was used. *"124"* indicates the use of GenBank version 124, as in *"BE674469_1_124"* of Example 10b.

"ProDG" following the internal accession number indicates an EST sequence data from a proprietary source, e.g., Examples 3d and 3e.

"han" represents the use of GenBank version 125. This version was used in the annotation of lung and colon cancer specific expressed sequences.

"lab" indicates expressed sequences which differential pattern of expression has been confirmed in the laboratory.

Transcript accession number identifies each sequence in the nucleotide sequence data files "Transcripts_nucleotide_seqs_part1", "Transeripts_nucleotide_seqs_part2", "Transcripts_nucleotide_segs_part3" and "Transcripts_nucleotide_seqs_part4" on CD-ROMs 1 and 2, and in the respective amino acid sequences data file "Protein.seqs" on CD-ROM2. Of note, some nucleotide sequence data files of the above, do not have respective amino acid sequences in the amino acid sequence file "Protein.seqs" attached on CD-ROM2.

Additional lines of the file contain the following information:

"*" indicates optional fields; "**" indicates repeatable features.

**"#EST"** represents a list of GenBank accession numbers of all expressed sequences (ESTs and RNAs) clustered to a contig, from which a respective transcript is derived. The GenBank accession numbers of these expressed sequences are listed only for the first transcript in the contig, e.g. *"#EST BC006216,BE674469,BE798748,NM032716"* in Example 10b. The rest of the transcripts derived from the same contig, are indicated by an #EST field marked with *"the same".*

Expressed sequences, marked with *"ProDGyXXX",* e.g., *"ProDGy933"* in Example 10d, and expressed sequences, marked with *"GeneID XXX",* e.g., *"GeneID1007Forward"* in Example 10e, are proprietary sequences which do not appear in GenBank database. These sequences are deposited in the nucleotide sequence file "ProDG_seqs" in the attached CD-ROM2.

Data pertaining to differentially expressed alternatively spliced sequences is presented in the following format:

*, ** **"#TAA_CD"** represents the coordinates of the differentially expressed sequence segment. A single number represents a differentially expressed edge, corresponding to the specific junction between 2 exons. "TAA_CD" represented by a pair of numbers represents the start and end positions of a differentially expressed sequence node. For example, *"#TAA_CD 269 296"* in Example 10a indicates that the transcript identified as N62228_4 contains a differentially expressed segment, located between the nucleotides at positions 269 and 296.

*, ** **"#TAA_TIS"** contains information pertaining to specific tissue(s), in which the respective transcript is predicted to be expressed differentially. Tumor tissues are indicated accordingly. For example, *"#TAA_TIS lung Tumor"* indicates that transcript BE674469_0 in Example 10b is predicted to be differentially expressed in lung tumor tissues.

*, ** **"#DN"**. represents information pertaining to transcripts, which contain altered functional interpro domains. The Interpro domain is either lacking in this protein (as compared to another expression product of the gene) or scored low (i.e., includes sequence alteration within the domain when compared to another expression product of the gene). This domain alteration can have a functional consequence in which the altered protein product can either gain a function, lose of function (e.g., acting, at times, as dominant negative, inhibitor of the respective protein) or obtain a function which is different than that of the wild-type protein, as described hereinabove (see the definition for "functionally altered biomolecular sequences" in the Terminology section).

This field lists the description of the functional domain(s), which is altered in the respective splice variants e.g., *"#DN EGF-like domain"* in Example 10a.

Functional annotations of transcripts based on Gene Ontology (GO) are indicated by the following format.

*, ** **"#GO_P",** annotations related to Biological Process,

*, ** **"#GO_F",** annotations related to Molecular Function, and

*, ** **"#GO_C'**,annotations related to Cellular Component.

For each category the following features are optionally addressed:

**"#GOPR"** represents internal arbitrary accession number of the predicted protein corresponding to the functionally annotated transcript. This internal accession number identifies the protein in the amino acid sequence file "Protein.seqs" in the attached CD-ROM2, together with the internal arbitrary transcript accession number. For example, *"#GOPR human_281192"* in Example 10a, is a protein sequence encoded by transcript N62228_4, which appears in the amino acid sequence file "Protein.seqs" in the attached CD-ROM2 and is identified by both numbers, "N62228_4" and "human_281192".

**"#GO_Acc"** represents the accession number of the assigned GO entry, corresponding to the following "#GO_Desc" field.

**"#GO_Desc"** represents the description of the assigned GO entry, corresponding to the mentioned "#GO_Acc" field. For example, *"#GO_Acc 7165 #GO_Desc signal transduction"* in Example 10a, means that the respective transcript is assigned to GO entry number 7165, corresponding to signal transduction pathway.

**"#CL"** represents the confidence level of the GO assignment, when #CL1 is the highest and #CL5 is the lowest possible confidence level.

**"#DB"** marks the database on which the GO assignment relies on. The "sp", as in Example 10a, relates to SwissProt Protein knowledgebase, available from http://www.expasy.ch/sprot/. "InterPro", as in Example 10c, refers to the InterPro combined database, available from http://www.ebi.ac.uk/interpro/, which contains information regarding protein families, collected from the following databases: SwissProt (http://www.ebi.ac.uk/swissprot/), Prosite (http://www.expasy.ch/prosite/), Pfam (http://www.sanger.ac.uk/Soflware/Pfam/), Prints (http://www.bioinf.man.ac.uk/dbbrowser/PRINTS/), Prodom (http://prodes.toulouse.inra.fr/prodom/), Smart (http://smart.embl-heidelberg.de/ ) and Tigrfams (http://www.tigr.org/TIGRFAMs/).

"#EN" represents the accession of the entity in the database(#DB), corresponding to the best hit of the predicted protein. For example, *"#DB sp #EN NRG2_HUMAN"* in Example 10a means that the GO assignment in this case was based on SwissProt database, while the closest homologue to the assigned protein is depicted in SwissProt entry **"NRG2_HUMAN"**, corresponding to protein named "Pro-neuregulin-2" (http://www.expasy.org/.cgi-bin/niceprot.pl?O14511). *"#DB interpro #EN IPR001609"* in Example 10c means that GO assignment in this case was based on InterPro database, while the best hit of the assigned protein is to protein family depicted in SwissProt accession number *"IPR001609",* corresponding to "Myosin head (motor domain)" protein family (http://www.ebi.ac.uk/interpro/IEntry?ac=IPR001609).

The following two fields correspond to the hierarchical assignment of the differentially expressed sequences to a specific tissue(s), based on the EST content and EST libraries' origin within the contig.

\*, \*\* **"#SA"** indicates that tissue assignment requires a contig, containing at least 3 ESTs, where at least 80 % thereof are assigned to a selected tissue.

\*, \*\* **"#RA"** indicates that tissue assignment requires a contig derived from at least two different EST libraries, originally constructed from a specific tissue.

### Example 10a

251470 N62228_4 #EST the_same #TAA_CD 269 296 #TAA_TIS ovary , #TAA_CD 269 296 #TAA_TIS ovary Tumor, #TAA_CD 269 296 #TAA_TIS skin Tumor, #TAA_CD 59 269 #TAA_TIS ovary , #TAA_CD 59 269 #TAA_TIS ovary Tumor, #TAA_CD 59 269 #TAA_TIS skin Tumor #DN EGF-like domain #GO_F #GOPR human_281192 #GO_Acc 3823 #GO_Desc antibody #CL 2 #DB sp
#EN NRG2_HUMAN #GO_P #GOPR human_281192 #GO_Acc 7165 #GO_Desc signal transduction #CL 2 #DB sp
#EN NRG2_HUMAN

### Example 10b

445259 BE674469_0 #EST BC006216,BE674469,BE798748,NM032716 #TAA_CD 0 2537 #TAA_TIS lung,
#TAA_CD 0 2537 #TAA_TIS lung Tumor
445260 BE674469_0_124 #124EST BC006216,BE674469,BE798748,NM_032716 #SA Lung Tumor #RA lung_cancer
445261 BE674469_1 #EST the_same #TAA_CD 0 2537 #TAA_TIS lung , #TAA_CD 0 2537 #TAA_TIS lung Tumor
445262 BE674469_1_124 #124EST BC006216,BE674469,BE798748,NM_032716 #SA Lung Tumor #RA lung_cancer

### Example 10c

314251 HUMM7BA_0  #EST  BF804381,BF805793,BF805830,BG978076,HUMM7BA  #GO_C #GOPR human_313276 #GO_Acc 16459 #GO_Desc myosin #CL 2 #DB interpro #EN IPR001609 #GO_F #GOPR human_313281 #GO_Acc 3774 #GO_Desc motor #CL 1 #DB sp #EN Q14786 #GO_F #GOPR human_313281 #GO_Acc 5524 #GO_Desc ATP binding #CL 1 #DB sp #EN Q14786 #GO_P #GOPR human_313281 #GO_Acc 5983 #GO_Desc starch catabolism #CL 4 #DB sp #EN Q14786 #SA colon, colonic, gut #RA colon_normal

### Example 10d

723873 AA157684_TO_ProDG #EST
AA157684,AA157764,AK057980,BF355351,ProDGy933
#GO_C #GO_Acc 0016021 #GO_Desc "integral membrane protein" #GO_F
#GO_Acc 0005978 #GO_Desc "glycogen biosynthesis" #GO_P #GO_Acc 0003707 #GO_Desc "steroid hormone receptor"

### Example 10e

723928 GeneID1007Forward_T0_ProDG #EST
AC018755CDS1,AC018755mRNA1,AW403840,AY040820CDS0,BF359557,
BF896787,BF898989,BF899932,BF900235,BF905509,BI518761,BI756629,BI822428,BI
906477,BI906754,BM550096,BM922784,GeneII71007Forward,Gene1D285Forward,ProD Gy1006 #GO_C #GO_Acc 0005887 #GO_Desc "integral plasma membrane protein" #GO_F #GO_Acc 0007267 #GO_Desc "cell-cell signaling"#GO_P #GO_Acc 0005530 #GO_Desc "lectin"

### EXAMPLE 11

### Description of the sequence files on the enclosed CD-ROMs 1-2

The sequences in the sequence files "Transcripts_nucleotide_seqs_part1", "Transcripts_nucleotide_seqs_part2", "Transcripts_nucleotide_seqs_part3", "Transcripts_nucleotide_seqs_part4", "protein_seqs", "ProDG_seqs" of the enclosed CD_ROMs 1-2 are in FastA text format. Each transcript sequence starts with ">" mark, followed by the transcript internal accession number. The proprietary ProDG EST sequences starts with ">" mark, followed by the internal sequence accession. An example of the sequence file is presented below.

### Example 11a

>R42278_0 (SEQ ID NO: 41)

TGTTTTAGAAATCTCATGATTCCCAGGAAAAAAATTTTAAATTGTGATACAGG
TTTGACAGCCTTTTAGTCAAATAAGTTAAAACACACACGCAAACTCATTTACT
CACTTTGCCATTATAATTCAATCACAAAGAAATTTTGGCCAGGCGTGGTGGTT
ACGCCTGTAATCCCAGCACTTTGGGAGGCCGAGGCAGGTGGATCACGAGGTC
AGGGGATCAAGATCATCCTGGCTAACATGTGAAACCCCGTCTCTATTAAAAAT
AAAAAATTAGCCTGGTGTGGTGGCGGGTGCCTGTAGTCCCAGCTACTCGGGAG
GCTGAGGCAGCAGAATGGCGTGAACTCAGGAGGCGGAGCTTGCAGTGAGCCG
AGATCGCGCCACTGCACTCCAGCCTGGATGACAGAGCGAGACTCCATCTCAAA
AAAAAAA

*Example 11b*

>GeneID3Reverse #TY RNA #DE ProDGy sequence #DT 18-JUN-1000 #DR 5 #LN 348 (SEQ ID NO: 1)

GTGGTTATTACAGCATGGTTCCCAGCCTTACAGTGTCTAAGTGCTTCTCTTGTG
TCCTGTAGATGTTGTGAAAAAGAAAAAAACAAAAAATACACCACACTGTACTT
TTTCCCCCTGCCCCCGTTACTGCCGGTGATTATTATTAAAAATTAGTTTTTTTCA
CATCATTATATCTGGCTTCCTATAAACAACAGCCTTAATTCAGTCAAGACTCCC
TTTGGGGAATTCATTTTATTAAAAATTGGTGTCTGGATACTTCCCTGTACATGC
ATAAATATGCATGCATGTACAGAAGACTGTATGTGTGTGCCTTGCACACACA
CCCATACCTCTCAGAAAAAGTGTTT

*Example 11c*

>ProDGy1339 #OS Homo sapiens #DE ProDGy sequence #DT 26-JUL-2002 #TY EST #DR 5 #AC ProDGy1339 #LN 132 (SEQ ID NO: 2)

CAGAAAGCCCAGAGTAGTCCCTGTAAGAAGCTGAGGGGCGCATACCTCTGGG
GTTTGGGTTCCCTTCAGGGAAGCGAAGGGAGATGACCTCTTTCCAGGCTGGGG
ACCAAGAGGGCTCCCTAGAAGATATTA

*EXAMPLE 12*

*Description of CD-ROM1-3 content*

The CD-ROMs enclosed herewith contain the following files:

*CD-ROM1 (2 files):*

1. "Transcripts_nucleotide_seqs_part1", containing nucleotide sequences of all the transcripts based on genomic production of GenBank version 126.
2. "GC_new.txt", includes a title of the invention and reference numbers.

*CD-ROM2 (4 files):*

1. "Transcripts_nucleotide_seqs_part2", containing nucleotide sequences of all the transcripts based on expressed production of GenBank version 126 (in cases where no genomic data support was available).
2. "Transcripts_nucleotide_seqs_part3.new", containing nucleotide sequences of all the transcripts based on Gen-Bank versions 124, 125, and transcripts containing ProDG proprietary sequences.
3. "Protein.seqs", containing all the amino acid sequences encoded by the transcripts based on GenBank versions 126.
4. "ProDG_seqs", containing the proprietary EST sequences.

5. "Transcripts_nucleotide_seqs_part4", containing nucleotide sequences of transcripts based on GenBank version 131, as described in Example 21.

**CD-ROM3** (*1 file):*

1. "Summary_table", containing all the annotation information, as described in Example 10.

**EXAMPLE 13**

**In-vitro confirmation** *of Differentially Expressed Transcripts Experimental procedures and reagents*

In-vitro confirmation of in-silico obtained differentially expressed polynucleotide sequences was effected utilizing laboratorial methodologies, based on nucleotide hybridization including northern analysis, RT-PCR and real-time PCR.

**RNA preparation** - Total RNA was isolated from the indicated cell lines or tumor tissues using the Tri-Reagent (Molecular Research Center Inc.) following the manufacturer's recommendations. Poly(A) RNA was purified from total RNA using oligo(dT)$_{25}$ Dynabeads (Dynal).

**Northern blotting** - 20 $\mu$g of total RNA or 2$\mu$g of poly(A) RNA were electrophoresed on 1% agarose gels containing formaldehyde, and blotted onto Nytran Super Charge membranes (Shcleicher & Schuell). Hybridization was carried out using a DNA probe (SEQ ID NO: 3) in EZ-Hybridization Solution (Biological Industries, Beit Haemek, Israel) at 68°C for 18 hrs. The membranes were rinsed twice with 2XSSC, 0.1% SDS at room temperature, followed by two washes with 0.1XSSC, 0.1%SDS at 50°C. Autoradiograms were obtained by exposing the membranes to X-ray films.

**RT-PCR analysis** - Prior to RT reactions, total RNA was digested with DNase (DNA-free™, Ambion) in the presence of RNasin. Reverse transcription was carried out on 2 $\mu$g of total RNA, in a 20 $\mu$l reaction; using 2.5 units of Superscript II Reverse Transcriptase (Bibco/BRL) in the buffer supplied by the manufacturer, with 10 pmol of oligo(dT)$_{25}$ (Promega), and 30 units of Rnasin (Promega). RT reactions were standardized by PCR with.GAPDH-specific primers, for 20 cycles. The calibrated reverse transcriptase samples were then analyzed with gene-specific primers either at 35 cycles, or at lower cycles (15 and 20 cycles). PCR products of lower number of cycles were visualized by southern blotting, followed by hybridization with the appropriate probe (the same PCR product).

**Real-Time RT-PCR -** Total RNA samples were treated with Dnasel (Ambion) and purified with Rneasy columns (Qiagen). 2 $\mu$g of treated RNA samples were added into 20 $\mu$l RT-reaction mixture including. RT-PCR end product 200 units SuperscriptII (Invitrogen), 40 units RNasin, and 500pmol oligo dT. All components were incubated for 1 hr at 50°C and then inactivated by incubation for 15 min at 70°C. Amplification products were diluted, 1:20, in water. 5$\mu$l of diluted products were used as templates in Real-Time PCR reactions using specific primers and the intercalating dye Sybr Green.

The amplification stage was effected as follows, 95 °C for 15 sec, 64 °C for 7 sec, 78 °C for 5 sec and 72 °C for 14 sec . Detection was effected using Roch light cycler detector. The cycle in which the reactions achieved a threshold level of fluorescence was registered and served to calculate the initial transcript copy number in the RT reaction. The copy number was calculated using a standard curve created using serial dilutions of a purified amplicon product. To minimize inherent differences in the RT reaction, the resulting copy number was normalized to the levels of expression of the housekeeping genes Proteasome 26S subunit (GenBank Accession number D78151) or GADPH (GenBank Accession number: AF261085).

**Semiquantitative PCR -** RT-PCR reaction was performed with sample specific primers, for 16 cycles. PCR products were used as probes. Labeling procedure was carried out using "Random primer DNA labeling mix" according to manufacturer's instructions (Cat. No: 20-101-25). Briefly, 25ng of template DNA were denatured by heating to 100 °C for 5 minutes, and then chilled on ice for 5 minutes. Labeling solution contained 11 $\mu$l of denatured DNA, 4 $\mu$l of labeling mix solution (Biological industries), 5 $\mu$l of $^{32}$(p)dCTP (Amersham, Pharmacia, AA0005). Labeling was effected for 10 minutes in 37°C. Removal of unincorporated nucleotides was effected using Sephadex G-50 columns. Prior to hybridization, labeled DNA was denatured by heating to 100°C for 5 minutes and then rapidly cooled on ice.

**Southern blotting -** PCR products were separated on 1.5 % agarose gel and size separated. The gel was denatured by two consecutive washes for 20 min in 1X denaturation buffer, containing 1.5M NaCl, 0.5M NaOH. Thereafter a neutralization procedure was effected by washing twice for 20 min in 1X neutralization buffer, containing 1.5M Nacl, 0.5m Tris/HCL pH=7.0. Blotting of the denatured DNA to the nylon membrane was performed overnight with 20XSSC. DNA was UV crosslinked (Stratalinker) to a nylon membrane prior to prehybridization step. Prehybridization was performed using EZ-hybridization solution (Biological Industries, Cat no: 01-889-1B) at 68°C for 1 hour. The DNA blot was subjected to Southern hybridization using specific oligonucleotides end-labeled with adenosine 5'-[$\gamma$-$^{32}$P]triphosphate (>5000 Ci/mmol, Amersham Biosciences, Inc.). Hybridization step was effected at 68°C for 16 hours.

Following hybridization the membrane was washed at gradually increasing stringent conditions: twice in 2X SSC, 0.1%SDS, for 15 min. at room temperature and twice in 0.1XSSC, 0.1%SDS, for 15 min, at 6o°C. Radioactive signal was visualized by autoradiography.

## EXAMPLE 14

### Colorectal cancer specific expression of AA535072

AA535072 (SEQ ID NO: 39) is a common sequence feature to a series of overlapping sequences (SEQ ID NOs: 4, 24-28) with predicted amino acid sequences provided in SEQ ID NOs: 35-38.

The indicated tissues and cell lines were examined for AA535072 (SEQ ID NOs: 39) expression by RT-PCR analysis. Primers for AA535072 were GTGACAGCCAGTAGCTGCCATCTC (SEQ ID NO: 5) and TCCGTTTCTAGCGGCCAGACCTTT (SEQ ID NO: 6). PCR reactions were denatured at 94 °C for 2 minutes followed by 35 cycles at 94 °C for 30 sec, 64 °C for 30 sec and 72 °C for 60 sec. All PCR products were separated on an ethidium bromide stained gel.

As shown in Figure 7 amplification yielded a major PCR product of 1000 bp. Evidently, AA535072 expression was limited to colorectal cancer tissues; adenocarcinoma, colon carcinoma cell line and colon carcinoma Duke A cells. Since colon carcinoma Duke A cells represent an early stage of colon cancer progression, differentially expressed AA535072 can be used as a putative marker of polyps and benign stages of colon cancer. Furthermore, corresponding protein products (SEQ ID NOs: 35-38) may be utilized as important colon cancer specific diagnostic and prognostic tools.

## EXAMPLE 15

### Bone Tumor Ewing's Sarcoma Specific expression of AA513157 (SEQ ID NO: 7)

The indicated tissues and cell lines were examined for AA513157 (SEQ ID NO: 7) expression by RT-PCR analysis. Primers for SEQ ID NO: 7 were GAAGGCAGGCGGATGCTACC (SEQ ID NO: 8) and AGCCTTCCACGCTGTACACGCCA (SEQ ID NO: 9). PCR reactions were denatured at 94 °C for 2 minutes followed by 35 cycles at 94 °C for 30 sec, 64 °C for 30 sec and 72 °C for 45 sec. All PCR products were separated on an ethidium bromide stained gel.

As shown in Figure 8, amplification reaction yielded a specific PCR product of 600 bp. As shown in Figure 8, in the presence of reverse transcriptase (indicated by +) high expression of AA513157 was evident in both samples of Ewing sarcoma , while only residual expression of AA513157 was seen in Ln-Cap cells, brain and splenic adenocarcinoma.

To substantiate these, Northern blot analysis of AA513157 was effected. The following primers were used, GAAGGCAGGCTGGATGCTACC (SEQ ID NO: 10), GGTAGTATAACCGGGCTCTGT (SEQ ID NO: 11). Figure 9 illustrates RNA expression of AA513157 in various tissues. Several transcripts were evident upon Northern analysis: two major transcripts of 800 bp and 1800 bp from ployA RNA preparation and total RNA preparation, respectively. Expression of both transcripts was limited to the Ewing sarcoma cell line. Low expression of the 1800 bp transcript was evident in Bone Ewing sarcoma tissue as well.

These results corroborate AA513157 as a putative Ewing sarcoma marker and a putative pharmaceutical target.

## EXAMPLE 16

### Colorectal cancer specific expression of AA469088

AA469088 (SEQ ID NO: 40) is a common sequence feature to a series of overlapping sequences (SEQ ID NOs: 12 and 29-31).

The indicated tissues and cell lines were examined for AA469088 (SEQ ID NO: 40) expression by semi quantitative RT-PCR analysis. Primers for AA469088 were CATATTTCACTCTGTTCTCTCACC (SEQ ID NO: 13) and CAGAATGGGATTATGGTAGTCTATCT (SEQ ID NO: 14). PCR reactions were effected as follows: 14 cycles at 92 °C for 20 sec, 59 °C for 30 sec and 68 °C for 45 sec. The PCR products were size separated on agarose 1.5 % gel, and undergone Southern blot analysis using the PCR products as specific probe, as described in details in Example 13. The visualization of the hybridization signal of the PCR products was performed by autoradiogram exposure to X-ray film. As shown in Figure 10 amplification reaction yielded a major PCR product of 484 bp. Evidently, AA469088 expression was limited to colorectal tumor tissues, normal colon and adenocarcinoma with only minor expression in the spleen and kidney.

## EXAMPLE 17

### HUMMCDR - A lung cancer specific marker

Real-time quantitative RT-PCR was used to measure the mRNA steady state levels of HUMMCDR (SEQ ID NO: 15). The following primers were used CTTCAATTGGATTATGTTGACCTCTAC (SEQ ID NO: 16) and CACTATAGGCAACCAGAACAATGTC (SEQ ID NO: 17).
Real-time PCR analysis (Figure 11) indicates that SEQ ID NO: 15 is specifically expressed in lung squamous cell carcinoma with an evident 2-10 fold higher expression than in normal lung samples.

## EXAMPLE 18

### SEQ ID NO: 18-A lung cancer specific transcript

Real-time quantitative RT-PCR was used to measure the mRNA steady state levels of SEQ ID NO: 18. The following primers were used GCGAGGACCGGGTATAAGAAGC (SEQ ID NO: 19) and TCGGCTCAGCCAAACACTGTCAG (SEQ ID NO: 20).
Real-time PCR analysis indicates that SEQ ID NO: 18 is specifically expressed in lung adenocarcinoma samples and in lung alveolus cell carcinoma (Figure 13).

## EXAMPLE 19

### SEQ ID NO: 21-A lung cancer specific transcript

Real-time quantitative RT-PCR was used to measure the mRNA steady state levels of SEQ ID NO: 21. The following primers were used GCTTCGACCGGCTTAGAACT (SEQ ID NO: 22) and GGTGAGCACGATACGGGC (SEQ ID NO: 23).
Real-time PCR analysis indicates that SEQ ID NO: 21 is specifically expressed in small lung cell carcinoma and in adenocarcinoma (Figure 14).

## EXAMPLE 20

### HSGPGI-A lung cancer specific transcript

Real-time quantitative RT-PCR was used to measure the mRNA steady state levels of HSGPGI (SEQ ID NO: 32). The following primers were used GAGCCCTGTGCGCCGCTCAGATGTG (SEQ ID NO: 33) and AGCCCAAGTTGAATCACCAACCAG (SEQ ID NO: 34).
As shown in Figure 12, real-time PCR analysis exhibited specific expression of SEQ ID NO: 32 in lung adenocarcinoma and lung squamos cell carcinoma, as compared to the expression in normal lung tissue (2-25 fold).

## EXAMPLE 21

### Comparative analysis of human and mouse alternatively spliced exons Rationale and Experimental Procedures

Alternatively spliced internal exons were identified as described hereinabove [Sorek (2002) Genome Res. 12:1060-1067], essentially screening for reliable exons according to canonical splice sites and discarding possible genomic contamination events. A constitutively spliced internal exon was defined as an internal exon when supported by at least 4 sequences, for which no alternative splicing was observed. Alternatively, a spliced internal exon was defined as such if there was at least one sequence that contained both the internal exon and the 2 flanking exons (exon inclusion), and at least one sequence which contained the two flanking exons without the middle one (exon skipping).
To identify exons, which are conserved in mice, mouse ESTs (from GenBank version 131) were aligned to the human genome using a spliced alignment model, which allows opening of long gaps. Single hits of mouse expressed sequences to the human genome shorter than 20 bases, or having less than 75% identity to the human genome, were discarded.
To determine if the borders of a human intron, which define the borders of the flanking exons, were conserved in mice, a mouse EST spanning the same intron-borders, while aligned to the human genome, was sought. Only mouse EST sequences which exhibited alignment of at least 25 bp on each side of the exon-exon junction were used. In addition, this mouse EST was sought to span an intron (i.e., open a long gap) at the same position along the EST, when aligned to the mouse genome.

A human exon-skipping was considered "conserved" in mice if both splice variants i.e., the variant that skips the exon and the variant that contains the exon, were supported by mouse ESTs.

As shown in Figures 15a-b, in 149 exon-skipping events, both variants were found in mouse ESTs. It will be appreciated though that when the variant which contains the alternatively spliced exon is a rare variant, or a variant unique to a tissue which is not represented in mouse EST libraries, there may be no mouse EST, which supports such an event. Nevertheless, if the human exon were really conserved in the mouse transcriptome, it would be expected that the DNA genomic sequence would be conserved between the species.

The basic assumption is that although exons are conserved between the human and mouse genomes to an average level of 85%, introns are conserved to a much lower extend. Therefore, in cases where there was a skipping variant evident in the mouse transcriptome, but there was no mouse EST showing the variant that contains the exon, the sequence of the human exon was aligned to the relevant intron in the mouse genome. The exon was declared conserved when the following terms were achieved (i) a significant conservation above 80% identity was found, (ii) the alignment spanned the full length of the human exon; and (iii) the exon was flanked by the canonical AG/GT acceptor and donor sites in the mouse genome. Using this approach 94 additional exon-skipping events conserved between human and mouse were identified (see Figures 15a-c).

*Results*

Two exon sequence sets are available (compiled from Gencarta 3.2, gb131):

1. 243 alternatively spliced exons that are conserved between human and mouse and are therefore probably functional for the normal organisms' life. The sequences are described by serial number 725901-7261386 in the attached "Summary_table" on CD-ROM3 and listed in the "Transcripts_nucleotide_seqs_part4" file of the attached CD-ROM2.

2. 737 alternatively spliced exons for which no conservation was detected. These sequences can represent alternative splicing which is unique to humans and therefore may define human-specific characteristics. Alternatively, there sequences can represent aberrant splicing which causes or is caused by pathological states. The sequences are described by serial number 726387-727860 in the attached "Summary_table" of CD-ROM3 and listed in the "Transcripts_nucleotide_seqs_part4" file of the attached CD-ROM2)

For both sequence sets, each alternative splicing is represented by two transcripts, the first represents the variant that skips the alternatively spliced exon and the second represents the variant that contains the exon. Example for the documentation is illustrated hereinunder.

#TRS_SKIP AA325140_0_8 #SKIP BM721749 #RETENT AW583868 #MOUSE_SKIP BC006836 BE226286 BG916003 B1738124 BI854105 BI905506
#MOUSE_RET AA116659 AF026259 AI326485 AW320664 BE569976 BG866594 BI412163 BI655027 BI689102 BI689989 BI695087 BI904805 MUSCAK NM007584
#TRS_RETENT AA325140_1_8 #SKIP BM721749 #RETENT AW583868 #MOUSE_SKIP BC006836 BE226286 BG916003 BI738124 BI854105 BI905506
#MOUSE_RET AA116659 AF026259 AI326485 AW320664 BE569976 BG866594 BI412163 BI655027 BI689102 BI689989 BI695087 BI904805 MUSCAK NM007584
#TRS_SKIP - indicates if this transcript represents a skipping variant or a retention variant, which includes the exon.
AA325140_0_8 - (contig_name)_(0 or 1, where 0 is the skipping transcript and 1 is the retention one)_(number of node which represents the exon)
#SKIP - list of human sequences which skip the exon, i.e., match to the "#TRS_SKIP" transcript.
#RETENT - list of human sequences which contain the exon, i.e., match to the "#TRS_RETENT" transcript.
#MOUSE_SKIP - list of mouse sequences which skip the exon.
#MOUSE_RET - list of mouse sequences which contain the exon.

*EXAMPLE 22*

*Description of data*

Following is a description of the data table in "Annotations.gz" file, on the attached CD-ROM4. The data table shows a collection of annotations for biomolecular sequences, which were identified according to the teachings of the present invention using transcript data based on GenBank versions 136 (June 15, 2003 ftp://ftp.ncbi.nih.gov/genbank/release.notes/gb136.release.notes) and NCBI genome assembly of April 2003. Each feature in the data table is identified by "#".

#INDICATION - This field designates the indications (i.e., diseases, disorders, pathological conditions) and therapies that the polypeptide of the present invention can be utilized for. Specifically, an indication lists the disorders or diseases in which the polypeptide of the present invention can be clinically used. A therapy describes a postulated mode of action of the polypeptide for the above-mentioned indication. For example, an indication can be "Cancer, general" while the therapy will be "Anticancer".

Each Protein of the present invention was assigned a SwissProt/TrEMBL human protein accession as described in section "Assignment of SwissProt/ TrEMBL accessions to Gencarta contigs" hereinbelow. The information contained in this field is the indication concatenated to the therapies that were accumulated for the SwissProt and/or TrEMBL human protein from drug databases, such as PharmaProject (PJB Publications Ltd 2003 http://www.pjbpubs.com/cms.asp?pageid=340) and public databases, such as LocusLink (http://www.genely,nx.org/cgi-bin/resource?res=locuslink) and Swissprot (http://www.ebi.ac.uk/swissprot/index.html). The field may includes more than one term wherein a ";" separates each adjacent terms.

Example- #INDICATION Alopecia, general; Antianginal; Anticancer, immunological; Anticancer, other; Atherosclerosis; Buerger's syndrome; Cancer, general; Cancer, head and neck; Cancer, renal; Cardiovascular; Cirrhosis, hepatic; Cognition enhancer; Dermatological; Fibrosis, pulmonary; Gene therapy; Hepatic dysfunction, general; Hepatoprotective; Hypolipaemic/Antiatherosclerosis; Infarction, cerebral; Neuroprotective; Ophthalmological; Peripheral vascular disease; Radio/chemoprotective; Recombinant growth factor; Respiratory; Retinopathy, diabetic; Symptomatic antidiabetic; Urological;

Assignment of SwissProt/TrEMBL accessions to Gencarta contigs - Gencarta contigs were assigned a Swissprot/TremB1 human accession as follows. SwissProt/TrEMBL data (SwissProt version 41.13 June 2003, TrEMBL and TrEMBL _new version 23.17 June 2003) were parsed and for each Swissprot/TremB1 accession (excluding Swissprot/TremB1 that are annotated as partial or fragment proteins) cross-references to EMBL and Genbank were obtained. The alignment quality of the SwissProt/TrEMBL protein to their assigned mRNA sequences was checked by frame+p2n alignment analysis. A good alignment was considered as having the following properties:

• For partial mRNAs (those that in the mRNA description have the phrase "partial cds" or annotated as "3'" or "5'")- an overall identity of 97% and coverage of 80 % of the Swissprot/TremB1 protein.
• All the rest mRNA sequences were considered as fully coding mRNAs and for them an overall identity of 97% identity and coverage of the SwissProt/TrEMBL protein of over 95 %.

The mRNAs were searched in the LEADS database for their corresponding contigs, and the contigs that included these mRNA sequences were assigned the Swissprot/TremB1 accession.

#PHARM- This field indicates possible pharmacological activities of the polypeptide. Each polypeptide was assigned with a SwissProt and/or TrEMBL human protein accession, as described above. The information contained in this field is the proposed pharmacological activity that was associated to the SwissProt and/or TrEMBL human protein from drug databases such as PharmaProject (PJB Publications Ltd 2003 http://www.pjbpubs.com/cms.asp?pageid=340) and public databases, such as LocusLink and Swissprot. Note that in some cases this field can include opposite terms in cases where the protein can have contradicting activities - such as:

1. Stimulant - inhibitor
2. Agonist - antagonist
3. Activator- inhibitor
4. Immunosuppressant - Immunostimulant

In these cases the pharmacology was indicated as "modulator". For example, if the predicted polypeptide has potential agonistic/antagonistic effects (e.g. Fibroblast growth factor agonist and Fibroblast growth factor antagonist) then the annotation for this code will be "Fibroblast growth factor modulator".

A documented example for such contradicting activities has been described for the soluble tumor necrosis factor receptors [Mohler et al., J. Immunology 151, 1548-1561]. Essentially, Mohler and co-workers showed that soluble receptor can act as a carrier of TNF (i.e., agonistic effect) and as an antagonist of TNFR activity.

#THERAPEUTIC_PROTEIN - This field predicts a therapeutic role for a protein represented by the contig. A contig was assigned this field if there was information in the drug database or the public databases (e.g., described hereinabove) that this protein, or part thereof, is used or can be used as a drug. This field is accompanied by the SwissProtaccession of the therapeutic protein, which this contig most likely represents. Example: # THERAPEUTIC_PROTEIN UROK_HUMAN

#SEQLIST- This field lists all ESTs and/or mRNA sequences supporting the transcript and the predicted protein derived from Genbank version 136 (June 15 2003 ftp://ftp.ncbi.nih.gov/genbank/release.notes/gb136.release.notes).

These sequences are the sequences which encompass the transcript. For example: BX394917 BX327693 AA894600 AA032291 AK027130 BM665029 BC025257 BE785231 BX371447 BX371446 BG821626 BX394918 BE737007 BE737043 AF213678 AB038318 AB038317 BE315017

#DN represents information pertaining to transcripts, which contain altered functional interpro domains. The Interpro domain is either lacking in this protein (as compared to another expression product of the gene) or scored low (i.e., includes sequence alteration within the domain when compared to another expression product of the gene). This field lists the description of the functional domain(s), which is altered in the respective splice variants.

Method: all proteins in a contig were analysed through BLASTP analysis against each other. All proteins were also analysed by Interpro domain analysis software (Interpro default parameters, the analyses that were run are HMMPfam, HMMSmart, ProfileScan, FprintScan, and BlastProdom). Each pair of proteins that shared at least 20 % coverage of one or the other with an identity of at least 80 % were analysed by domain comparison. If the proteins share a common domain (i.e., same domain accession) and in one of the proteins this domain has a decreased score (escore of 20 magnitude for HMMPfam, HMMSmart, BlastProdom, FprintScan or Pscore difference of ProfileScan of 5), or lacking a domain contained in another protein of the same contig, the protein with the reduced score or without the domain is annotated as having lost of this interpro domain. This domain alteration can have a functional consequence in which the altered protein product can either gain a function, lose of function (e.g., acting, at times, as dominant negative inhibitor of the respective protein) or obtain a function which is different than that of the wild-type protein, as described hereinabove (see the definition for "functionally altered biomolecular sequences" in the Terminology section). Interpro domains, which have no functional attributes were omitted from this analysis. The domains that were omitted are:

IPR000694 Proline-rich region
IPR001611 Leucine-rich repeat
IPR001893 Cysteine rich repeat
IPR000372 Cysteine-rich flanking region, N-terminal
IPR000483 Cysteine-rich flanking region, C-terminal
IPR003591 Leucine-rich repeat, typical subtype
IPR003885 Leucine-rich repeat, cysteine-containing type
IPR006461 Uncharacterized Cys-rich domain
IPR006553 Leucine-rich repeat, cysteine-containing subtype
IPR007089 Leucine-rich repeat, cysteine-containing

The results of this analysis are denoted in terms of the Interpro domain that is missing or altered in the protein. Example: #DN IPR002110 Ankyrin.

#SECRETED_FORM_OF_MEMBRANAL_PROTEINS_(BY_PROLOC/BY_SW ISSPROT) - This field indicates if a protein of the present invention is a secreted form of a membrane-bound protein. To uncover this, two different methods were applied:

Method1: all proteins in a contig were analyzed by BLASTP analysis against each other. The Proloc algorithm was applied to all the proteins. Each pair of proteins that shared at least 20 % coverage of one or the other with an identity of at least 80 % was further examined. A protein was considered a soluble form of a membrane protein (i.e., cognate protein) if it was shown to be a secreted protein (as further described below) while the cognate partner was a membrane-bound protein.

A protein was considered secreted or extracellular if it had at least one of the following properties.

(i) Proloc's highest subcellular localization prediction is EXTRACELLULAR.
(ii) Proloc's prediction of a signal peptide sequence is more reliable than the prediction of a lack of signal peptide sequence. Furthermore, no transmembrane regions are predicted in the non N-terminus part of the protein (following 30 N-terminal amino acids)
(iii) Proloc's prediction of only one transmembrane domain, which is localized to the N-terminus part of the protein (in a region less than the first 30 amino acids)

The cognate protein was considered to be a membrane-bound protein if it obeyed at least one of the following rules:

(i) Proloc's highest subcellular localization prediction is either CELL_INTEGRAL_MEMBRANE, CELL_MEMBRANE_ANCHORL or CELL_MEMBRANE_ANCHORII.
(ii) Proloc's prediction of at least one transmembrane domain which is not in the N-terminus part of the protein (in a region greater than the first 30 amino acids) The output header of this method is #SECRETED_FORM_OF_MEMBRANNEL_PROTEINS_BY_PROLOC. Example: AA283884_P3 #SECRETED_FORM_OF_MEMBRANAL_PROTEINS_ BY_PROLOC

Method 2: the transcripts were compared to the SwissProt protein representing the Gencarta contig by BLASTP analysis. If the SwissProt protein had an annotation of a signal peptide domain and a transmembrane domain and the protein aligned to the protein in the signal peptide region but not in the transmembrane domain(s), the protein encoded by the transcript was annotated a soluble form of a membrane-bound protein. In this case the header is BY_SWISSPROT (indicating the method).

#MEMBRANE_FORM_OF_SOLUBLE_PROTEINS_BY_PROLOC - This fields denotes if the indicated protein is a membranal form of a secreted protein.

Method: all proteins in a contig were analysed through BLASTP analysis against each other. The Proloc algorithm was applied to all the proteins. Each pair of proteins that shared at least 20 % coverage with an identity of at least 80 % was further examined. A protein was considered a membrane form of a secreted protein if it was shown to be (i.e., annotated) a membrane-bound protein and the other protein it was compared to (i.e., cognate) was a secreted protein.

A protein is annotated membrane-bound if is had at least one of the following properties:

(i) Proloc's highest subcellular localization prediction is either CELL_INTEGRAL_MEMBRANE, CELL_MEMBRANE_ANCHORI, or CELL_MEMBRANE_ANCHORII.

(ii) Proloc's prediction of at least one transmembrane domain which is not in the N-terminus part of the protein (i.e. the transmembrane domain is in a region greater than the first N-terminal 30 amino acids)

The cognate protein is considered secreted if it obeyed at least one of the following rules:

(i) Proloc's highest subcellular localization prediction is EXTRACELLULAR.

(ii) Proloc's prediction of the existence of a signal peptide sequence is more reliable than the prediction of a lack of signal peptide sequence and no transmembrane regions are predicted in the non N-terminus part of the protein (after its N-terminal 30 amino acids)

(iii) Proloc's prediction of only one transmembrane domain which is in the N-terminus part of the protein (in a region less than the N-terminal 30 amino acids).

The annotation will be in the form of this header, for example, AA176800_P7#MEMBRANE_FORM_OF_SOLUBLE_PROTEINS_BY_PROLOC

GO annotations were predicted as described in "The ontological annotation approach" section hereinabove. Additions to the GO prediction, other than the GO engine will be described below. These additions are to the cellular component attribute and biological process.

Functional annotations of transcripts based on Gene Ontology (GO) are indicated by the following format.

**"#GO_P",** annotations related to Biological Process,

**"#GO F",** annotations related to Molecular Function, and

"**#GO_G**",annotations related to Cellular Component.

The Gene Ontology and gene association files were updated using the following databases: SWISS-PROT and TrEMBL release Dec. 18, 2002; Medline databases of April 6, 2001 and the following files from Gene Ontology Consortium, which were downloaded on Oct. 22, 2003: gene_association.fb; gene_association.mgi; gene_association.sgd; gene_association.wb; and gene_association.goa_sptr.

Proloc was used for protein subcellular localization prediction that assigns GO cellular component annotation to the protein. The localization terms were assigned GO entries.

For this assignment two main approaches were used: (i) the presence of known extracellular domain/s in a protein (as appears in Table 12); (ii) calculating putative transmembrane segments, if any, in the protein and calculating 2 p-values for the existence of a signal peptide. The latest is done by a search for a signal peptide at the N-terminal sequence of the protein generating a score. Running the program on real signal peptides and on N-terminal protein sequences that lack a signal peptide resulted in two score distributions the first is the score distribution of the real signal peptides and the second is the score distribution of the N-termmal protein sequences that lack the signal peptide. Given a new protein, ProLoc calculates its score and outputs the percentage of the scores that are higher than the current score, in the first distribution, as a first p-value (lower p-values mean more reliable signal peptide prediction) and the percentage of the scores that are lower than the current score, in the second distribution, as a second p-value (lower p-values mean more reliable non signal peptide prediction)

Assignment of an extracellular localization (#GO_Acc 5576 #GO_Desc extracellular) was also based on Interpro domains. A list of Interpro domains that characterize secreted proteins was compiled A Protein of the present invention that had a hit to at least one of these domains was annotated with an extracellular GO annotation. Interpro indices for secreted domains are listed in Table 12, below

*Table 12*

| | |
|---|---|
| IPR000874 | Bombesin-like peptide |
| IPR001693 | Calcitonin-like |
| IPR001651 | Gastrin/cholecystokinin peptide hormone |
| IPR000532 | Glucagon/GIP/secretin/VIP |
| IPR001545 | Gonadotropin, beta chain |
| IPR004825 | Insulin/IGF/relaxm |
| IPR000663 | Natriuretic peptide |
| IPR001955 | Pancreatic hormone |
| IPR001400 | Somatotropin hormone |
| IPR002040 | Tachykinin/Neurokinin |
| IPR006081 | Alpha defensin |
| IPR001928 | Endothelin-like toxm |
| IPR001415 | Parathyroid hormone |
| IPR001400 | Somatotropm hormone |
| IPR001990 | Chromogranin/secretogranin |
| IPR001819 | Chromogranin A/B |
| IPR002012 | Gonadotropin-reloasing hormone |
| IPR001152 | Thymosin beta-4 |
| IPR000187 | Corticotropin-releasing factor, CRF |
| IPR001545 | Gonadotropin, beta chain |
| IPR000476 | Glycoprotein hormones alpha chain |
| IPR000476 | Glycoprotein hormones alpha chain |
| IPR001323 | Erythronoietin/thrombopoeitin |
| IPR001894 | Cathelicidm |
| IPR001894 | Cathelicidin |
| IPR001483 | Urotensin II |
| IPR006024 | Opioid neuropeptide precursor |
| IPR000020 | Anaphylatoxin/fibulin |
| IPR000074 | Apolipoprotein A1/A4/E |
| IPR001073 | Complement C1q protein |
| IPR000117 | Kappa casein |
| IPR001588 | Casein, alpha/beta |
| IPR001855 | Beta defensin |
| IPR001651 | Gastrin/cholecystokinin peptide hormone |
| IPR000867 | Insulin-like growth factor-binding protein, IGFBP |
| IPR001811 | Small chemokine, interleukin-8 like |
| IPR004825 | Insulin/IGF/relaxin |

(continued)

| IPR002350 | Serine protease inhibitor, Kazal type |
|-----------|---------------------------------------|
| IPR000001 | Kringle |
| IPR002072 | Nerve growth factor |
| IPR001839 | Transforming growth factor beta (TGFb) |
| IPR001111 | Transforming growth factor beta (TGFb), N-terminal |
| IPR001820 | Tissue inhibitor of metalloproteinase |
| IPR000264 | Serum albumin family |
| IPR005817 | Wnt superfamily |

For each category the following features are optionally addressed:

"#GO_Acc" represents the accession number of the assigned GO entry, corresponding to the following "#GO_Desc" field.

"#GO_Desc" represents the description of the assigned GO entry, corresponding to the mentioned "#GO_Acc" field.

The assignment of Immune response GO annotation (#GO_Acc 6955 # GO_Desc immune response) to transcripts and proteins of the present invention was based on a homology to a viral protein, as described in U.S. Pat. Appl. 60/480,752.

"#CL" represents the confidence level of the GO assignment, when #CL1 is the highest and #CL5 is the lowest possible confidence level. This field appears only when the GO assignment is based on a SwissProt/TrEMBL protein accession or Interpro accession and (not on Proloc predictions or viral proteins predictions). Preliminary confidence levels were calculated for all public proteins as follows:

PCL 1: a public protein that has a curated GO annotation,
PCL 2: a public protein that has over 85 % identity to a public protein with a curated GO annotation,
PCL 3: a public protein that exhibits 50 - 85 % identity to a public protein with a curated GO annotation,
PCL 4: a public protein that has under 50 % identity to a public protein with a curated GO annotation.

For each Protein of the present invention a homology search against all public proteins was done. If the Protein of the present invention has over 95 % identity to a public protein with PCL X than the Protein of the present invention gets the same confidence level as the public protein. This confidence level is marked as "#CL X". If the Protein of the present invention has over 85 % identity but not over 95 % to a public protein with PCL X than the Protein of the present invention gets a confidence level lower by 1 than the confidence level of the public protein. If the Protein of the present invention has over 70 % identity but not over 85 % to a public protein with PCL X than the Protein of the present invention gets a confidence level lower by 2 than the confidence level of the public protein. If the Protein of the present invention has over 50 % identity but not over 70 % to a public protein with PCL X than the Protein of the present invention gets a confidence level lower by 3 than the confidence level of the public protein. If the Protein of the present invention has over 30 % identity but not over 50 % to a public protein with PCL X than the Protein of the present invention gets a confidence level lower by 4 than the confidence level of the public protein.

A Protein of the present invention may get confidence level of 2 also if it has a true interpro domain that is linked to a GO annotation http://www.geneontology.org/external2go/interpro2go/.

When the confidence level is above "1", GO annotations of higher levels of the GO hierarchy are assigned (e.g. for "#CL 3" the GO annotations provided, is as appears plus the 2 GO annotations above it in the hierarchy).

"#DB" marks the database on which the GO assignment relies on. The "sp", as in Example 10a, relates to SwissProt/TremBI Protein knowledgebase, available from http://www.expasy:ch/sprot/. "InterPro", as in Example 10c, refers to the InterPro combined database, available from http://www.ebi.ac.uk/interpro/, which contains information regarding protein families, collected from the following databases: SwissProt (http://www.ebi.ac.uk/swissprot/), Prosite (http://www.expasy.ch/prosite/), Pfam (http://www.sanger.ac.uk/Software/Pfam/), Prints (http://www.bioinf.man.ac.uk/dbbrowser/PRINTS/), Prodom (http://prodes.toulouse.inra.fr/prodom/), and Smart (http://smart.embl-heidelberg.de/). PROLOC means that the method used for predicting the Gene Ontology cellular component is based on Proloc prediction, where the database is the statistical data the Proloc software employs to predict the subcellular localization of proteins. "Viral protein database" -All viral proteins (Total 294,805 proteins) were downloaded from NCBI GenBank on 1/10/2003. All the Baculoviridae and Entomopoxvirinae proteins, which are known to infect only insects, were removed and then a non-redundant set was prepared using 95 % identity as a cutoff (Holm L, Sander C. Removing near-neighbor redundancy from large protein sequence collections. Bioinformatics 1998 Jun;14(5):423-9). This resulted in 97,979 proteins. The cluster members of each of the viral proteins are described in U.S. Pat. Appl. 60/480,752.

"#EN" represents the accession of the entity in the database (#DB), corresponding to the accession of the protein/domain why the GO was predicted. If the GO assignment is based on a protein from the SwissProt/TremBl Protein database this field will have the locus name of the protein. Examples, *"#DB sp #EN NRG2_HUMAN"* means that the GO assignment in this case was based on a protein from the SwissProt/Trembl database, while the closest homologue (that has a GO assignment) to the assigned protein is depicted in SwissProt entry "NRG2_HUMAN "#DB *interpro #EN IPR001609*" means that GO assignment in this case was based on InterPro database, and the protein had an Interpro domain, *IPR001609,* that the assigned GO was based on. In Proloc predictions this field will have a Proloc annotation "#EN Proloc". In predictions based on viral proteins this field will have the gi. viral protein accession, "#EN 1491997".

#GENE_SYMBOL - for each Gencarta contig a HUGO gene symbol was assigned in two ways:

(i) After assigning a Swissprot/TremBl protein to each contig (see *Assignment of Swissprot/TremBl accessions to Gencarta contigs)* all the gene symbols that appear for the Swissprot entry were parsed and added as a Gene symbol annotation to the gene.

(ii) LocusLink information- LocusLink was downloaded from NCBI ftp://ftp.ncbi.nih.gov/refseq/LocusLink/ (files loc2acc, loc2ref, and LL.out_hs). The data was integrated producing a file containing the gene symbol for every sequence. Gencarta contigs were assigned a gene symbol if they contain a sequence from this file that has a gene symbol

Example: #GENE_SYMBOL MMP15

#DIAGNOSTICS- secreted/membrane-bound proteins get an annotation of "can be used as a diagnostic markers" preferably for the corresponding list of indications appearing in the # INDICATION field, described hereinabove. All proteins that were identified as secreted or membrane-bound proteins (as described in the GO field section), excluding membrane-bound proteins of intracellular components such as nuclear membrane, will be assigned with this field.

In addition, known contigs representing known diagnostic markers (such as listed in Table 13, below) and all transcripts and proteins deriving from this contig will be assigned to this field and will get the above mentioned annotation followed by "as indicated in the Diagnostic markers table"

*Table 13*

| *Enzymes* | | |
|---|---|---|
| *Test* | *Gencarta Contig* | *Comments* |
| GPT | R35137 (GPT glutamic-pyruvate transaminase (alanine aminotransferase)) Z24841 (GPT2 glutamic pyruvate transaminase (alanine ammotransferase) 2) | Also called ALT - alanine aminotransferase. Standard liver function test |
| GOT | M78228 (GOT1 glutamic-oxaloacetic transaminase 1, soluble (aspartate aminotransferase 1)) M86145 (GOT2 glutamic-oxaloacetic transaminase 2, mitochondrial (aspartate aminotransferase 2) | Also called AST - aspartate aminotransferase. Standard liver function test |
| GGT | HUMGGTX (GGT1: gamma-glutamyltransferase 1) | Liver disease |
| CPK | T05088 (CKB creatine kinase, brain) HUMCKMA (CKM creatine kinase, muscle) H20196 (CKMT1 creatine kinaso, mitochondrial 1 (ubiquitous)) HUMSMCK (CKMT2 creatine kinase mitochondrial 2 (sarcomeric)) | Also called CK. Mostly used for muscle pathologies. The MB variant is heart specific and used in the diagnosis of myocardial infarction |
| CPK-MB | T05088 (CKB creatine kinase, brain) HUMCKMA (CKM creatine kinase, muscle) | Cardiac problems - hetro-dimer of CKB and CKM |

(continued)

| Enzymes | | |
|---|---|---|
| Test | Gencarta Contig | Comments |
| Alkaline Phosphatase | HSAPHOL- ALPL: alkalino phosphatase, liver/bone/kidney<br>HUMALPHB - ALPI: alkaline phosphatase, intestinal<br>HUMALPP- ALPP: alkaline Phosphatase, placental (Regan isozyme) | Bone related syndromes and liver diseases, mostly with biliary involvement |
| Amylase | AA367524- (AMY1A: amylase, alpha 1A; salivary)<br>T10898- (AMY2B: amylase, alpha 2B; pancreatic and 2A) | Blood/Urine. Pancreas related diseases |
| LDH | HSLDHAR (LDHA lactate dehydrogenase A)<br>M77886 (LDHB lactate dehydrogenase B)<br><br>HSU13680 (LDHC lactate dehydrogenase C)<br>AA398148 (LDHL lactate dehydrogenase A-like)<br>R09053 (LDHD lactate dehydrogenase D) | Lactate Dehydrogenase Used for myocardial infarction diagnosis and neoplastic syndromes assessment. |
| G6PD | S58359 (G6PD glucose-6-phosphate dehydrogenase) | Glucose 6-phosphate dehydrogonase. Levels measured when deficiency is suspected (leading to susceptibility to hemolysis) |
| Alpha1 antiTrypsin | HUMA1ACM (SERPINA3 serine (or cysteine) proteinase inhibitor, clade A (alpha 1 antiprotemase, antitrypsin), member 3)<br>T10891 (AGT angiotensinogen (serine (or cysteme) proteinase inhibitor, clade A (alpha-1 antiprotemase, antitrypsin), memb er 8))<br>R83168 (SERPINA6 serine (or cysteme) proteinase inhibitor, clade A (alpha 1 anhproteinase, antitrypsin), member 6)<br>HUMCINHP (SERPINA5 serine (or cysteine) proteinase inhibitor, clade A (alpha-1 antiprotemase antitrypsin), member 5)<br>HSA1ATCA (SERPINA1 serine (or cysteine) proteinase inhibitor, clade A (alpha 1 antiproteinase, antitrypsin), member 1)<br>HUMKALLS (SERPINA4 serine (or cysteine) proteinase inhibitor, clade A (alpha-1 antiprotemase, antitrypsin), member 4)<br>HUMTBG (SERPINA7 serine (or cysteine) proteinase inhibitor, clade A (alpha-1 antiproteinase, antitrypsin), member 7)<br>T60354 (SERPINA10 serine (or cysteme) proteinase inhibitor, clade A (alpha-1 antiproteinase, antitrypsin), member 10) | Chronic lung diseases |
| Renin | HSRENK (REN renin) | Some hypertension syndromes |

(continued)

| Enzymes | | |
|---|---|---|
| Test | Gencarta Contig | Comments |
| Acid Phosphatase | HUMAAPA (ACP1: acid phosphatase 1, soluble)<br>T48863 (ACP2. acid phosphatase 2, lysosomal)<br><br>HSMRACP5 (ACP5: acid phosphatase 5, tartrate resistant)<br>T85211 (ACP6: lysophosphatidic acid phosphatase)<br>HSPROSAP (ACPP acid phosphatase, prostate)<br>AA005037 (ACPT acid phosphatase, testicular) | Used to differentiate multiple myeloma with other monoclonal gammopathies of uncertain significance |
| Beta glucoromdase | T11069 (GUSB glucuromdase, beta) | Used to differentiate multiple myeloma with other monoclonal gammopathies of uncertain significance |
| Aldolase | HSALDAR (ALDOA aldolase A, fructose-bisphosphate)<br>HSALDOBR (ALDOB aldolase B, fructose-bisphosphate)<br>M62176 (ALDOC aldolase C, fructose-bisphosphate) | Glycogen storage diseases |
| Choline esterase | HUMCHEF (BCHE butyrylcholinesterase)<br><br>F00931 (ACHE acetylcholinesterase (YT blood group)) | organophosphates/"nerve gases" intoxications |
| Pepsinogen | HUMPGCA PGC. progastriesin (pepsmogen C) | (m the stomach), high in gastritis, low in pernicious anemia[ |
| ACE | HSACE (ACE: angiotensin I converting enzymo (peptidyl-dipeptidase A) 1)<br>AA397955 (ACE2: angiotensin I converting enzyme (peptidyl-dipeptidase A) 2) | Angiotensin-converting enzyme. Sarcoidosis |
| Miscelleneous | | |
| Test | Gencarta contig | Comments |
| Pnon Protem | HUMPRP0A (PKNP pnon protein (p27-30) (Creutzfeld-Jakob disease, Gerstmann-Straus ler-Scheinker syndrome, fatal familial insomnia))<br>W73057 (PRND pnon protoin 2 (dublet)) | BSE diagnosis |
| Myelin basic protein | M78010 (MBP myelin basic protem)<br>R13982 (MOBP myelin-associated oligodendrocyte basic protem) | In CSF. In Multiple sclerosis |
| Albumin | HSALB1 (ALB albumin) | Mostly liver function and failure of intestine absorption |
| Proalbumin | HSALB1 (ALB albumin) | early diagnosis of malabsorption |
| Ferritin | HUMFERLS (FTL ferritin, light polypeptide)<br>HUMFERHA (FTH1 ferritin, heavy polypeptide 1) | Iron deficiency anemia |
| Transferrin | S95936 (TF transferring) | Iron deficiency anemia |
| Haptoglobm | HUMHPA1B (HP haptoglobin) | Used in anemia states and neoplastic syndromes |

(continued)

| Miscelleneous | | |
|---|---|---|
| Test | Gencarta contig | Comments |
| CRP | HSCREACT (CRP C-reactive protein, pentraxin-related) | C reactive protein. Associated with active inflammation |
| AFP | D11581 (AFP alpha-fetoprotein) | Alpha Feto Protein Used in pregnancy for abnormalities screening and as a cancer marker. |
| C3 | T40158 (C3 complement component 3) | Various auto-immune and allergy syndromes |
| C4 | HSCOC4 (C4A complement component 4A, C4B complement component 4B) | Various auto-immune and allergy syndromes |
| Ceruloplasmin | HSCP2 (CP ceruloplasmin (ferroxidase)) | Wilson's disease (liver disease) |
| Myoglobin | T11628 (MB myoglobin) | Rhabdomyolysis, Myocardial infarction |
| FABP | S67314 (FABP3: fatty acid binding protein 3, muscle and heart) D11754 (FABP1 liver- L-FABP- fatty acid binding protein 1) AW605378 (FABP2 fatty acid binding protein 2, intestinal) HUMALBP (FABP4: fatty acid binding protein 4, adipocyte) T06152 (FABP5 fatty acid binding protein 5 (psoriasis associated) HSI15PGN1 (FABP6: fatty acid binding protein 6, ileal (gastrotropm) R60348 (FABP7. fatty acid binding protem 7, brain) | myoglobm and Fatty Acid Bmding |
| Troponin I | HUMTROPNIN (TNNI2 tropomn I, skeletal, fast) Z25083 (TNNI1 troponin I, skeletal, slow) HUMTROPIA (TNNI3 tropomn I, cardiac) | Acute myocardial infarction |
| Beta 2-microglobulin | HSB2MMU (B2M beta-2-microglobulin) | |
| Macroglobin | M62177 (A2M. alpha-2-macroglobulin) | Elevated in inflammation |
| Alpha 1 glycoprotem | T72188 (A1BG alpha-1-B glycoprotem) | Elevated m inflammation and tumors, |
| Apo A-I | HUMAPOAIP (APOA1 apolipoprotein A-I) | Risk for coronary artery disease |
| Apo B 100 | HSAPOBR2 (APOB: apolipoprotemB (including Ag(x) antigen)) | Atherosclerotic heart disease |
| Apo E | T61627 (APOE apolipoprotein E) | diagnosis of Type III hyperlipoproteinemia, evaluate a possible genetic component to atherosclerosis, or to help confirm a diagnosis of late onset AD |
| CF gene | HUMCFTRM (CFTR: cystic fibrosis transmembrane conductance regulator, ATP-binding cassette (sub-family C, member 7)) | Cystic fibrosis disease (a DNA test - blood sample) |
| PSEN1 gene | T89701 (PSEN1: presemlin 1 (Alzheimer disease 3)) | Early onset of familial AD (a DNA test - blood sample) |

(continued)

| Hormones | | |
|---|---|---|
| Test | Gencarta Contig | Comments |
| Erythropoietin | HSERPR (EPO erythropoietin) | Hardly used for diagnosis Used as treatment |
| GH | HSGROW1 (GH1 growth hormone 1) HUMCS2 (GH2 growth hormone 2) | Growth Hormone. Endocrine syndromes |
| TSH | AV745295 (TSHB thyroid stimulating hormone beta) | Part of thyroid functions tests |
| betaHCG | R27266 (CGB5 chorionic gonadotropin, beta polypeptide 5) | Pregnancy, malignant syndromes in men and women |
| LH | HUMCGBB50 (LHB luteinizing hormone beta polypeptide) | Part of standard hormonal profile for fertility, gynecological syndromes and endocrine syndromes |
| FSH | AV754057 (FSHB follicle stimulating hormone, beta polypeptide) | Part of standard hormonal profile for fertility, gynecological syndromes and endocrine syndromes |
| TBG | S40807 (TG thyroglobulin) | Thyroxin bindmg globulin. Thyroid syndromes |
| Prolactin | HSLACT (PRL prolactin) | Various endocrine syndromes |
| Thyroglobulin | S40807 (TG thyroglobulin) | Follow up of thyroid cancer patients |
| PTH | HSTHYR (PTH parathyroid hormone) | Parathyroid Hormone Syndromes of calcium management |
| Insulin/Pre Insulin | HSPPI (INS insulin) | Diabetes |
| Gastrin | HSGAST (GAS gastrin) | Peptic ulcers |
| Oxytocm | HUMOTCB (OXT oxytocin, prepro-(nourophysin I)) | Endocrine syndromes related to lactation |
| AVP | HUMVPC (AVP arginine vasopressin (neurophysin II, antidiuretic hormone, diabetes insipidus, neurohypophyseal)) | Arginine Vasopressin. Endocrine syndromes related to the osmotic pressure of body fluids |
| ACTH | HUMPOMCMTC (POMC: proopiomelanocortin (adrenocorticotropin/ beta-lipotropin/ alpha-melanocyte stimulating hormone/ beta melanocyte stimulating hormone/ beta-endorphin)) | Secreted from the anterior pituitary gland. Regulation of cortisol Abnormalities are indicative of Cushing's disease, addison's disease and adrenal tumors |
| BNP | HUMNATPEP (NPPB. natnuretic peptide precursor B) | Heart failure |

| Blood Clotting | | |
|---|---|---|
| Test | Gencarta Contig | Comments |
| Protein C | S50739 (PROC protem C (mactivator of coagulation factors Va and VIIIa)) | Inhented Clottmg disorders |
| Protem S | HSSPROTR (PROS1 protein S (alpha)) | Inherited Clotting disorders |
| Fibrinogen | D11940 (FGA: fibrinogen, A alpha polypeptide) HUMFBRB (FGB fibrinogen, B beta polypeptide) T24021 (FGG: fibrinogen, gamma polypeptide) | Clotting disorders |
| Factors 2, 5, 7, 9, 10,11, 12, 13 | HUMPTHROM (F2 coagulation factor II (thrombin)) | Inherited Clotting disorders |

**Blood Clotting**

| Test | Gencarta Contig | Comments |
|---|---|---|
| | HUMTFPC (F3 coagulation factor III (thromboplastin, tissue factor))<br>HUMF5A (F5 coagulation factor V (proaccelerin, labile factor))<br>M78203 (F7 coagulation factor VII (serum prothrombm conversion accelerator))<br>HUMF8C (F8 coagulation factor VIII, procoagulant component (hemophilia A))<br>HUMCFIX (F9 coagulation factor IX (plasma thromboplastic component, Chnstmas dis ease, hemophilia B))<br>HUMCFX (F10: coagulation factor X)<br>HUMFXI (F11 coagulation factor XI (plasma thromboplastin antecedent))<br>HUMCFXIIA (F12 coagulation factor XII (Hageman factor))<br>HUMFXIIIA (F13A1 coagulation factor XIII, A1 polypeptide)<br>R28976 (F13B coagulation factor XIII, B polypeptide) | |
| vWF | HUMVWF (VWF von Willebrand factor) | Von Willebrand factor Inherited Clotting disorders |
| Antithrombin III | T62060 (SERPINC1 serine (or cysteine) proteinase inhibitor clade C (antithrombin ), member 1) | Inherited Clotting disorders |

**Cancer Markers**

| Test | Gencarta Contig | Comments |
|---|---|---|
| AFP | D11581 (AFP alpha-fetoprotein) | Pregnancy, testicular cancer and hepatocellular cancer |
| CA125 | HSIAI3B (M17S2 membrane component, chromosome 17, surface marker 2 (ovarian carcinoma antigen CA125)) | Ovarian cancer |
| CA-15-3 | HSMUC1A (MUC1 mucin 1 transmembrane) | Breast cancer |
| CA-19-9 | HSAFUTF (FUT3: fucosyltransferase 3 (galactoside 3(4)-L-fucosyltransferase, Lewis blood group included)) | Gastrointestinal cancer, pancreatic cancer |
| CEA | T10888 HUMCEA (CEACAM3 carcinoembryonic antigen-related cell adhesion molecule 3) | Carcinoembryomc Antigen. Colorectal cancer |
| PSA | HSCDN9 (KLK3: kallikrein 3, (prostate specific antigen)) | |
| PSMA | HUMPSM (FOLH1: folate hydrolase (prostate-specific membrane antigen) 1) | |
| TPA, TATI, OVX1, LASA, CA54/81 | HSPSTI (SPINK1: serine protease inhibitor, Kazal type 1) | Ovanan cancer |
| BRCA 1 | H90415 (BRCA1 breast cancer 1, early onset) | |
| BRCA 2 | H47777 (BRCA2. breast cancer 2, early onset) | Breast cancer (ovarian cancer) |

(continued)

| Cancer Markers | | |
|---|---|---|
| Test | Gencarta Contig | Comments |
| HER2/Neu | S57296 (ERBB2: v-erb-b2 erythroblastic leukemia viral oncogene homolog 2, neuro/glioblastoma denved oncogene homolog (avian)) | Breast cancer |
| Estrogen receptor | HSERG5UTA (ESR1: estrogen receptor 1) HSRNAERB (ESR2: estrogen receptor 2 (ER beta)) | Breast cancer |
| Progesterone receptor | T09102 (PGRMC1: progesterone receptor membrane component 1) Z32891 (PGRMC2: progesterone receptor membrane component 2) | Breast cancer |
| Note: (i) Small portion of these "markers" are also drug targets, whether already for approved drugs (such as alphal anti-Trypsin) or under development (e g., GOT) (ii) Some of these "markers" are also used as therapeutic proteins (e g, Erythropoietin). (iii) All markers are found m the blood/serum unless otherwise specified. | | |

#FR denotes the start position of the coding region on the transcript.

#TO denotes the end position of the coding region on the transcript.

Example: #FR 102 #TO 1052

#ALTERNATIVE_MET_POS - This field designates the position of an alternative translation start site that is suggested based on a homology to a public protein that starts from a Met different than the Met predicted by the protein prediction algorithm. This field includes the position of the alternative Met relative to the original protein prediction.

Method:

All proteins were BLASTP analyzed relatively to a public protein database containing proteins from Swissprot and TremBl. If the Protein of the present invention had an homology to a public protein and this homology was identified by the following:

• The homology started on a Met in the query protein that was different from the original coding start position
• The escore was below 1e-10 or the identity was above 70%
• The homology on the hit protein started on a position in the first 30 amino acids
• The annotation in the hit protein did not contain one of the following words:

   ◦ Hypothetical
   ◦ Predicted
   ◦ Partial
   ◦ Fragment

A protein starting from this Met was translated in addition to proteins from upstream Met.

Example: #ALT_MET_POS 11 means that the protein started on the 11 amino acid (where the first amino acid is counted as 0).

#ALTERNATIVE_MET_AC This field contains the accession numbers of the public proteins that this alternative Met was predicted by, as described in the # ALTERNATIVE_MET_POS field.

#SN - This field represents the polymorphisms that were found. If the annotation is for a protein sequence than only SNPs that changed the amino acid were denoted as well as the change in the amino acid. An example of this field #SN 38 A=>T where the number (38) denotes the position of the amino acid, "A" represents the original amino acid, and "T" represents the amino acid that was changed as a result of the polymorphism. A field can have multiple entries such as #SN 38 A=>T 38 A=>E 167 A=>G 237 M=>K 245 S=>F . In case of SNPs on the protein sequence the position defines the amino acid position where the first amino acid is denoted as 1.

If there are more than two nucleotides at the same SNP position in the transcript and both can change the amino acid then this will be denoted as #SN 201 A=>V,I which means that the amino acids at position 201 can change from Aln to Valine or to Isoleucine. If the SNP is at the same codon but at different positions in the transcript this is denoted as #SN 38 A=>T 38 A=>E.

If the annotation is for a transcript sequence then all SNPs will be included in the output, including silent SNPs. The position will be on the transcript sequence and the SNPs will be depicted as for the protein but in nucleotides. Example: #SN 200 C=>G. In case there is more than one possibility, the nucleotides are separated by a comma (#SN 200 C=>G,A).

If there is a SNP that is an insertion or a deletion this will be denoted as "." (examples: #SN 100 . =>G means that the SNP is an insertion of a G in the transcript at position 100, #SN 100 G=> . means that the SNP is a deletion of a G in the transcript at position 100). This can result in a frameshift at the protein level.

Criteria used in the SNP detection process - This process scans the multiple alignment of the sequences versus the consensus of the gene and searches for SNPs as follows (see Figure 16a).

Stage 1: Masking - Scanning the multiple alignment for problematic regions where no SNPs should be predicted:

- Masking of dirty regions (i.e., more than 3 bp differing from the consensus on a 20 bp stretch).
- Masking of regions with repetitive characters at least 4 bp long.
- Masking of the ends of the sequences (the last 30 bps of the sequences).

Stage 2. Searching for SNPs - Looking at every position, and calculating a weighted score for each nucleic acid that appears in any of the sequences in that position:

- Each type of sequence is allocated a different weight: EST -1 point, HTG 1.5 points, RNA 1.9 points and DNA 3 points.
- Searching the multiple alignment columns that contain 2 or 3 different characters, Each position is considered a putative SNP if:

    (i) it has a weight of at least 2 points (Meaning it came from at least 2 ESTs, or from one DNA sequence, for example) if the total number of "clean" sequences is no more than 10;
    (ii) it has a weight of more than 2 points if the total number of clean sequences is between 10 and 50;
    (iii) it has a weight of more than 4 % of the total number of clean sequences if the total number of clean sequences is between 50 and 100;
    (iv) the total number of "clean" sequences is more than 100 the SNP's points must be a minimum of 5.5 percents;
    (v) masked sequences contribute to a SNP with a very high score (at least 10), their contribution is not discarded.

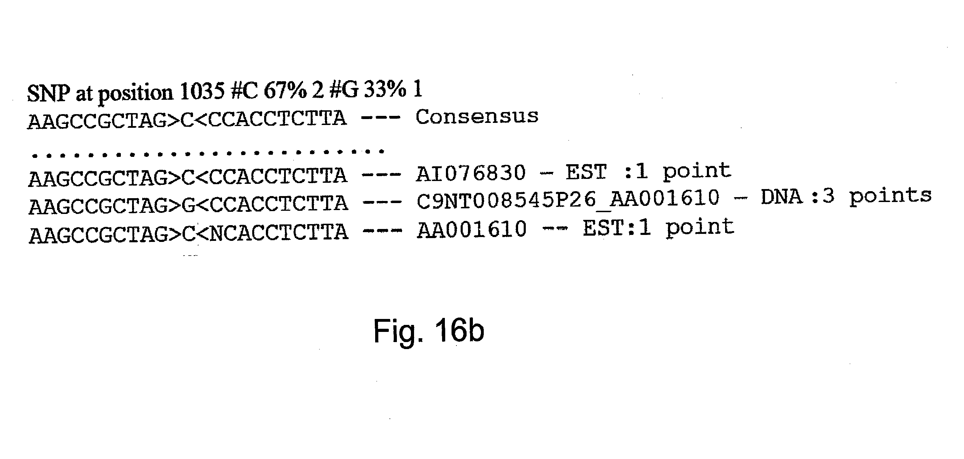

For example see Figure 16b.

Stage 3: Filtering out false positives as follows:

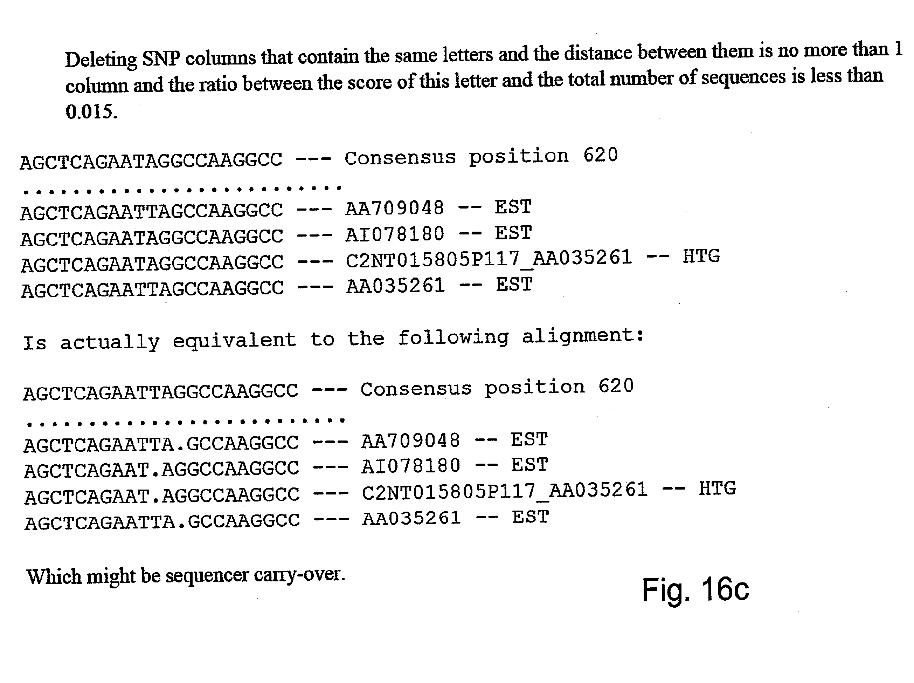

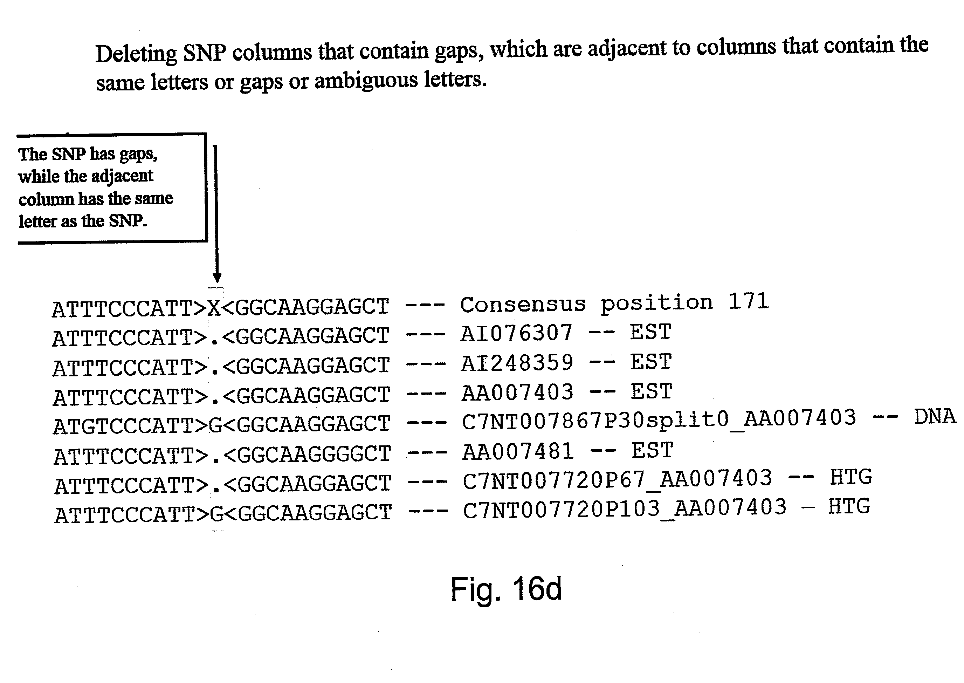

- Deleting SNP columns that contain the same letters and the distance between them is no more than 1 column and the ratio between the score of this letter and the total number of sequences is less than 0.015 (see Figure 16c).
- Deleting SNP columns that contain gaps, which are adjacent to columns that contain the same letters or gaps or ambiguous letters (see Figure 16d).

If the number of points from both clean and masked sequences is more than 10 and we have 2 different characters we exclude the gaps (if they exist) in this position.

The remaining SNP columns are the SNP program output.

#DISEASE_RELATED_CLINICAL_PHENOTYPE - This field denotes the possibility of using biomolecular sequences of the present invention for the diagnosis and/or treatment of genetic diseases such as listed in the following URL: http://www.geneclinics.org/servlet/access?id=8888891&key=X9D79005re1Az&db=genet ests&res=&fcn=b&grp=g&genesearch=true&testtype=both&ls=1&type=e&qry=&submit= Search and in Table 14, below. This list includes genetic diseases and genes which may be used for the detection and/or treatment thereof. As such, newly uncovered variants of these genes, including novel SNPs or mutations, may be used for improved diagnosis and/or treatment when used singly or in combination with the previously described genes. For example, in genetic diseases where the diseased phenotype has a different splice variant profile than the healthy phenotype, like that seen in Thalasemia and in Duchenne Mascular Dystrophy, the novel splice variants may distinguish between healthy and diseased phenotype.

Another example is in cases of autosomal recessive genetic diseases. Some publicly available sequences were sequenced from malfunctioning alleles derived from healthy carriers of the disease, and therefore contain the mutation that leads to the disease. Identification of novel SNPs based on sequence alignment can assist in identifying disease-causing mutations.

*Table 14*

| Gencarta Contig | Gene Symbol | Disease |
|---|---|---|
| HSCFTRMA | CFTR | Congenital Bilateral Absence of the Vas Deferens ;Cystic Fibrosis |
| HUMCFTRM | CFTR | Congenital Bilateral Absence of the Vas Deferens ;Cystic Fibrosis |
| HUMFGFR3 | FGFR3 | Achondroplasia ;Crouzon Syndrome with Acanthosis Nigricans ;FGFR-Related Craniosynostosis Syndromes ;Hypochondroplasia ;Muenke Syndrome ;Severe Achondroplasia with Developmental Delay and Acanthosis Nigricans (SADDAN) ;Thanatophoric Dysplasia |
| HSU11690 | FGD1 | Aarskog Syndrome |
| HSCA1III | COL3A1 | Ehlers-Danlos Syndrome, Vascular Type |
| HUMCOL2A1B | COL2A1 | Achondrogenesis Type 2 ;Knicst Dysplasia ;Spondyloepimetaphyseal Dysplasia, Strudwick Type ;Spondyloepiphyseal Dysplasia, Congenita ;Stickler Syndrome ;Stickler Syndrome Type I |
| R68817 | APRT | Adenino Phosphoribosyltransferase Deficiency |
| HUMAMPD1 | AMPD1 | Adenosine Monophosphate Deaminase 1 |
| M62124 | PXR1 | Zellweger Syndrome Spectrum |
| HSXLALDA | ABCD1 | Adrenoleukodystrophy, X-Linked |
| T28718 | BTK | X-Linked Agammaglobulinemia |
| R91110 | IL2RG | X-Linked Severe Combined Immunodeficiency |
| HUMPEDG | OCA2 | Oculocutaneous Albinism Type 2 |
| HSU01873 | TYR | Oculocutaneous Albinism Type 1 |
| BSOA1MRNA | OA1 | Ocular Albinism, X-Linked |
| R14843 | TYRP1 | Oculocutaneous Albinism Type 3 (TRP1 Related) |
| HSALDAR | ALDOA | Aldolase A Deficiency |
| T40633 | HBA1 | Alpha-Thalassemia |
| T40633 | HBA2 | Alpha-Thalassemia ;Hemoglobin Constant Spring |
| HSU09820 | ATRX | Alpha-Thalassemia X-Linked Mental Retardation Syndrome |
| HUMCOL4A5 | COL4A5 | Alport Syndrome ;Alport Syndrome, X-Linked |
| T61627 | APOE | Apolipoprotein E Genotypmg ;Familial Combined Hyperbpidemia ;Hyperlipoproteinemia Type III |
| T89701 | PSEN1 | Alzheimer Disease Type 3 ;Early-Onset Familial Alzheimer Disease |
| R05822 | PSEN2 | Alzheimer Disease Type 4 ;Early-Onset Familial Alzheimer Disease |
| HSTTRM | TTR | Transthyretin Amyloidosis |
| T23978 | SOD1 | Amyotrophic Lateral Sclerosis |
| HUMANDREC | AR | Androgen Insensitivity Syndrome ;Spinal and Bulbar Muscular Atrophy |
| Z19491 | UBE3A | Angelman Syndrome |
| HUMPAX6AN | PAX6 | Aniridia ;Anophthalmia ;Isolated Aniridia ,Peters Anomaly ;Peters Anomaly with Cataract ;Wilms Tumor-Aniridia-Genital Anomalies Retardation Syndrome |
| HUMKGFRA | FGFR2 | Apert Syndrome ;Beare-Stevenson Syndrome ;Crouzon Syndrome ;FGFR-Related Cramosynostosis Syndromes ;Jackson-Weiss Syndrome ;Pfeiffer Syndrome Type 1, 2, and 3 |

(continued)

| Gencarta Contig | Gene Symbol | Disease |
|---|---|---|
| HSU03272 | FBN2 | Congenital Contractural Arachnodactyly |
| Z19459 | AMCD1 | Arthrogryposis Multiplex Congenita, Distal, Type I |
| T88756 | ATM | Ataxia Telangiectasia |
| H30056 | BBS1 | Bardet-Biedl Syndrome |
| Z25009 | BBS2 | Bardet Biedl Syndrome |
| T64876 | BBS4 | Bardet Biedl Syndrome |
| N27125 | PTCH | Nevoid Basal Cell Carcinoma Syndrome |
| N31453 | VMD2 | Best Vitelliform Macular Dystrophy |
| HUMHBB3E | HBB | Beta-Thalassemia ;Hemoglobm E ,Hemoglobm S Beta-Thalassemia ,Hemoglobin SC ;Hemoglobin SD ,Hemoglobm SO ;Hemoglobin SS ;Sickle Cell Disease |
| H53763 | BLM | Bloom Syndrome |
| N22283 | EYA1 | Branchiootorenal Syndrome |
| H90415 | BRCA1 | BRCA1 and BRCA2 Hereditary Breast/Ovarian Cancer ;BRCA1 Hereditary Breast/Ovarian Cancer |
| H47777 | BRCA2 | BRCA1 and BRCA2 Hereditary Broast/Ovarian Cancer ;BRCA2 Hereditary Breast/Ovarian Cancer |
| Z33575 | SOX9 | Campomelic Dysplasia |
| S67156 | ASPA | Canavan Disease |
| T52465 | CPS1 | Carbamoylphosphate Synthetase I Deficiency |
| HSVD3HYD | CYP27A1 | Cerebrotendmous Xanthomatosis |
| S66705 | MPZ | Charcot Marie-Tooth Neuropathy Type 1 ,Charcot-Marie-Tooth Neuropathy Type 1B ;Congenital Hypomyelination |
| HSGAS3MR | PMP22 | Charcot-Marie-Tooth Neuropathy Type 1 ;Charcot-Mane-Tooth Neuropathy Type 1A ;Charcot Mane-Tooth Neuropathy Type 1E Hereditary Neuropathy with Liability to Pressure Palsies |
| T93208 | PMP22 | Charcot-Marie-Tooth Neuropathy Type 1 ;Charcot-Mane-Tooth Neuropathy Type 1A ;Charcot-Mane-Tooth Neuropathy Type IE ;Hereditary Neuropathy with Liability to Pressure Palsies |
| HSGAPJR | GJB1 | Charcot-Marie-Tooth Neuropathy Type X |
| HSXCGD | CYBB | Chronic Granulomatous Disease |
| S67289 | CYBB | Chronic Granulomatous Disease |
| HSASD | ASS | Citrullinemia |
| HUMPAX2A | PAX2 | Anophthalmia ;Renal-Coloboma Syndrome |
| HUMP45C21 | CYP21A2 | 21-Hydroxylase Deficiency |
| S74720 | NR0B1 | Complex Glycerol Kinase Deficiency ;Dosage-Sensitive Sex Reversal ;Isolated X-Linked Adrenal Hypoplasia Congenita ;X-Linked Adrenal Hypoplasia Congenita |
| HSKERTRNS | TGM1 | Autosomal Recessive Congenital Ichthyosis |
| BF928311 | CPO | Hereditary Coproporphyria |

(continued)

| Gencarta Contig | Gene Symbol | Disease |
|---|---|---|
| HSCPPOX | CPO | Hereditary Coproporphyria |
| HUMTGFBIG | TGFBI | Avellino Corneal Dystrophy ;Granular Corneal Dystrophy ;Lattice Corneal Dystrophy Type I |
| R08437 | MSX2 | Craniosynostosis Type II ;Parietal Foramina 1 |
| HUMPRP0A | PRNP | Prion Diseases |
| T08652 | DRPLA | DRPLA |
| Z46151 | DRPLA | DRPLA |
| HSWT1 | WT1 | Denys-Drash Syndrome ;Wilms Tumor ;Wilms Tumor-Aniridia-Genital Anomalies-Retardation Syndrome ;WT1-Related Disorders |
| HUMWT1X | WT1 | Denys-Drash Syndrome ;Wilms Tumor ;Wilms Tumor-Aniridia-Genital Anomalies-Retardation Syndrome ;WT1-Related Disorders |
| M78080 | ATP2A2 | Darier Disease |
| Z30219 | DCR | Down Syndrome Critical Region |
| T11279 | DKC1 | Dyskeratosis Congenita |
| T08131 | DYT1 | Early-Onset Primary Dystonia (DYT1) |
| T50729 | ED1 | Hypohidrotic Ectodermal Dysplasia ;Hypohidrotic Ectodermal Dysplasia, X-Linked |
| HUMPA1V | COL5A1 | Ehlers-Danlos Syndrome, Classic Type |
| HUMLYSYL | PLOD | Ehlers-Danlos Syndrome, Kyphoscoliotic Form |
| HSCOLIA | COL1A2 | Ehlers-Danlos Syndrome, Arthrochalasia Type ;Osteogenesis Imperfecta |
| HUMCG1PA1 | COL1A1 | Ehlers-Danlos Syndrome, Arthrochalasia Type ;Osteogenesis Imperfecta |
| Z30171 | TAZ | 3-Methylglutaconic Aciduria Type 2 ;Cardiomyopathy ;Dilated Cardiomyopathy ;Endocardial Fibroelastosis ;Familial Isolated Noncompaction of Left Ventrical Myocardium |
| Z39302 | TAZ | 3-Methylglutaconic Aciduria Type 2 ;Cardiomyopathy ;Dilated Cardiomyopathy ;Endocardial Fibroelastosis ;Familial Isolated Noncompaction of Left Ventrical Myocardium |
| HUMKERK5A | KART5 | Epidermolysis Bullosa Simplex |
| R72295 | KRT14 | Epidermolysis Bullosa Simplex |
| HUMKTEP2A | KRT1 | Epidermolytic Hyperkeratosis ;Nonepidermolytic Palmoplantar Hyperkeratosis |
| HUMK10A | KRT10 | Epidermolytic Hyperkeratosis |
| M78482 | CHS1 | Chediak-Higashi Syndrome |
| HSTCD1 | CHM | Choroideremia |
| HSAGALAR | GLA | Fabry Disease |
| T79651 | GLA | Fabry Disease |
| HUMF5A | F5 | Factor V Leiden Thrombophilia ;Factor V R2 Mutation Thrombophilia |
| HUMFXI | F11 | Factor XI Deficiency |
| M79108 | APC | Colon Cancer (APC I1307K related) ;Familial Adenomatous Polyposis |
| T10619 | IKBKAP | Familial Dysautonomia |

(continued)

| Gencarta Contig | Gene Symbol | Disease |
|---|---|---|
| HUMFMR1 | FMR1 | Fragile X Syndrome |
| M78417 | FMR2 | FRAXE Syndrome |
| R06415 | FRDA | Friedreich Ataxia |
| HSALDOBR | ALDOB | Hereditary Fructose Intolerance |
| HUMALFUC | FUCA1 | Fucosidosis |
| M85904 | FH | Fumarate Hydratase Deficiency |
| H85361 | ABCA4 | Age-Related Macular Degeneration ;Retinitis Pigmentosa, Autosomal Recessive ,Stargardt Disease 1 |
| R31596 | GALK1 | Galactokinase Deficiency |
| T53762 | GALT | Galactosemia |
| HUMGCB | GBA | Gaucher Disease |
| T48672 | GBA | Gaucher Disease |
| HSGCRAR | NR3C1 | Glucocorticoid Resistance |
| S58359 | G6PD | Glucose-6-Phosphate Dehydrogenase Deficiency |
| HSGKTS1 | GK | Glycerol Kinase Deficiency |
| HSRNAGLK | GK | Glycerol Kmase Deficiency |
| U01120 | G6PC | Glycogen Storage Disease Type Ia |
| HUMGAAA | GAA | Glycogen Storage Disease Type II |
| F00985 | AGL | Glycogen Storage Disease Type III |
| HUMHGBE | GBE1 | Glycogen Storage Disease Type IV |
| HSPHOSR1 | PYGM | Glycogen Storage Disease Type V |
| D12179 | PYGL | Glycogen Storage Disease Type VI |
| HSHMPFK | PFKM | Glycogen Storage Disease Type VII |
| HUMGLI3A | GLI3 | GLI3-Related Disorders ;Greig Cephatopolysyndactyly Syndrome ;Pallister Hall Syndrome |
| F09335 | ATP2C1 | Hailey-Hailey Disease |
| M62210 | CCM1 | Angiokeratoma Corporis Diffusum with Arteriovenous Fistulas ;Familial Cerebral Cavernous Malformation |
| T59431 | HFE | HFE- Associated Hereditary Hemochromatosis |
| HSALK1A | ACVRL1 | Hereditary Hemorrhagic Telangiectasia |
| HUMENDO | ENG | Hereditary Hemorrhagic Telangiectasia |
| HUMF8C | F8 | Hemophilia A |
| HUMFVIII | F8 | Hemophilia A |
| HUMCFIX | F9 | Hemophilia B |
| HSU03911 | MSH2 | Hereditary Non-Polyposis Colon Cancer |
| Z24775 | MLH1 | Hereditary Non-Polyposis Colon Cancer |
| HSRETTT | RET | Hirschsprung Disease ;Multiple Endocrine Neoplasia Type 2 |
| HUMSHH | SHH | Holoprosencephaly 3 |

(continued)

| Gencarta Contig | Gene Symbol | Disease |
|---|---|---|
| N81026 | TBX5 | Holt-Oram Syndrome |
| M78262 | CBS | Homocystinuria |
| T06035 | IDS | Mucopolysaccharidosis Type II |
| T03828 | HD | Huntington Disease |
| H27612 | IDUA | Mucopolysaccharidosis Type I |
| M62205 | GFAP | Alexander Disease |
| HUMCD40L | TNFSF5 | Hyper IgM Syndrome, X Linked |
| HUMPTHROM | F2 | Prothrombin G20210A Thrombophilia |
| T61466 | MTHFR | MTHFR Deficiency ;MTHFR Thermolabile Vanant |
| HUMSKM1A | SCN4A | Hyperkalemic Periodic Paralysis Type 1 ;Hypokatemic Periodic Paralysis ;Hypokalemic Periodic Paralysis Type 2 ;Myotonia Congenita, Dominant ;Paramyotonia Congenita |
| HSU09784 | CACNA1S | Hypokalemic Periodic Paralysis ;Hypokalemic Periodic Paralysis Type 1 ;Malignant Hyperthermia Susceptibility |
| HUMLPLAA | LPL | Familial Lipoprotein Lipase Deficiency |
| HUMPEX | PHEX | Hypophosphatemic Rickets, X-Linked Dominant |
| M78626 | STS | Ichthyosis, X-Linked |
| R56102 | IKBKG | Incontinentia Pigmenti |
| Z39843 | IVD | Isovaleric Acidemia |
| S60085S1 | KAL1 | Kallmann Syndrome, X-Linked |
| T55061 | KEL | Kell Antigen Genotyping |
| HUMGALC | GALC | Krabbe Disease |
| HUMZFPSREB | ZNF9 | Myotonic Dystrophy Type 2 |
| Z19342 | KIF1B | Charcot-Marie-Tooth Neuropathy Type 2 |
| T11351 | NPC2 | Niemann-Pick Disease Type C |
| Z39096 | NDRG1 | Charcot-Marie-Tooth Neuropathy Type 4 |
| AA984421 | PRX | Charcot-Mane-Tooth Neuropathy Type 4 ;Charcot-Marie-Tooth Neuropathy Type 4F |
| HUMRETGC | GUCY2D | Leber Congenital Amaurosis |
| HSU18991 | RPE65 | Leber Congenital Amaurosis ;Retinitis Pigmentosa, Autosomal Recessive |
| C16899 | MTND6 | Leber Hereditary Optic Neuropathy ;Mitochondrial Disorders ;Mitochondrial DNA Associated Leigh Syndrome and NARP |
| AA069417 | MTND4 | Leber Hereditary Optic Neuropathy ;Mitochondrial Disorders ,Mitochondnal DNA Associated Leigh Syndrome and NARP |
| HUMCYP3A | MTND4 | Leber Hereditary Optic Neuropathy ;Mitochondrial Disorders ;Mitochondrial DNA-Associated Leigh Syndrome and NARP |
| HSCPHC22 | MTND1 | Leber Hereditary Optic Neuropathy ;Mitochondrial Disorders ;Mitochondrial DNA-Associated Leigh Syndrome and NARP |
| HUMHPRT | HPRT1 | Leach-Nyhan Syndrome |

(continued)

| Gencarta Contig | Gene Symbol | Disease |
|---|---|---|
| HUMLHHCGR | LHCGR | Leydig Cell Hypoplasia/Agenesis ;Male-Limited Precocious Puberty |
| HSP53 | TP53 | Li-Fraumeni Syndrome |
| Z19198 | HADHB | Long Chain 3-Hydroxyacyl-CoA Dehydrogenase Deficiency |
| M79018 | HADHA | Long Chain 3-Hydroxyacyl CoA Dehydrogenase Deficiency |
| W93500 | KCNQ1 | Atrial Fibrillation ,Jervell and Lange-Nielsen Syndrome ;LQT 1 ;Romano-Ward Syndrome |
| S62085 | OCRL | Lowe Syndrome |
| T48981 | FBN1 | Marfan Syndrome |
| HUMASFB | ARSB | Mucopolysaccharidosis Type VI |
| M62202 | GNAS | Albright Hereditary Osteodystrophy ;McCune-Albright Syndrome ;Osseus Heteroplasia, Progressive |
| N46342 | SACS | ARSACS |
| T81605 | FANCD2 | Fancom Anemia |
| H47777 | FANCD1 | Fancom Anemia |
| T23877 | ACADM | Medium Chain Acyl-Coenzyme A Dehydrogenase Deficiency |
| AA906866 | PARK2 | Parkin Type of Juvenile Parkinson Disease |
| BE140729 | GJB4 | Erythrokeratodermia Variabilis |
| HSU26727 | CDKN2A | Familial Malignant Melanoma |
| T47218 | SPINK5 | Netherton Syndrome |
| HSMNKMBP | ATP7A | ATP7A-Related Copper Transport Disorders |
| R37821 | SHFM4 | Ectrodactyly |
| M78183 | GSN | Amyloidosis V |
| HSARYA | ARSA | Chromosome 22q13.3 Deletion Syndrome ;Metachromatic Leukodystrophy |
| S68531 | COL10A1 | Metaphyseal Chondrodysplasia, Schmid Type |
| T59742 | CACNA1A | Episodic Ataxia Type 2 ;Familial Hemiplegic Migraine ,Spinocerebellar Ataxia Type 6 |
| HSCP2 | HPS3 | Hermansky-Pudlak Syndrome ;Hermansky-Pudlak Syndrome 3 |
| R21301 | HPS3 | Hermansky-Pudlak Syndrome ;Hermansky-Pudlak Syndrome 3 |
| HUMDGALRP | GLB1 | GM1 Gangliosidosis ;Mucopolysaccharidosis Type IVB |
| HSU12507 | KCNJ2 | Andersen Syndrome |
| R28488 | MEN1 | Multiple Endocrine Neoplasia Type 1 |
| HUMCOMP | COMP | COMP-Related Multiple Epiphyseal Dysplasia ;Multiple Epiphyseal Dysplasia, Dominant ;Pseudoachondroplasia |
| H30258 | COL9A2 | Multiple Epiphyseal Dysplasia, Dommant |
| T48133 | EXT1 | Hereditary Multiple Exostoses ;Multiple Exostoses Type I |
| T06129 | EXT2 | Hereditary Multiple Exostoses ;Multiple Exostoses, Type II |
| T05624 | LAMA2 | Congenital Muscular Dystrophy with Merosin Deficiency |

(continued)

| Gencarta Contig | Gene Symbol | Disease |
|---|---|---|
| HSDYSTIA | DMD | Duchenne/Becker Muscular Dystrophy ;Dystrophinopathies ;X-Linked Dilated Cardiomyopathy |
| HSSTA | EMD | Emery-Dreifuss Muscular Dystrophy, X-Linked |
| HSU20165 | BMPR2 | Primary Pulmonary Hypertension |
| M79239 | CAPN3 | Calpamopathy ;Limb-Girdle Muscular Dystrophies, Autosomal Recessive |
| HSU34976 | SGCG | Gamma-Sarcoglycanopathy ;Limb-Girdle Muscular Dystrophies, Autosomal Recessive ;Sarcoglycanopathies |
| HUMADHA | SGCA | Alpha-Sarcoglycanopathy ;Limb-Girdle Muscular Dystrophies, Autosomal Recessive ;Sarcoglycanopathies |
| Z25374 | SGCB | Beta-Sarcoglycanopathy ;Limb-Girdle Muscular Dystrophies, Autosomal Recessive ;Sarcoglycanopathies |
| N29439 | SGCD | Delta-Sarcoglycanopathy ;Dilated Cardiomyopathy ;Limb-Girdle Muscular Dystrophies, Autosomal Recessive ;Sarcoglycanopathies |
| N56180 | CASQ2 | Catecholaminergic Ventricular Tachycardia, Autosomal Recessive |
| T23560 | CHRNB2 | Nocturnal Frontal Lobe Epilepsy, Autosomal Dominant |
| HSCBRNA44 | CHRNA4 | Nocturnal Frontal Lobe Epilepsy, Autosomal Dominant |
| M78654 | CHRNA4 | Nocturnal Frontal Lobe Epilepsy, Autosomal Dominant |
| T86329 | CDH23 | Usher Svndromo Type 1 |
| D11677 | PABPN1 | Oculopharyngeal Muscular Dystrophy |
| AW449267 | PCDH15 | Usher Syndrome Type 1 |
| HUMCLC | CLCN1 | Myotoma Congenita, Dominant ;Myotoma Congenita, Recessive |
| S86455 | DMPK | Myotomc Dystrophy Type 1 |
| T70260 | MTM1 | Myotubular Myopathy, X-Linked |
| T12579 | LMX1B | Nail Patella Syndrome |
| HSTRKT1 | TPM3 | Nemaline Myopathy |
| HUMTROPCK | TPM3 | Nemaline Myopathy |
| Z19248 | NEB | Nemaline Myopathy |
| AF030626 | AVPR2 | Nephrogenic Diabetes Insipidus ;Nephrogenic Diabetes Insipidus, X-Liked |
| AA780862 | NPHS1 | Congenital Finnish Nephrosis |
| T08860 | ABCC8 | ABCC8-Related Hyperinsulinism ;Familial Hyperinsulinism |
| AA679741 | KCNJ11 | Familial Hyperinsulinism ;KCNJ11-Related Hyperinsulinism |
| M77935 | NF1 | Neurofibromatosis 1 |
| HSMEORPRA | NF2 | Neurofibromatosis 2 |
| T08995 | CLN3 | CLN3-Related Neuronal Ceroid-Lipofuscinosis ,Neuronal Ceroid-Lipofuscinoses |
| T72120 | CLN2 | CLN2-Related Neuronal Ceroid-Lipofuscinosis ;Neuronal Ceroid-Lipofuscinoses |
| T41059 | GRHPR | Hyperoxaluria, Primary Type 2 |
| HUMGCRFC | FCGR3A | Neutrophil Antigen Genotyping |
| R21657 | NPC1 | Niemann-Pick Disease Type C ;Niemann-Pick Disease Type C1 |

(continued)

| Gencarta Contig | Gene Symbol | Disease |
|---|---|---|
| M77961 | SMPD1 | Niemann-Pick Disease Due to Sphingomyelinase Deficiency |
| T87256 | SUOX | Sulfocysteinuria |
| D79813 | SOST | SOST-Related Sclerosing Bone Dysplasias |
| T94707 | MATN3 | Multiple Epiphyseal Dysplasia, Dominant |
| HSCOL9AL | COL9A1 | Multiple Epiphyseal Dysplasia, Dominant |
| S69208 | TNNT1 | Nemaline Myopathy |
| Z19459 | TPM2 | Nemaline Myopathy |
| D11793 | SLC2A1 | Glucose Transporter Type 1 Deficiency Syndrome |
| HSCHRX | NDP | Nome Disease |
| T62791 | OPA1 | Optic Atrophy 1 |
| Z24812 | OFD1 | Oral-Facial-Digital Syndrome Type I |
| HUMOTC | OTC | Ornithine Transcarbamvlase Deficiency |
| R66505 | MKKS | Bardet-Biedl Syndrome ;McKusick-Kaufman Syndrome |
| Z19438 | CHAC | Choreoacanthocytosis |
| HUMRDSA | RDS | Patterned Dystrophy of Retinal Pigment Epithelium ;Retinitis Pigmentosa, Autosomal Dominant |
| Z30072 | PLP1 | Hereditary Spastic Paraplegia, X-Linked ;PLP-Related Disorders |
| HSFGR1IG | FGFR1 | FGFR-Related Craniosynostosis Syndromes ;Pfeiffer Syndrome Type 1, 2, and 3 |
| HUMPHH | PAH | Phenylalanine Hydroxylase Deficiency |
| HSKITCR | KIT | Gastrointestinal Stromal Tumor ;Piebaldism |
| HSGROW1 | GH1 | Pituitary Dwarfism I |
| F00079 | GHR | Pituitary Dwarfism II |
| HSPIT1 | POU1F1 | Pituitary-Specific Transcription Factor Defects (PIT1) |
| T58874 | SDHD | Familial Nonchromaffin Paragangliomas |
| HUMINTB3 | ITGB3 | Integrin, Beta 3 ;Platelet Antigen Genotyping |
| T09245 | PKD1 | Polycystic Kidney Disease 1, Autosomal Dominant ;Polycystic Kidney Disease, Autosomal Dominant |
| T55657 | PKD2 | Polycystic Kidney Disease 2, Autosomal Dominant ;Polycystic Kidney Disease, Autosomal Dominant |
| T77325 | PKD2 | Polycystic Kidney Disease 2, Autosomal Dominant ;Polycystic Kidney Disease, Autosomal Dominant |
| W27963 | PKD2 | Polycystic Kidney Disease 2, Autosomal Dominant ;Polycystic Kidney Disease, Autosomal Dominant |
| R05352 | PKHD1 | Polycystic Kidney Disease, Autosomal Recessive |
| M77871 | PCLD | Polycystic Liver Disease |
| M78097 | UROD | Porphyria Cutanea Tarda |
| HUMPBG | HMBS | Acute Intermittent Porphyria |
| HUMRODSA | UROS | Congenital Erythropoietic Porphyria |

(continued)

| Gencarta Contig | Gene Symbol | Disease |
|---|---|---|
| T10891 | AGT | Angiotensinogen |
| T67463 | CTSK | Pycnodysostosis |
| M77954 | PDHA1 | Pyruvate Dehydrogenaso Deficiency, X-linked |
| Z19400 | PHYH | Refsum Disease, Adult |
| R07476 | PEX1 | Zellweger Syndrome Spectrum |
| Z24965 | RCA1 | Renal Cell Carcinoma |
| H37900 | RHO | Retinitis Pigmentosa, Autosomal Dommant ;Retinitis Pigmentosa, Autosomal Recessive |
| T24020 | RB1 | Retinoblastoma |
| Z44098 | RS1 | X-Linked Juvenile Retinoschisis |
| HSRH30A | RHCE | Rh C Genotyping ;Rh E Genotypmg |
| S57971 | RHCE | Rh C Genotyping :Rh E Genotyping |
| T89255 | RHCE | Rh C Genotyping :Rh E Genotyping |
| R60192 | PEX7 | Refsum Disease, Adult ;Rhizomelic Chondrodysplasia Punctata Type 1 |
| HUMMLC1AA | MLC1 | Megalencephalic Leukoencephalopathy with Subcortical Cysts |
| M79106 | MLC1 | Megalencephalic Leukoencephalopathy with Subcortical Cysts |
| T64905 | PITX2 | Anophthalmia ;Peters Anomaly ;Rieger Syndrome |
| Z41163 | CREBBP | Rubinstein-Taybi Syndrome |
| HSBHLH | TWIST1 | Saethre-Chotzen Syndrome |
| F00367 | EIF2B1 | Childhood Ataxia with Central Nervous System Hypomyelination/Vanishing White Matter |
| Z20030 | EIF2B2 | Childhood Ataxia with Central Nervous System Hypomyelination/Vanishing White Matter |
| Z41323 | EIF2B3 | Childhood Ataxia with Central Nervous System Hypomyelination/Vanishing White Matter |
| Z17882 | EIF2B4 | Childhood Ataxia with Central Nervous System Hypomyelmation/Vanishing White Matter |
| R13846 | EIF2B5 | Childhood Ataxia with Central Nervous System Hypomyelination/Vanishing White Matter ;Cree Leukoencephalopathy |
| T03917 | HEXB | Sandhoff Disease |
| HUMSRYA | SRY | XX Male Syndrome ;XY Gonadal Dysgenesis |
| HUMSCAD | ACADS | Short Chain Acyl-CoA Dehydrogenase Deficiency |
| HSALAS2R | ALAS2 | Sideroblastic Anemia, X-Linked |
| T47846 | GPC3 | Simpson-Golabi-Behmel Syndrome |
| T11069 | GUSB | Mucopolysaccharidosis Type VII |
| T08813 | SPG3A | Hereditary Spastic Paraplegia, Dominant ;SPG 3 |
| Z40639 | SPG3A | Hereditary Spastic Paraplegia, Dominant ;SPG 3 |
| M77964 | SPG4 | Hereditary Spastic Paraplegia, Dominant ;SPG 4 |
| N36808 | SMN1 | Spinal Muscular Atrophy |

(continued)

| Gencarta Contig | Gene Symbol | Disease |
|---|---|---|
| Z38265 | SMN1 | Spinal Muscular Atrophy |
| T06490 | SCA1 | Spinocerebellar Ataxia Type 1 |
| T55469 | SCA2 | Spinocerebellar Ataxia Type 2 |
| Z41764 | SCA2 | Spinocerebellar Ataxia Type 2 |
| T61453 | MJD | Spinocerebellar Ataxia Type 3 |
| HUMELASF | ELN | Cutis Laxa, Autosomal Dominant ;Supravalvular Aortic Stenosis |
| T05970 | HEXA | Hexosamimdase A Deficiency |
| M79184 | THRB | Thyroid Hormone Resistance |
| Z20729 | TCOF1 | Treacher Collins Syndrome |
| R48739 | TRPS1 | Trichorhinophalangeal Syndrome Type I |
| T77655 | TSC1 | Tuberous Sclerosis 1 ,Tuberous Sclerosis Complex |
| M78940 | TSC2 | Tuberous Sclerosis 2 ;Tuberous Sclerosis Complex |
| HSFAA | FAH | Tyrosinemia Type I |
| T39510 | TBX3 | Ulnar-Mammary Syndrome |
| HUMM7AA | MYO7A | Usher Syndrome Type 1 |
| W22160 | USHIC | Usher Syndrome Type 1 |
| T08506 | ACADVL | Very Long Chain Acyl-CoA Dehydrogenase Deficiency |
| HUMHIPLIND | VHL | Von Hippel-Lmdau Syndrome |
| HUMVWF | VWF | Von Willebrand Disease |
| HSU02368 | PAX3 | Waardenburg Syndrome Type I |
| H80461 | WRN | Werner Syndrome |
| HUMWND | ATP7B | Wilson Disease |
| T40645 | WAS | WAS Related Disorders |
| HSLAL | LIPA | Wolman Disease |
| HSASL1 | ASL | Argininosuccinicaciduria |
| HSAGAGENE | AGA | Aspartylglycosaminuria |
| T88756 | ATD | Asphyxiating Thoracic Dystrophy |
| Z19164 | ASAH | Farber Disease |
| HUMALD | FBP1 | Fructose 1,6 Bisphosphatase Deficiency |
| HSLDHAR | LDHA | Lactate Dehydrogenase Deficiency |
| M77886 | LDHB | Lactate Dehydrogenase Deficiency |
| HSU13680 | LDHC | Lactate Dehydrogenase Deficiency |
| Z46189 | MAN2B1 | Alpha-Mannosidosis |
| M79249 | MANBA | Beta-Mannosidosis |
| H26723 | GALNS | Mucopolysaccharidosis Type IVA |
| H23053 | SLC26A4 | DFNB 4 ;Enlarged Vestibular Aqueduct Syndrome ,Nonsyndromic Hearing Loss and Deafness, Autosomal Recessive ;Pendred Syndrome |

(continued)

| Gencarta Contig | Gene Symbol | Disease |
|---|---|---|
| HSPGK1 | PGK1 | Phosphoglycerate Kinase Deficiency |
| HSU08818 | MET | Papillary Renal Carcinoma |
| M79231 | PRCC | Papillary Renal Carcinoma |
| T08200 | GNS | Mucopolysaccharidosis Type IIID |
| HUMNAGB | NAGA | Schindler Disease |
| T08881 | NEU1 | Mucolipidosis I |
| R81783 | SLC17A5 | Free Sialic Acid Storage Disorders |
| HUMAUTONH | MTATP6 | Mitochondnal Disorders ;Mitochondrial DNA-Associated Leigh Syndrome and NARP |
| F09306 | SCA7 | Spinocerebellar Ataxia Type 7 |
| AF248482 | DAZ | Y Chromosome Infertility |
| HSU21663 | DAZ | Y Chromosomo Infertility |
| T47024 | JAG1 | Alagille Syndrome |
| HSRYRRM1 | RBMY1A1 | Y Chromosome Infertility |
| HSRYRRM2 | RBMY1A1 | Y Chromosome Infertility |
| HSVD3R | VDR | Osteoporosis ;Rickets Alopecia Syndrome |
| T40157 | FMO3 | Trimethylaminuria |
| HUMPHOSLIP | PPGB | Galactosialidosis |
| HUMPPR | PPGB | Galactosialidosis |
| H22222 | FANCC | Fanconi Anemia |
| D12009 | RPS6KA3 | Coffin-Lowry Syndrome |
| M78282 | PTEN | PTEN Hamartoma Tumor Syndrome (PHTS) |
| M78802 | FY | Duffy Antigen Genotypmg |
| HSU04270 | KCNH2 | LQT 2 ;Romano Ward Syndrome |
| T19733 | SCN5A | Brugada Syndrome .LOT 3 ;Romano-Ward Syndrome |
| HSTFIIDX | TBP | Spinocerebellar Ataxia Type17 |
| HUMKCHA | KCNA1 | Episodic Ataxia Type 1 |
| HSU78110 | NRTN | Hirschsprung Disease |
| HSET3AA | EDN3 | Hirschsprung Disease |
| Z17351 | ACE1 | Hirschsprung Disease |
| T47284 | DHCR7 | Smith-Lemli-Opitz Syndrome |
| HUMXIHB | HBZ | Alpha-Thalassemia |
| HSCP2 | CP | Aceruloplasminemia |
| N25320 | CLN6 | CLN6 Related Neuronal Ceroid-Lipofuscinosis ,Neuronal Ceroid-Lipofuscinoses |
| T11340 | NBS1 | Nijmegen Breakage Syndrome |
| Z40114 | NBS1 | Nijmegen Breakage Syndrome |
| HSU03688 | CYP1B1 | Glaucoma, Recessive (Congenital) ;Peters Anomaly |

(continued)

| Gencarta Contig | Gene Symbol | Disease |
|---|---|---|
| D62980 | MYOC | Glaucoma. Dommant (Juvenile Onset) |
| T98453 | NAGLU | Mucopolysaccharidosis Type IIIB |
| AA779817 | RUNX2 | Cleidocranial Dysplasia |
| HUMCBFA | RUNX2 | Cleidocranial Dysplasia |
| HSMARENO | MEFV | Familial Mediterranean Fever |
| F02180 | PHKB | Phosphorylase Kinase Deficiency of Liver and Muscle |
| D11905 | HPS1 | Hermansky-Pudlak Syndrome ;Hermansky-Pudlak Syndrome 1 |
| R95987 | CRX | Retinitis Pigmentosa, Autosomal Dominant |
| T05762 | EVC | Ellis-van Croveld Syndrome |
| T12126 | FLNA | Frontometaphyseal Dysplasia ;Melnick-Needles Syndrome ;Otopalatodigital Syndrome ;Periventricular Heterotopia, X-Linked |
| T60913 | EBP | Chondrodysplasia Punctata, X Linked Dominant |
| HSHNF4 | HNF4A | Maturity-Onset Diabetes of the Young Type I |
| HUMBGLUKIN | GCK | Familial Hyperinsulinism ;GCK-Related Hyperinsulinism ;Maturity-Onset Diabetes of the Young Type II |
| M62026 | GCK | Familial Hyperinsulinism ;GCK-Related Hyperinsulinism ,Maturity-Onset Diabetes of the Young Type II |
| R94860 | CIAS1 | Chronic Infantile Neurological Cutaneous and Articular Syndrome ;Familial Cold Urticana ;Muckle-Wells Syndrome |
| T08221 | SMARCAL1 | Schimke Immunoosseous Dysplasia |
| T95621 | SLC25A15 | Hyperornithinemia-Hyperammonemia-Homocitrullinuria Syndrome |
| HUMOATC | OAT | Ornithine Aminotransferase Deficiency |
| R08989 | MLYCD | Malonyl-CoA Decarboxylase Deficiency |
| T20008 | PMM2 | Congenital Disorders of Glycosylation |
| HSRPMI | MPI | Congenital Disorders of Glycosylation |
| HSSRECV6 | MGAT2 | Congenital Disorders of Glycosylation |
| T91755 | MGAT2 | Congenital Disorders of Glycosylation |
| HSCPTI | CPT1A | Carnitine Palmitoyltransferase IA (liver) Deficiency |
| HUMCPT | CPT2 | Carnitine Palmitoyltransferase II Deficiency |
| HSA1ATCA | SERPINA1 | Alpha-1-Antitrypsin Deficiency |
| N36808 | SMN2 | Spinal Muscular Atrophy |
| Z38265 | SMN2 | Spinal Muscular Atrophy |
| HUMACADL | ACADL | Long Chain Acyl-CoA Dehydrogenase Deficiency |
| Z25247 | CACT | Carnitine-Acylcarnitine Translocase Deficiency |
| HUMETFA | ETFA | Glutaricacidemia Type 2 |
| HSETFBS | ETFB | Glutaricacidemia Type 2 |
| S69232 | ETFDH | Glutaricacidemia Type 2 |
| T09377 | MEB | Muscle-Eye-Brain Disease |

(continued)

| Gencarta Contig | Gene Symbol | Disease |
|---|---|---|
| Z40427 | G6PT1 | Glycogen Storage Disease Type Ib |
| AI002801 | SLC14A1 | Kidd Genotyping |
| Z19313 | SLC14A1 | Kidd Genotyping |
| HUMPGAMM | PGAM2 | Phosphoglycerate Mutase Deficiency |
| H86930 | MPP4 | Retinitis Pigmentosa, Autosomal Recessive |
| HSU14910 | RGR | Retinitis Pigmentosa, Autosomal Recessive |
| AA775466 | CARD15 | Crohn Disease |
| AA306952 | GAN | Giant Axonal Neuropathy |
| T99245 | CLCN5 | Dent Disease |
| T23537 | NR3C2 | Pseudohypoaldosteronism Type 1, Dominant |
| HSLASNA | SCNN1A | Pseudohypoaldosteronism Type 1, Recessive |
| H26938 | SCNN1B | Pseudoaldosteronism ;Pseudohypoaldosteronism Type 1, Recessive |
| HUMGAMM | SCNN1G | Pseudoaldosteronism ;Pseudohypoaldosteronism Type 1, Recessive |
| HSP450AL | CYP11B2 | Familial Hyperaldosteronism Type 1 ;Familial Hypoaldosteronism Type 2 |
| HUMCYPADA | CYP11B1 | Familial Hyperaldosteronism Type 1 |
| AF017089 | COL11A1 | Stickler Syndrome ;Stickler Syndrome Type II |
| HUMCA1XIA | COL11A1 | Stickler Syndrome ;Stickler Syndrome Type II |
| HUMA2XICOL | COL11A2 | Stickler Syndrome |
| S61523 | PIGA | Paroxysmal Nocturnal Hemoglobinuria |
| T58881 | PHKA2 | Glycogen Storage Disease Type IX |
| Z39614 | DHAPAT | Rhizomelic Chondrodysplasia Punctata Type 2 |
| N89899 | SH2D1A | Lymphoproliferative Disease, X-Linked |
| HUMUGT1FA | UGT1A1 | Gilbert Syndrome |
| HUMNC1A | COL7A1 | Epidermolysis Bullosa Dystrophica, Bart Type ;Epidermolysis Bullosa Dystrophica, Cockayne-Touraine Type ;Epidermolysis Bullosa Dystrophica, Hallopeau-Siemens Type ;Epidermolysis Bullosa Dystrophica, Pasini Type ;Epidermolysis Bullosa, Pretibial |
| T49684 | ITGB4 | Epidermolysis Bullosa Letalis with Pyloric Atresia |
| S66196 | ITGA6 | Epidermolysis Bullosa Letalis with Pyloric Atresia |
| T10988 | LAMC2 | Epidermolysis Bullosa Junctional, Herlitz-Pearson Type |
| HUMLAMAA | LAMA3 | Epidermolysis Bullosa Junctional, Herlitz-Pearson Type |
| Z24848 | LAMA3 | Epidermolysis Bullosa Junctional, Herlitz-Pearson Type |
| T10484 | LAMB3 | Epidermolysis Bullosa Junctional, Disentis Type ;Epidermolysis Bullosa Junctional, Herlitz-Pearson Type |
| HUMBP180AA | COL17A1 | Epidermolysis Bullosa Junctional, Disentis Type |
| M78889 | PLEC1 | Epidermolysis Bullosa with Muscular Dystrophy |
| Z38659 | SLC22A5 | Carnitine Deficiency, Systemic |
| T85099 | CTNS | Cystinosis |

(continued)

| Gencarta Contig | Gene Symbol | Disease |
|---|---|---|
| W27253 | CNGA3 | Achromatopsia ;Achromatopsia 2 |
| HSU66088 | SLC5A5 | Thyroid Hormonogenesis Defect I |
| HUMTEKRPTK | TEK | Venous Malformation, Multiple Cutaneous and Mucosal |
| R69741 | SLC26A2 | Achondrogenesis Type 1B ;Atelosteogenesis Type 2 ;Diastrophic Dysplasia ;Multiple Epiphyseal Dysplasia, Recessive |
| Z46092 | PEX10 | Zellweger Syndrome Spectrum |
| S55790 | COL4A3 | Alport Syndrome ;Alport Syndrome, Autosomal Recessive |
| HSCOL4A4 | COL4A4 | Alport Syndrome ;Alport Syndrome, Autosomal Recessive |
| T10559 | SHFM3 | Ectrodactyly |
| T93670 | FANCA | Fanconi Anemia |
| H47777 | FANCB | Fanconi Anemia |
| AA542822 | FANCE | Fanconi Anemia |
| HUMPSPB | PSAP | Metachromatic Leukodystrophy |
| HUMSAPA1 | PSAP | Metachromatic Leukodystrophy |
| S69686 | PSAP | Metachromatic Leukodystrophy |
| AA252786 | NCF1 | Chronic Granulomatous Disease |
| HUMNCF1A | NCF1 | Chronic Granulomatous Disease |
| HSTGFB1 | TGFB1 | Camurati-Engelmann Disease |
| R24242 | CYBA | Chronic Granulomatous Disease |
| HUMNOXF | NCF2 | Chronic Granulomatous Disease |
| S41458 | PDE6B | Retinitis Pigmentosa, Autosomal Recessive |
| R21727 | DYSF | Dysferlinopathy ;Limb-Girdle Muscular Dystrophies, Autosomal Recessive |
| AF055580 | USH2A | Usher Syndrome Type 2 ;Usher Syndrome Type 2A |
| N36632 | MITF | Waardenburg Syndrome Type II ;Waardenburg Syndrome Type IIA |
| M78027 | MYH9 | DFNA 17 ;Epstein Syndrome ;Fechtner Syndrome ;May-Hegglin Anomaly ;Sebastian Syndrome |
| Z40194 | HPS4 | Hermansky-Pudlak Syndrome |
| AA333774 | GP1BA | Platelet Antigen Genotyping |
| M79110 | GP1BB | Platelet Antigen Genotyping |
| HUMGPIIBA | ITGA2B | Platelet Antigen Genotyping |
| T29174 | ITGA2 | Glycoprotein 1a Deficiency ;Platelet Antigen Genotyping |
| HSGST4 | GSTM1 | Lung Cancer |
| AA338271 | CHEK2 | Li-Fraumeni Syndrome |
| T78869 | CHEK2 | Li-Fraumeni Syndrome |
| T03839 | SH3BP2 | Cherubism |
| T67412 | IRF6 | IRF6-Related Disorders |
| AB037973 | FGF23 | Hypophosphatemic Rickets, Dominant |
| T60199 | FBLN5 | Cutis Laxa, Autosomal Recessive |

(continued)

| Gencarta Contig | Gene Symbol | Disease |
|---|---|---|
| T03890 | ARX | ARX-Related Disorders |
| M79175 | NSD1 | Sotos Syndrome |
| T07860 | NSD1 | Sotos Syndrome |
| M79181 | COH1 | Cohen Syndrome |
| MIHS75KDA | NDUFS1 | Leigh Syndrome (nuclear DNA mutation) ;Mitochondrial Respiratory Chain Complex I Deficiency |
| T09312 | NDUFV1 | Leigh Syndrome (nuclear DNA mutation) ;Mitochondrial Respiratory Chain Complex I Deficiency |
| AA399371 | SALL4 | Acrorenoocular Syndrome ;Okihiro Syndrome |
| HUMA8SEQ | TIMP3 | Pseudoinflammatory Fundus Dystrophy |
| Z40623 | GDAP1 | Charcot-Marie-Tooth Neuropathy Type 4 ;Charcot-Marie-Tooth Neuropathy Type 4A |
| AA128030 | FOXL2 | Blepharophimosis, Epicanthus Inversus, Ptosis |
| HUMCRTR | SLC6A8 | Creatine Deficiency Syndrome, X-Linked |
| T08882 | JPH3 | Huntington Disease-Like 2 |
| T07283 | SNRPN | Autistic Disorder ;Pervasive Developmental Disorders |
| Z38837 | SPR | Sepiapterin Reductase Deficiency (SR) |
| HUMANTIR | AGTR1 | Angiotensin II Receptor, Type 1 |
| T46961 | SEPN1 | Congenital Muscular Dystrophy with Early Spine Rigidity ;Multiminicore Disease |
| Z43954 | TRIM32 | Limb-Girdle Muscular Dystrophies, Autosomal Recessive |
| Z19219 | TTID | Limb-Girdle Muscular Dystrophies, Autosomal Dominant |
| HSECADH | CDH1 | Hereditary Diffuse Gastric Cancer |
| Z41199 | WFS1 | Nonsyndromic Low-Frequency Sensorineural Hearing Loss ;Wolfram Syndrome |
| HUMLORAA | LOR | Progressive Symmetric Erythrokeratoderma |
| Z38324 | HR | Alopecia Universalis ;Papular Atrichia |
| T09039 | RYR1 | Central Core Disease of Muscle ;Malignant Hyperthermia Susceptibility ;Multiminicore Disease |
| T10442 | GALE | Galactose Epimerase Deficiency |
| D82541 | PDB2 | Paget Disease of Bone |
| HSU20759 | CASR | Autosomal Dominant Hypocalcemia ;Familial Hypocalciuric Hypercalcemia, Type I ;Familial Isolated Hypoparathyroidism ;Neonatal Severe Primary Hyperparathyroidism |
| AA071082 | SALL1 | Townes-Brocks Syndrome |
| T81692 | EDAR | Hypohidrotic Ectodermal Dysplasia ;Hypohidrotic Ectodermal Dysplasia, Autosomal |
| HUMHPA1B | HP | Anhaptoglobinemia |
| HSU01922 | TIMM8A | Deafness-Dystonia-Optic Neuronopathy Syndrome |
| HUMHSDI | HSD3B2 | Prostate Cancer |
| HSU05659 | HSD17B3 | Prostate Cancer |

(continued)

| Gencarta Contig | Gene Symbol | Disease |
|---|---|---|
| Z38915 | NPHP4 | Nephronophthisis 4 ;Senior-Loken Syndrome |
| HSC1INHR | SERPTNG1 | Hereditary Angioneurotic Edema |
| D62739 | BBS7 | Bardet-Biedl Syndrome |
| T64266 | SLC7A7 | Lysinuric Protein Intolerance |
| S52028 | CTH | Cystathioninuria |
| Z30254 | EFEMP1 | Doyne Honeycomb Retinal Dystrophy ;Patterned Dystrophy of Retinal Pigment Epithelium |
| D59254 | ELOVL4 | Stargardt Disease 3 |
| S43856 | GCH1 | Dopa-Responsive Dystoma ;GTP Cyclohydrolase 1-Deficient DRD ;GTP Cyclohydrolase-1 Deficiency (GTPCH) |
| M78468 | PAFAH1B1 | 17-Linked Lissencephaly |
| M78473 | PAFAH1B1 | 17 Linked Lissencephaly |
| S51033 | MID1 | Opitz Syndrome, X Linked |
| Z40343 | MIDI | Opitz Syndrome, X Linked |
| HUM6PTHS | PTS | Pyruvoyltetrahydropterin Synthase Deficiency |
| M62103 | CIRH1A | North American Indian Childhood Cirrhosis |
| HSDHPR | QDPR | Dihydropteridine Reductase Deficiency (DHPR) |
| T23665 | FKRP | Congemtal Muscular Dystrophy Type 1C ;Limb-Girdle Muscular Dystrophies, Autosomal Recessive |
| T60498 | LRPPRC | Leigh Syndrome, French Canadian Type |
| HSACHRA | CHRNA1 | Congenital Myasthenic Syndromes |
| HSACHRB | CHRNB1 | Congenital Myasthemc Syndromes |
| HSACHRG | CHRND | Congenital Myasthemc Syndromes |
| HSACETR | CHRNE | Congenital Myasthenic Syndromes |
| HSACRAP | RAPSN | Congenital Myasthenic Syndromes |
| M78334 | COLQ | Congenital Myasthenic Syndromes |
| S56138 | CHAT | Congenital Myasthenic Syndromes |
| D11584 | SDHC | Familial Nonchromaffin Paragangliomas |
| HSPSTI | SPINK1 | Hereditary Pancreatitis |
| HSSPROTR | PROS1 | Protem S Heerlen Variant |
| HUMLAP | ITGB2 | Leukocyte Adhesion Deficiency, Type 1 |
| T12572 | ADAMTS13 | Familial Thrombotic Thrombocytopenia Purpura |
| HUMCOMIIP | SDHB | Carotid Body Tumors and Multiple Extraadrenal Pheochromocytomas |
| NM005912 | MC4R | Obesity |
| HUMPAX8A | PAX8 | Congenital Hypothyroidism |
| AA037119 | FOXE1 | Bamforth-Lazarus Syndrome ;Congenital Hypothyroidism |
| AV754057 | FSHB | Isolated Follicle Stimulating Hormone Deficiency |
| HUMHOMEOA | PCBD | Pterin-4a Carbinolamine Dehydratase Deficiency (PCD) |

(continued)

| Gencarta Contig | Gene Symbol | Disease |
|---|---|---|
| HSTHR | TH | Dopa-Responsive Dystoma ;Tyrosine Hydroxylase-Deficient DRD |
| AA219596 | ZIC3 | Heterotaxy Syndrome |
| HSU20324 | CSRP3 | Dilated Cardiomyopathy |
| HUMPHLAM | PLN | Dilated Cardiomyopathy |
| F10219 | ALMS1 | Alstrom Syndrome |
| T06612 | VCL | Dilated Cardiomyopathy |
| AF388566 | USH3A | Usher Syndrome Type 3 |
| Z40797 | SGCE | Myoclonus-Dystonia |
| T08448 | RAB7 | Charcot-Mane-Tooth Neuropathy Type 2 |
| D12383 | GARS | Charcot-Marie-Tooth Neuropathy Type 2 |
| Z36734 | HRPT2 | HRPT2-Related Disorders |
| H19914 | EDARADD | Hypohidrotic Ectodermal Dysplasia ;Hypohidrotic Ectodermal Dysplasia, Autosomal |
| T08852 | PPT1 | Neuronal Ceroid-Lipofuscinoses ;PPT1-Related Neuronal Ceroid-Lipofuscinosis |
| HUMDRA | SLC26A3 | Familial Chloride Diarrhea |
| R16324 | AGPAT2 | Berardinelh-Seip Congenital Lipodystrophy |
| Z38569 | BSCL2 | Berardinelli-Seip Congenital Lipodystrophy |
| W28410 | OPN1MW | Blue-Mono-Cone-Monochromatic Type Colorblindness |
| T27896 | OPN1LW | Blue-Mono-Cone Monochromatic Type Colorblindness |
| AI469991 | PHOX2A | Congenital Fibrosis of Extraocular Muscles |
| HSFSTHR | FSHR | Premature Ovarian Failure, Autosomal Recessive |
| HSLPH | LCT | Hypolactasia, Adult Type |
| Z41000 | BCS1L | Gracile Syndrome ;Mitochondnal Respiratory Chain Complex III Deficiency |
| HSCGJP | GJA1 | Oculodentodigital Dysplasia |
| HSPERFP1 | PRF1 | Familial Hemophagocytic Lymphohistiocytosis 2 |
| M78112 | GLUD1 | Familial Hyperinsulinism ;GLUD1-Related Hyperinsulinism |
| W79230 | RAX | Anophthalmia |
| AF041339 | PITX3 | Anophthalmia |
| AA151708 | HESX1 | Anophthalmia |
| HSSOXB | SOX3 | Anophthalmia ;Mental Retardation, X-Linked, with Growth Hormone Deficiency |
| HUMHMGBOX | SOX2 | Anophthalmia |
| HSGM2APA | GM2A | GM2 Activator Deficiency |
| Z19280 | GLC1E | Glaucoma, Dominant (Adult Onset) |
| T20165 | PHF6 | Borjeson-Forssman-Lehmann Syndrome |
| Z40394 | CMT4B2 | Charcot Marie-Tooth Neuropathy Type 4 |
| HUMIHH | IHH | Brachydactyly Type A1 |
| HUMCDPK | CDK4 | Familial Malignant Melanoma |

(continued)

| Gencarta Contig | Gene Symbol | Disease |
|---|---|---|
| T39355 | SBDS | Shwachman-Diamond Syndrome |
| HSHMPLK | MPL | Amegakaryocytic Thrombocytopenia, Congemtal |
| Z38860 | TRIM37 | Mulibrey Nanism |
| M62027 | DTNA | Familial Isolated Noncompaction of Left Ventrical Myocardium |
| Z39175 | DDB2 | Xeroderma Pigmentosum |
| T09329 | MUTYH | MYH-Associated Polyposis |
| HUMAPA | APP | Alzheimer Disease Type 1 ;Early-Onset Familial Alzheimer Disease |
| M79090 | GSS | 5-Oxoprolinuria |
| Z26981 | OXCT | 3-Oxoacid CoA Transferase |
| D12046 | PMS1 | Hereditary Non Polyposis Colon Cancer |
| T08186 | PMS2 | Hereditary Non Polyposis Colon Cancer |
| R00471 | MSH6 | Hereditary Non Polyposis Colon Cancer |
| T60457 | NDUFS4 | Leigh Syndrome (nuclear DNA mutation) ;Mitochondrial Respiratory Chain Complex I Deficiency |
| D30864 | NDUFS8 | Leigh Syndrome (nuclear DNA mutation) |
| M78107 | SDHA | Leigh Syndrome (nuclear DNA mutation) |
| R15290 | NDUFS7 | Leigh Syndrome (nuclear DNA mutation) |
| HUMPCBA | PC | Pyruvate Carboxylase Deficiency |
| W32719 | AASS | Hyperlysinemia |
| T23789 | PEX3 | Zellweger Syndrome Spectrum |
| T09086 | STK11 | Peutz Jeghers Syndrome |
| T87335 | HAL | Histidinemia |
| Z19082 | ALDH4A1 | Hyperprolinemia, Type II |
| Z25227 | MADH4 | Juvenile Polyposis Syndrome |
| M78130 | XPB | Xeroderma Pigmentosum |
| T08987 | XPD | Xeroderma Pigmentosum |
| D81449 | XPF | Xeroderma Pigmentosum |
| HSXPGAA | XPG | Xeroderma Pigmentosum |
| HSAUHMR | AUH | 3-Methylglutaconic Aciduria Type 1 |
| T19530 | MMAB | Methylmalonicaciduria |
| Z40169 | MMAA | Methylmalonicaciduria |
| T93695 | BCAT1 | Hynerleucine-Isoleucinemia |
| Z41266 | BCAT2 | Hyperleucine-Isoleucinemia |
| HSU03506 | SLC1A1 | Dicarboxylicaminoaciduria |
| R88591 | PRODH | Hyperprolinemia, Type I |
| T05380 | EPM2A | Progressive Myoclonus Epilepsy, Lafora Type |
| T27227 | FANCF | Fanconi Anemia |

(continued)

| Gencarta Contig | Gene Symbol | Disease |
|---|---|---|
| Z41736 | FANCG | Fanconi Anemia |
| R66178 | ED4 | Ectodermal Dysplasia, Margarita Island Type |
| L25197 | KCNE1 | Jervell and Lange-Nielsen Syndrome ;LQT 5 ;Romano-Ward Syndrome |
| HUMUMOD | UMOD | Familial Nephropathy with Gout ;Medullary Cystic Kidney Disease 2 |
| HSU66583 | CRYGD | Cataract, Crystalline Aculeiform |
| HSPHR | PTHR1 | Chondrodysplasia, Blomstrand Type |
| T97980 | MTRR | Homocystinuria-Megaloblastic Anemia |
| S60710 | ADSL | Adenylosuccinase deficiency |
| Z38216 | SLC25A19 | Amish Lethal Microcephaly |
| T11501 | DBH | Dopamine Beta-Hydroxylase Deficiency |
| H11439 | NLGN3 | Autistic Disorder ;Pervasive Developmental Disorders |
| R12551 | NLGN4 | Autistic Disorder ;Pervasive Developmental Disorders |
| M78212 | ATP1A2 | Familial Hemiplegic Migraine |
| T96957 | SPCH1 | Severe Speech Delay |
| AI266171 | PHOX2B | Congenital Central Hypoventilation Syndrome |
| BG723199 | DSG4 | Localized Autosomal Recessive Hypotrichosis |
| T46918 | HSD11B2 | Apparent Mineralocorticoid Excess Syndrome |
| HUMFERLS | FTL | Hyperferritinemia Cataract Syndrome |
| HUMCKRASA | KRAS2 | Familial Pancreatic Cancer |
| S39383 | PTPN11 | LEOPARD Syndrome ;Noonan Syndrome |
| HUMSTAR | STAR | Cholesterol Desmolase Deficiency |
| Z20453 | STAR | Cholesterol Desmolase Deficiency |
| HUMVPC | AVP | Neurohypophyseal Diabetes Insipidus |
| M62144 | MECP2 | Rett Syndrome |
| HSCA2VR | COL5A2 | Ehlers-Danlos Syndrome, Classic Type |
| HUMGENX | TNXB | Ehlers-Danlos-like Syndrome Due to Tenascin-X Deficiency |
| R02385 | TNXB | Ehlers-Danlos-like Syndrome Due to Tenascin-X Deficiency |
| T39901 | LITAF | Charcot-Marie-Tooth Neuropathy Type 1 |
| AA621310 | FOXE3 | Anophthalmia |
| H18132 | CFC1 | Heterotaxy Syndrome |
| R36719 | EBAF | Heterotaxy Syndrome |
| HSACTIIRE | ACVR2B | Heterotaxy Syndrome |
| T52017 | CRELD1 | Heterotaxy Syndrome |
| D11851 | LMNA | Dilated Cardiomyopathy ;Emery-Dreifuss Muscular Dystrophy, Autosomal Dominant ;Familial Partial Lipodystrophy, Dunnigan Type ;Hutchinson-Gilford Progeria Syndrome ;Limb-Girdle Muscular Dystrophies, Autosomal Dominant ;Mandibuloacral Dysplasia |

(continued)

| Gencarta Contig | Gene Symbol | Disease |
|---|---|---|
| D12062 | DSP | Cardiomyopathy, Dilated, with Woolly Hair and Keratoderma ;Keratosis Palmoplantaris Striata |
| H99382 | MSH3 | Hereditary Non-Polyposis Colon Cancer |
| AW205295 | NOG | Multiple Synostoses Syndrome |
| AA135181 | GJB3 | Erythrokeratodermia Variabilis |
| F10278 | PEO1 | Mitochondrial DNA Deletion Syndromes |
| M62022 | MASS1 | Febrile Seizures |
| Z42549 | UQCRB | Mitochondrial Respiratory Chain Complex III Deficiency |
| HUMEGR2A | EGR2 | Charcot-Marie-Tooth Neuropathy Type 1 ;Charcot-Marie-Tooth Neuropathy Type ID ;Charcot-Marie-Tooth Neuropathy Type 4 ;Charcot-Marie-Tooth Neuropathy Type 4E |
| HSFLT4X | FLT4 | Milroy Congenital Lymphedema |
| Z28459 | PEX26 | Zellweger Syndrome Spectrum |
| HUMRPS24A | RPS19 | Diamond-Blackfan Anemia |
| T11633 | RPS19 | Diamond-Blackfan Anemia |
| HSACMHCP | MYH7 | Dilated Cardiomyopathy ;Familial Hypertrophic Cardiomyopathy |
| Z25920 | TNNT2 | Dilated Cardiomyopathy ;Familial Hypertrophic Cardiomyopathy |
| HUMTRO | TPM1 | Dilated Cardiomyopathy ;Familial Hypertrophic Cardiomyopathy |
| Z18303 | MYBPC3 | Dilated Cardiomyopathy ;Familial Hypertrophic Cardiomyopathy |
| HSU09466 | COX10 | Leigh Syndrome (nuclear DNA mutation) |
| S72487 | ECGF1 | Mitochondrial Neurogastrointestinal Encephalopathy Syndrome |
| M62196 | KIF5A | Hereditary Spastic Paraplegia, Dominant |
| T07578 | KIF5A | Hereditary Spastic Parapleaia, Dominant |
| D11648 | HSPD1 | Hereditary Spastic Paraplegia, Dominant |
| T47330 | SOX18 | Hypotrichosis-Lymphedema-Telangiectasia Syndrome |
| AA448334 | CAV3 | Caveolinopathy ;Limb-Girdle Muscular Dystrophies, Autosomal Dominant |
| AWO71529 | ALX4 | Parietal Foramina 2 |
| M61973 | CD2AP | Focal Segmental Glomerulosclerosis |
| W21801 | NR2E3 | Enhanced S-Cone Syndrome |
| Z20305 | TREM2 | PLOSL |
| T05421 | ANK2 | LQT 4 ;Romano-Ward Syndrome |
| HUMROR2A | ROR2 | ROR2-Related Disorders |
| Z25920 | CMD1D | Dilated Cardiomyopathy |
| AA887962 | HLXB9 | Currarino Syndrome |
| R00281 | ALDH5A1 | Succinic Semialdehyde Dehydrogenase Deficiency |
| HSPCCAR | PCCA | Propionic Acidemia |
| N43992 | DLL3 | Spondylocostal Dysostosis, Autosomal Recessive ;Syndactyly, Type IV |
| Z39790 | MUT | Methylmalonicaciduria |

(continued)

| Gencarta Contig | Gene Symbol | Disease |
|---|---|---|
| HUMARGL | ARG1 | Argininemia |
| HUMRENBAT | SLC3A1 | Cystinuria |
| T80665 | SLC7A9 | Cystinuria |
| T27286 | HGD | Alkaptonuria |
| HUMBCKDH | BCKDHA | Maple Syrup Urine Disease |
| HUMBCKDHA | BCKDHB | Maple Syrup Urine Disease |
| HSTRANSP | DBT | Maple Syrup Urine Disease |
| Z44722 | HLCS | Holocarboxylase Synthetase Deficiency |
| Z38396 | BTD | Biotinidaso Deficiency |
| T48178 | POMT1 | Walker-Warburg Syndrome |
| T28737 | GJB2 | DFNA 3 Nonsyndromic Hearing Loss and Deafness ;DFNB 1 Nonsyndromic Hearing Loss and Deafness ;GJB2-Related DFNA 3 Nonsyndromic Hearing Loss and Deafness ;GJB2-Related DFNB 1 Nonsyndromic Hearing Loss and Deafness ;Nonsyndromic Hearing Loss and Deafness, Autosomal Dominant ;Nonsyndromic Hearing Loss and Deafness, Autosomal Recessive ;Vohwinkel Syndrome |
| T05861 | COCH | DFNA 9 (COCH) ;Nonsyndromic Hearing Loss and Deafness, Autosomal Dominant |
| HSBRN4 | POU3F4 | DFN 3 |
| HSU21938 | TTPA | Ataxia with Vitamin E Deficiency (AVED) |
| T93783 | K1AA1985 | Charcot-Marie-Tooth Neuropathy Type 4 |
| BE735997 | SANS | Usher Syndrome Type 1 |
| AA548783 | HOXD13 | Syndactyly, Type II |
| R33750 | HOXA13 | Hand-Foot-Uterus Syndrome |
| HUMPP | GLDC | GLDC-Related Glycine Encephalopathy ;Glycine Encephalopathy |
| F04230 | AMT | AMT-Related Glycine Encephalopathy ;Glycine Encephalopathy |
| T54795 | DECR | 2,4-Dienoyl-CoA Reductase Deficiency |
| R07295 | ACAT1 | Ketothiolase Deficiency |
| S70578 | ACAT1 | Ketothiolase Deficiency |
| HUMMEVKIN | MVK | Hyper IgD Syndrome ;Mevalonicaciduria |
| T11245 | HMGCL | 3-Hydroxy-3-Methylglutaryl-Coenzyme A Lyase Deficiency |
| Z41427 | GCDH | Glutaricacidemia Type 1 |
| HSSHOXA | SHOX | Langer Mesomelic Dwarfism ;Leri-Weill Dyschondrosteosis ;Short Stature |
| HUMDOPADC | DDC | Aromatic L-Amino Acid Decarboxylase Deficiency |
| HSCOL3A4 | COL6A3 | Limb-Girdle Muscular Dystrophies, Autosomal Dominant |
| HSCOL1A4 | COL6A1 | Limb-Girdle Muscular Dystrophies, Autosomal Dominant |
| HSCOL2C2 | COL6A2 | Limb-Girdle Muscular Dystrophies, Autosomal Dominant |
| H16770 | RECQL4 | Rothmund-Thomson Syndrome |
| H11473 | SGSH | Mucopolysaccharidosis Type IIIA |

(continued)

| Gencarta Contig | Gene Symbol | Disease |
|---|---|---|
| H67137 | MCCC1 | 3-Methylcrotonyl-CoA Carboxylase Deficiency |
| R88931 | MCCC2 | 3-Methylcrotonyl-CoA Carboxylase Deficiency |
| Z24865 | TCAP | Dilated Cardiomyopathy ;Limb-Girdle Muscular Dystrophies, Autosomal Recessive |
| M86030 | DCX | DCX-Related Malformations |
| HUMACTASK | ACTA1 | Nemaline Myopathy |
| HSDGIGLY | DSG1 | Keratosis Palmoplantaris Striata |
| HSRETSA | SAG | Retinitis Pigmentosa, Autosomal Recessive |
| HSAPHOL | ALPL | Hypophosphatasia |
| N73784 | XPA | Xeroderma Pigmentosum |
| T28958 | XPC | Xeroderma Pigmentosum |
| N69543 | POLH | Xeroderma Pigmentosum |
| T54103 | POLH | Xeroderma Pigmentosum |
| H56484 | CKN1 | Cockayne Syndrome |
| Z38185 | ERCC6 | Cockayne Syndrome |
| F07041 | PI12 | Familial Encephalopathy with Neuroserpin Inclusion Bodies |
| AA633404 | KCNE2 | LQT 6 ;Romano-Ward Syndrome |
| HSTITINC2 | CMD1G | Dilated Cardiomyopathy |
| N99115 | NPHP1 | Nephronophthisis 1 ;Senior-Loken Syndrome |
| HUMELANAA | ELA2 | ELA2-Related Neutropenia |
| S67325 | PCCB | Propionic Acidemia |
| HSGA7331 | M1S1 | Corneal Dystrophy, Gelatinous Drop-Like |
| HSACE | ACE | Angiotensin I Converting Enzyme 1 |
| S49816 | TSHR | Congenital Hypothyroidism ;Familial Non-Autoimmune Hyperthyroidism |
| Z30221 | VMGLOM | Multiple Glomus Tumors |
| H88042 | COL9A3 | Multiple Epiphyseal Dysplasia, Dominant |
| M78119 | ADA | Adenosine Deaminase Deficiency |
| T55785 | GAMT | Guanidinoacetate Methyltransferase Deficiency |
| HUMCST4BA | CSTB | Myoclonic Epilepsy of Unverricht and Lundborg |
| S73196 | AQP2 | Nephrogenic Diabetes Insipidus ;Nephrogenic Diabetes Insipidus, Autosomal |
| HSU76388 | NR5A1 | XY Sex Reversal with Adrenal Failure |
| HSCPHC22 | MTRNR1 | MTRNR1-Related Hearing Loss and Deafness |
| H21596 | PPARG | Diabetes Mellitus with Acanthosis Nigricans and Hypertension |
| D56550 | FOXC1 | Anophthalmia ;Rieger Syndrome |
| M78868 | AP3B1 | Hermansky-Pudlak Syndrome |
| T47068 | NOTCH3 | CADASIL |
| HSHMF1C | TCF1 | Maturity-Onset Diabetes of the Young Type III |

(continued)

| Gencarta Contig | Gene Symbol | Disease |
|---|---|---|
| AF049893 | IPF1 | Maturity-Onset Diabetes of the Young Type IV |
| HSU30329 | IPF1 | Maturity-Onset Diabetes of the Young Type IV |
| HSVHNF1 | TCF2 | Maturity-Onset Diabetes of the Young Type V |
| HUMLDLRFMT | LDLR | Familial Hypercholesterolemia |
| HSAPOBR2 | APOB | Familial Hypercholesterolemia Type B |
| T78010 | ABCB7 | Sideroblastic Anemia and Ataxia |
| AF076215 | PROP1 | PROP 1-Related Combined Pituitary Hormone Deficiency |
| S99468 | ALAD | Acute Hepatic Porphyria |
| T61818 | ABCC2 | Dubin-Johnson Syndrome |
| HUMLCAT | LCAT | Lecithin Cholesterol Acyltransferase Deficiency |
| Z38510 | HADHSC | Short Chain 3-Hydroxyacyl-CoA Dehydrogenase Deficiency, Liver |
| AF041240 | PPOX | Variegate Porphyria |
| T77011 | PPOX | Variegate Porphyria |
| Z40014 | ALDH10 | Sjogren-Larsson Syndrome |
| S79867 | KRT16 | Nonepidermolytic Palmoplantar Hyperkeratosis ;Pachyonychia Congenita |
| HUMKER56K | KRT6A | Pachyonychia Congenita |
| HSKERELP | KRT17 | Pachyonychia Congenita ;Steatocystoma Multiplex |
| R11850 | KRT6B | Pachyonychia Congenita |
| S69510 | KRT9 | Epidermolytic Palmoplantar Keratoderma |
| HSCYTK | KRT13 | White Sponge Nevus of Cannon |
| T92918 | KRT4 | White Sponge Nevus of Cannon |
| S54769 | SPG7 | Hereditary Spastic Paraplegia, Recessive ;SPG 7 |
| T50707 | FECH | Erythropoiedc Protoporphyria |
| HUMPOMM | PXMP3 | Zellweger Syndrome Spectrum |
| R05392 | PEX6 | Zellweger Syndrome Spectrum |
| Z38759 | PEX12 | Zellweger Syndrome Spectrum |
| R14480 | PEX16 | Zellweger Syndrome Spectrum |
| R10031 | PEX13 | Zellweger Syndrome Spectrum |
| R13532 | PXF | Zellweger Syndrome Spectrum |
| Z30136 | AGPS | Rhizomelic Chondrodysplasia Punctata Type 3 |
| HSU07866 | ACOX | Pseudoneonatal Adrenoleukodystrophy |
| N63143 | ALG6 | Congenital Disorders of Glycosylation |
| HSTNFR1A | TNFRSF1A | Familial Hibernian Fever |
| AA018811 | RP1 | Retinitis Pigmentosa, Autosomal Dominant |
| HSG11 | RP1 | Retinitis Pigmentosa, Autosomal Dominant |
| T07942 | RP1 | Retinitis Pigmentosa, Autosomal Dominant |
| H28658 | PRPF31 | Retinitis Pigmentosa, Autosomal Dominant |

(continued)

| Gencarta Contig | Gene Symbol | Disease |
|---|---|---|
| T07062 | PRPF8 | Retinitis Pigmentosa, Autosomal Dominant |
| T05573 | RP18 | Retinits Pigmentosa, Autosomal Dominant |
| HUMNRLGP | NRL | Retinitis Pigmentosa, Autosomal Dominant |
| T87786 | CRB1 | Retinitis Pigmentosa, Autosomal Recessive |
| H92408 | TULP1 | Retinitis Pigmentosa, Autosomal Recessive |
| S42457 | CNGA1 | Retinitis Pigmentosa, Autosomal Recessive |
| H30568 | PDE6A | Retinitis Pigmentosa, Autosomal Recessive |
| M78192 | RLBP1 | Retinitis Pigmentosa, Autosomal Recessive ;Retinitis Pigmentosa, Autosomal Recessive, Bothnia Type |
| T10761 | SLC4A4 | Proximal Renal Tubular Acidosis with Ocular Abnormalities |
| N64339 | GJB6 | DFNA 3 Nonsyndromic Hearing Loss and Deafness ;DFNB 1 Nonsyndromic Hearing Loss and Deafness ;GJB6-Related DFNB 1 Nonsyndromic Hearing Loss and Deafness ;GJB6-Related DFNA 3 Nonsyndromic Hearing Loss and Deafness ;Hidrotic Ectodermal Dysplasia 2 ;Nonsyndromic Hearing Loss and Deafness, Autosomal Dominant ;Nonsyndromic Hearing Loss and Deafness, Autosomal Recessive |
| T67968 | MAT1A | Isolated Persistent Hypermethioninemia |
| HUMUMPS | UMPS | Oroticaciduria |
| HSPNP | NP | Purine Nucleoside Phosphorylase Deficiency |
| AB006682 | AIRE | Autoimmune Polyendocrinopathy Syndrome Type 1 |
| BE871354 | JUP | Naxos Disease |
| T08214 | JUP | Naxos Disease |
| F00120 | DES | Dilated Cardiomyopathy |
| R28506 | MOCS1 | Molybdenum Cofactor Deficiency |
| T70309 | MOCS2 | Molvbdenum Cofactor Deficiency |
| T08212 | SNCA | Parkinson Disease |
| R99091 | ABCC6 | Pseudoxanthoma Elasticum |
| T69749 | ABCC6 | Pseudoxanthoma Elasticum |
| AA207040 | PRG4 | Arthropathy Camptodactyly Syndrome |
| T07189 | PRG4 | Arthropathy Camptodactyly Syndrome |
| F07016 | OPPG | Osteoporosis Pseudoghoma Syndrome |
| H27782 | SCO2 | Fatal Infantile Cardioencephalopathy due to COX Deficiency |
| S54705S1 | PRKAR1A | Carney Complex |
| Z25903 | SCA10 | Spinocerebellar Ataxia Type10 |
| AA592984 | WISP3 | Progressive Pseudorheumatoid Arthropathy of Childhood |
| Z39666 | MCOLN1 | Mucolipidosis IV |
| HSEMX2 | EMX2 | Familial Schizencephaly |
| HUMSP18A | SFTPB | Pulmonary Surfactant Protein B Deficiency |

(continued)

| Gencarta Contig | Gene Symbol | Disease |
|---|---|---|
| T10596 | ATP8B1 | Benign Recurrent Intrahepatic Cholestasis ;Progressive Familial Intrahepatic Cholestasis ;Progressive Familial Intrahepatic Cholestasis 1 |
| U46845 | CYP27B1 | Pseudovitamin D Deficiency Rickets |
| Z21585 | MAPT | Frontotemporal Dementia with Parkinsonism-17 |
| HSPPD | HPD | Tyrosinemia Type III |
| HUMUGT1FA | UGT1A | Crigler-Najjar Syndrome |
| R20880 | SLC19A2 | Thiamine-Responsive Megaloblastic Anemia Syndrome |
| H42203 | TFAP2B | Char Syndrome |
| Z30126 | RYR2 | Catecholaminergic Ventricular Tachycardia, Autosomal Dominant |
| HSSPYRAT | AGXT | Hyperoxaluria, Primary, Type 1 |
| T80758 | SEDL | Spondyloepiphyseal Dysplasia Tarda, X-Linked |
| T89449 | SEDL | Spondyloepiphyseal Dysplasia Tarda, X-Linked |
| AA373083 | FOXC2 | Lymphedema with Distichiasis |
| HUMPROP2AB | SCA12 | Spinocerebellar Ataxia Type12 |
| Z30145 | ACTC | Dilated Cardiomyopathy |
| HS1900 | GDNF | Hirschsprung Disease |
| M62223 | NEFL | Charcot-Marie-Tooth Neuropathy Type 1F/2E ;Charcot-Marie-Tooth Neuropathy Type 2 ;Charcot-Marie-Tooth Neuropathy Type 2E/1F |
| T10920 | SERPINE1 | Plasminogen Activator Inhibitor I |
| HSNCAML1 | L1CAM | Hereditary Spastic Paraplegia, X-Linked ;L1 Syndrome |
| T11074 | L1CAM | Hereditary Spastic Paraplegia, X-Linked ;L1 Syndrome |
| HUMHPROT | GCSH | Glycine Encephalopathy |
| HSTATR | TAT | Tyrosinemia Type II |
| Z19514 | CPT1B | Carnitine Palmitoyltransferase IB (muscle) Deficiency |
| HSALK3A | BMPR1A | Juvenile Polyposis Syndrome |
| T78581 | CLN5 | CLN5-Related Neuronal Ceroid-Lipofuscinosis ;Neuronal Ceroid-Lipofuscinoses |
| N32269 | CLN8 | CLN8-Related Neuronal Ceroid-Lipofuscinosis ;Neuronal Ceroid-Lipofuscinoses |
| HSU44128 | SLC12A3 | Gitelman Syndrome |
| AI590292 | NPHS2 | Focal Segmental Glomerulosclerosis ;Steroid-Resistant Nephrotic Syndrome |
| M62209 | ACTN4 | Focal Segmental Glomerulosclerosis |
| H53423 | CNGB3 | Achromatopsia ;Achromatopsia 3 |
| HSEPAR | HCI | Hemangioma, Hereditary |
| R14741 | ZIC2 | Holoprosencephaly 5 |
| H84264 | SIX3 | Anophthalmia ;Holoprosencephaly 2 |
| T10497 | TGIF | Holoprosencephaly 4 |
| Z30052 | USP9Y | Y Chromosome Infertility |
| N85185 | DBY | Y Chromosome Infertility |
| T11164 | SPTLC1 | Hereditary Sensory Neuropathy Type I |

(continued)

| Gencarta Contig | Gene Symbol | Disease |
|---|---|---|
| T68440 | GNE | GNE-Related Myopathies ;Sialuria, French Type |
| HSPROPERD | PFC | Properdin Deficiency, X-Linked |
| T46865 | SURF1 | Leigh Syndrome (nuclear DNA mutation) |
| AI015025 | VAX1 | Anophthalmia |
| BM727523 | VAX1 | Anophthalmia |
| AA310724 | SIX6 | Anophthalmia |
| R37821 | TP63 | TP63-Related Disorders |
| AF091582 | ABCB11 | Progressive Familial Intrahepatic Cholestasis |
| HUMHOX7 | MSX1 | Hypodontia, Autosomal Dominant ;Tooth-and-Nail Syndrome |
| R15034 | CACNB4 | Episodic Ataxia Type 2 |
| T52100 | TYROBP | PLOSL |
| F09012 | MTMR2 | Charcot-Marie-Tooth Neuropathy Type 4 |
| T08510 | APTX | Ataxia with Oculomotor Apraxia;Ataxia with Oculomotor Apraxia 1 |
| HUMHAAC | HF1 | Hemolytic Uremic Syndrome |
| C16899 | MTND5 | Leber Hereditary Optic Neuropathy ,Mitochondnal DNA Associated Leigh Syndrome and NARP |

#AUTOANTIGEN_IN_AUTOIMMUNE_DISEASE - Secreted splice variants of known autoantigens associated with a specific autoimmune syndrome, such as for example, those listed in Table 15, below ("contig" column), can be used as therapeutic tools for the treatment of such disorders as described hereinabove It is also contemplated that variants of autoantigens are of a diagnostic value Novel splice variant of the genes listed in Table 15, may be revealed as true autoantigens, therefore their use for detection of autoantibodies is expected to result in a more sensitive and specific test

*Table 15*

| Contig | Disease | Description |
|---|---|---|
| HUMROSSA | Sjogren's syndrome | 52 kDa Ro protein |
| HUMI69KAA | Insulin dependent diabetes Mellitus | 69 kDa islet cell autoantigen |
| S55790 | Goodpasture's syndrome | alpha 3 chain of collagen IV |
| HSACHRA | Myasthenia Gravis | Alpha chain of nicotinic Acetyl Choline receptor |
| Z21711 | Rheumatoid Arthritis | Annexin A11 |
| Z21711 | Sjogren's syndrome | Annexin A11 |
| Z21711 | SLE | Annexin A11 |
| S38729 | SLE | ATP-dependent DNA helicase II, 70 kDa subunit |
| M77907 | SLE | ATP-dependent DNA helicase II, 80 kDa subunit |

(continued)

| Contig | Disease | Description |
|---|---|---|
| T08224 | scleroderma | Autoantigen p27 |
| T08224 | Sjogren's syndrome | Autoantigen p27 |
| M85815 | Pemphigus | bullous pemphigoid antigen 1 |
| HUMROSSAA | SLE | calreticulin |
| HUMCENPRO | General autoimmune response | Centromere autoantigen C |
| HSU14518 | General autoimmune response | Centromere protein A |
| M62116 | dermatomyositis | Chromodomain helicase-DNA-binding protein 3 |
| T05980 | dermatomyositis | Chromodomain helicase-DNA-binding protein 4 |
| H18687 | Autoimmune demyelinating disease | claudin 11 |
| M79258 | dermatomyositis | Dermatomyositis associated with cancer putative autoantigen-1 |
| HSDGIGLY | Pemphigus foliaceus | Desmoglein 1 |
| HUMPVA | Pemphigus vulgaris | Desmoglein 3 |
| BG723199 | Pemphigus vulgaris | desmoglein 4 |
| M77924 | Primary billiary cirrhosis | Dihydrolipoamide acetyltransferase component of pyruvate dehydrogenase complex mitochondrial |
| D11598 | Polymyositis | Exosome complex oxonuclease RRP45 |
| D11598 | scleroderma | Exosome complex exonuclease RRP45 |
| HUMACTINBI | Grave's disease | Filamin B |
| Z17837 | Rheumatoid Arthritis | follistatin-like 1 |
| HUMGAD | Insulin dependent diabetes Mellitus | glutamate decarboxylase 1 (GAD 1) |
| HSGLAD2A | Insulin dependent diabetes Mellitus | glutamate decarboxylase 2 (GAD 2) |
| D12383 | dermatomyositis | glycyl tRNA synthetase |
| D12383 | Polymyositis | glycyl-tRNA synthetase |
| Z40013 | Sjogren's syndrome | Golgi autoantigen, golgin subfamily A member 1 |
| HSMAC | Rheumatoid Arthritis | Golgi autoantigen, golgin subfamily B member 1 |

(continued)

| Contig | Disease | Description |
|---|---|---|
| HSMAC | Sjogren's syndrome | Golgi autoantigen, golgin subfamily B member 1 |
| HUMMSCA | Graves disease | Grave's disease carrier protein |
| HUMGRAVIN | Myasthema Gravis | gravin |
| HUMRNPSMBA | SLE | Homo sapiens small nuclear ribonucleoprotein polypeptides B and B1 |
| HUMINSR | Insulin resistant diabetes Mellitus | insulin receptor |
| HSRNAIFMH | Pernicious Anemia | intrinsic factor |
| D12018 | dermatomyositis | isoleucine-tRNA synthetase |
| D12018 | Polymyositis | isoleucme-tRNA synthetase |
| T97710 | Pemphigus | ladinin 1 |
| HSAUTAN64 | Antoimmune thyroid disease | Leiomodin 1 |
| HSLAANT | SLE | Lupus La protein |
| HUM60RO | SLE | Lupus Ro Protem |
| T08267 | dermatomyositis | lysyl-tRNA synthetase |
| T08267 | Polymyositis | lysyl-tRNA synthetase |
| F01282 | General autoimmune response | Major centromere autoantigen B |
| M78010 | multiple sclerosis | myelin basic protein |
| R89508 | Autoimmune demyelinating disease | Myelin oligodendrocyte glycoprotein (MOG) |
| HUMHSTNBP | Autoimmune infertility | Nuclear autoantigenic sperm protein |
| S80305 | Antiphospholipid syndrome | Phospholipid beta 2 glycoprotein 1 complex |
| D11598 | Polymyositis | polymyositis/scleroderma autoantigen 1 |
| D11598 | scleroderma | polymyositis/scleroderma autoantigen 1 |
| HUMAUA | Polymyositis | Polymyositis/scleroderma autoantigen 2 |
| HUMAUA | scleroderma | Polymyositis/scleroderma autoantigen 2 |
| HUMMCH | Vitiligo | Pro-melanin-concentrating hormone |
| T05361 | Insulin dependent diabetes Mellitus | protein tyrosine phosphatase |
| HSP3MY | Wegener's granulomatosis | Protemase 3 (ANCA - antineutrophil cytoplasmic antibody) |
| F02560 | Insulin dependent diabetes Mellitus | Protein-tyrosine phosphatase-like N [Precursor] |

(continued)

| Contig | Disease | Description |
|---|---|---|
| T05361 | Insulin dependent diabetes Mellitus | Receptor type protein-tyrosine phosphatase N2 |
| HUM60RO | Sjogren's syndrome | Sjogren syndrome antigen A2 |
| H81770 | Sjogren's syndrome | Sjogren's syndrome nuclear autoantigen 1 |
| HUMSNRNPD | SLE | Small nuclear ribonucleoprotein Sm D1 |
| HUMMSCA | Grave's disease | solute carrier family 25 |
| Z17347 | Insulin dependent diabetes Mellitus | SOX-13 protein |
| N79953 | Autoimmune infertility | Sperm surface protein Spl7 |
| T08224 | scleroderma | SSSCA1 |
| T08224 | Sjogren's syndrome | SSSCA1 |
| R54783 | interstitial cystitis | synaptonemal complex protein SC65 (SC65) |
| S40807 | Hashimoto's thyroditis | thyroglobulin |
| S38729 | Autoimmune thyroid disease | thyroid autoantigen 70kDa |
| HUMTPOA | Hashimoto's thyroditis | Thyroid peroxidase |
| HUMBF7A | Celiac disease | transglutaminase 2 |
| S49816 | Grave's disease | TSH receptor |

#DRUG_DRUG_INTERACTION. refers to proteins involved m a biological process which mediates the interaction between at least two consumed drugs. Novel splice variants of known proteins involved in interaction between drugs may be used, for example, to modulate such drug-drug interactions Examples of proteins involved in drug-drug interactions are presented in Table 16 together with the corresponding internal gene contig name, enabling to allocate the new splice variants within the data files "Annotations.gz"' "Transcripts.gz" and "Proteins gz" in the attached CD-ROM4.

*Table 16*

| Contig | Gene Symbol | Description |
|---|---|---|
| HUMANTLA | SLC3A2 | 4f2 cell surface antigen heavy chain |
| Z43093 | HTR6 | 5-hydroxytryptamine 6 receptor |
| HSXLALDA | ABCD1 | Adrenoleukodystrophy protein |
| R35137 | GPT | Alanine aminotransferase |
| D11683 | ALDH1 | Aldehyde dehydrogenase, cytosolic |
| T53833 | AOX1 | Aldehyde oxidase |
| HUMAGP1A | ORM1 | Alpha-1-acid glycoprotein 1 |
| HUMAGP1A | ORM2 | Alpha-1-acid glycoprotein 2 |

(continued)

| Contig | Gene Symbol | Description |
|---|---|---|
| HUMABPA | ABP1 | Amiloride-sensitive amine oxidase [copper-containing] |
| S62734 | MAOB | Amine oxidase [flavin-containing] b |
| AA526963 | SLC6A14 | Ammo acid transporter b0+ |
| HSAE2 | SLC4A2 | Amon exchange protein 2 |
| M78110 | SLC4A3 | Amon exchange protein 3 |
| M78052 | ABCB2 | Antigen peptide transporter 1 |
| HUMMHCILAB | ABCB3 | Antigen peptide transporter 2 |
| F02693 | APOD | Apolipoprotein d |
| M62234 | ASNA1 | Arsenical pump-driving ATPaso |
| HUMNORTR | NAT1 | Arylamme n-acetyltransferase 1 |
| T67129 | NAT1 | Arylamine n-acetytransferase 1 |
| AI262683 | NAT2 | Arylamine n-acetyltransferase 2 |
| Z39550 | ABCB9 | ATP-binding cassette protein abcb9 |
| Z44377 | ABCA1 | ATP-binding cassette, sub-family a, member 1 |
| M78056 | ABCA2 | ATP-binding cassette, sub-family a, member 2 |
| M85498 | ABCA3 | ATP binding cassette, sub-family a, member 3 |
| T79973 | ABCB6 | ATP-binding cassette, sub-family b, member 6, mitochondrial |
| T78010 | ABCB7 | ATP-binding cassette, sub-family b, member 7, mitochondrial |
| R89046 | ABCB8 | ATP-binding cassette, sub-family b, member 8, mitochondrial |
| H64439 | ABCD2 | ATP-binding cassette, sub-family d, member 2 |
| M85760 | ABCD3 | ATP-binding cassette, sub-family d, member 3 |
| Z21904 | ABCD4 | ATP-binding cassette, sub-family d, member 4 |
| Z39977 | ABCG1 | ATP-binding cassette, sub-family g, member 1 |
| Z45628 | ABCG2 | ATP-binding cassette, sub-family g, member 2 |
| T80665 | SLC7A9 | B(0,+)-type amino acid transporter 1 |
| AF091582 | ABCB11 | Bile salt export pump |
| Z38696 | BLMH | Bleomycin hydrolase |
| T08127 | BNPI | Brain-specific na-dependent inorganic phosphate cotransporter |
| F00545 | SLC12A2 | Bumetanide-sensitive sodium-(potassium)-chloride cotransporter 2 |
| HSU07969 | CDH17 | Cadherin-17 |
| T10238 | SLC25A12 | Calcium-binding mitochondrial carrier protein aralarl |
| Z40674 | SLC25A13 | Calcium-binding mitochondrial carrier protein aralar2 |
| T61818 | ABCC2 | Canalicular multispecific organic anion transporter 1 |
| T39953 | ABCC3 | Canalicular multispecific organic anion transporter 2 |
| HUMCRE | CBR1 | Carbonyl reductase [nadph] 1 |
| AA320697 | CBR3 | Carbonyl reductase [nadph] 3 |
| F03362 | COMT | Catechol o-methyltransferase, membrane-bound form |
| T11004 | COMT | Catechol o-methyltransferase, membrane-bound form |

(continued)

| Contig | Gene Symbol | Description |
|--------|-------------|-------------|
| T39368 | SLC7A4 | Cationic amino acid transporter-4 |
| S74445 | RBP5 | Cellular retinol-binding protein iii |
| T55952 | RBP5 | Cellular retinol-binding protein iii |
| HSU39905 | SLC18A1 | Chromaffin granule amine transporter |
| R52371 | SLC35A1 | Cmp-sialic acid transporter |
| D20754 | CNT3 | Concentrative nucleoside transporter 3 |
| HSMNKMBP | ATP7A | Copper-transporting ATPase 1 |
| HUMWND | ATP7B | Copper-transporting ATPase 2 |
| HUMCFTRM | ABCC7 | Cystic fibrosis transmembrane conductance regulator |
| F10774 | SLC7A11 | Cystine/glutamate transporter |
| HUMCYPADA | CYP11B1 | Cytochrome P450 11B1, mitochondrial |
| HUMARM | CYP19 | Cytochrome P450 19 |
| HUMCYP145 | CYP1A1 | Cytochrome P450 1A1 |
| R21282 | CYP26 | Cytochrome P450 26 |
| AF209774 | CYP2A13 | Cytochrome P450 2A13 |
| HSC45B2C | CYP2A6 | Cytochrome P450 2A6 |
| HSC45B2C | CYP2A7 | Cytochrome P450 2A7 |
| HSP452B6 | CYP2B6 | Cytochrome P450 2B6 |
| HUM2C18 | CYP2C18 | Cytochrome P450 2C18 |
| HSCP450 | CYP2C19 | Cytochrome P450 2C19 |
| HUM2C18 | CYP2C19 | Cytochrome P450 2C19 |
| HUMCYPAX | CYP2C8 | Cytochrome P450 2C8 |
| HSCP450 | CYP2C9 | Cytochrome P450 2C9 |
| HSP450 | CYP2D6 | Cytochrome P450 2D6 |
| M77918 | CYP2E1 | Cytochrome P450 2E1 |
| HUMCYPIIF | CYP2F1 | Cytochrome P450 2F1 |
| H09076 | CYP2J2 | Cytochrome P450 2J2 |
| R07010 | CYP39A1 | Cytochrome P450 39A1 |
| HUMCYPHLP | CYP3A3 | Cytochrome P450 3A3 |
| HUMCYPHLP | CYP3A4 | Cytochrome P450 3A4 |
| AA416822 | CYP3A43 | Cytochrome P450 3A43 |
| HUMCYP3A | CYP3A5 | Cytochrome P450 3A5 |
| T82801 | CYP3A7 | Cytochrome P450 3A7 |
| HSCYP4AA | CYP4A11 | Cytochrome P450 4A11 |
| S67580 | CYP4A11 | Cytochrome P450 4A11 |
| HUMCP45IV | CYP4B1 | Cytochrome P450 4B1 |
| T98002 | CYP4F12 | Cytochrome P450 4F12 |
| AA377259 | CYP4F2 | Cytochrome P450 4F2 |

(continued)

| Contig | Gene Symbol | Description |
|---|---|---|
| AI400898 | CYP4F8 | Cytochrome P450 4F8 |
| HSU09178 | DPYD | Dihydropyrimidine dehydrogenase [nadp+] |
| WO3174 | DPYD | Dihydropyrimidine dehydrogenase [nadp+] |
| HUMFMO1 | FMO1 | Dimethylaniline monooxygenase [n-oxide forming] 1 |
| HSFLMON2R | FMO2 | Dimethylaniline monooxygenase [n-oxide forming] 2 |
| T64494 | FMO2 | Dimethylaniline monooxygenase [n-oxide forming] 2 |
| T40157 | FMO3 | Dimethylaniline monooxygenase [n-oxide forming] 3 |
| HSFLMON2R | FMO4 | Dimethylaniline monooxygenase [n-oxide forming] 4 |
| D12220 | FMO5 | Dimethylaniline monooxygenase [n-oxide forming] 5 |
| H25503 | HET | Efflux transporter like protein |
| T12485 | HET | Efflux transporter like protein |
| M78151 | EPHX1 | Epoxide hydrolase 1 |
| T66884 | SLC29A1 | Equilibrative nucleoside transporter 1 |
| HSHNP36 | SLC29A2 | Equilibrative nucleoside transporter 2 |
| T08444 | SLC1A3 | Excitatory amino acid transporter 1 |
| HSU01824 | SLC1A2 | Excitatory amino acid transporter 2 |
| HSU03506 | SLC1A1 | Excitatory amino acid transporter 3 |
| F07883 | SLC1A6 | Excitatory amino acid transporter 4 |
| N39099 | SLC1A7 | Excitatory amino acid transporter 5 |
| F00548 | SLC2A9 | Facilitative glucose transporter family member glut9 |
| T95337 | SLC27A1 | Fatty acid transport protein |
| Z44099 | SLC27A1 | Fatty acid transport protein |
| HUMALBP | FABP4 | Fatty acid-binding protein, adipocyte |
| S67314 | FABP3 | Fatty acid-binding protein, heart |
| AW605378 | FABP2 | Fatty acid-binding protein, intestinal |
| L25227 | SLC19A1 | Folate transporter 1 |
| HSI15PGN1 | FABP6 | Gastrotropin |
| Z40427 | G6PT1 | Glucose 5-phosphate transporter |
| D11793 | SLC2A1 | Glucose transporter type 1,erythrocyte/brain |
| N27535 | SLC2A10 | Glucose transporter type 10 |
| T52633 | SLC2A11 | Glucose transporter type 11 |
| HUMLGTPA | SLC2A2 | Glucose transporter type 2,liver |
| HUMLGTPA | SLC2A2 | Glucose transporter type 2,liver |
| T07239 | SLC2A3 | Glucose transporter type 3,brain |
| HUMIRGT | SLC2A4 | Glucose transporter type 4,insulin-responsive. |
| M62105 | SLC2A5 | Glucose transporter type 5,small intestine |
| T59518 | SLC2A8 | Glucose transporter type 8 |
| HUMLGTH1 | GSTA21 | Glutathione s-transferase al |

(continued)

| Contig | Gene Symbol | Description |
|--------|-------------|-------------|
| HUMLGTH1 | GSTA2 | Glutathione s-transferase a2 |
| T98291 | GSTA3 | Glutathione s-transferase a3-3 |
| Z21581 | GSTA4 | Glutathione s-transferase a4-4 |
| HSGST4 | GSTM1 | Glutathione s-transferase mu 1 |
| D31291 | GSTM2 | Glutathione s-transferase mu 2 |
| HSGST4 | GSTM2 | Glutathione s-transferase mu 2 |
| T08311 | GSTM3 | Glutathione s-transferase mu 3 |
| HUMGSTM4B | GSTM4 | Glutathione s-transferase mu 4 |
| HUMGSTM5 | GSTM5 | Glutathione s-transferase mu 5 |
| T05391 | GSTP1 | Glutathione s-transferase p |
| Z32822 | GSTT1 | Glutathione s-transferase theta 1 |
| R08187 | GSTT2 | Glutathione s-transferase theta 2 |
| Z25318 | GSTK1 | Glutathione s-transferase, mitochondrial |
| H03163 | SLC37A1 | Glycerol-3-phosphate transporter |
| AA363955 | SLC5A7 | High affinity choline transporter |
| HSRRMRNA | SLC7A1 | High-affinity catiomc amino acid transporter-1 |
| R22196 | SLC31A1 | High-affinity copper uptake protein 1 |
| AA918012 | SLC10A2 | Ileal sodium/bile acid transporter |
| F00840 | SLC7A5 | Large neutral amino acid transporter small subumt 1 |
| M79133 | SLC7A5 | Large neutral ammo acid transporter small subunit 1 |
| Z38621 | SLC7A8 | Large neutral amino acids transporter small subunit 2 |
| HUMCARAA | CES1 | Liver carboxylesterase |
| S52379 | CES1 | Liver carboxylesterase |
| T55488 | SLC21A6 | Liver-specific organic anion transporter |
| W78748 | SLC5A4 | Low affinity sodium-glucose cotransporter |
| T54842 | SLC7A2 | Low-affinity cationic ammo acid transporter-2 |
| T87799 | ABCA7 | Macrophage abc transporter |
| Z17844 | LRP | Major vault protein |
| Z24885 | GSTZ1 | Maleylacetoacetate isomerase |
| T39939 | MT1A | Metallothionem-IA |
| R99207 | MT1B | Metallothionein-IB |
| T39939 | MT1E | Metallothionem-IE |
| D11725 | MT1F | Metallothionein-IF |
| S68949 | MT1G | Metallothionein-IG |
| S68954 | MT1G | Metallothionein-IG |
| HSFMET | MT1H | Metallothionein-IH |
| S52379 | MT2A | Metallothionein-II |
| M78846 | MT3 | Metallothionem-III |

(continued)

| Contig | Gene Symbol | Description |
|---|---|---|
| AA570216 | MT1K | Metallothionein-IK |
| S68954 | MT1K | Metallothionein-IK |
| D11725 | MT1L | Metallothionem-IL |
| HSPP15 | MT1L | Metallothionem-IL |
| HSPP15 | MT1R | Metallothionein-IR |
| NM032935 | MT4 | Metallothionem-IV |
| HUMGST | MOST1 | Microsomal glutathione s-transferase 1 |
| H59104 | MGST2 | Microsomal glutathione s transferase 2 |
| T47062 | MGST3 | Microsomal glutathione s-transferase 3 |
| SSMPCP | SLC25A3 | Mitochondrial phosphate carrier protein |
| H39996 | SULT1A3 | Monoamme-sulfating phenol sulfotransferase |
| HUMARYTRAB | SULT1A3 | Monoamine-sulfating phenol sulfotransferase |
| M62141 | SLC16A1 | Monocarboxylate transporter 1 |
| H90048 | SLC16A6 | Monocarboxylate transporter 2 |
| F02520 | SLC16A2 | Monocarboxvlate transporter 3 |
| AI005004 | SLC16A8 | Monocarboxylate transporter 4 |
| T59354 | SLC16A3 | Monocarboxylate transporter 5 |
| R22416 | SLC16A4 | Monocarboxylate transporter 6 |
| T78890 | SLC16A5 | Monocarboxylate transporter 7 |
| F01173 | SLC16A7 | Monocarboxylate transporter 8 |
| Z41819 | ABCB1 | Multidrug resistance protein 1 |
| HUMMDR3 | ABCB4 | Multidrug resistance protein 3 |
| SATHRMRP | ABCC1 | Multidrug resistance-associated protein 1 |
| R00050 | ABCC4 | Multidrug resistance-associated protein 4 |
| M78673 | ABCC5 | Multidrug resistance-associated protein 5 |
| R99091 | ABCC6 | Multidrug resistance-associated protein 6 |
| T69749 | ABCC6 | Multidrug resistance-associated protein 6 |
| D11495 | DIA4 | Nad(p)h dehydrogenase [quinone] 1 |
| HUMNRAMP | SLC11A1 | Natural resistance-associated macrophage protein 1 |
| Z38360 | SLC11A2 | Natural resistance-associated macrophage protein 2 |
| HUMASCT1A | SLC1A4 | Neutral ammo acid transporter a |
| T10696 | SLC1A5 | Neutral amino acid transporter b(0) |
| HUMRENBAT | SLC3A1 | Neutral and basic ammo acid transport protein rbat |
| HSU08021 | NNMT | Nicotinamide n-methyltransferase |
| T87759 | SLC22A4 | Novel organic cation transporter 1 |
| Z41935 | SLC15A2 | Oligopeptide transporter, kidney isoform |
| HSU21936 | SLC15A1 | Oligopeptide transporter, small intestine isoform |
| M62053 | OAT1 | Orgamc anion transporter 1 |

(continued)

| Contig | Gene Symbol | Description |
|---|---|---|
| H18607 | OAT3 | Organic amon transporter 3 |
| R16970 | OAT4 | Orgamc amon transporter 4 |
| T39111 | SLC21A9 | Organic anion transporter b |
| Z41576 | SLC21A11 | Organic amon transporter oATP-d |
| T23657 | SLC21A12 | Organic amon transporter oATP-e |
| Z21041 | SLC21A14 | Organic amon transporting polypeptide 14 |
| H75435 | SLC21A8 | Organic anion transporting polypeptide 8 |
| HSU77086 | SLC22A1 | Organic cation transporter 1 |
| HSOCTK | SLC22A2 | Organic cation transporter 2 |
| T53187 | SLC22A3 | Organic cation transporter 3 |
| H30224 | ORCTL4 | Organic cation transporter like 4 |
| H25503 | ORCTL2 | Organic cation transporter-like 2 |
| Z38659 | SLC22A5 | Organic cation/carmtme transporter 2 |
| AB010438 | ORCTL3 | Organic-cation transporter like 3 |
| T95621 | ORNT1 | Ornithine transporter |
| AA398593 | ORNT2 | Ornithine transporter 2 |
| R79412 | NTT5 | Orphan sodium- and chloride-dependent neurotransmitter transporter ntt5 |
| H82347 | NTT73 | Orphan sodium- and chloride-dependent neurotransmitter transporter ntt73 |
| Z43484 | NTT73 | Orphan sodium- and chlonde dependent neurotransmitter transporter ntt73 |
| Z44749 | SLC25A17 | Peroxisomal membrane protein pmp34 |
| HUMARYLSUL | SULT1A1 | Phenol-sulfating phenol sulfotransferase 1 |
| HUMARYLSUL | SULT1A2 | Phenol-sulfating phenol sulfotransferase 2 |
| D12243 | RBP4 | Plasma retinol-binding protein |
| HUMATPAD | ATP12A | Potassium-transporting ATPase alpha chain 2 |
| Z40030 | ATP8A1 | Potential phospholipid-transporting ATPase ia |
| T10596 | FIC1 | Potential phospholipid-transporting ATPase ic |
| T86800 | SLC31A2 | Probable low affinity copper uptake protein 2 |
| Z41717 | PTGIS | Prostacyclin synthase |
| S78220 | PTGS1 | Prostaglandin g/h synthase l |
| HUMBNDOSYN | PTGS2 | Prostaglandin g/h synthase 2 |
| T85296 | SLC21A2 | Prostaglandin transporter |
| M62053 | SLC22A6 | Renal organic anion transport protem 1 |
| HSU26209 | SLC13A2 | Renal sodium/dicarboxylate cotransporter |
| Z40774 | SLC13A2 | Renal sodium/dicarboxylate cotransporter |
| HSNAPI1 | SLC17A1 | Renal sodium-dependent phosphate transport protein 1 |
| HUMNAPI3X | SLC34A1 | Renal sodium-dependent phosphate transport protem 2 |
| H85361 | ABCA4 | Retinal specific ATP-binding cassette transporter |
| S74445 | CRABP1 | Retinoic acid-binding protein i, cellular |

(continued)

| Contig | Gene Symbol | Description |
|--------|-------------|-------------|
| HUMCRABP | CRABP2 | Retinoic acid-binding protein ii, cellular |
| HUMCRBP | RBP1 | Retinol binding protein i, cellular |
| S57153 | RBP1 | Retinol binding protein i, cellular |
| T07054 | RBP2 | Retinol-binding protein ii, cellular |
| T63266 | RBP2 | Retinol-binding protein ii, cellular |
| HUMBGT1R | SLC6A12 | Sodium- and chloride-dependent betaine transporter, |
| HUMCRTR | SLC6A8 | Sodium- and chloride-dependent creatine transporter 1 |
| R20043 | SLC6A13 | Sodium- and chloride-dependent gaba transporter 2 |
| S70609 | SLC6A9 | Sodium- and chloride-dependent glycine transporter 1 |
| AA625644 | SLC6A5 | Sodium- and chloride-dependent glycine transporter 2 |
| M78677 | SLC6A6 | Sodium- and chloride-dependent taurine transporter |
| T10761 | SLC4A4 | Sodium bicarbonate cotransporter nbcl |
| AA452802 | NBC4 | Sodium bicarbonate cotransporter nbc4a |
| HUMCNC | SLC8A1 | Sodium/calcium exchanger 1 |
| R20720 | SLC8A2 | Sodium/calcium exchanger 2 |
| T07666 | SLC8A3 | Sodium/calcium exchanger 3 |
| T07666 | SLC8A3 | Sodium/glucose cotransporter 1 |
| HUMSGLCT | SLC5A2 | Sodium/glucose cotransporter 2 |
| S83549 | SLC9A2 | Sodium/hydrogen exchanger 2 |
| HSU66088 | SLC5A5 | Sodium/iodide cotransporter |
| HSU62966 | SLC28A1 | Sodium/nucleoside cotransporter 1 |
| AA358822 | SLC28A2 | Sodium/nucleoside cotransporter 2 |
| HUMNTCP | SLC10A1 | Sodium/taurocholate cotransporting polypeptide |
| HSGAT1MR | SLC6A1 | Sodium-and chloride-dependent gaba transporter 1 |
| F05686 | SLC6A11 | Sodium-and chloride-dependent gaba transporter 3 |
| AA604857 | SVCT1 | Sodium-denpendent vitamin c transporter 1 |
| T27309 | SVCT2 | Sodium-denpendent vitamin c transporter 2 |
| S44626 | SLC6A3 | Sodium-dependent dopamine transporter |
| Z39412 | NADC3 | Sodium-dependent high-affinity dicarboxylate transporter |
| T77525 | SLC5A6 | Sodium-dependent multivitamin transporter |
| HUMNORTR | SLC6A2 | Sodium-dependent noradrenaline transporter |
| HSZ83953 | SLC17A3 | Sodium-dependent phosphate transport protein 3 |
| R06460 | SLC17A3 | Sodium-dependent phosphate transport protein 3 |
| HSZ83953 | SLC17A4 | Sodium-dependent phosphate transport protein 4 |
| R09122 | SLC17A4 | Sodium-dependent phosphate transport protein 4 |
| H40741 | SLC6A7 | Sodium-dependent proline transporter |
| HSSERT | SLC6A4 | Sodium-dependent serotonin transporter |
| T64950 | SLC21A3 | Sodium-independent organic anion transporter |

(continued)

| Contig | Gene Symbol | Description |
|--------|-------------|-------------|
| M79233 | EPHX2 | Soluble epoxide hydrolase |
| Z39813 | SLC25A18 | Solute carrier |
| HUMSTAR | STAR | Steroidogenic acute regulatory protein |
| Z20453 | STAR | Steroidogenic acute regulatory protein |
| R69741 | SLC26A2 | Sulfate transporter |
| T08860 | ABCC8 | Sulfonylurea receptor 1 |
| R73927 | ABCC9 | Sulfonylurea receptor 2 |
| T84623 | SULT1C1 | Sulfotransferase 1C1 |
| R58632 | SULT1C2 | Sulfotransferase 1C2 |
| HSVMT | SLC18A2 | Synaptic vesicle amine transporter |
| AF080246 | TRAG3 | Taxol resistant associated protein 3 |
| R20880 | SLC19A2 | Thiamine transporter 1 |
| HSU44128 | SLC12A3 | Thiazide-sensitive sodium-chloride cotransporter |
| S62904 | TPMT | Thiopurine s-methyltransferase |
| HSPBX2 | G17 | Transporter protein |
| T62038 | G17 | Transporter protein |
| R53836 | SLC35A3 | UDP n-acetylglucosamine transporter |
| T60594 | SLC35A2 | UDP-galactose translocator |
| HUMUGT1FA | UGT1 | UDP-glucuronosyltransferase 1-1, microsomal |
| HUMUGT1FA | UGT1A10 | UDP-glucuronosyltransferase 1A10 |
| HUMUGT1FA | UGT1A7 | UDP-glucuronosyltransferase 1A7 |
| HUMUGT1FA | UGT1A8 | UDP-glucuronosyltransferase 1A8 |
| HUMUGT1FA | UGT1A9 | UDP-glucuronosyltransferase 1A9 |
| HSUGT2BIO | UGT2B10 | UDP-glucuronosyltransferase 2B10, microsomal |
| HSUDPGT | UGT2B11 | UDP-glucuronosyltransferase 2B11, microsomal |
| N70316 | UGT2B11 | UDP-glucuronosyltransferase 2B11, microsomal |
| HSU08854 | UGT2B15 | UDP-glucuronosyltransferase 2B15, microsomal |
| T24450 | UGT2B17 | UDP-glucuronosyltransferase 2B17, microsomal |
| HSUDPGT | UGT2B4 | UDP-glucuronosyltransferase 2B4, microsomal |
| HUMUDPGTA | UGT2B7 | UDP-glucuronosyltransferase 2B7, microsomal |
| AI002801 | SLC14A1 | Urea transporter, erythrocyte |
| Z19313 | SLC14A1 | Urea transporter, erythrocyte |
| AI002801 | SLC14A2 | Urea transporter, kidney |
| HSU09210 | SLC18A3 | Vesicular acetylcholine transporter |
| HUMKCHB | KCNA4 | Voltage-gated potassium channel protein kv1.4 |
| R09608 | XDH | Xanthine dehydrogenase/oxidase |
| T64266 | SLC7A7 | Y+1 amino acid transporter 1 |
| T10628 | SLC30A1 | Zinc transporter 1 |

(continued)

| Contig | Gene Symbol | Description |
|--------|-------------|-------------|
| AA322641 | SLC30A4 | Zinc transporter 4 |

*Differentially expressed biomolecular sequences - field description*

#TS - This field denotes tissue-specific genes i.e., genes upregulated in a specific tissue or tissues. As described hereinabove, such gene may be used as markers for tissue proliferation, differentiation and/or tissue damage. These proteins also have therapeutic significance as described above.

The annotation format is as follows:

#TS tissue-name -the "tissue name" field specifies the list of tissues for which tissue-specific genes/variants were searched, as follows: amniotic+placenta; Blood; Bone; Bone marrow; Brain; Cervix+uterus; Colon; Endocrine, adrenal gland; Endocrine, pancreas; Endocrine, parathyroid+thyroid; Gastrointestinal tract; Genitourinary; Head and neck; Immune, T-cells; Kidney; Liver; Lung; Lymph node; Mammary gland; Muscle; Ovary; Prostate; Skin; Thymus.
#TAA - This field denotes genes or transcript sequences over-expressed in cancer. The annotation format is as follows:

#TAA tissue-name - where the "tissue name" field specifies the list of tissues for which tissue-tumor specific genes/variants were searched, as follows: All tumor types; All epithelial tumors; prostate-tumor; lung-tumor; head and neck-tumor; stomach-tumor; colon-tumor, mammary-tumor; kidney-tumor; ovary-tumor; uterus/cervix-tumor; thyroid-tumor; adrenal-tumor; pancreas-tumor; liver-tumor; skin-tumor; brain-tumor; bone-tumor; bone marrow-tumor; blood-cancer; T-cells-tumor; lymph nodes-tumor; muscle-tumor.

#TAAT - This field denotes splice variants over expressed in cancer. The annotation format is as follows:
#TAAT tissue-name start nucleotide - end nucleotide, where the "start nucleotide-end nucleotide" field denotes the start and end nucleotides are the location on the transcript of the unique exon/s of this transcript which are over expressed in cancer.

The following are examples of annotational data, described hereinabove, for differentially expressed biomolecular sequences uncovered using the methodology of the present invention.

>125 T12234_S7 (124 T12234-S5) #PHARM B cell inhibitor #PHARM B cell stimulant #INDICATION Allergy, general; Anaemia, general; Anti-inflammatory; Antiallergic, non-asthma, Antianaemic; Antiarthritic, immunological; Antiarthritic, other; Antiasthma; Anticancer, immunological; Anticancer, other; Antidiabetic; Arthritis, rheumatoid; Asthma; Cancer, basal cell; Cancer, breast; Cancer, colorectal; Cancer, leukaemia, general; Cancer, lung, non-small cell; Cancer, lymphoma, B-cell; Cancer, lymphoma, general; Cancer, lymphoma, non-Hodgkin's; Cancer, melanoma; Cancer, myeloma; Cancer, prostate; Cancer, renal; Cancer, sarcoma, Kaposi's; Cancer, stomach; Chemotherapy-induced injury, bone marrow, general; Chemotherapy-induced injury, general; Cytokine; Diabetes, Type I; Diagnosis, cancer; Gene therapy; Haematological; Immunoconjugate, other; Immunodeficiency, IgA deficiency; Immunodeficiency, IgG deficiency; Immunomodulator, anti-infective; Immunostimulant, anti-AIDS; Immunostimulant, other; Immunosuppressant; Infection, HIV/AIDS; Infection, cytomegalovirus; Infection, hepatitis-B virus; Infection, hepatitis-B virus prophylaxis; Infection, hepatitis-C virus; Infection, influenza virus; Infection, respiratory tract, lower; Inflammation, general; Lupus erythematosus, systemic; Lupus nephritis; Menstruation disorders; Monoclonal antibody, chimaeric; Monoclonal antibody, human; Monoclonal antibody, other; Non-antisense oligonucleotides; Prophylactic vaccine; Radio/chemoprotective; Recombinant growth factor; Recombinant interleukin; Recombinant vaccine; Releasing hormones; Renal failure; Reproductive/gonadal, general; Stomatological; Transplant rejection, general; Urological; Vaccine adjunct; **#TS amniotic+placenta** #SEQLIST CB959801 CB993198 BG723218 CB988266 CB990001 CB960437 CB960673 AY152547 HSU88047 NM005224 BM560075 BG480550 BG481613 BG336181 BC033163 BM914890 BM915483 BG774041 BE407615 BE278788 BU553664 AL528528 BE281155 BG335245 AW502116 AW502448 AW502360 T12234 BG336194 BG336792 BG471353 BE251115 BM728646 BF988865 BG480658 BF752956 BI055866 BX349962 AW874049 BX327713 AW361327 AW604456 AA705382 AI394608 R36384 AWO09403 CA424222 BU953740 BC007077 AA371391 AI635170 BU616621 BE018489 CA420992 BX344903 AL563180 BI090573 BX282372 AA232770 AI343403 BE350191 AA219626 AI128378

>89 AA176616_T0 (88 AA176616_P2) **#TS brain** #SEQLIST AA176616 AL706148 AF188700 BC032777 AL710268 AL706541 NM021638 AI878896 AL708077 AL044957 BI561136 BG818703 AL597876 BF931341

>121 AA542845_T6 **#TAA all tumor types** #SEQLIST BM821505 BM820228 BM833450 BM822871 BM450551 BM822584 BG685476 BG759086 BF975093 BG758047 BG684967 BE879584 BG613292 BF670091 BM741097 BI226181 BC032142 CD248060 BG033600 BU935172 BG616080 BF238873 BG496847 AY028916 BE513408 NM032117 BX118316 AW803742 CA430591 BU622320 AW173084 BG027970 CB053175 BG109991 BQ876910 BU533354 CB053174 BQ888320 BF513683 AA782986 BG678591 BG213307 BE775171 AA971073 BG187870 BG201266 BG211199 BG190562 BG188927 BU953916 AW972924 AA542845 BG031442

>1780 D12188_T22 (1779 D12188_P10) **#TAA stomach-tumor** #SEQLIST BI667214 AA069168 CB120972 AA146921 BF339541 BE697327 AA018956 BI868974 AW977547 AWO16369 BF994680 BF994678 AA768226 AA482525 AA417892 AV747968 AV749122 BI018849 BF327760 AA815174 T11015 CB121829 CB265681 CB114032 T10894 R07220 AU099455 BE940424 AA034472 AA085190 CB122775 CD110517 AW812500 BF445602 BM835953 AL702485 CB137205 AA317134 BM698061 AV686120 BM844438 BF963067 R84427 BQ347914 CB132190 BE812639 H53309 H54062 CB322047 BX420238 AW752802 BG008882 AW752803 AL712969 AW752822 AW838203 BM844307 AW403110 BQ694780 BM843951 BQ272011 W56384 CB119170 BQ291729 AA037057 AA063367 AA021068 BM468187 WO5307 BU561523 AV689084 CB122111 AW674114 AA058777 CB115968 BQ340054 R18396 CB119210 AA975948 AA374973 BG898631 BM888115 BM462720 BG704216 CB114864 BE894309 AA348659 BM847309 AL559362 CB114023 BM843812 CA391445 BQ227099 BM747740 CB115337 R86059 AW838393 BE000940 AW376878 BG940230 BG988188 H44528 H44511 BI056192 R83531 H44513 R73359 AA551357 H44512 BQ271689 AW973514 AA994108 BU948701 BG940229 AI280227 AA534047 AA953711 AA094698 BF832976 BF856679 BM843946 D79108 AV708137 AV703503 CB045840 CB115801 CB110101 AA307112 AA309647 BM819549 BF115653 AA019960 BM761384 CB119259 CB178328 BM788339 BI915305 AI125690 W56155 CB140821 CB123983 CB114859 CB149671 CB122938 CB122913 BG898806 CA406239 BM542792 Z21191 AWO68861 CB122934 CB144641 BU599940 BF665043 CA395566 BF945470 BM791398 CB134041 BQ231812 BM456716 BU164262 BQ777351 BE894021 BM791005 AU137511 BQ953788 BM843126 BM452319 BE540905 CA773780 BI551564 CB216095 CB215747 AW239473 BE269198 BQ214343 BM791465 AU135994 AA303881 BF082675 AA877149 BF893173 BE068965 BQ331544 CB119266 BM772290 CA406825 CB158897 CB122643 BM760734 BM765063 BF082716 BG949629 BI549175 BI010948 BI016251 BF893182 BF773210 BF768828 BI015143 BI013525 WO5482 BE892227 CA442266 BE886787 BM999021 AA363541 AL036270 CB110183 BG773048 AU137419 BI092416 CB988632 H16540 R16060 BF852596 BQ108743 CB242845 AV708995 CD251708 BI029212 BI030865 BI030862 BG723362 BG107552 BG772916 AW800206 F06911 BU189109 BU177966 AA216699 BI468513 CB993967 BF341343 BG171853 BE888095 BE890937 BF967377 BM707195 BI091903 N94298 BI090331 AA325593 BG171642 AA037516 BE565830 CB119330 BM752427 BE562276 BQ424269 BQ437514 BU186557 AA322781 BG390997 BG114948 BQ310814 BM837070 BQ720930 BE547324 R58206 BE897153 BG388576 AF498929 BG899293 BQ681067 CB128905 AU132656 BG698150 BE773333 BG705788 BQ433491 BF540961 BQ377040 BI764787 BF692590 BQ424046 BE885985 BQ308854 BU195290 AW956847 BE935829 AW954378 CD105507 BU162355 BI912425 BI599480 BQ308017 AA393842 AA868907 AV728310 BI760445 AV661126 NM004161 AV727669 HUMRAB1A AW627895 BE786127 BG250484 AI208230 BQ437146 BG534065 AV661125 AA282775 BG250152 AA525489 BG281078 BF970841 BQ223273 BF530743 BI858729 BM452068 BQ921303 R31123 BM450994 BF821830 BF822942 CD556388 CD519333 BU170353 BX345433 BU170821 BM756987 BX460643 AA165326 AV717718 BM786746 BF691745 BI601531 CB164305 BM800733 BI598835 BM476507 BM922791 BF029031 BF247598 WO0963 T29874 BE958017 BX345434 BF211990 R14095 AV708027 CB121142 BQ314772 BM919860 N28650 BG573345 AW850068 AW849755 BG743352 CA771560 BG500384 BI495590 BG168366 BI496921 BM829716 C03749 CA942358 BX426888 CB108527 BG619962 AV702665 BX448589 BM452262 BM542833 AA609771 BF673431 AF170935 AA447942 BX463467 BF890884 BF932035 AW605322 CB131651 BF792766 BE568870 BM784959 BG547236 CD108335 BM767367 BG111725 BG562818 BF090111 BE000976 AW888620 BM450140 BI087362 AW955054 BG538626 BF037863 BG563261 BM904432 CD245285 BU193816 AL539022 CB161342 AA229813 R25145 AL530265 WO4313 BX440905 H04049 BM694415 BG776554 BE617480 BM686049 BG676937 BG432954 BE786784 AW389890 BG779464 BU945327 AA393153 AA112860 R31365 BI913132 CA867672 CB161701 BX452629 AI342700 BM706159 AA962389 CB164662 BG032817 BX332699 BM702777 BQ276789 BM747028 BE818819 AA604440 BG622470 BU927812 AW949877 AL580999 BE771083 AV702319 BE617921 BF967807 CA389222 BX345431 BM826571 BI092003 H01861 BE771069 BI913092 BF447660 BI869965 BX332698 D51100 AA825801 BU567689 BX411609 BG617277 BM783973 AA903879 BE771068 BX345432 AA229649 R88420 AI299811 N51901 AA115325 AI422754 AA857140 BG178268 AI285303 AA782737 BF215497 BM983826 BQ003293 CA443454 BQ276678 R16059 BM983670 N94989 BQ788033 AA047226 CB178572 BG434409 BE972858 AU185510 AA448877 HSM800023 AV645424 N73941 CB116472 AU156411 AU154149 BM973320 CB114088 CB122944 CB119169 AA702144 BC000905 N36763 CB119152 AA283077 CB116486 CB118471 AI056955 CB119061 BE465097 AI636837 AV645778 CB118460 AA043751 AA058471 AI858694 H03362 CB122915 CB114037 CB110114 N75497 CB110081 CB113929 CB122736 CB113962 CB119817 AI872853 CB121359 CB118415 N34579 BQ448090 CB115729 AI026998 AA018921 AW169620 BU677700 AA019266 AWO02352 BU622272 N70762 CA311086 BU736924 AW663003 BM667225 BM971301 BE714687 AI434392 BM991470 BG223478 BU688425 AW136631 AA020983 AA019890 N66759 AW104753 R31083 AI860577 AI889183 AW575163 BM999282 AI628146 BQ772048 AI350328 AA746643 BU626516 BU680296 BM984215 BQ014597 BU608906 BI468512 CB306393 N22842 BM984471 AWO69359 CA503384 AI754132 AW673786 AA435590 BX424956 AI828874 AA844547 C75589 AI287282 AA035154 CB118341 AW473264 AI343795 BF372829 AI191816 C75414 BG231998 CA867063 BU069071 AI066620 C75465 AA805211 C75659 AI097435 C75516 W60992 BM969765 H88552 AW166902 R25146 AW471315 AI884351 AI127749 C75610 BQ000946 AI143341 AA855141 R42459 AI148222 AI952757 AA860442 AI800097 AW150848 AI191331 AI684028 N69689 CB107598 AA601550 AI089357 CB113484 AI097427 AA037361 N74146 D58246 AA776990 BG939358 AA165327 AW972204 AA778332 AI799192 AW236263 N70637 AI245751 D12188 CB994890 BE879644 BF440024 BE962443 AI094813 AA769867 AI720190 AA553840 N70238 AA983962 AA033620 AA216604 BF029770 AA069169 BQ776896 F03178 BG257928 AA962096 AA600022 BQ010358 CD239850 BI495589 AI886405 BG059991 BU726083 CA441504 AA551680 CA446990 CB219015 CA422823 BF382544 BG059705 AA586815 BM975245 AI096519 CA425640 H01862 AW190066 BG236221 AI025608 AA507519 AA398553 BE568059 AA918487 C75502 AI680344 BQ776581 BF433185 CA771253 C75521 AW969792 C75459 AI335718 AA484873 BF238483 AU146032 AWO86107 BE139600 BE646347 AA076117 BM472577 BG938435 BI086445 BX413207 CD514144 BX452630 AA253286 AA456890 Z32881 BM766511 BI917513 W74145 W74146 W74151 BM472811 BF029576 N45488 BG498271 CB157466 BG498187 W30880 AA400752 BE874417 BX448588 W30883 BM689897 BF667421 BF692063 BF028711 BE564328 BM827080 BE566877 BE564359 BE564278 BG538932 AA493231 BI090805 BG492697 AA418454 BF246949 AI697924 BX417813 AA628947 AL530264 AW970415 BI764324 BF433701 BE670383 AI765971 AI805951 AI690022 AI291415 AW188359 AA908254 BE464880 AI694931 BM795518 AI188743 BF224091 BE503079 BE669944 AI302751 AI693340 C01263 AI871744 AW263291 AB73523 AW235080 BF590042 BF593086 AI633918 AI962999 AWO78858 AW262562 AI377218 AI804431 AK055927 AI656152 AI683808 BG150110 AT394179 BQ017287 CA418030 AW300526 AI797649 BU753351 AI933975 AI685760 AI2837I0 AI221410 AI623655 AI146623 AA535127 AI950013 AA418384 AI768809 AW771276 AI245073 AA400670 AA506113 CD369826 BQ030029 AW236683 AI913948 AI500621 BU620635 AI085359 AW571693 BE673936 AW299978 R39965 CA429063 AWO69008 AW194519 AI378576 BU619001 AI288901 BU634305 BM968348 AI204696 AI276084 BE671896 AI096452 BM661969 AI290774 AA514463 BM981294 AA906864 AW196314 AA457046 BF878685 H00768 H00677 BX112077 BQ023552 BF431990 AI223034 AW631338 AI216459 #DN IPR003577 Ras small GTPase, Ras type #DN IPR002041 GTP-binding nuclear protein Ran #DN IPR003578 Ras small GTPase, Rho type #DN IPR001806 Ras GTPase superfamily #DN IPR006688 ADP-ribosylation factor #DN IPR003579 Ras small GTPase, Rab type

>44100 D63246_T1 (44099 D63246_P2) **#TAAT all tumor types 1-447,** #SEQLIST AI459211 AI298516 BQ336762 AI218063 D63246 BM983853 BG200539 BQ186241 BQ184762 BE549966 AWO87501 AW589555 BF061478 BU603861 BU536429 BU954011 BG198439 BQ267681 AA346773 AA642108 AA807781 AI632300 AI633800 AI479561 AA405485 AI419510 AWO16718 BU678979 BM311591 BM692249 BM673518 AA652250 CA771710 AI492091 BM310984 AI494386 CA950854 BM311000 AW961666 AA346774 CA772543 CA951103 CA848186 BM126029 BI837048 BI834774 BI559674 AA327608 BG705044 BG703547 BF967333 BG168937 BC015348 BX119411 AA405635 BG722153 NM152773 BC021177 BM548106 AI380016 AI990640 BX098544 AA917719 CA308507 BU633848 CA430273 AI002739 BG490753 CD368238 BE897067 AA380953 BC013113 NM138461 BM550337 BI860838 BQ678650 CA489370 BM808243 BM810125 BG027765

>20301 D45585_TO (20300 D45585-P1) **#TAA brain-tumor #TAAT all tumor types** 5350-5769 #SEQLIST AA078583 BF852870 N42349 BX100987 N30436 AA078590 BF325559 BF358933 BG979863 BE254942 BF817778 AW504141 BM458377 BM011407 BU501666 BG398407 BG759894 AL134029 BE408840 BF026970 AA077540 CA309755 BE890305 AI085174 BF372046 AW815926 AW815924 AU124991 AK022628 BI224200 BG272215 AI002796 AA077835 BG950470 CD171714 BG575647 BF871631 BM462627 BF811628 BM467542 BF933509 BF838980 CB854836 CB854837 BF515576 BG675707 BC039159 BM479268 BG111365 BQ017628 BE547671 BM716560 BM711371 BI094547 AA463437 BE881465 BI036534 R72665 BU619478 BU682838 BG117492 BQ001621 AWO07319 AA663735 CA444773 CA444806 AI459241 AA987211 BE222061 AW341312 AU148750 AI914217 AI683508 BF001419 BM055310 AWO58367 BE674110 AI309597 AI356881 BM055031 AI540797 AA938193 AA632081 AI357119 BF059293 BE503366 T96349 AU121951 AT356665 BE646431 AI913226 AA760871 AI128965 AW193657 AWO50889 D45585 C20562

>93 H63975_TO (92 H63975_P1) **#TS lung #TAA all tumor types** #SEQLIST BF832090 BM917407 BF087575 BG008463 BC017022 NM152426 BE888971 BX340829 BF841711 BE827866 AL598990 BF879160 AI621256 CB215343 BX368513 BX326934 BE885482 N79740 BX279693 H63975 BX116531 AI022304 WO7257

>137 AA985547_TO **#TS kidney** #SEQLIST AI681733 AI733428 AA985547 CB132776 AI791772 CB959047 BM467433 AI791738 BG249301 BE162114

>2298 AA337524_T0 (2297 AA337524__P1) **#TS ovary #TS cervix+uterus #TAA all tumor types** #SEQLIST AI889508 BX093157 AI820938 AA482061 AA828779 A

I829497 AA337524

*EXAMPLE 23*

*Identification of differentially expressed gene products - Algorithm*

In order to distinguish between differentially expressed gene products and constitutively expressed genes (i.e., house keeping genes) an algorithm based on an analysis of frequencies was configured. A specific algorithm for identification of transcripts over expressed in cancer is described hereinbelow.

**Dry analysis**

**Library annotation** - EST libraries are manually classified according to:

(i) Tissue origin

(⊐) Biological source - Examples of frequently used biological sources for construction of EST libraries include cancer cell-lines; normal tissues; cancer tissues; fetal tissues; and others such as normal cell lines and pools of normal cell-lines, cancer cell-lines and combinations thereof.

($\lambda$) Protocol of library construction - various methods are known in the art for library construction including normalized library construction; non-normalized library construction; subtracted libraries; ORESTES and others. It will be appreciated that at times the protocol of library construction is not indicated.

The following rules are followed:

EST libraries originating from an identical biological samples are considered as a single library.
EST libraries which include above-average DNA contaminations are eliminated.
**Dry computation** - development of engines which are capable of identifying genes and splice variants that are temporally and spacially expressed.
Contigs (genes) having at least five sequences including at least two sequences from the tissue of interest are analyzed.

**EXAMPLE 23a**

**Identification of genes over expressed in cancer.**

Two different scoring algorithms were developed.
**Libraries score** -candidate sequences which are supported by a number of cancer libraries, are more likely to serve as specific and effective diagnostic markers.
**The basic algorithm -** for each contig the number of cancer and normal libraries contributing sequences to the contig was counted. Fisher exact test was used to check if cancer libraries are significantly over-represented in the contig as compared to the total number of cancer and normal libraries.
**Library counting:** Small libraries (e.g., less than 1000 sequences) were excluded from consideration unless they participate in the contig. For this reason, the total number of libraries is actually adjusted for each contig).
**Clones no. score -** Generally, when the number of ESTs is much higher in the cancer libraries relative to the normal libraries it might indicate real over-expression.

**The algorithm** -

Clone counting: For counting EST clones each library protocol class was given a weight based on our belief of how much the protocol reflects real expression levels:

(i) non-normalized : 1
(ii) normalized: 0.2
(iii) all other classes : 0.1

Clones number score - The total weighted number of EST clones from cancer libraries was compared to the EST clones from normal libraries. To avoid cases where one library contributes to the majority of the score, the contribution of the library that gives most clones for a given contig was limited to 2 clones.

The score was computed as

$$\left. \cfrac{c+1}{C} \middle/ \cfrac{n+1}{N} \right.$$

where:

c - weighted number of "cancer" clones in the contig.
C- weighted number of clones in all "cancer" libraries.
n - weighted number of "normal" clones in the contig.
N- weighted number of clones in all "normal" libraries.

Clones number score significance - Fisher exact test was used to check if EST clones from cancer libraries are significantly over-represented in the contig as compared to the total number of EST clones from cancer and normal libraries.

**Two search approaches were used** to find either general cancer-specific candidates or tumor specific candidates.

- Libraries/sequences originating from tumor tissues are counted as well as libraries originating from cancer cell-lines ("normal" cell-lines were ignored).
- Only libraries/sequences originating from tumor tissues are counted

### EXAMPLE 23b

### Identification of tissue specific genes

For detection of tissue specific contigs, tissue libraries/sequences were compared to the total number of libraries/sequences in contig. Similar statistical tools to those described in Example 23 a were employed to identify tissue specific genes.

**The algorithm -** for each tested tissue T and for each tested contig the following were examined:

1. Each contig includes at least 2 libraries from the tissue T. At least 3 clones (weighed - as described above) from tissue T in the contig; and
2. Clones from the tissue T are at least 40 % from all the clones participating in the tested contig

Fisher exact test P-values were computed both for library and weighted clone counts to check that the counts are statistically significant

### EXAMPLE 23c

### Identification of splice variants over expressed in cancer of contigs which are not over expressed in cancer

Cancer-specific splice variants containing a unique region were identified.

### Identification of unique sequence regions in splice variants

A Region is defined as a group of adjacent exons that always appear or don't appear together in each splice variant.

Only reliable ESTs were considered for region analysis. An EST was defined as unreliable if:

(i) Unspliced;
(ii) Not covered by RNA;

(iii) Not covered by spliced ESTs; and

(iv) Alignment to the genome ends in proximity of long poly-A stretch or starts in proximity of long poly-T stretch.

Only reliable regions were selected for further scoring. Unique sequence regions were considered reliable if:

(i) Aligned to the genome; and
(⬜) Regions supported by more than 2 ESTs.

**The algorithm**

Each unique sequence region divides the set of transcripts into 2 groups:

(i) Transcripts containing this region          (group TA).
(ii) Transcripts not containing this region          (group TB).

The set of EST clones of every contig is divided into 3 groups:

(i) Supporting (originating from) transcripts of group TA (S1).
(ii) Supporting transcripts of group TB (S2).
(iii) Supporting transcripts from both groups (S3).

Library and clones number scores described above were given to S1 group.
Fisher Exact Test P-values were used to check if:

S1 is significantly enriched by cancer EST clones compared to S2; and
S1 is significantly enriched by cancer EST clones compared to contig background (S1+S2+S3).

Identification of unique sequence regions and division of the group of transcripts accordingly is illustrated in Figure 17.

**EXAMPLE 23d**

**Identification of cancer specific splice variants of genes over expressed in cancer**

A search for EST supported (no mRNA) regions for genes of:

(i) known cancer markers
(ii) Genes shown to be over-expressed in cancer in published micro-array experiments.

Reliable EST supported-regions were defined as supported by minimum of one of the following:

(i) 3 spliced ESTs; or
(ii) 2 spliced ESTs from 2 libraries;
(iii) 10 unspliced ESTs from 2 libraries, or
(iv) 3 libraries.

*EXAMPLE 24*

*Granulocyte colony stimulating factor (GCSF) splice variant, SEQ ID NOs. 68 and 71*

To confirm that the teachings of the present invention are effective in identifying biomolecular sequences, which can be of clinical use, the present inventors applied those teachings to uncover numerous sequences of clinical relevance. Examples 24-26 describe three such sequences and clinical uses thereof. These sequences were previously identified by patent applications: WO2003018612 and WO9627007, thereby validating the robustness of the present invention.

*Background*

The first line of defense against infectious agents is comprised primarily of polymorphonuclear granulocytes, macrophages, natural killer cells and cytotoxic lymphocytes. GCSF, a central mediator of the endogenous response to infection and inflammation, plays a critical role in the process of hematopoiesis, regulating the proliferation, differentiation and survival of neutrophils and neutrophilic progenitor cells. GCSF is produced mainly by haematopoietic cells, such as monocytes/ macrophages and lymphocytes. Other cells , such as fibroblast, endotheliai cells, astrocytes and bone marrow stromal cells can also produce GCSF following activation by LPS, IL-1 or TNF-$\alpha$. Indeed, GCSF production is increased sharply in response to bacterial infection and cell-mediated immune responses, supporting its role in vivo is host defense against microorganisms. In vitro, GCSF exhibits stimulation of neutrophil production from precursor cells and enhancement of mature neutrophil function as augmentation of their antibody-dependant cellular cytotoxicity (ADCC). The dual action of GCSF in vitro, suggested that it would be useful clinically to stimulate haematopoietic recovery in situations of reduced bone marrow capacity or to enhance the ability to resolve infections in immunocompromised hosts. In its native form, the GCSF protein is O-glycosylated with a molecular mass of approximately 20 kD. It is a member of a family of cytokines that have a four-$\alpha$-helical bundle structure which contribute importantly to its three-dimensional structure. GCSF mediates its biological actions by binding to a specific cell surface receptor, the GCSF-R, which is expressed on neutrophils, their precursors and some leukemic cell lines. Binding of GCSF causes receptor dimerization and activation of signaling cascades such as the Jak-STAT and mitogen-activated kinase pathways. The receptor has no intrinsic tyrosine kinases activity but rather it activates a number of cytoplasmic tyrosine kinases that initiate the cascade of signaling events. There are four tyrosine residues in the cytoplasmic region of the GCSF-R that are rapidly phosphorylated following ligand binding and have been shown to have specific roles in mediating the various activities of GCSF.

### Clinical applications

Neutropenia is still the leading factor limiting the use of chemotherapy for the treatment of neoplastic diseases and a major cause of morbidity and mortality following hematopoietic stem cell transplantation. GCSF is widely employed clinically to treat cancer patients undergoing chemotherapy in order to alleviate the depression of white blood cells levels produced by cytotoxic therapeutic agents. It has been also used to accelerate hematopoietic recovery after transplantation and therefore reduce the risks of serious infection. Use of this cytokine reduces the duration of neutropenia, enhances hematopoietic reconstitution and increases the progenitor cell yields. Since GCSF treatment leads to rapid expansion of bone marrow cellularity and the appearance of progenitors in peripheral blood, it has been used to mobilize CD34+ hematopoietic stem cells from the marrow to the blood (peripheral blood stem cells) for use in hematopoietic transplantation. Approved pharmaceutical forms of GCSF for human use include a recombinant nonglycosylated protein expressed in Escherichia coli (filgrastim, produced by Amgen, Thousand Oaks, Calif., USA) and a glycosylated form expressed in Chinese hamster ovary cells (lenograstim, produced by Chugai Pharmaceuticals,Tokyo,Japan). Both forms have similar biological activities and bioavailability following subcutaneous or intravenous administration.

GCSF is widely employed clinically to treat cancer patients undergoing chemotherapy in order to alleviate the depression of white blood cells levels and to accelerate hematopoietic recovery after transplantation. Furthermore, much interest has focused on the use of GCSF to mobilize CD34+ hematopoietic stem cells from the marrow to the blood for use in hematopoietic transplantation. GCSF cannot be administered orally. Instead, frequent injections of significant quantities of the cytokine are necessary throughout the course of the treatment. In addition, GCSF requires stringent formulation and storage conditions. Much effort was placed in developing alternative or improved molecules that demonstrate cytokine function but have superior pharmacological properties. GCSF splice variants might fulfill these requirements, exhibiting increased stability while retaining the biological activity of GCSF (Basu et al. 2002. International Journal of molecular Medicine. 10: 3-10; Layton J. E. 1992. Growth Factors Vol. 6, pp. 17-186; Young et al. 1997. Protein Science. 6 :1228-1236; Layton et al. 1999. The Journal of Biological Chemistry. Vol. 274, No. 25, pp. 17445-17451; Bishop et al. 2001. The Journal of Biological Chemistry. Vol. 276, No. 36, pp. 33465-33470; Hubel et al. 2003. Ann Hematol. 82:207-213; Kuga et al. 1989. Biochemical and biophysical research communications. Vol. 159, No. 1. Pp 103-111; Clark-Lewis et al. 1988. The Journal of Immunology. Vol. 141, No. 3, Pp 881-889).

By applying the teachings of the present invention the present inventors uncovered GCSF splice variant (i.e., GCSF splice variant T2, SEQ ID NOs: 68 and 71, see Figures 18a-e), which can be used for the above described clinical applications and which may be devoid,of the above listed limitations.

GCSF splice variant T2 results from alternative splicing of the GCSF gene, thus leading to the skipping of exon 3 (according to refsec MN000759), and the generation of a protein lacking amino acids 66-104 of w.t GCSF. GCSF splice variant T2 encodes a 168 amino acids long protein, which contains the N-terminal signal sequence (residues 1-30) and most of the IL6/GCSF/ MGF family domain (residues 51-163, out of 51-202 of the w.t).

*EXAMPLE 25*

*Interleukin-7 splice variant, SEQ ID NOs. 69 and 72*

*Background*

Interleukin-7 is a cytokine that was originally identified as a growth factor for murine B cell progenitors and was isolated from bone marrow stromal cells. Subsequently, it was demonstrated that IL-7 has a crucial role in normal B and T cell lymphopoiesis. It acts as a differentiation and proliferation factor in B cells and a survival factor in activated T cells. Receptors for IL-7 have been found on cells of both the lymphoid and myeloid lineages. IL-7 is a member of the family of cytokines that signal through the common cytokine gamma chain ($\gamma$c). The heterodimeric IL-7R complex is composed of two subunits, a unique alpha ($\alpha$) subunit and the p64 gamma ($\gamma$) subunit, which is common to the receptors for IL-2, IL-4, IL-9 and IL-15. While IL-7R expression is important in early pre-B and pro-B cell development, mature B cells lack expression of high affinity receptor and demonstrate no proliferation response to IL-7. In addition to its expression on immature B cells, IL-7R has been identified also on thymocyte and on most mature T cells with transient down-regulation upon activation. IL-7 signaling involves a number of nonreceptor tyrosine kinase pathways that associate with the cytoplasmic tail of the receptor. These include the Janus kinase/signal transducer and activator of transcription (Jak/STAT) pathway, phosphatidylinositol 3-kinase (PI3-kinase), and Src family tyrosine kinases.

*Clinical applications*

Due to the numerous effects of IL-7 on mature T cells it may serve to modulate immune responses in infectious disease or tumor models. IL-7, administered systemically can be used as an anti-cancer therapy by enhancing the immune responses against tumor through a variety of mechanisms. In addition to the expansion and maintenance of T cells expressing TCRs with high affinity for tumor antigens, IL-7, combined with other factors, such as GM-CSF, enhances the generation of mature monocyte-derived dendritic cells. Furthermore, IL-7, along with other cytokines, may contribute to the induction of a type 1 immune response and LAK cells. Finally, by diminishing TGF-$\beta$ production, IL-7 can potentially down-regulate one mechanism through which tumors suppress local immune responses. In contrast, IL-7 stimulates the growth of pre-B and T acute lymphoblastic leukemia cells in vitro. It also induces proliferation of chronic lymphocytic leukemia cells and acute myelogenous leukemia cells, as well as cells from patients with Sezary syndrome. IL-7R is expressed on the majority of neoplastic lymphoid cells and on a subset of myeloid neoplasms. The demonstration of IL-7 secretion by neoplastic B lymphocytes from patients with Burkitt's lymphoma, Sezary leukemia cells, Hodgkin's lymphoma cells and normal keratinocytes suggests the possibility of both autocrine and paracrine growth-stimulatory mechanisms for IL-7 in neoplastic diseases. Therefore inhibiting IL-7 signaling might have a therapeutic potential in cancer therapy (Cosenza et al. 2000. Protein Science. 9:916-926; VanderSpek et aL 2002, Cytokine. Vol. 17, No. 5, Pp. 227-233; Gorgun et al. 2002. Cytokine. Vol. 20, No. 1, Pp 17-22; Fry et al. 2002. Blood. Vol. 99, No. 11, Pp. 3892-3904).

By applying the teachings of the present invention, the present inventors uncovered an alternatively spliced isoform of IL-7 (i.e., Splice variant T3, see Figures 19a-e, SEQ ID NOs: 69 and 72), which may be used alone or in combination with wild-type IL-7 for the above-listed applications. IL-7 splice variant T3 results from alternative splicing of the IL-7 gene, thus leading to the skipping of exon 4, and the generation of a protein lacking amino acids 77-121 of the w.t IL-7. IL-7 splice variant T3 encodes a 132 amino acids long protein which contains the N-terminal signal sequence (residues 1-27) and part of the IL-7/IL-9 family domain (residues 28-129, out of 28-173 of the wild-type.

*EXAMPLE 26*

*Vascular endothelial growth factor-B (VEGF-B) Splice Variant*, *SEQ ID NOs*. *70 and 73*

*Background*

The VEGF family of growth factors has been implicated as key regulators of blood vessel formation. VEGF is required for both vasculogenesis, the de novo formation of endothelial channels from differentiating angioblasts and for angiogenesis, the sprouting or splitting of capillaries from pre-existing vessels. While vasculogenesis is restricted to embryonic development, angiogenesis continues to operate throughout life when neovascularization is required. Physiological angiogenesis is mainly restricted to the female reproductive cycle and wound healing, but the angiogenic machinery can also be recruited by pathological processes such as tumor growth. VEGF is an endothelial-cell-specific mitogen. It stimulates endothelial cell migration and vessel permeability and promotes survival of the newly formed vessels. Several members of the VEGF family have been identified namely the VEGF-A,B,C,D,E and placenta growth factor (PIGF). VEGF-B is expressed early during fetal development and is widely distributed, being prominently expressed in the cardiac myocytes, in skeletal muscle and smooth muscle cells of large vessels. Interestingly, VEGF-B is also expressed in the perichondrium of developing bone and in the nervous system, especially in the cerebral cortex. Two mRNA splice variants are generated from the VEGF-B gene which share the same 115 amino-terminal amino acid residues but have distinct carboxy termini. After the 21 amino acid signal sequence has been cleaved off, the two polypeptides are 167 (VEGF-B167) and 186 (VEGF-B186) amino acids in length. The carboxy terminus of VEGF-B167 is homologous to that of VEGF165; both encode protein sequences rich in basic amino acid residues, which after secretion bind the growth factor to cell-surface heparan sulphate proteoglycans. In contrast, the carboxy-terminal domain of VEGF-B186 is hydrophobic and contains many serine, threonine and proline residues. Thereby, the two isoforms differ in their affinity for heparin and thus release and bioavailability. VEGF-B167 and VEGF-B186 also differ in their glycosylation pattern; whereas VEGF-B167 is not glycosylated, VEGF-B186 contains O-linked glycans. Furthermore, VEGF-B 186 is proteolytically processed at Arg127, giving rise to a 34 kDa dimer. VEGF-B belongs to a growth factor superfamily containing a cystein knot motif. In addition to the disulfide bridges in the cystein knot, two disulfide bridges join the two antiparallel monomers into a homodimer. VEGF-B167 can form a heterodimer with VEGF, a property likely to alter the receptor specificity and biological effects of VEGF-B. VEGF exerts its functions through binding to two receptor tyrosine kinases, VEGFR-1/Flt-1 and VEGFR-2/ KDR. These receptors are expressed almost exclusively on endothelial cells, although VEGFR-1 is also found in monocytes where it mediates migration.

VEGF-B and PIGF interact exclusively with VEGFR-1 and VEGF competes with VEGF-B for VEGFR-1 binding. Mutagenesis of VEGF-B identified the charged residues Asp63, Asp64 and Glu67 as important for VEGFR-1 binding. In addition to its binding to VEGFR-1, VEGF-B was shown to interact with neuropilin-1, a receptor for semaphorins/collapsins involved in axonal guidance. Besides to its neuronal expression, neuropilin-1 is also present in the developing embryo in endothelial cells of capillaries and blood vessels and in mesenchymal cells surrounding the blood vessels, as well as in certain other non-neuronal tissues, including the endocardial cells of the embryonic heart. The interaction of VEGF-B167 with neuropilin-1 is mediated by the carboxy-terminal domain, which contains a sequence homologous to the neuropilin-binding peptide of VEGF165 and mediates heparin binding of VEGF-B167. Surprisingly, the non-heparin-binding isoform VEGF-B186 also bound neuropilin-1, but only in its proteolytically cleaved form. The neuropilin-binding epitope in VEGF-B186 was mapped to the first 12 amino acid residues following the core region that is common to both VEGF-B167 and VEGF-B186. Thus, VEGF-B exhibits, in addition to the VEGF-like core, a distinct domain with unique characteristics, which confers binding specificity. VEGF-B and VEGF have only partially overlapping biological roles due to their different receptor binding specificities. VEGF knockout mice embryos are lethal, indicating that no other growth factor could compensate for the loss of even a single VEGF allele. VEGF-B might modulate VEGF signaling by forming heterodimers with VEGF. VEGF has been shown to regulate most steps of the angiogenesis process, including endothelial cell degradation of ECM, migration, proliferation and tube formation. In keeping with its ability to induce ECM degradation, VEGF increases the expression and activity of plasminogen activators, uPA and tPA. These serine proteases convert plasminogen to plasmin and are thereby involved in tissue remodeling, cell invasion and thrombolysis. VEGF up-regulates also the expression of the plasminogen activator inhibitor (PAI-1), which inhibits both of the proteases and thus protects ECM from excessive proteolysis. Both PAI-1 and uPA have been implicated in regulating cell adhesion and migration and their concerted expression has been observed during physiological angiogenesis in vivo. Similarly to VEGF, VEGF-B has been shown to induce the expression and activity of uPA and PAI-1.

### Clinical applications

Angiogenesis is suggested to be a rate-limiting step in tumor development, thus the control of tumoral angiogenesis is one of the promising therapeutic ways in cancer therapy. During the so-called angiogenic switch, the transition from the latent phase to the invasive and metastatic phase of a cancer, the balance between angiogenesis inhibitors (e.g. endostatin and thrombospondin-1) and angiogenesis inducers (e.g. VEGF) is shifted and rapid vessel ingrowth follows, supporting tumor expansion. VEGF-B was shown to be expressed in tumour tissues. Its expression is upregulated in ovarian carcinoma relative to normal ovarian surface epithelium and is commonly present in both benign and malignant human tumours (e.g. in breast carcinoma, melanoma and fibrosar-coma), as well as in a variety of cultured tumour cell lines. VEGF-B may therefore serve as an important target in the development of angiogenesis inhibitors. Thus, tumour growth, metastasis and diabetic retinopathy could be prevented by inhibition of angiogenesis, whereas pro-angiogenic stimuli could help patients with myocardial or peripheral ischemia. Rapid progress is being made to control vascular responses to arterial injury based on gene transfer or local delivery of the VEGF protein. In this context, additional VEGF-like molecule , such as VEGF-B, might be useful (Olofson et al. 1996. Proc. Natl. Acad. Sci. USA. Vol. 93, pp. 2576-2581; Clauss M. 2000. Seminars in Thrombosis and Hemostasis. Vol. 26, No. 5, pp 561-569; Scotney et al. 2002. Clinical and experimental Pharmacology and physiology 29: 1024-1029; Olofsson et al. 1998. Vol. 95, Issue 20:11709-11714.

Shinkaruk et al. 2003. Curr. Med. Chem - Anti Cancer Agents. Vol 3, No. 2, Pp. 95-117; Olofsson et al. 1999. Current Opinion in Biotechnology 10:528-535).

By applying the teachings of the present invention the present inventors uncovered a VEGF-B splice variant (SEQ ID NOs. 70 and 73, see Figures 20a-e), which may be used alone, or with other angiogenic factors (e.g., wild-type VEGF-B) for diagnosis and/or treatment of the above listed indications.

VEGF-B splice variant results from alternative splicing of the VEGF-B gene, thus causing the skipping of exon 6 and the generation of a protein lacking amino acids 138-207 of the w.t VEGF-B. VEGF-B splice variant encodes a 143 amino acids long protein which contains the N-terminal signal sequence (residues 1-21), the complete PDGF domain (residues 47-124) and a unique sequence of 6 amino acids in the carboxy terminus of the protein.

### EXAMPLE 27

#### Overexpression of a Troponin variant in cancer

#### Background

The regulatory protein troponin (Tn) located on actin filament consists of three subunits: TnT--binds troponin to tropomyosin, TnC--binds divalent calcium ions, and TnI--affects myosin-actin interactions. Tn subunits display several molecular and calcium binding variations. During ontogenetic development of cardiac and skeletal muscles the synthesis of multiple isoforms of Tn subunits was detected. Expression of Tn isoforms and the extent of phosphorylation of both TnT and TnI via protein kinase C or protein kinase A under different pathological situations (e.g. ischemia, congenital heart disease, heart failure) can affect the Ca2+-stimulated contraction function and the myofibrillar ATPase activity of the heart [Adamcova (1999) Physiol. Res. 48:235-247]. Troponin is commonly used as a marker for predicting cancer-therapy-induced cardiotoxicity. To date no reliable association has been made between cancer onset or progression and troponin expression.

By applying the teachings of the present invention, the present inventors uncovered eleveated levels of novel troponin isoforms (see Figure 21 and SEQ ID NOs. 74-85, 66 and 67) in lung, ovarian and colon cancers, suggesting the use of troponin alone or in combination with wild type troponin for diagnosis and treatment of cancer (see Examples 27a-c).

#### Materials and Exprerimental Procedures

*RNA preparation* - RNA was purchased from Clontech (Franklin Lakes, NJ USA 07417, www.clontech.com) or BioChain Inst. Inc. (www.biochain.com) or ABS or Ambion. Alternatively, RNA was purified from tissue samples using TRI-Reagent (Molecular Research Center), according to Manufacturer's instructions. Tissue samples were obtained from cancer patients or from postmortem. Total RNA samples were treated with DNaseI (Ambion) then purified using RNeasy columns (Qiagen).

*RT PCR -* 1 $\mu$g of DNaseI-treated RNA was mixed with 150 ng Random Hexamer primers (Invitrogen) and 500 $\mu$M dNTP in a total volume of 15.6 $\mu$l. The mixture was incubated for 5 min at 65 °C and then quickly chilled on ice. Thereafter, 5 $\mu$l of 5X SuperscriptII first strand buffer (Invitrogen), 2.4$\mu$l 0.1M DTT and 40 units Rnasin (Promega) were added, and the mixture was incubated for 10 min at 25 °C, followed by further incubation at 42 °C for 2 min. Then, 1$\mu$l (200 units) of SuperscriptII (Invitrogen) was added and the reaction (final volume of 25 $\mu$l) was incubated for 50 min at 42 °C and then inactivated at 70 °C for 15 min. The resulting cDNA was diluted 1:20 in TE (10 mM Tris pH=8, 1 mM EDTA pH=8).

*Real-Time RT-PCR analysis-* 5$\mu$l of diluted cDNA generated as described above were used as a template in Real-Time PCR reactions using the SYBR Green I assay (PE Applied Biosystem) with specific primers (SEQ ID NOs: 42 and 43). UNG Enzyme (Eurogentech Cat No. 2L, or ABI Cat. No. D12107 or Roche Cat. No. 10232921) was also included in the reactions. The amplification stage was effected as follows, 50 °C for 2 min, 95 °C for 10 min, and then 40 cycles of 95 °C for 15s ec, followed by 60 °C for 1min. Detection was effected using PE Applied Biosystem SDS 7000. The cycle in which the reactions achieved a threshold level (Ct) of fluorescence was registered and served to calculate the relative transcript quantity in the RT reactions. The relative quantity was calculated using the equation Q=efficiency^-Ct. The efficiency of the PCR reaction was calculated from a standard curve created using serial dilutions of reverse transcription (RT) reactions prepared from RNA purified from 5 cell-lines (HCT116, H1299, DU145, MCF7, ES-2). To minimize inherent differences in the RT reaction, the resulting relative quantities were normalized to the geometric mean of the relative quantities of several housekeeping genes.

### EXAMPLE 27a

#### Expression of troponin isoforms in normal benign and cancerous ovary tissues

Expression of troponin isoforms of the present invention was measured by real time PCR using a fragment derived therefrom (SEQ ID NO: 44, primers are set forth in SEQ ID NOs. 42 and 43), In addition the expression of four housekeeping genes - PBGD (GenBank Accesion No. BC019323), HPRT (GenBank Accesion No. NM_000194), GAPDH (GenBank Accesion No. BC026907) and SDHA (GenBank Accesion No. NM_004168) was measured by real time PCR. In each RT sample, the expression of troponin-S69208_unique_region was normalized to the geometric mean of the quantities of the housekeeping genes. The normalized quantity of each RT sample was then divided by the averaged quantity of the normal post-mortem samples (no. 45-48,71, Table 17 below) to obtain a value of fold up-regulation of each sample relative to averaged normal samples.

As shown in Figure 22, the expression of troponin-S69208_unique_region in normal samples (samples no 45-52, 67-69, 71-75, Table 17, below) and benign samples (samples 56-64, Table 17, below) was significantly lower than in cancerous samples. Notably, tropomn-S69208_unique_region up-regulation of at least 10 fold was found in 8 out of 15 adenocacinoma, 2 out of 7 Mucinus adenocarcinoma, 4 out of 9 Serous adenocarcinoma, 3 out of 5 mix serous-endometroid adenocarcinoma, 1 out of 3 endometroid adenocarcmoma, and in 2 of 2 clear-cell adenocarcmoma samples. 10-15 fold up-regulation was observed also m 2 of the 11 matched normal samples However, since matched samples are histologically non-cancerous tissue that surrounds the tumor, such samples could have been contaminated with cancer or pre-cancer cells

*Table 17*

| Sample name | Lot number | Source | Tissue | Pathology |
|---|---|---|---|---|
| 2-A-Pap Adeno G2 | ILS-1408 | ABS | ovary | Papillary adenocarcinoma |
| 3-A-Pap Adeno G2 | ILS-1431 | ABS | ovary | Papillary adenocarcinoma |
| 4-A-Pap CystAdeno G2 | ILS-7286 | ABS | ovary | Papillary cystadenocarcinoma |
| 1-A-Pap Adeno G3 | ILS-1406 | ABS | ovary | Papillary adenocarcinoma |
| 14-B-Adeno G2 | A501111 | BioChain | ovary | Adenocarcinoma |
| 5-G-Adeno G3 | 99-12-G432 | GOG | ovary | Adenocarcinoma (Stage3C) |
| 6-A-Adeno G3 | A0106 | ABS | ovary | adenocarcinoma |
| 7-A-Adeno G3 | IND-00375 | ABS | ovary | adenocarcinoma |
| 8-B-Adeno G3 | A501113 | BioChain | ovary | adenocarcinoma |
| 9-G-Adeno G3 | 99-06-G901 | GOG | ovary | Adenocarcinoma (maybe serous) |
| 10-B-Adeno G3 | A407069 | Biochain | ovary | Adenocarcinoma |
| 11-B-Adeno G3 | A407068 | Biochain | ovary | Adenocarcinoma |
| 12-B-Adeno G3 | A406023 | Biochain | ovary | Adenocarcinoma |
| 13-G Adeno G3 | 94-05-7603 | GOG | right ovary | Metastasis adenocarcmoma |
| 15-B-Adeno G3 | A407065 | BioChain | ovary | Carcinoma |
| 16 Ct Adeno | 1090387 | Clontech | ovary | Carcinoma NOS |
| 22-A-Muc CystAde G2 | A0139 | ABS | ovary | Mucinous cystadenocarcinoma (Stage1C) |
| 21-G- Muc CystAde G2-3 | 95-10-G020 | GOG | ovary | Mucinous cystadenocarcinoma (Stage2) |
| 23-A-Muc CystAde G3 | VNM-00187 | ABS | ovary | Mucinous cystadenocarcinoma with low malignant |
| 17-B-Muc Adeno G3 | A504084 | BioChain | ovary | Mucinous adenocarcinoma |
| 18-B-Muc Adeno G3 | A504083 | BioChain | ovary | Mucinous adenocarcinoma |
| 19- B-Muc Adeno G3 | A504085 | BioChain | ovary | Mucinous adenocarcinoma |

(continued)

| Sample name | Lot number | Source | Tissue | Pathology |
|---|---|---|---|---|
| 20- A-Pap Muc CystAde | USA 00273 | ABS | ovary | Papillary mucinous cystadenocarcmoma |
| 33-B-Pap Sero CystAde G1 | A503175 | BioChain | ovary | Serous papillary cystadenocarcinoma |
| 25-A-Pap Sero Adeno G3 | N0021 | ABS | ovary | Papillary serous adenocarcinoma (StageT3CN1MX) |
| 24-G- Pap Sero Adeno G3 | 2001-07-G801 | GOG | ovary | Papillary serous adenocarcinoma |
| 30-G-Pap Sero Adeno G3 | 2001-08-G011 | GOG | ovary | Papillary serous carcinoma (Stage1C) |
| 70-G-Pap Sero Adeno G3 | 95-08-G069 | GOG | ovary | Papillary serous adenocarcmoma |
| 31-B-Pap Sero CystAde G3 | A503176 | BioCham | ovary | Serous papillary cystadenocarcinoma |
| 32-G-Pap Sero CystAde G3 | 93-09-4901 | GOG | ovary | Serous papillary cystadenocarcinoma |
| 66-G-Pap Sero Adeno G3 SIV | 2000-01 -G413 | GOG | ovary | Papillary serous carcinoma (metastais of primary peritoneum) (Stage4) |
| 29-G Sero Adeno G3 | 2001-12-G035 | GOG | right ovary | Serous adenocarcinoma (Stage3A) |
| 41-G-Mix Sero/Muc/Endo G2 | 98-03-G803 | GOG | ovary | Mixed epithelial cystadenocarcinoma with mucinous, endometrioid, squamous and papillary serous (Stage2) |
| 40-G-Mix Sero/Endo G2 | 95-11-G006 | GOG | ovary,endometrium | Papillary serous and endometrioid cystadenocarcmoma (Stage3C) |
| 37-G-Mix Sero/Endo G3 | 2002-05-G513 | GOG | ovary | Mixed serous and andometrioid adenocarcinoma |
| 38-G-Mix Sero/Endo G3 | 2002-05-G509 | GOG | ovary | Mixed serous and endometrioid adenocarcmoma of mullenan (Stage3C) |
| 39--G-Mix Sero/Endo G3 | 2001-12-G037 | GOG | ovary | Mixed serous and endometrioid adenocarcinoma |
| 36-G-Endo Adeno G1-2 | 2000-09-G621 | GOG | ovary | Endometrial adenocarcinoma |
| 35-G-Endo Adeno G2 | 94-08-7604 | GOG | right ovary | Endometrioid adenocarcinoma |
| 34-G-Pap Endo Adeno G3 | 95-04-2002 | GOG | ovary | Papillary endometrioid adenocarcinoma (Stage3C) |
| 43-G-Clear cell Adeno G3 | 2001-10-G002 | GOG | ovary | Clear cell adenocarcinoma |
| 44-G-Clear cell Adeno | 2001-07-G084 | GOG | ovary | Clear cell adenocarcinoma (Stage3A) |

(continued)

| Sample name | Lot number | Source | Tissue | Pathology |
|---|---|---|---|---|
| 42-G-Adeno borderline | 98-08-G001 | GOG | ovary | Epithelial adenocarcinoma of borderline malignancy |
| 59-G-Sero CysAdenoFibroma | 98-12-G401 | GOG | ovary | Serous CysAdenoFibroma |
| 63-G-Sero CysAdenoFibroma | 2000-10-G620 | GOG | ovary | Serous CysAdenoFibroma of borderline malignancy |
| 64-G-Ben Sero CysAdenoma | 99-06-G039 | GOG | ovary | Bengin Serous CysAdenoma |
| 56-G-Ben Muc CysAdeno | 99-01-G407 | GOG | left ovary | Bengin mucinus cysadenoma |
| 62-G-Ben Muc CysAdenoma | 99-10-G442 | GOG | ovary | Bengin mucinus cysadenoma |
| 60-G- Muc CysAdenoma | 99-01-G043 | GOG | ovary | Mucinous Cysadenoma |
| 61-G- Muc CysAdenoma | 99-07-G011 | GOG | ovary | Mucinous Cysadenoma |
| 57-B-Thecoma | A407066 | BioChain | ovary | Thecoma |
| 58-CG-Stru teratoma | CG-177 | Ichilov | ovary | Struma ovary/monodermal teratoma |
| 50-B-N M8 | A501114 | BioChain | ovary | Normal (matched tumor A501113) |
| 49-B-N M14 | A501112 | BioChain | ovary | Normal (matched tumor A501111) |
| 69-G-N M24 | 2001-07-G801N | GOG | ovary | Normal (matched tumor 2001-07-G801) |
| 67-G-N M38 | 2002-05-509N | GOG | ovary | Normal (matched tumor 2002-05-G509) |
| 51-G-NM41 | 98-03-G803N | GOG | ovary | Normal (matched tumor 98-03-G803) |
| 52-G-N M42 | 98-08-G001N | GOG | ovary | Normal (matched tumor 98-08-G001) |
| 68-G-N M56 | 99-01-G407N | GOG | ovary | Normal (matched bengin 99-01-G407) |
| 72-G-N M66 | 2000-01-G413N | GOG | ovary | Normal (matched tumor 2000-01-G413) |
| 73-G-N M59 | 98-12-G401N | GOG | ovary | Normal (matched tumor 98-12-G401) |
| 74-G-N M65 | 97-11-G320N | GOG | ovary | Normal (matched tumor 97-11G320) |
| 75-G-N M60 | 99-01-G043N | GOG | ovary | Normal (matched tumor 99-01-G043) |
| 45-B-N | A503274 | BioChain | ovary | Normal PM |
| 46-B-N | A504086 | BioChain | ovary | Normal PM |
| 48-B-N | A504087 | BioChain | ovary | Normal PM |
| 47-Am-N | 061P43A | Ambion | ovary | Normal PM |
| 71-CG-N | CG-188-7 | Ichilov | ovary | Normal PM |

*EXAMPLE 27b*

*Expression of troponin isoforms in normal and cancerous lung tissues*

Expression of the unique region of troponin-S69208 (SEQ ID NO: 67) was measured by real time PCR using a fragment derived therefrom (SEQ ID NO: 44, primers are set forth in SEQ ID NOs. 42 and 43). In addition the expression of four housekeeping genes - PBGD (GenBank Accession No. BC019323), HPRT1 (GenBank Accession No. NM_000194), Ubiquitin (GenBank Accession No. BC000449) and SDHA (GenBank Accession No. NM_004168) was measured by real time PCR. In each RT sample, the expression of troponin-S69208_unique_region was normalized to the geometric mean of the quantities of the housekeeping genes. The normalized quantity of each RT sample was then divided by the averaged quantity of the normal post-mortem samples (no. 47-50, 90-93, 96-99, Table 18 below) to obtain a value of fold up-regulation of each sample relative to averaged normal samples

As shown in Figure 23, the expression of troponin-S69208_unique_region was upregulated in several cancer samples relative to the normal samples. Specifically, troponin-S69208_unique_region up-regulation of at least 10 fold was found in 2 of 15 adenocarcmoma, 2 out of 16 squamous, 3 out of 4 large cell, and 2 out of 8 small cell samples Notably, up-regulation of troponin-S69208_unique_region seems to be more specific to large cell tumors.

*Table 18*

| sample rename | Lot No. | source | pathology | Grade | gender/age |
|---|---|---|---|---|---|
| 1-B-Adeno G1 | A504117 | Biochain | Adenocarcinoma | 1 | F/29 |
| 2-B-Adeno G1 | A504118 | Biochain | Adenocarcinoma | 1 | M/64 |
| 95-B-Adeno G1 | A610063 | Biochain | Adenocarcinoma | 1 | F/54 |
| 12-B-Adeno G2 | A504119 | Biochain | Adenocarcinoma | 2 | F/74 |
| 75 B-Adeno G2 | A609217 | Biochain | Adenocarcinoma | 2 | M/65 |
| 77 B-Adeno G2 | A608301 | Biochain | Adenocarcinoma | 2 | M/44 |
| 13-B-Adeno G2-3 | A504116 | Biochain | Adenocarcinoma | 2-3 | M/64 |
| 89-B-Adeno G2-3 | A609077 | Biochain | Adenocarcinoma | 2-3 | M/62 |
| 76 B-Adeno G3 | A609218 | Biochain | Adenocarcinoma | 3 | M/57 |
| 94-B-Adeno G3 | A610118 | Biochain | Adenocarcinoma | 3 | M/68 |
| 3-CG-Adeno | CG-200 | Ichilov | Adenocarcinoma | | NA |
| 14-CG- Adeno | CG-111 | Ichilov | Adenocarcinoma | | M/68 |
| 15-CG-Bronch adeno | CG-244 | Ichilov | Bronchioloalveolar adenocarcinoma | | M/74 |
| 45-B-Alvelous Adeno | A501221 | Biochain | Alveolus carcinoma | | F/50 |
| 44-B-Alvelous Adeno G2 | A501123 | Biochain | Alveolus carcinoma | 2 | F/61 |
| 19-B-Squamous G1 | A408175 | Biochain | Squamous carcinoma | 1 | M/78 |
| 16-B-Squamous G2 | A409091 | Biochain | Squamous carcinoma | 2 | F/68 |
| 17-B-Squamous G2 | A503183 | Biochain | Squamous carcinoma | 2 | M/57 |
| 21-B-Squamous G2 | A503187 | Biochain | Squamous carcinoma | 2 | M/52 |
| 78-B-Squamous G2 | A607125 | Biochain | Squamous Cell Carcmoma | 2 | M/62 |
| 80-B-Squamous G2 | A609163 | Biochain | Squamous Cell Carcinoma | 2 | M/74 |
| 18 B-Squamous G2-3 | A503387 | Biochain | Squamous Cell Carcinoma | 2-3 | M/63 |
| 81-B-Squamous G3 | A609076 | Biochain | Squamous Carcinoma | 3 | m/53 |
| 79-B-Squamous G3 | A609018 | Biochain | Squamous Cell Carcinoma | 3 | M/67 |
| 20-B-Squamous | A501121 | Biochain | Squamous Carcinoma | | M/64 |
| 22-B-Squamous | A503386 | Biochain | Squamous Carcmoma | | M/48 |

(continued)

| sample rename | Lot No. | source | pathology | Grade | gender/age |
|---|---|---|---|---|---|
| 88-B-Squamous | A609219 | Biochain | Squamous Cell Carcmoma | | M/64 |
| 100-B-Squamous | A409017 | Biochain | Squamous Carcinoma | | M/64 |
| 23-CG-Squamous | CG-109 (1) | Ichilov | Squamous Carcinoma | | M/65 |
| 24-CG-Squamous | CG-123 | Ichilov | Squamous Carcinoma | | M/76 |
| 25-CG-Squamous | CG-204 | Ichilov | Squamous Carcinoma | | M/72 |
| 87-B-Large cell G3 | A609165 | Biochain | Large Cell Carcmoma | 3 | F/47 |
| 38-B-Large cell | A504113 | Biochain | Large cell | | M/58 |
| 39-B-Large cell | A504114 | Biochain | Large cell | | F/35 |
| 82-B-Large cell | A609170 | Biochain | Large Cell Neuroendocrine Carcinoma | | M/68 |
| 30-B-Small cell carci G3 | A501389 | Biochain | small cell | 3 | M/34 |
| 31-B-Small cell carci G3 | A501390 | Biochain | small cell | 3 | F/59 |
| 32-B-Small cell carci G3 | A501391 | Biochain | small cell | 3 | M/30 |
| 33 B-Small cell carci G3 | A504115 | Biochain | small cell | 3 | M |
| 86-B-Small cell carci G3 | A608032 | Biochain | Small Cell Carcinoma | 3 | F/52 |
| 83-B-Small cell carci | A609162 | Biochain | Small Cell Carcinoma | | F/47 |
| 84-B-Small cell carci | A609167 | Biochain | Small Cell Carcinoma | | F/59 |
| 85-B-Small cell carci | A609169 | Biochain | Small Cell Carcinoma | | M/66 |
| 46-B-N-M44 | A501124 | Biochain | Normal M44 | | F/61 |
| 47-B-N | A503205 | Biochain | Normal PM | | M/26 |
| 48-B-N | A503206 | Biochain | Normal PM | | M/44 |
| 49-B-N | A503334 | Biochain | Normal PM | | M/27 |
| 50-B-N | A503385 | Biochain | Normal PM | | M/28 |
| 90-B-N | A608152 | Biochain | Normal (Pool 2) PM | | pool 2 |
| 91-B-N | A607257 | Biocham | Normal (Pool 2) PM | | pool 2 |
| 92-B-N | A503204 | Biochain | Normal PM | | m/28 |
| 93-Am-N | 111P0103A | Ambion | Normal ICH | | F/61 |
| 96-Am-N | 36853 | Ambion | Normal PM | | F/43 |
| 97-Am-N | 36854 | Ambion | Normal PM | | M/46 |
| 98-Am-N | 36855 | Ambion | Normal PM | | F/72 |
| 99-Am-N | 36856 | Ambion | Normal PM | | M/31 |

### EXAMPLE 27c

### Expression of troponin isoforms in normal and cancerous colon tissues

Expression of the unique region of troponin-S69208 (SEQ ID NO: 67) was measured by real time PCR using a fragment derived therefrom (SEQ ID NO: 44, primers are set forth in SEQ ID NOs 42 and 43) In addition the expression of four housekeeping genes - PBGD (GenBank Accession No BC019323), HPRT1 (GenBank Accession No NM_000194), RPS27A (GenBank Accession No NM_002954) and G6PD (GenBank Accession No NM_000402) was measured by real time PCR. In each RT sample, the expression of troponin-S69208_unique_region was normalized to the geometric mean of the quantities of the housekeeping genes The normalized quantity of each RT sample was then divided by the averaged quantity of the normal post-mortem samples (no. 41,52, 62-67, 69-71 Table 19, below) to obtain a value of fold up-regulation of each sample relative to avargaed normal samples.

As shown in Figure 24, the expression of troponin-S69208_unique_region was upregulated at least 10 fold (4 samples showed at least 5 fold) in two cancer samples relative to the normal samples. One of the 3 autoimmune disease samples also showed up-regulation in the expression of troponin S69208_unique_region

*Table 19*

| sample rename | Lot No. | Tissue | source | pathology |
|---|---|---|---|---|
| 68-B-Adeno G1 | A610024 | Sigmoid colon | biochain | Adenocarcinoma |
| 58-B-Adeno G1 | A609152 | Colon | biochain | Adenocarcinoma |
| 59-B-Adeno G1 | A609059 | Colon | biochain | Adenocarcinoma, Ulcer |
| 14-CG-Polypoid Adeno G1 D-C | CG-222 (2) | Rectum | Ichilov | Well polypoid adeocarcinoma Duke's C |
| 17-CG-Adeno G1-2 | CG-163 | Rectum | Ichilov | Adenocarcinoma |
| 10-CG-Adeno G1-2 D-B2 | CG-311 | Sigmod colon | Ichilov | Adenocarcinoma Astler-Coller B2. |
| 11-CG-Adeno G1-2 D-C2 | CG-337 | Colon | Ichilov | Adenocarcinoma Astler-Coller C2. |
| 6-CG-Adeno G1-2 D-C2 | CG-303 (3) | Colon | Ichilov | Adenocarcinoma Astler-Coller C2. |
| 5-CG-Adeno G2 | CG-308 | Colon Sigma | Ichilov | Adenocarcinoma. |
| 16-CG-Adeno G2 | CG-278C | Colon | Ichilov | Adenocarcinoma |
| 56-B-Adeno G2 | A609148 | Colon | biochain | Adenocarcinoma |
| 61-B-Adeno G2 | A606258 | Colon | biochain | Adenocarcinoma, Ulcer |
| 60-B-Adeno G2 | A609058 | Colon | biochain | Adenocarcinoma, Ulcer |
| 22-CG-Adeno G2 D-B | CG-229C | Colon | Ichilov | Adenocarcinoma Duke's B |
| 1-CG-Adeno G2 D-B2 | CG-335 | Cecum | Ichilov | Adenocarcinoma Dukes B2. |
| 12-CG-Adeno G2 D-B2 | CG-340 | Colon Sigma | Ichilov | Adenocarcinoma Astler-Coller B2. |
| 28-CG-Adeno G2 D- B2 | CG-284 | sigma | Ichilov | Adenocarcinoma Duke's B2 |
| 2-CG-Adeno G2 D-C2 | CG-307 X2 | Cecum | Ichilov | Adenocarcinoma Astler-Coller C2. |
| 9-CG-Adeno G2 D-D | CG-297 X2 | Rectum | Ichilov | Adenocarcinoma Dukes D. |
| 13-CG-Adeno G2 D -D | CG-290 X2 | Rectosigmoidal colon | Ichilov | Adenocarcinoma Dukes D. |
| 26-CG-Adeno G2D-D | CG-283 | sigma | Ichilov | Colonic adenocarcinoma Duke's D |
| 4-CG-Adena G3 | CG-276 | Colon | Ichilov | Carcinoma. |
| 53-B-Adeno G3 | A609161 | Colon | biochain | Adenocarcinoma |
| 54-B-Adeno G3 | A609142 | Colon | biochain | Adenocarcinoma |
| 55-B-Adeno G3 | A609144 | Colon | biochain | Adenocarcinoma |
| 57-B-Adeno G3 | A609150 | Colon | biochain | Adenocarcinoma |
| 72-CG-Adeno G3 | CG-309 | colon | Ichilov | Adenocarcinoma |
| 20-CG-Adeno G3 D-B2 | CG-249 | Colon | Ichilov | Ulcerated adenocarcinoma Duke's B2 |
| 7-CG-Adeno D-A | CG-235 | Rectum | Ichilov | Adenocarcinoma intramucosal Duke's A. |
| 23-CG-Adeno D-C | CG-282 | sigma | Ichilov | Mucinus adenocarcinoma Astler Coller C |
| 3-CG-Muc adeno D-D | CG-224 | Colon | Ichilov | Mucinois adenocarcinoma Duke's D |

(continued)

| sample rename | Lot No. | Tissue | source | pathology |
|---|---|---|---|---|
| 18-CG-Adeno | CG-22C | Colon | Ichilov | Adenocarcinoma |
| 19-CG-Adeno | CG-19C (1) | Colon | Ichilov | Adenocarcinoma |
| 21-CG-Adeno | CG-18C | Colon | Ichilov | Adenocarcinoma |
| 24-CG-Adeno | CG-12 (2) | Colon | Ichilov | Adenocarcinoma |
| 25-CG-Adeno | CG-2 | Colon | Ichilov | Adenocarcinoma |
| 27-CG-Adeno | CG-4 | Colon | Ichilov | Adenocarcinoma |
| 8-CG-diverticolosis, diverticulitis | CG-291 | Wall of sigma | Ichilov | Diverticolosis and diverticulitis of the Colon |
| 46-CG-Crohn's disease | CG-338C | Cecum | Ichilov | Crohn's disease |
| 47-CG-Crohn's disease | CG-338AC | Colon | Ichilov | Crohn's disease. |
| 42-CG-N M20 | CG-249N | Colon | Ichilov | Normal |
| 43-CG-NM8 | CG-291N | Wall of sigma | Ichilov | Formal |
| 44-CG-N M21 | CG-18N | Colon | Ichilov | Normal |
| 45-CG-N M11 | CG-337N | Colon | Ichilov | Normal |
| 49-CG-N M14 | CG-222N | Rectum | Ichilov | Normal |
| 50-CG-N M5 | CG-308N | Sigma | Ichilov | Within normal limits |
| 51-CG-N M26 | CG-283N | Sigma | Ichilov | Normal |
| 41-B-N | A501156 | Colon | biochain | Normal PM |
| 52-CG-N | CG-309TR | Colon | Ichilov | Within normal limits |
| 62-B-N | A608273 | Colon | biochain | Normal PM |
| 63-B-N | A609260 | Colon | biochain | Normal PM |
| 64-B-N | A609261 | Colon | biochain | Normal PM |
| 65-B-N | A607115 | Colon | biochain | Normal PM |
| 66-B-N | A609262 | Colon | biochain | Normal PM |
| 67-B-N | A406029 | Colon | biochain | Normal PM (Pool 10) |
| 69-B-N | A411078 | Colon | biochain | Normal PM (Pool 10) |
| 70-C1-N | 1110101 | Colon | clontech | Normal PM (Pool of 3) |
| 71-Am-N | 071P10B | Colon | Ambion | Normal (IC BLEED) |

Altogether the results presented herein suggest the use of troponin as a valuable diagnostic marker for cancer, preferably, lung, ovarian and colon cancers.

***EXAMPLE 28***

***Example of sequence files "Transcripts.gz" and "Proteins.gz" and the annotation file "Annotations.gz" on the enclosed CD-ROM4***

***EXAMPLE** 28a*

***Description of the sequence files on the enclosed CD-ROM4***

The sequences in the sequence files "Transcripts.gz" and "Proteins.gz" of the enclosed CD_ROM4 are in FastA text format. Each transcript sequence starts with ">" mark, followed by the transcript internal accession number. An example of the sequence file is presented below.

## Example 28(i) (nuc)

>1 (AL589416_T0)

CAATATCTGCCAGAGTTCTTCCTTCCTTTTTTGCTTGCCTGACACCCTTCCCTTCAGAGTTCAGTT
CTTGCTAGTAGAAAAAGGCTATGCAGCATGATAATACAG


TGATTTTAAAAGTTGATTATCTTGAAGTATTTTGGGACAGTTTTGGGGAATGGGTTATGTGGTA
TATATTTATTTGGCTCTTTGCTGAATATGGCTGTATAAATC
CAGCCTCCTGGATGTTACAAGTCATCCCCTTGATTTCTTCTTTTGAATAAAAAAGGAGCATTGCT
TCATTTTTCTTGCAGAAACCCTTATTTAAACAATGAAAAC
TTCA

## Example 28(ii) (prot)

>57 (BX394567_P1)

ASLAPASLRTRWDGNRVQELLSAFETSAPSPAFNLKGLNQTAASVHPYPPLIPSPLRRKQKTHSSSGR
CEAWPRRPLSGFPIDLTPGIVIGLFIYVLPAPTVNAF
QNYKPMS

## EXAMPLE 28b

## Description of the annotation file "Annotations.gz" on the enclosed CD-ROM4

Each transcript annotation starts with ">" mark. Each annotation field starts with "#" mark.

## Example 28(iii)

>542937 HSMUC1A_R73 (542739 HSMUC1A_P20) #GENE_SYMBOL MUC1 #INDICATION Anticancer, antibiotic ;Anticancer, immunological Cancer, breast ; Cancer, general ;Cancer, lung, general ;Cancer, lung, non-small cell ;Cancer, ovarian ;Cancer, pancreatic ;Cancer, prostate ;Cancer, stoma ch ;Immunoconjugate, other ;Immunostimulant, other ;Immunotoxin ;Monoclonal antibody, murine ;Recombinant vaccine #PHARM CD8 agonist ;Immu nostimulant ;Interferon gamma agonist ;MUC-1 inhibitor #DIAGNOSTICS as indicated in the Diagnostic markers table #THERAPEUTIC_PROTEIN MUC1 _HUMAN #TS gastrointestinal tract #TAA GEN #TAA brain-tumor #TAA pancreas-tumor #SEQLIST HSMUC1A HSMUC1B BF876040 BE001495 S81736 S81781 R 48730 BF876382 BE706360 BF881208 BF985614 BM759495 HUMEPISIB1 HUMEPISIA1 HUMDF3AA BQ943809 BG775565 CA489836 T27692 BM046583 BF934279 BF75 9082 BM852195 BF881341 CB120860 BM791359 BQ923149 BQ082925 AI905082 BU680357 BM795232 BG979899 AW799571 BU542454 AL703494 BF869858 BF84987 8 AW862410 CB122585 AW610387 AW610438 AW860264 AA847094 AW845083 BE140784 CB216759 AW579078 BF338440 AW579048 AI795839 AW610422 AW604927 A W610473 AW862376 AI909787 AW579037 AW579021 AI909790 AI909811 AW610386 AI795813 AI816584 AI909798 AI795823 AW177757 AI834269 AW610437 AI90 5191 AW610477 AW862372 AW610454 AW604911 AW860163 AW604913 AW604917 AW604891 AW579067 AW604897 AW610403 AW610426 AW862360 AW862369 AW86237 0 BQ317003 AW862447 BM795113 AI909813 BE140766 AW610427 AW604981 AW610421 AW610478 AW610472 AW610416 AW610467 BM856029 AL543598 AW579047 A W610456 AW610461 AW610410 AW610405 BM850782 BI014455 BG900038 BM829853 AI245417 BG541121 AI909251 BM820231 BG896519 BM828885 AA112487 AW60 2769 AA336046 AI910233 AI909279 BM797973 BM852127 BM745435 AA367451 AA292565 BM831319 AA367457 BM833236 BQ084348 BM686872 AI905920 AA15135 1 BM793904 BG742381 R07092 AW391803 R06201 T84561 BM746832 HSSETA BF880547 AL046435 CA489602 BF917653 AI909863 BF913521 AI909332 AI904561 AA847972 BQ083205 BG740690 AA344985 AF125525 HSMUC1 HSU60261 HSU60259 NM002456 HSU60260 AF348143 AW387919 BM741056 BM822982 BM817771 BM794 696 AW175638 HSTEYMA AA429321 H02932 AW369405 AW369398 AW369452 AW369432 AW369441 AW369450 AW369421 AW369436 AW369449 T29335 CA489620 BM79 1452 AA429320 AA641284 BG774910 BQ918845 BM825463 AI884519 BM706491 BQ928921 BG775831 BG775373 AA486365 R72486 AA158865 AA775299 BM849640 AL543556 H04235 AI903204 AWO81805 AI567905 AI589353 AA149270 AA573028 AI270578 AI659618 BM045706 AI624362 BQ029166 BI761034 AI625260 AW131 341 BM982303 AI802528 AA536189 BQ029112 AWO26623 AI648569 AWO01395 AWO01137 BM990638 AA530908 BU754181 AA826345 AW138860 AI697120 BM049643 BU683186 AA480067 AI952140 HUMPANMU AI497674 AI985760 AI187328 BM993267 AI382404 AW263844 BM984687 AI922289 AW130017 HUMMUCAB BM982837 BM 997733 BQ029959 AI991289 BG698098 AA887919 AA885865 CB306106 N27731 AI973169 BU677135 CB053053 BQ003028 AI678846 AA158866 BM673313 AWO0993 7 BQ025188 BU689421 AA908325 AA889527 BU680339 AI264942 AI269893 AA937026 AI926172 AI270379 BM769180 BM770034 AW151995 AA932409 AA099243 A 1299297 BM991344 BQ446679 AW572126 BU686752 AA580280 BM795831 AA872219 AA112420 AI624443 HUMEPISIB2 AI570806 AA970828 BM976097 AW951288 AA 627845 HUMEPISIA2 AI870710 AI866858 AWO01116 AI279932 AI858369 AI610869 AI886098 AA425062 BM673704 AI918060 BM971650 BQ447959 AI933677 BM9 69296 BM969363 AW192838 AI275395 BM974802 AI962981 BG150966 BG151071 BG370389 AA569793 AI672970 BG370420 AI582464 AI923310 AI631295 AW4404 71 AA877326 AW243914 BU565754 #FR 74 #TO 1330 #SECRETED_FORM_OF_MEMBRANNEL_PROTEINS_BY_PROLOC #SECRETED_FORM_OF_MEMBRANNEL_PROTEINS_BY_SWI SSPROT #DIAGNOSTICS can be used as a diagnostic marker #DN IPR000082 SEA domain #GO_F #GO_Acc 3779 #GO_Desc actin binding #CL 4 #DB sp #EN MUC1_HYLLA #GO_P #GO_Acc 6955 #GO_Desc immune response #DB Viral protein database #EN 583834 #GO_C #GO_Acc 5576 #GO_Desc extracellular #C L 3 #DB PROLOC #EN PROLOC

## EXAMPLE 29

### Description of CD-ROM4

Enclosed CD-ROM4 contains the following files:

1. "Annotations.gz", containing all the annotation information, as described in Example 22.
2. "Proteins.gz", containing all the amino acid sequences encoded by the transcripts based on GenBank version 136
3. "Transcripts.gz", containing nucleotide sequences of all the transcripts based on GenBank versions 136.

## EXAMPLE 30

### Description of CD-ROM5

Enclosed CD-ROM5 contains the following files:

1. "GC_localization_1", "GC_localization_2", "GC_localization_3", "GC_localization_4", containing protein cellular localization information.
2. "pos_proteins_ipr_report_1_dos", "pos_proteins_ipr_report_2_dos", "pos_proteins_ipr_report_3_dos", "pos_proteins_ipr_report_4_dos", containing information related to Interpro analysis of domains.
3. "GC_expression_x", wherein "x" may be from 1 to 46, containing information related to expression of transcripts according to oligonucleotide data.
4. "GC_expression_y", wherein "y" may be from 47 to 75, containing information related to expression of transcripts according to EST expression data.
5. "oligo probs abbreviations for patent", containing the information about abbreviations of tissue names for oligonucleotide probe binding.

All tables are best viewed by using a text editor with the "word wrap" function disabled (to preserve line integrity) and in a fixed width font, such as Courier for example, preferably in font size 10. Table spacing is described for each table as a guide to assist in reading the tables.
With regard to protein cellular localization information, table structure is as follows: column 1 features the protein identifier as used throughout the application to identify this sequence; column 2 features the name of the protein; column 3 shows localization (which may be intracellular, membranal or secreted); and column 4 gives the reason for this localization in terms of results from particular software programs that were used to determine localization. Spacing for this table is as follows: column 1: characters 1-9; column2: characters 10-45; column 3: characters 46-61; and column 4: characters 62-121.

Information given in the text with regard to cellular localization was determined according to four different software programs: (i) tmhmm (from Center for Biological Sequence Analysis, Technical University of Denmark DTU, http://www.cbs.dtu.dk/services/TMHMM/TMHMM2.0b.guide.php) or (ii) tmpred (from EMBnet, maintained by the ISREC Bionformatics group and the LICR Information Technology Office, Ludwig Institute for Cancer Research, Swiss Institute of Bioinformatics, http://www.ch.embnet.org/software/TMPRED_form.html) for transmembrane region prediction; (iii) signalp_hmm or (iv) signalp_nn (both from Center for Biological Sequence Analysis, Technical University of Denmark DTU, http://www.cbs.dtu.dk/services/SignalP/background/prediction.php) for signal peptide prediction. The terms "signalp_hmm" and "signalp_nn" refer to two modes of operation for the program SignalP: hmm refers to Hidden Markov Model, while nn refers to neural networks. Localization was also determined through manual inspection of known protein localization and/or gene structure, and the use of heuristics by the individual inventor. In some cases for the manual inspection of cellular localization prediction inventors used the ProLoc computational platform [Einat Hazkani-Covo, Erez Levanon, Galit Rotman, Dan Graur and Amit Novik; (2004) "Evolution of multicellularity in metazoa: comparative analysis of the subcellular localization of proteins in Saccharomyces, Drosophila and Caenorhabditis." Cell Biology International 2004;28(3): 171-8.], which predicts protein localization based on various parameters including, protein domains (e.g., prediction of trans-membranous regions and localization thereof within the protein), pI, protein length, amino acid composition, homology to pre-annotated proteins, recognition of sequence patterns which direct the protein to a certain organelle (such as, nuclear localization signal, NLS, mitochondria localization signal), signal peptide and anchor modeling and using unique domains from Pfam that are specific to a single compartment.

With regard to to Interpro analysis of domains, table structure is as follows: column 1 features the protein identifier as used throughout the application to identify this sequence; column 2 features the name of the protein; column 3 features the Intepro identifier; column 4 features the analysis type; column 5 features the domain description; and column 6 features the position(s) of the amino acid residues that are relevant to this domain on the protein (amino acid sequence). Spacing for this table is as follows: column 1: characters 1-8; column 2: characters 9-48; column 3: 49-72; column 4: characters 73-96; column 5: characters 97-136; and column 6: 137-168.

Interpro provides information with regard to the analysis of amino acid sequences to identify domains having certain functionality (see Mulder et al (2003), The InterPro Database, 2003 brings increased coverage and new features, Nucleic Acids Res. 31, 315-318 for a reference). It features a database of protein families, domains and functional sites in which identifiable features found in known proteins can be applied to unknown protein sequences. The analysis type relates to the type of software used to determine the domain: Pfam (see Bateman A, et al (2004) The Pfam protein families database. Nucleic Acids Res. 32, 138-41), SMART (see Letunic I, et al (2004) SMART 40: towards genomic data integration. Nucleic Acids Res. 32, 142-4), TIGRFAMs (see Haft DH, et al (2003) The TIGRFAMs database of protein families. Nucleic Acids Res. 31, 371-373), PIRSF (see Wu CH et al (2003) The Protein Information Resource. Nucleic Acids Res. 31, 345-347), and SUPERFAMILY (see Gough J et al (2001) Assignment of homology to genome sequences using a library of Hidden Markov Models that represent all proteins of known structure. Journal Molecular BioL 313, 903-919) all use hidden Markov models (HMMs) to determine the location of domains on protein sequences.

With regard to transcript expression information, table structure is as follows: column 1 features the transcript identifier as used throughout the application to identify this sequence; column 2 features the name of the transcript; column 3 features the name of the probeset used in the chip experiment; and column 4 relates to the tissue and level of expression found. Spacing for this table is as follows: column 1: characters 1-9; column 2: characters 10-27; column 3: 28-41; and column 4: characters 42-121.

Information given in the text with regard to expression was determined according to oligonucleotide binding to arrays. Information is given with regard to overexpression of a cluster in cancer based on microarrays. As a microarray reference, in the specific segment paragraphs, the unabbreviated tissue name was used as the reference to the type of chip for which expression was measured. Oligonucleotide microarray results were taken from Affymetrix data, available from Affymetrix Inc, Santa Clara, CA, USA (see for example data regarding the Human Genome U133 (HG-U133) Set at www.affymetrix.com/products/arrays/specific/hgu133.affx; GeneChip Human Genome U133A 2.0 Array at www.affymetrix.com/products/arrays/specific/hgu133av2.affx; and Human Genome U133 Plus 2.0 Array at www.affymetrix.com/products/arrays/specific/hgu133plus.affx). The data is available from NCBI Gene Expression Omnibus (see www.ncbi.nlm.nih.gov/projects/geo/ and Edgar et al, Nucleic Acids Research, 2002, VoL 30, No. 1 207-210). The dataset (including results) is available from www.ncbi.nlm.nih.gov/geo/query/acc.cgi?acc=GSE1133 for the Series GSE1133 database (published on March 2004); a reference to these results is as follows: Su et al (Proc Natl Acad Sci U S A. 2004 Apr 20;101(16):6062-7. Epub 2004 Apr 09).

With regard to EST distribution information, table structure is as follows: column 1 features the transcript identifier as used throughout the application to identify this sequence; column 2 features the name of the transcript; and column 3 relates to the tissue and level of expression found in terms of numbers of ESTs. Spacing for this table is as follows: column 1: characters 1-9; column 2: characters 10-27; and column 3: characters 28-107.

***EXAMPLE 31***

***Description of CD-ROM6 and CD-ROM7***

Enclosed CD-ROM6 contains the following files:

1. "pos_proteins_x_y", wherein "x" may be from 1 to 45, and "y" may be 1 or 2, containing comparison reports between known protein sequences and variant protein sequences according to the present invention, including identifying unique regions therein.

2. "variants_report.txt", containing the information about the different variants of the known protein sequences (for example, due to known amino acid changes because of an SNP).

Enclosed CD-ROM7 contains the following files:

1. "not_mapped_report_x_y", wherein "x" may be from 1 to 45, and "y" may be 1 or 2, also containing comparison reports between known protein sequences and variant protein sequences according to the present invention, including identifying unique regions therein.

All tables are best viewed by using a text editor with the "word wrap" function disabled (to preserve line integrity) and in a fixed width font, such as Courier for example, preferably in font size 10. Table spacing is described for each table as a guide to assist in reading the tables.

With regard to comparison reports between variant protein according to the present invention and known protein, table structure is as follows: column 1 features the protein identifier as used throughout the application to identify this sequence; column 2 features the name of the protein; column 3 reports on the differences between the variant protein sequence and the known protein sequence (including the name of the known protein); and column 4 shows the alignment between the variant protein sequence and the known protein sequence. Spacing for this table is as follows: characters 1-18: column 1; characters 19-32: column 2; characters 33-92: column 3; and characters 97-170: column 4.

Information given in the text with regard to the Homology to the known proteins was determined by Smith-Waterman version 5.1.2 using special (non default) parameters as follows:

- model=sw.model
- GAPEXT=0
- GAPOP=100.0
- MATRIX=blosum100

In some cases, the known protein sequence was included with one or more known variations in order to assist in the above comparison. These sequences are given in variants_report.txt: column 1 features the name of the protein sequence as it appears in the comparison to the variant protein(s); column 2 features the altered protein sequence; column 3 features the type of variation (for example init_met refers to lack of methionine at the beginning of the original sequence); column 4 states the location of the variation in terms of the amino acid(s) that is/are changed; column 5 shows FROM; and column 6 shows TO (FROM and TO - start and end of the described feature on the protein sequence). Spacing for this table is as follows: column 1: characters 1-24; column 2: characters 25-96; column 3: characters 97-120; column 4: characters 121-144; and column 5: characters 145-169.

The comparison reports herein may optionally include such features as bridges, tails, heads and/or insertions (unique regions), and/or analogs, homologs and derivatives of such peptides (unique regions).

As used herein a "tail" refers to a peptide sequence at the end of an amino acid sequence that is unique to a splice variant according to the present invention. Therefore, a splice variant having such a tail may optionally be considered as a chimera, in that at least a first portion of the splice variant is typically highly homologous (often 100% identical) to a portion of the corresponding known protein, while at least a second portion of the variant comprises the tail.

As used herein a "head" refers to a peptide sequence at the beginning of an amino acid sequence that is unique to a splice variant according to the present invention. Therefore, a splice variant having such a head may optionally be considered as a chimera, in that at least a first portion of the splice variant comprises the head, while at least a second portion is typically highly homologous (often 100% identical) to a portion of the corresponding known protein.

As used herein "an edge portion" refers to a connection between two portions of a splice variant according to the present invention that were not joined in the wild type or known protein. An edge may optionally arise due to a join between the above "known protein" portion of a variant and the tail, for example, and/or may occur if an internal portion of the wild type sequence is no longer present, such that two portions of the sequence are now contiguous in the splice variant that were not contiguous in the known protein. A "bridge" may optionally be an edge portion as described above, but may also include a join between a head and a "known protein" portion of a variant, or a join between a tail and a "known protein" portion of a variant, or a join between an insertion and a "known protein" portion of a variant.

Optionally and preferably, a bridge between a tail or a head or a unique insertion, and a "known protein" portion of a variant, comprises at least about 10 amino acids, more preferably at least about 20 amino acids, most preferably at least about 30 amino acids, and even more preferably at least about 40 amino acids, in which at least one amino acid is from the tail/head/insertion and at least one amino acid is from the "known protein" portion of a variant. Also optionally, the bridge may comprise any number of amino acids from about 10 to about 40 amino acids (for example, 10, 11, 12, 13...37, 38, 39, 40 amino acids in length, or any number in between).

It should be noted that a bridge cannot be extended beyond the length of the sequence in-either direction, and it should be assumed that every bridge description is to be read in such manner that the bridge length does not extend beyond the sequence itself.

Furthermore, bridges are described with regard to a sliding window in certain contexts below. For example, certain descriptions of the bridges feature the following format: a bridge between two edges (in which a portion of the known protein is not present in the variant) may optionally be described as follows: a bridge portion of CONTIG-NAME_P1 (representing the name of the protein), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise XX (2 amino acids in the center of the bridge, one from each end of the edge), having a structure as follows (numbering according to the sequence of CONTIG-NAME_P1): a sequence starting from any of amino acid numbers 49-x to 49 (for example); and ending at any of amino acid numbers 50 + ((n-2) - x) (for example), in which x varies from 0 to n-2. In this example, it should also be read as including bridges in which n is any number of amino acids between 10-50 amino acids in length. Furthermore, the bridge polypeptide cannot extend beyond the sequence, so it should be read such that 49-x (for example) is not less than 1, nor 50 + ((n-2) - x) (for example) greater than the total sequence length.

In another embodiment, this invention provides antibodies specifically recognizing the splice variants and polypeptide fragments thereof of this invention. Preferably such antibodies differentially recognize splice variants of the present invention but do not recognize a corresponding known protein, optionally and more preferably through recognition of a unique region as described herein.

All nucleic acid sequences and/or amino acid sequences shown herein as embodiments of the present invention relate to their isolated form, as isolated polynucleotides (including for all transcripts), oligonucleotides (including for all segments, amplicons and primers), peptides (including for all tails, bridges, insertions or heads, optionally including other antibody epitopes as described herein) and/or polypeptides (including for all proteins). It should be noted that oligonucleotide and polynucleotide, or peptide and polypeptide, may optionally be used interchangeably.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences identified by their accession numbers mentioned in this specification are herein incorporated in their entirety by reference into the specification, to the same extent as if each individual publication, patent, patent application or sequence identified by their accession number was specifically and individually indicated to be incorporated herein by reference. In addition, citation or identification of any reference in this application shall not be construed as an admission that such reference is available as prior art to the present invention.

*CD-ROM* Content

The following lists the file content of the CD-ROMs which are enclosed herewith and filed with the application. File information is provided as: File name/byte size/date of creation/operating system/machine format

CD-ROM1 (2 files):

1. "Transcripts_nucleotide_seqs_part1"/ 594,303,263 bytes/ September 4, 2002/ PC/ Internet Explorer
2. "GC_new"/ 167 bytes/ January 4,2004/ PC/ Notepad

CD-ROM2 (5 files):

1. "Transcripts_nucleotide_seqs_part2"/ 132,371,321 bytes/ September 4, 2002/ PC/ Internet Explorer
2. "Transcripts_nucleotide_seqs_part3."/ 27,709,397 bytes/ September 4, 2002/ PC/ Internet Explorer
3. "Protein.seqs"/ 97,838,818 bytes/ September 4, 2002/ PC/ Internet Explorer

4. "ProDG_seqs"/ 404,161 bytes/ September 4, 2002/ PC/ Internet Explorer

5. "Transcripts_nucleotide_seqs_part4"/ 5,463,018 bytes/ April 24, 2003/ PC/ Internet Explorer

CD-ROM3 (1 file):

1. "Summary_table"/ 590,263,484 bytes/ April 24, 2003/ PC/ Internet Explorer

CD-ROM4 (3 files):

1. "Annotations.gz"/ 56,350,000 bytes/ January 12, 2004/ PC/ Internet Explorer (winzip file)

2. "Proteins.gz"/ 12,855,000 bytes/ January 12, 2004/ PC/ Internet Explorer (winzip file)

3. "Transcripts.gz"/ 100,834,000 bytes/ January 12, 2004/ PC/ Internet Explorer (winzip file)

CD-ROM5 (84 files):

1. GC_localization_1 /2,378,737 bytes/ January 21, 2005/PC/text file

2. GC_localization_2/2,380,602 bytes/ January 21, 2005/PC/text file

3. GC_localization_3 /2,379,257 bytes/ January 21, 2005/PC/text file

4. GC_localization_4/87,253 bytes/ January 21, 2005/PC/text file

5. pos_proteins_ipr_report_1_dos / 10,586,666 bytes/ January 22, 2005/PC/text file

6. pos_proteins_ipr_report_2_dos / 10,586,269 bytes/ January 22, 2005/PC/text file

7. pos_proteins_ipr_report_3_dos / 10,585,825 bytes/ January 22, 2005/PC/text file

8. pos_proteins_ipr_report_4_dos / 6,496,110 bytes/ January 22, 2005/PC/text file

9-54. "GC_expression_x", wherein "x" may be from 9 to 54 / (9) 9,214,636 bytes; (10) 9,081,780 bytes; (11) 9,154,407 bytes; (12) 9,308,696 bytes; (13) 9,308,992 bytes; (14) 9,299,294 bytes; (15) 9,534,459 bytes; (16) 9,673,720 bytes; (17) 9,465,217 bytes; (18) 9,442,226 bytes; (19) 9,447,735 bytes; (20) 9,626,304 bytes; (21) 9,400,354 bytes; (22) 9,469,312 bytes; (23) 9,396,605 bytes; (24) 9,465,028 bytes; (25) 9,439,944 bytes'; (26) 9,412,413 bytes; (27) 9,397,960 bytes; (28) 9,365,528 bytes; (29) 9,375,167 bytes; (30) 9,363,929 bytes; (31) 9,437,120 bytes; (32) 9,732,771 bytes; (33) 9,713,720 bytes; (34) 9,848,090 bytes; (35) 9,721,763 bytes; (36) 9,678,045 bytes; (37) 9,763,448 bytes; (38) 9,649,552 bytes; (39) 9,314,050 bytes; (40) 9,645,203 bytes; (41) 9,831,288 bytes; (42) 9,710,696 bytes; (43) 9,511,299 bytes; (44) 9,549,547 bytes; (45) 9,487,768 bytes; (46) 9,469,180 bytes' (47) 9,388,181 bytes; (48) 9,412,450 bytes; (49) 9,668,768 bytes; (50) 9,526,664 bytes; (51) 9,416,802 bytes; (52) 9,557,697 bytes; (53) 9,525,028 bytes; (54) 3,704,972 bytes/ January 21, 2005/PC/text files

55-83. "GC_expression_y", wherein "y" may be from 55 to 83 /(55) 3,817,168 bytes; (56) 3,799,248 bytes; (57) 3,817,532 bytes; (58) 3,874,264 bytes; (59) 3,830,725 bytes; (60) 3,956,329 bytes; (61) 3,946,376 bytes; (62) 3,929,059 bytes; (63) 3,970,538 bytes; (64) 3,934,979 bytes; (65) 3,922,081 bytes; (66) 3,913,641 bytes; (67) 3,921,419 bytes; (68) 3,890,506 bytes; (69) 3,897,223 bytes; (70) 3,889,876 bytes; (71) 4,104,832 bytes; (72) 4,060,342 bytes; (73) 4,100,080 bytes; (74) 3,942,622 bytes; (75) 4,044,765 bytes; (76) 4,044,765 bytes; (77) 3,953,800 bytes; (78) 3,939,035 bytes; (79) 3,917,616 bytes; (80) 4,005,800 bytes; (81) 3,934,991 bytes; (82) 3,945,777 bytes; (83) 823,700 bytes/January 21, 2005/PC/text files

84. oligo probs abbreviations for patent /1,256 bytes/ January 23, 2005/PC/text file

CD-ROM6 (46 files)

1-45. "pos_proteins_x_y", wherein "x" may be from 1 to 45, and "y" may be 1 or 2 /(1_1) 10,570,916 bytes; (1_2) 4,161,830 bytes; (2_1) 10,588,485 bytes; (2_2) 2,459,781 bytes; (3_1) 10,427,688 bytes; (4_1) 10,559,367 bytes; (4_2) 3,235,926 bytes; (5_1) 10,558,697 bytes; (5_2) 2,280,702 bytes; (6_1) 10,560,297 bytes; (6_2) 2,018,993 bytes; (7_1) 10,219,364 bytes; (8_1) 9,649,567 bytes; (9_1) 10,714,690 bytes; (9_2) 1,467,287 bytes; (10_1) 9,071,523 bytes; (11_1) 8,280,109 bytes; (12_1) 9,632,356 bytes; (13_1) 8,638,177 bytes; (14_1) 10,561,478 bytes; (14_2) 5,407,861 bytes; (15_1) 10,557,873 bytes; (15_2) 6,259,819 bytes; (16_1) 10,557,317 bytes; (16_2) 4,263,683 bytes; (17_1) 10,558,573 bytes; (17_2) 5,965,301 bytes; (18_1) 10,560,130 bytes (18_2) 2,396,758 bytes ; (19_1) 10,558,010 bytes; (19_2) 2,847,184 bytes; (20_1) 10,588,298 bytes; (20_2) 3,768,855 bytes (21_1) 10,573,416 bytes; (21_2) 4,445,746 bytes (22_1) 10,557,051 bytes; (22_2) 1,331,734 bytes; (23_1) 10,557,956 bytes; (23_2) 4,864,012 bytes; (24_1) 10,570,905 bytes; (24_2) 7,634,772 bytes; (25_1) 10,611,377 bytes; (25_2) 5,093,347 bytes; (26_1) 10,563,842 bytes; (26_2) 1,195,230 bytes; (27_1) 10,591,727 bytes; (27_2) 3,616,606 bytes; (28_1) 10,623,887 bytes; (28_2) 2,939,329 bytes; (29_1) 10,560,510 bytes; (29_2) 1,680,816 bytes; (30_1) 10,566,104 bytes; (30_2) 2,494,098 bytes; (31_1) 10,561,616 bytes; (31_2) 3,155,447 bytes; (32_1) 10,558,910 bytes; (32_2) 1,758,624 bytes; (33_1) 10,559,701 bytes;

(33_2) 1,293,659 bytes; (34_1) 10,556,906 bytes; (34_2) 1,999,767 bytes; (35_1) 10,593,569 bytes; (35_2) 4,607,901 bytes; (36_1) 10,562,098 bytes; (36_2) 2,172,735 bytes (37_1) 10,582,541 bytes; (37_2) 7,449,260 bytes; (38_1) 10,556,188 bytes; (38_2) 3,864,084 bytes; (39_1) 10,569,544 bytes (39_2) 2,713,147 bytes; (40_1) 10,561,012 bytes; (40_2) 5,248,633 bytes; (41_1) 10,569,007 bytes;(41_2) 5,343,005 bytes; (42_1) 10,556,579 bytes; (42_2) 5,605,359 bytes; (43_1) 10,563,105 bytes; (43_2) 10,563,105 bytes; (44_1) 10,556,778 bytes; (44_2) 7,308,690 bytes; (45_1) 58,980 bytes/ January 21, 2005/PC/text files

46. "variants_report.txt" /2,867,745 bytes/ January 22, 2005/PC/text file

CD-ROM7 (45 files)

1-45. "not_mapped_report_x_y", wherein "x" maybe from 1 to 45, and "y" may be 1 or 2 /(1_1) 10,576,298 bytes; (1_2) 4,222,791 bytes; (2_1) 10,562,054 bytes; (2_2) 248,826 bytes; (3_1) 10,559,026 bytes; (3_2) 1,446,960 bytes; (4_1) 10,575,598 bytes; (4_2) 4,742,743 bytes); (5_1) 10,559,843 bytes; (5_2) 2,176,599 bytes; (6_1) 10,574,002 bytes; (6_2) 1,446,117 bytes; (7_1) 9,749,754 bytes; (8_1) 9,017,086 bytes; (9_1) 10,602,966 bytes; (9_2) 4,402,068 bytes; (10_1) 8,913,636 bytes; (11_1) 8,720,771 bytes; (12_1) 9,981,606 bytes; (13_1) 10,567,765 bytes; (13_2) 474,113 bytes; (14_1) 10,560,625 bytes; (14_2) 1,087,116 bytes; (15_1) 7,569,541 bytes; (16_1) 10,563,539 bytes; (16_2) 3,339,041 bytes; (17_1) 10,556,970 bytes; (17_2) 492,893 bytes; (18_1) 10,565,983 bytes; (18_2) 7,189,913 bytes; (19_1) 10,556,650 bytes; (19_2) 3,134,978 bytes; (20_1) 10,559,149 bytes; (20_2) 2,925,497 bytes; (21_1) 10,563,142 bytes (21_2) 2,959,111 bytes; (22_1) 10,569,992 bytes; (22_2) 3,995,958 bytes; (23_1) 10,560,900 bytes; (23_2) 7,750,412 bytes; (24_1) 10,555,999 bytes; (24_2) 1,085,007 bytes; (25_1) 10,566,649 bytes; (25_2) 2,891,369 bytes; (26_1) 10,558,186 bytes; (26_2) 9,456,211 bytes; (27_1) 10,563,619 bytes; (27_2) 4,379,336 bytes; (28_1) 10,577,845 bytes; (28_2) 2,759,632 bytes; (29_1) 10,572,719 bytes; (29_2) 4,280,054 bytes; (30_1) 10,559,701 bytes; (30_2) 3,800,438 bytes; (31_1) 10,560,665 bytes; (31_2) 3,683,403 bytes; (32_1) 10,557,516 bytes; (32_2) 1,816,935 bytes; (33_1) 10,562,496 bytes; (33_2) 1,122,816 bytes; (34_1) 10,561,495 bytes; (34_2) 3,487,803 bytes; (35_1) 10,563,439 bytes; (35_2) 2,209,562 bytes; (36_1) 10,559,147 bytes; (36_2) 2,423,910 bytes; (37_1) 10,575,905 bytes; (37_2) 4,668,663 bytes; (38_1) 10,560,131 bytes; (38_2) 4,748,251 bytes; (39_1) 10,670,985 bytes; (39_2) 10,568,385 bytes; (39_3) 2,939,389 bytes; (40_1) 10,574,754 bytes; (40_2) 2,443,940 bytes; (41_1) 10,565,694 bytes; (41_2) 3,818,655 bytes; (42_1) 10,559,922 bytes; (42_2) 6,467,236 bytes; (43_1) 10,577,714 bytes; (43_2) 5,650,119 bytes; (44_1) 10,556,832 bytes; (44_2) 4,295,811 bytes; (45_1) 10,566,176 bytes; (45_2) 5,734,325 bytes / January 21, 2005/PC/text files

1. A computer readable storage medium, comprising a database stored in a retrievable manner, said database including biomolecular sequence information as set forth in files "Transcripts.gz", and/or "Proteins.gz" of enclosed CD-ROM4, and biomolecular sequence annotations, as set forth in file "Annotations.gz" of enclosed CD-ROM4.

2. The computer readable storage medium of item 1, wherein said database further includes information pertaining to generation of said data and potential uses of said data.

3. The computer readable storage medium of item 1, wherein the medium is selected from the group consisting of a magnetic storage medium, an optical storage medium and an optico-magnetic storage medium.

4. A method of comparing an expression level of a gene of interest in at least two types of tissues, the method comprising:

(a) obtaining a contig representing the gene of interest, said contig being assembled from a plurality of expressed sequences; and
(b) comparing a number of said plurality of expressed sequences corresponding to said contig which are expressed in each of the at least two tissue types, to thereby compare the expression level of the gene of interest in the at least two tissue types.

5. The method of item 4, wherein said plurality of expressed sequences present complete exonal coverage of the gene of interest.

6. The method of item 4, wherein said plurality of expressed sequences present partial exonal coverage of the gene of interest.

7. The method of item 4, wherein said obtaining said contig is effected by a sequence assembly software.

8. The method of item 4, further comprising scoring each of said plurality of said expressed sequences prior to (b), wherein said scoring is effected according to:

(i) expression level of each of said plurality of said expressed sequences; and
(ii) a quality of each of said plurality of said expressed sequences.

9. The method of item 4, wherein comparing is effected using statistical pairing analysis.

10. The method of item 9, wherein said statistical pairing analysis is Fisher exact test.

11. The method of item 4, wherein the at least two types of tissues are selected from the group consisting of tissues of different pathological origin, tissues of different developmental origin and tissues of a different cellular composition.

12. The method of item 4, further comprises computationally aligning sequences expressed in each of the at least two types of tissue with said contig to thereby identify said expressed sequences corresponding to said contig prior to (b).

13. A method of comparing an expression level of at least two splice variants of a gene of interest in a tissue, the method comprising:

(a) obtaining a contig having exonal sequences of the at least two splice variants of the gene of interest, said contig being assembled from a plurality of expressed sequences;
(b) identifying at least one contig sequence region unique to one of the at least two splice variants of the gene of interest; and
(c) comparing a number of said plurality of expressed sequences in the tissue having said at least one contig sequence region with a number of said plurality of expressed sequences not-having said at least one contig sequence region, to thereby compare the expression level of the at least two splice variants of the gene of interest in the tissue.

14. The method of item 13, wherein said plurality of expressed sequences present complete exonal coverage of the gene of interest.

15. The method of item 13, wherein said plurality of expressed sequences present partial exonal coverage of the gene of interest.

16. The method of item 13, wherein said obtaining said contig is effected by a sequence assembly software.

17. The method of item 13, further comprising scoring each of said plurality of said expressed sequences prior to (c), wherein said scoring is effected according to:

(i) expression level of each of said plurality of said expressed sequences; and
(ii) a quality of each of said plurality of said expressed sequences.

18. The method of item 13, wherein comparing is effected using statistical pairing analysis.

19. The method of item 18, wherein said statistical pairing analysis is Fisher exact test.

20. The method of item 13, wherein the tissue is selected from the group consisting of a tissue of a pathological origin of interest, a tissue of a cellular composition of interest.

21. The method of item 13, further comprising comparing the number of said plurality of expressed sequences in the tissue having said at least one contig sequence region with a number of said plurality of expressed sequences of the contig.

22. A computer readable storage medium comprising data stored in a retrievable manner, said data including sequence information of differentially expressed mRNA sequences as set forth in files "Transcripts.gz", and/or "Proteins.gz" of enclosed CD-ROM4, and sequence annotations as set forth in annotation categories "#TS", "#TAA" and/or "#TAAT", in the file "Annotations.gz" of enclosed CD-ROM4.

23. The computer readable storage medium of item 22, wherein said database further includes information pertaining to generation of said data and potential uses of said data.

24. The computer readable storage medium of item 22, wherein said medium is selected from the group consisting of a magnetic storage medium, an optical storage medium and an optico-magnetic storage medium.

25. The computer readable storage medium of item 22, wherein said database further includes information pertaining to gain and/or loss of function of said differentially expressed mRNA splice variants or polypeptides encoded thereby.

26. A kit useful for detecting differentially expressed polynucleotide sequences, the kit comprising at least one oligonucleotide being designed and configured to be specifically hybridizable with a polynucleotide sequence selected from the group consisting of sequence files "Transcripts.gz" of enclosed CD-ROM4 under moderate to stringent hybridization conditions.

27. The kit of item 26, wherein said at least one oligonucleotide is labeled.

28. The kit of item 26, wherein said at least one oligonucleotide is attached to a solid substrate.

29. The kit of item 28, wherein said solid substrate is configured as a microarray and whereas said at least one oligonucleotide includes a plurality of oligonucleotides each being capable of hybridizing with a specific polynucleotide sequence of the polynucleotide sequences set forth in the files "Transcripts.gz" of enclosed CD-ROM4 under moderate to stringent hybridization conditions.

30. The kit of item 29, wherein each of said plurality of oligonucleotides is being attached to said microarray in a regio-specific manner.

31. The kit of item 26, wherein said at least one oligonucleotide is designed and configured for DNA hybridization.

32. The kit of item 26, wherein said at least one oligonucleotide is designed and configured for RNA hybridization.

33. A system for generating a database of differentially expressed genes, the system comprising a processing unit, said processing unit executing a software application configured for:

(a) obtaining contigs representing genes of interest, each of said contigs being assembled from a plurality of expressed sequences;
(b) comparing a number of said plurality of expressed sequences corresponding to each of said contigs which are expressed in each of at least two tissue types, to thereby compare the expression level of the genes of interest in said at least two tissue types; and
(c) storing contigs which are supported by different numbers of said plurality of expressed sequences in each of said at least two tissue types, to thereby generate the database of differentially expressed genes.

34. An isolated polynucleotide comprising a nucleic acid sequence being at least 80 % identical to a nucleic acid sequence of the sequences set forth in file "Transcripts.gz" of the enclosed CD-ROM4.

35. The isolated polynucleotide of item 34, wherein said nucleic acid sequence is set forth in the file "Transcripts.gz" of the enclosed CD-ROM4.

36. An isolated polynucleotide comprising a nucleic acid sequence encoding a polypeptide having an amino acid sequence at least 80 % homologous to a sequence set forth in the file "Proteins.gz" of the enclosed CD-ROM4.

37. An isolated polynucleotide comprising a nucleic acid sequence at least 80 % identical to a sequence set forth in the file "Transcripts.gz" of the enclosed CD-ROM4.

38. An isolated polypeptide having an amino acid sequence at least 80 % homologous to a sequence set forth in the file "Proteins.gz" of the enclosed CD-ROM4.

39. Use of a polynucleotide or polypeptide set forth in the file "Transcripts.gz" or "Proteins.gz" of the enclosed CD-ROM4 for the diagnosis and/or treatment of the diseases listed in herein.

**Claims**

1. An isolated monoclonal or polyclonal antibody or an antigen binding fragment thereof comprising an antigen binding site that binds specifically to an amino acid sequence as set forth in any one of:

   a. HUMDAF_P3 polypeptide, having the amino acid sequence set forth in SEQ ID NO: 1,499,531.
   b. HUMDAF_P2 polypeptide, having the amino acid sequence set forth in SEQ ID NO: 1,499,523.
   c. N30824_P3 (heparanase variant) polypeptide, having the amino acid sequence set forth in SEQ ID NO: 1,411,223.
   d. R31375_P7 (FXYD3) polypeptide, having the amino acid sequence set forth in SEQ ID NO: 1,439,368.
   e. T10306_P4 (TMEPAI) polypeptide, having the amino acid sequence set forth in SEQ ID NO: 1,439,186.
   f. T87597_P1 (FLJ13593) polypeptide, having the amino acid sequence set forth in SEQ ID NO: 1,461,794.

2. The antibody or fragment according to claim 1, wherein the antibody is a fully human antibody, a chimeric antibody, or humanized antibody.

3. The antibody or fragment according to claim 1, wherein the antibody is selected from the group consisting of Fab, Fab', F(ab')2, F(ab'), F(ab), Fv or scFv fragment and minimal recognition unit.

4. The antibody or fragment according to claim 1, wherein the antibody or fragment is coupled to a detectable label, selected from any one of a radioactive material, an enzyme, a fluorescent compound, a bioluminescent compound or a chemiluminescent compound, or to a toxin, a therapeutic agent, or a chemotherapeutic agent.

5. A pharmaceutical composition, comprising the antibody or fragment of any of claims 1-4.

6. The pharmaceutical composition of claim 5, wherein said antibody or fragment comprises an antigen binding site that binds specifically to an amino acid sequence as set forth in any one of SEQ ID NO: 1,499,531 and SEQ ID NO: 1,499,523, or differentially recognizes SEQ ID NO: 1,499,531 and SEQ ID NO: 1,499,523, splice variants of a corresponding known protein CD55 but does not recognize CD55, adapted for treatment of disorder selected from the group consisting of immunological disorder, rheumatoid arthritis, complement factor 1 inhibition related disorder, lung cancer and bone marrow tumor, or for use as an immunosuppressant.

7. The pharmaceutical composition of claim 5, wherein said antibody or fragment comprises an antigen binding site that binds specifically to the amino acid sequence set forth in SEQ ID NO: 1,411,223, adapted for treatment of disorder selected from the group consisting of inflammatory disease, cancer selected from the group consisting of lung cancer, myeloma and melanoma, cardiovascular disorder, multiple sclerosis, inflammatory bowel disease and rheumatoid arthritis.

8. The pharmaceutical composition of claim 5, wherein said antibody or fragment comprises an antigen binding site that binds specifically to amino acid sequences set forth in SEQ ID NO: 1,439,368, adapted for treatment of skin cancer.

9. The pharmaceutical composition of claim 5, wherein said antibody or fragment comprises an antigen binding site that binds specifically to amino acid sequences set forth in SEQ ID NO: 1,439,186, adapted for treatment of skin cancer and/or pancreas cancer.

10. The pharmaceutical composition of claim 5, wherein said antibody or fragment comprises an antigen binding site that binds specifically to amino acid sequences set forth in SEQ ID NO: 1,461,794, adapted for treatment of gastrointestinal disease.

11. A use of the antibody or fragment, wherein the antibody or fragment comprises an antigen binding site that binds specifically to amino acid sequences set forth in any one of SEQ ID NO: 1,499,531 and SEQ ID NO: 1,499,523, or differentially recognizes SEQ ID NO: 1,499,531 and SEQ ID NO: 1,499,523, splice variants of a corresponding known protein CD55 but does not recognize CD55, or the pharmaceutical composition according to claim 6, for treatment of a disorder selected from the group consisting of immunological disorder, rheumatoid arthritis, complement factor 1 inhibition related disorder, a cardiovascular disorder, lung cancer and bone marrow tumor, or for use as an immunosuppressant.

12. A use of the antibody or fragment, wherein said antibody or fragment comprises an antigen binding site that binds specifically to amino acid sequences set forth in SEQ ID NO: 1,411,223, or the pharmaceutical composition according to claim 7, for treatment of a disorder selected from the group consisting of inflammatory disease, cancer selected from the group consisting of lung cancer, myeloma and melanoma, cardiovascular disorder, multiple sclerosis, inflammatory bowel disease and rheumatoid arthritis.

13. A use of the antibody or fragment, wherein said antibody or fragment comprises an antigen binding site that binds specifically to amino acid sequences set forth in SEQ ID NO: 1,439,368, or the pharmaceutical composition according to claim 8, for treatment of skin cancer.

14. A use of the antibody or fragment, wherein said antibody or fragment comprises an antigen binding site that binds specifically to amino acid sequences set forth in SEQ ID NO: 1,439,186, or the pharmaceutical composition according to claim 9, for treatment of skin cancer and/or pancreas cancer.

15. A use of the antibody or fragment, wherein said antibody or fragment comprises an antigen binding site that binds specifically to amino acid sequences set forth in SEQ ID NO: 1,461,794, or the pharmaceutical composition according to claim 10, for treatment of gastrointestinal disease.

16. An assay for detecting the presence of polypeptide comprising the amino acid sequence as set forth in any one of SEQ ID NOs: 1,499,531; 1,499,523; 1,411,223, 1,439,368, 1,439,186 and 1,461,794 in a biological sample comprising a cell lysate or supernatant, or a histological section, comprising contacting the sample with the antibody of claim 1, and detecting the specific binding of said antibody to any one of SEQ ID NOs: 1,499,531; 1,499,523; 1,411,223, 1,439,368, 1,439,186 and 1,461,794 in the sample.

17. A use of the antibody of claim 1 for detecting a disease, comprising detecting in a subject or in a sample obtained from the subject a polypeptide having a sequence selected from those set forth in any one of SEQ ID NOs: 1,499,531; 1,499,523; 1,411,223, 1,439,368, 1,439,186 and 1,461,794, wherein a presence of said polypeptide in the subject or in the sample indicates detection of said disease.

18. The use of claim 17, further comprising one or more of diagnosing a disease, or treatment efficacy or selecting a therapy for said disease, or determining prognosis of said disease, according to said presence of said polypeptide.

19. The use of claim 17 wherein said detecting said polypeptide comprises performing an immunoassay on the sample from the subject to detect binding of an antibody to said polypeptide.

20. The use of claim 17, wherein the polypeptide having the sequence selected from any one of SEQ ID NOs: 1,499,531; 1,499,523; and wherein the disease is selected from the group consisting of immunological disorder, rheumatoid arthritis, complement factor 1 inhibition related disorder, lung cancer, a cardiovascular disorder and bone marrow tumor.

21. The use of claim 17, wherein the polypeptide having the sequence set forth in SEQ ID NO: 1,411,223, and wherein the disease is selected from the group consisting of inflammatory disease, cancer selected from the group consisting of lung cancer, myeloma and melanoma, cardiovascular disorder, multiple sclerosis, inflammatory bowel disease and rheumatoid arthritis.

22. The use of claim 17, wherein the polypeptide having the sequence set forth in SEQ ID NO: 1,439,368, and wherein the disease is skin cancer.

23. The use of claim 17, wherein the polypeptide having the sequence set forth in SEQ ID NO: 1,439,186, and wherein the disease is skin cancer and/or pancreas cancer.

24. The use of claim 17, wherein the polypeptide having the sequence set forth in SEQ ID NO: 1,461,794, and wherein the disease is gastrointestinal disease.

25. The pharmaceutical composition of claim 6, the use of claim 11 or the use of claim 20, wherein

   a. the bone marrow tumor is leukemia, selected from the group consisting of B-lymphoid leukemia, T-lymphoid leukemia, undifferentiated leukemia, erythroleukemia, megakaryoblastic leukemia, and monocytic leukemia, myeloid leukemia, chronic myelogenous leukemia, acute myelogenous leukemia, acute promyelocytic leukemia, acute nonlymphocytic leukemia with increased basophils, acute monocytic leukemia, acute myelomonocytic leukemia with eosinophilia, lymphoctyic leukemia, acute lumphoblastic leukemia, chronic lymphocytic leukemia, erythroblastic leukemia and acute megakaryoblastic leukemia, acute promyeloid leukemia (APML), acute myelogenous leukemia (AML) and chronic myelogenous leukemia (CML), acute lymphoblastic leukemia (ALL), B-lineage ALL and T-lineage ALL, chronic lymphocytic leukemia (CLL), prolymphocytic leukemia (PLL), hairy cell leukemia (HLL), Waldenstrom's macroglobulinemia (WM), myelo dysplastic syndrome, hematologic malignancies of monocyte-macrophage lineage, juvenile chronic myelogenous leukemia, secondary AML, and antecedent hematological disorder; and

b. wherein the lung cancer is selected from the group consisting of small cell lung cancer and non small cell lung cancer.

26. The pharmaceutical composition of claim 10, the use of claim 15 or the use of claim 24, wherein the gastrointestinal disease is selected from the group consisting of autoimmune diseases of the gastrointestinal tract, celiac disease, colitis, ileitis, Crohn's disease and ulcerative colitis.

27. An isolated polypeptide consisting of the polypeptide sequence set forth in anyone of:

a. HUMDAF_P3 polypeptide, having the amino acid sequence set forth in SEQ ID NO: 1,499,531.
b. HUMDAF_P2 polypeptide, having the amino acid sequence set forth in SEQ ID NO: 1,499,523.
c. N30824_P3 (heparanase variant) polypeptide, having the amino acid sequence set forth in SEQ ID NO: 1,411,223.
d. R31375_P7 (FXYD3) polypeptide, having the amino acid sequence set forth in SEQ ID NO: 1,439,368.
e. T10306_P4 (TMEPAI) polypeptide, having the amino acid sequence set forth in SEQ ID NO: 1,439,186.
f. T87597_P1 (FLJ13593) polypeptide, having the amino acid sequence set forth in SEQ ID NO: 1,461,794.

<u>10</u>

Fig. 1a

<u>10</u>

Fig. 1b

Fig. 2

Fig. 3

EP 2 816 351 A2

**1 tumor**

***1.1 epithelial cell tumors***

1.1.1 carcinoma

1.1.1.1 adenocarcinoma

1.1.1.2 lobullar carcinoma

***1.2 Mesenchimal cell tumors***

1.2.1 sarcoma

1.2.1.1 liposarcoma

1.2.1.2 rhabdomyosarcoma

1.2.1.3 pnet

1.2.1.4 ewing sarcoma

***1.3 blood tumors***

1.3.3 lymphoma

1.3.2 leukemia

1.3.3 myeloma

***1.4 endocrine tumors***

1.4.1 pheocromocytoma

1.4.2 carcinoid

**2 endocrine system**

***2.1 adrenal***

2.1.1 pheocromocytoma

***2.2 pancreas***

2.2.1 islets of Langerhans

***2.3 neuroendocrine***

2.3.1 hypothalamus

2.3.2 carcinoid

***2.4 thyroid***

**3 vascular tissue**

***3.1 arteries***

3.1.1 aorta

***3.2 vein***

# Fig. 4

**4 genitourinary system**

***4.1 urinary* system**

4.1.1 bladder

4.1.2 kidney

***4.2 genital system***

4.2.1 women genital system

4.2.1.1 cervix

4.2.1.2 ovary

4.2.1.3 uterus

*4.2.1.3.1 endometrium*

***4.2.2 men gentile system***

4.2.2.1 prostate

4.2.2.2 testis

*4.2.2.2.1 epididymis*

**5 muscles**

***5.1 rhabdomyosarcoma***

***5.2 tongue***

***5.3 bladder***

***5.4 heart***

***5.5 uterus***

**6 blood**

***6.1 peripheral blood***

6.1.1 erythroid line

6.1.2 leukocyte

6.1.2.1 lymphoid system

*6.1.2.1.1 lymphoma*

*6.1.2.1.2 spleen*

*6.1.2.1.3 thalamus*

***6.2 stem cells***

**6.2.1 myeloid**

**6.2.2 myeloma**

***6.3 Bone marrow***

***6.4 leukemia***

Fig. 4 (Cont.)

7 **nerve system**

**7.1 CNS, central nervous system**

7.1.1 brain

7.1.1.1 cerebrum

7.1.1.2 cerebellum

7.1.1.3 pitituary gland

7.1.1.4 hypothalamus

7.1.1.5 thalamus

7.1.1.6 olfactory

7.1.1.7 Hippocampus

7.1.1.8 amygdala

7.1.1.9 frontal lobe

7.1.1.10 pnet

**7.2 Embryonal nerve system**

7.2.1 primitive neuroectoderm

**7.3 retina**

7.3.1 retinoblastoma


8 **breast**

**8.1 ductal breast**

8.1.1. ductal carcinoma

**8.2 lobullar carcinoma**

**8.3 mammary**


9 **skeleton**

**9.1 bone**

9.1. ewing sarcoma

9.1.2 craniofacial

9.1.2.1 calvarium

**9.2 connective tissue**

9.2.1 trabeculae

9.2.2 cartilage

# Fig. 4 (Cont.)

**10 embryo**

*10.1 amnion*

*10.2 chorion*

*10.3 primitive neuroectoderm*

*10.4 placenta*


**11 exocrine system**

*11.1 pancreas*

11.1.1 islets of Langerhans

*11.2 prostate*

*11.3 salivary gland*


**12 face organs**

*12.1 nose*

*12.2 ear*

12.2.1 cochlea

*12.3 eye*

12.3.1 retina

12.3.1.1 retinoblastoma

12.3.2 lens

*12.4 mouth*

*12.5 tongue*


**13 gastrointestinal system**

*13.1 mucosa*

*13.2 stomach*

*13.3 intestine*

13.3.1 colorectal

13.3.1.1 colon

**13.4 hepatobiliary system**

13.4.1 liver

13.4.2 biliary system

13.4.2.1 gall bladder

*13.5 pancreas*

13.5.1 islets of Langerhans

**Fig. 4 (Cont.)**

**14 respiratory system**

*14.1 nasopharynx*

*14.2 lung*

14.2.1 small cell lung carcinoma

**15 skin**

*15.1 dermis*

15.1. melanocyte

**16 fat tissue**

*16.1 liposarcoma*

# Fig. 4 (Cont.)

Fig. 5

Fig. 6a

Fig. 6b

Fig. 6c

Physiological Process GO Nodes

Number of Contigs/Proteins

Fig. 7

Fig. 8

Fig. 9

Fig. 10

Fig. 11

Fig. 12

Fig. 13

Fig. 14

980 human alternative exons
with skipping mouse EST

Human DNA

Human RNA/EST

Human RNA/EST

Mouse RNA/EST

Fig. 15a

Human DNA

Mouse RNA/EST

Mouse RNA/EST

149 Exons

Mouse DNA

Mouse RNA/EST

94 Exons

243 conserved exon-skipping events

Fig. 15b

Fig. 15c

**Masking - Scanning the multiple alignment for problematic regions where no SNPs should be predicted:**

• Masking of dirty regions (more than 3 bp differing from the consensus on a 20 bp stretch).

```
GAAAACTGTCACGCAAGAACA --- Consensus
GAAAACTGTCACGCAAGAACA --- BE072817 -- EST
GAAAACTGTCACGCAAGAACA --- BE072881 -- EST
GAAAACTGTCTCGCAAGAACA --- BE072946 -- EST
GAAAACTGTCACGCAAGAACA --- BE674705 -- EST
GTAAACTGTCACGCCAGGACC --- AV744807 -- EST
←                     → Masked region in a sequence (AV744807)
GAAAACTGTCACGCAAGAACA --- AA001990 -- EST
GAAAACTGTCACGCAAGAACA --- C7NT007695P11_AA001990 -- HTG
```

• Regions with repetitive characters (repeat length : at least 4 bp) and and the following base pair..

```
AGAAAACTGTCACGCAAGAACA --- Consensus
AGAAAACTGTCACGCAAGAACA --- BE072817 -- EST
AGAAAACTGTCACGCAAGAACA --- BE072881 -- EST
AGAAAACTGTCTCGCAAGAACA --- BE072946 -- EST
AGAAAACTGTCACGCAAGAACA --- BE674705 -- EST
AGTAAACTGTCACGCCAGGACC --- AV744807 -- EST
AGAAAACTGTCACGCAAGAACA --- AA001990 -- EST
AGAAAACTGTCACGCAAGAACA --- C7NT007695P11_AA001990 -- HTG
←    → Masked region in the multiple alignment
```

## Fig. 16a

• End of sequences (the first and last 30 bps of the sequences).

SNP at position 1035 #C 67% 2 #G 33% 1

AAGCCGCTAG>C<CCACCTCTTA --- Consensus

........................

AAGCCGCTAG>C<CCACCTCTTA --- AI076830 – EST :1 point
AAGCCGCTAG>G<CCACCTCTTA --- C9NT008545P26_AA001610 – DNA :3 points
AAGCCGCTAG>C<NCACCTCTTA --- AA001610 -- EST:1 point

# Fig. 16b

Deleting SNP columns that contain the same letters and the distance between them is no more than 1 column and the ratio between the score of this letter and the total number of sequences is less than 0.015.

```
AGCTCAGAATAGGCCAAGGCC --- Consensus position 620
. . . . . . . . . . . . . . . . . . . . . .
AGCTCAGAATTAGCCAAGGCC --- AA709048 -- EST
AGCTCAGAATAGGCCAAGGCC --- AI078180 -- EST
AGCTCAGAATAGGCCAAGGCC --- C2NT015805P117_AA035261 -- HTG
AGCTCAGAATTAGCCAAGGCC --- AA035261 -- EST


Is actually equivalent to the following alignment:


AGCTCAGAATTAGGCCAAGGCC --- Consensus position 620
. . . . . . . . . . . . . . . . . . . . . .
AGCTCAGAATTA.GCCAAGGCC --- AA709048 -- EST
AGCTCAGAAT.AGGCCAAGGCC --- AI078180 -- EST
AGCTCAGAAT.AGGCCAAGGCC --- C2NT015805P117_AA035261 -- HTG
AGCTCAGAATTA.GCCAAGGCC --- AA035261 -- EST
```

Which might be sequencer carry-over.

Fig. 16c

EP 2 816 351 A2

Deleting SNP columns that contain gaps, which are adjacent to columns that contain the same letters or gaps or ambiguous letters.

The SNP has gaps, while the adjacent column has the same letter as the SNP.

```
ATTTCCCATT>X<GGCAAGGAGCT  --- Consensus position 171
ATTTCCCATT>.<GGCAAGGAGCT  --- AI076307 -- EST
ATTTCCCATT>.<GGCAAGGAGCT  --- AI248359 -- EST
ATTTCCCATT>.<GGCAAGGAGCT  --- AA007403 -- EST
ATGTCCCATT>G<GGCAAGGAGCT  --- C7NT007867P30split0_AA007403 -- DNA
ATTTCCCATT>.<GGCAAGGGGCT  --- AA007481 -- EST
ATTTCCCATT>.<GGCAAGGAGCT  --- C7NT007720P67_AA007403 -- HTG
ATTTCCCATT>G<GGCAAGGAGCT  --- C7NT007720P103_AA007403 - HTG
```

## Fig. 16d

Fig. 17

GCSF structure

Fig. 18a

(GCSF T_2) -NO UNIQUE

AGTCGTGGCCCCAGGTAATTTCCTCCCAGGCCTCCATGGGGTTATGTATAAAGGCCCCCC
tagagctgggccccaaaacagcccggagcctgcagcccagccccacccagaccc**atg**gct
ggacctgccacccagagccccatgaagctgatggccctgcagctgctgctgtggcacagt
gcactctggacagtgcaggaagccacccccctgggccctgccagctccctgccccagagc
ttcctgctcaagtgcttagagcaagtgaggaagatccagggcgatggcgcagcgctccag
gagaagctggcaggctgcttgagccaactccatagcggccttttcctctaccaggggctc
ctgcaggccctggaagggatctcccccgagttgggtcccaccttggacacactgcagctg
gacgtcgccgactttgccaccaccatctggcagcagatggaagaactgggaatggcccct
gccctgcagcccacccagggtgccatgccggccttcgcctctgctttccagcgccgggca
ggaggggtcctggttgcctcccatctgcagagcttcctggaggtgtcgtaccgcgttcta
cgccaccttgcccagccc**gag**gccaagccctccccatcccatgtatttatctctatttaa
tatttatgtctatttaagcctcatatttaaagacagggaagagcagaacggagccccagg
cctctgtgtccttccctgcatttctgagtttcattctcctgcctgtagcagtgagaaaaa
gctcctgtcctcccatcccctggactgggaggtagataggtaaataccaagtatttatta
ctatgactgctccccagccctggctctgcaatgggcactgggatgagccgctgtgagccc
ctggtcctgagggtccccacctgggacccttgagagtatcaggtctcccacgtgggagac
aagaaatccctgtttaatatttaaacagcagtgttccccatctgggtccttgcacccctc
actctggcctcagccgactgcacagcggcccctgcatccccttggctgtgaggcccctgg
acaagcagaggtggccagagctgggaggcatggccctggggtcccacgaatttgctgggg
aatctcgttttctcttcttaagacttttgggacatggtttgactcccgaacatcaccgacg
tgtctcctgtttttctgggtggcctcgggacacctgccctgcccccacgagggtcaggac
tgtgactcttttttagggccaggcaggtgcctggacatttgccttgctggacggggactgg
ggatgtgggagggagcagacaggaggaatcatgtcaggcctgtgtgtgaaaggaagctcc
actgtcaccctccacctcttcacccccactcaccagtgtcccctccactgtcacattgt
aactgaacttcaggataataaagtgtttgcctccaaaaacgtcc

Fig. 18b

(GCSF T_2) -NO UNIQUE

MAGPATQSPMKLMALQLLLWHSALWTVQEATPLGPASSLPQSFLLKCLEQVRKIQGDGAA
LQEKLAGCLSQLHSGLFLYQGLLQALEGISPELGPTLDTLQLDVADFATTIWQQMEELGM
APALQPTQGAMPAFASAFQRRAGGVLVASHLQSFLEVSYRVLRHLAQP

Fig. 18c

EP 2 816 351 A2

```
humgcsf_p3.pfs

Sequence name: /dir/tp/CGC/DATA/analysis_db/sw.fasta:CSF3_HUMAN

Sequence documentation:
Granulocyte colony-stimulating factor precursor (G-CSF) (Pluripoietin)
(Filgrastim) (Lenograstim). Homo sapiens (Human). P09919;

Alignment of: HUMGCSF_P3 x CSF3_HUMAN    ..
                     .         .         .         .         .
    1 MAGPATQSPMKLMALQLLLWHSALWTVQEATPLGPASSLPQSFLLKCLEQ 50
      |||||||||||||||||||||||||||||||||||||||||||||||||||
    1 MAGPATQSPMKLMALQLLLWHSALWTVQEATPLGPASSLPQSFLLKCLEQ 50
                     .         .         .         .         .
   51 VRKIQGDGAALQEK................................... 64
      ||||||||||||||
   51 VRKIQGDGAALQEKLVSECATYKLCHPEELVLLGHSLGIPWAPLSSCPSQ 100
                     .         .         .         .         .
   65 ...LAGCLSQLHSGLFLYQGLLQALEGISPELGPTLDTLQLDVADFATTI 111
         ||||||||||||||||||||||||||||||||||||||||||||||||
  101 ALQLAGCLSQLHSGLFLYQGLLQALEGISPELGPTLDTLQLDVADFATTI 150
                     .         .         .         .         .
  112 WQQMEELGMAPALQPTQGAMPAFASAFQRRAGGVLVASHLQSFLEVSYRV 161
      |||||||||||||||||||||||||||||||||||||||||||||||||
  151 WQQMEELGMAPALQPTQGAMPAFASAFQRRAGGVLVASHLQSFLEVSYRV 200

  162 LRHLAQP                                        168
      |||||||
  201 LRHLAQP                                        207
```

Fig. 18d

EP 2 816 351 A2

Fig. 18e

Granulocyte colony stimulating factor

Novel Transcripts

ESTs supporting new variant (T2)

IL-7 structure

W.T.   SP   IL-7/IL-9 family
       1-27        28-173

T3     SP   IL-7/IL-9 family
       1-27        28-129

Fig. 19a

EP 2 816 351 A2

## (IL7 T_3) -no unique

aagacgaatagtttgatttattagccaattcagataaatgtgcacgtggaagtcatagtt
aaatattatcgtcagtttccacgtcctgcgtttaatttggggtttgattttccaaataca
acacttaccagattaggtggacccacaggattattttttccttgaggtctcacctgagcag
gtgcatgtacagcagacggagcagaaagagactgattagagaggttggagtggtagaggg
cgtgaccctcttaatcattcttcacttccttttttaaaagacgacttggcatcgtccacc
acatccgcggcaacgcctccttggtgtcgtccgcttccaataacccagcttgcgtcctgc
acacttgtggcttccgtgcacacattaacaactcatggttctagctcccagtcgccaagc
gttgccaaggcgttgagagatcatctgggaagtctttacccagaattgctttgattcag
gccagctggttttttcctgcggtgattcggaaattcgcgaattcctctggtcctcatccag
gtgcgcgggaagcaggtgcccaggagagagggggataatgaagattccatgctgatgatcc
caaagattgaacctgcagaccaagcgcaaagtagaaactgaaagtacactgctggcggat
cctacggaagttatggaaaaggcaaagcgcagagccacgccgtagtgtgtgccgcccccc
ttgggatggatgaaactgcagtcgcggcgtgggtaagaggaaccagctgcagagatcacc
ctgcccaacacagactcggcaactccgcggaagaccagggtcctgggagtgactatgggc
ggtgagagcttgctcctgctccagttgcggtcatcatgactacgcccgcctcccgcagac
c▓▓▓ttccatgtttctttaggtatatctttggacttcctccctgatccttgttctgtt
gccagtagcatcatctgattgtgatattgaaggtaaagatggcaaacaatatgagagtgt
tctaatggtcagcatcgatcaattattggacagcatgaaagaaattggtagcaattgcct
gaataatgaatttaactttttaaaagacatatctgtgatgctaataaggttaaaggaag
aaaaccagctgccctgggtgaagcccaaccaacaaagagtttggaagaaaataaatcttt
aaaggaacagaaaaactgaatgacttgtgtttcctaaagagactattacaagagataaa
aacttgttggaataaaattttgatgggcactaaagaaca▓▓▓aaaatatggagtggcaa
tatagaaacacgaactttagctgcatcctccaagaatctatctgcttatgcagtttttca
gagtggaatgcttcctagaagttactgaatgcaccatggtcaaaacggattagggcattt
gagaaatgcatattgtattactagaagatgaatacaaacaatggaaactgaatgctccag
tcaacaaactatttcttatatatgtgaacatttatcaatcagtataattctgtactgatt
tttgtaagacaatccatgtaaggtatcagttgcaataatacttctcaaacctgtttaaat
atttcaagacattaaatctatgaagtatataatggtttcaaagattcaaaattgacattg
ctttactgtcaaaataattttatggctcactatgaatctattatactgtattaagagtga
aaattgtcttcttctgtgctggagatgtttagagttaacaatgatatatggataatgcc
ggtgagaataagagagtcataaaccttaagtaagcaacagcataacaaggtccaagatac
ctaaaagagatttcaagagatttaattaatcatgaatgtgtaacacagtgccttcaataa
atggtatagcaaatgttttgacatgaaaaaaggacaatttcaaaaaaataaaataaaata
aaaataaattcacctagtctaaggatgctaaaccttagtactgagttacattgtcattta
tatagattataacttgtctaaataagtttgcaatttgggagatatattttaagataata
atatatgtttacctttaattaatgaaatatctgtatttaattttgacactatatctgta
tataaaatattttcatacagcattacaaattgcttactttggaatacatttctcctttga
taaaataaatgagctatgt

## Fig. 19b

## (IL7 T_3) -no unique

MFHVSFRYIFGLPPLILVLLPVASSDCDIEGKDGKQYESVLMVSIDQLLDSMKEIGSNCL
NNEFNFFKRHICDANKVKGRKPAALGEAQPTKSLEENKSLKEQKKLNDLCFLKRLLQEIK
TCWNKILMGTKEH

## Fig. 19c

humil7a_p3.pfs


Sequence name: /dir/tp/CGC/DATA/analysis_db/sw.fasta:IL7_HUMAN

Sequence documentation:
Interleukin-7 precursor (IL-7). Homo sapiens (Human). P13232;

Alignment of: HUMIL7A_P3 x IL7_HUMAN ..

```
  1 MFHVSFRYIFGLPPLILVLLPVASSDCDIEGKDGKQYESVLMVSIDQLLD 50
    |||||||||||||||||||||||||||||||||||||||||||||||||||
  1 MFHVSFRYIFGLPPLILVLLPVASSDCDIEGKDGKQYESVLMVSIDQLLD 50

 51 SMKEIGSNCLNNEFNFFKRHICDANK...................... 76
    |||||||||||||||||||||||||||
 51 SMKEIGSNCLNNEFNFFKRHICDANKEGMFLFRAARKLRQFLKMNSTGDF 100

 77 ...................VKGRKPAALGEAQPTKSLEENKSLKEQKKL 106
                       ||||||||||||||||||||||||||||||||
101 DLHLLKVSEGTTILLNCTGQVKGRKPAALGEAQPTKSLEENKSLKEQKKL 150

107 NDLCFLKRLLQEIKTCWNKILMGTKEH                       133
    |||||||||||||||||||||||||||
151 NDLCFLKRLLQEIKTCWNKILMGTKEH                       177
```

# Fig. 19d

Interleukin 7

Novel Transcripts

ESTs supporting new variant (T3)

Fig. 19e

EP 2 816 351 A2

VEGF-B structure

Fig. 20a

W.T.

SP
1-21

PDGF domain
47-124

SV

SP
1-21

PDGF domain
47-124

6 a.a

(vegf t_4) -NO UNIQUE

atgagccgcctgcccgccagcccgggcccagcccccgccgcccccgccgtccccgccgc
cgctgcccgccgccaccggccgcccgcccgcccggctcctccggccgcctccgctgcgct
gcgctgcgctgcctgcacccagggctcgggagggggccgcggaggagccgcccccgcgc
ccggccccgcccgccgcgcccgggcccgcgccatggggctctggctgccgccgcccccc
gcgccgccgggctagggcgatgcgggcgcccccggcgggcggccccggcgggcacc█████a
gccctctgctccgccgcctgctgctcgccgcactcctgcagctggccccgcccaggccc
ctgtctccagcctgatgcccctggccaccagaggaaagtggtgtcatggatagatgtgt
atactcgcgctacctgccagccccgggaggtggtggtgcccttgactgtggagctcatgg
gcaccgtggccaaacagctggtgcccagctgcgtgactgtgcagcgctgtggtggctgct
gccctgacgatggcctggagtgtgtgcccactgggcagcaccaagtccggatgcagatcc
tcatgatccggtacccgagcagtcagctgggggagatgtccctggaagaacacagccagt
gtgaatgcagacctaaaaaaaggacagtgctgtgaagccagacaggtgccggaagctgc
gaagg█████cacatggcttttcagactcagcaggtgacttgcctcagaggctatatccca
gtgggggaacaaagaggagcctggtaaaaaacagccaagcccccaagacctcagcccagg
cagaagctgctctaggacctgggcctctcagagggctcttctgccatccttgtctccct
gaggccatcatcaaacaggacagagttggaagaggagactgggaggcagcaagagggtc
acataccagctcaggggagaatggagtactgtctcagtttctaaccactctgtgcaagta
agcatcttacaactggctcttcctcccctcactaagaagacccaaacctctgcataatgg
gatttgggctttggtacaagaactgtacccccaaccctgataaaagagatggaagga

(vegf t_4)

MSPLLRRLLLAALLQLAPAQAPVSQPDAPGHQRKVVSWIDVYTRATCQPREVVVPLTVEL
MGTVAKQLVPSCVTVQRCGGCCPDDGLECVPTGQHQVRMQILMIRYPSSQLGEMSLEEHS
QCECRPKKKDSAVKPDRCRKLRR

Fig. 20b

Fig. 20c

t08411_p5.pfs

Sequence name: /dir/tp/CGC/DATA/analysis_db/sw.fasta:VEGB_HUMAN

Sequence documentation:
Vascular endothelial growth factor B precursor (VEGF-B) (VEGF related
factor) (VRF). Homo sapiens (Human). P49765; Q16528;

Alignment of: T08411_P5 x VEGB_HUMAN   ..

```
       .         .         .         .         .         .
  1 MSPLLRRLLLAALLQLAPAQAPVSQPDAPGHQRKVVSWIDVYTRATCQPR 50
    |||||||||||||||||||||||||||||||||||||||||||||||||
  1 MSPLLRRLLLAALLQLAPAQAPVSQPDAPGHQRKVVSWIDVYTRATCQPR 50

       .         .         .         .         .         .
 51 EVVVPLTVELMGTVAKQLVPSCVTVQRCGGCCPDDGLECVPTGQHQVRMQ 100
    |||||||||||||||||||||||||||||||||||||||||||||||||
 51 EVVVPLTVELMGTVAKQLVPSCVTVQRCGGCCPDDGLECVPTGQHQVRMQ 100

        .         .         .         .         .
101 ILMIRYPSSQLGEMSLEEHSQCECRPKKKDSAVKPDRCRKLRR          143
    |||||||||||||||||||||||||||||||||||||||
101 ILMIRYPSSQLGEMSLEEHSQCECRPKKKDSAVKPDR......          137
```

## Fig. 20d

EP 2 816 351 A2

VEGF-b

Fig. 20e

EP 2 816 351 A2

Troponin - S69208 - WT transcript and variants 1,4,6,9,10,14 and 16

Fig. 21

Fig. 22

Endo-Endometroid
PM-post mortem

Fig. 23

Fig. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 480752 P
- US 09133987 B
- US 4235877 A
- US 4603112 A
- US 4769330 A
- US 5017487 A
- WO 8901973 A
- US 4777127 A
- GB 2200651 A
- EP 0345242 A
- WO 9102805 A
- US 4436727 A
- US 4877611 A
- US 4866034 A
- US 4912094 A
- US 6008200 A
- US 5856462 A
- US 5093246 A
- US 4987071 A
- US 5116742 A
- US 6326174 B, Joyce
- US 487331 A
- EP 264166 A
- US 4215051 A
- US 5876742 A
- US 5272071 A
- WO 9106667 A
- US 4946778 A
- US 4704692 A
- US 5223409 A
- WO 9218619 A
- WO 9117271 A
- WO 9220791 A
- WO 9215679 A
- WO 9301288 A
- WO 9201047 A
- WO 9209690 A
- WO 9002809 A
- WO 9100906 A
- WO 9110741 A
- WO 9203918 A
- WO 9203917 A
- EP 125023 A
- EP 184187 A
- EP 171496 A
- EP 173494 A
- WO 8601533 A
- US 4816567 A
- US 5585089 A, Queen

- US 5693761 A
- US 5693762 A
- US 5225539 A
- EP 519596 A1
- US 5631169 A
- US 4868103 A
- US 5283317 A
- WO 9410300 A
- US 5272057 A
- US 4683202 A
- US 5854033 A
- US 5695937 A
- US 5498531 A
- US 5459039 A
- US 5695940 A
- US 5143854 A
- US 5510270 A
- US 5527681 A
- US 5384261 A
- US 5288514 A
- US 9304145 B
- WO 9951773 A1
- US 6033862 A
- US 4522811 A
- US 5208020 A
- US 5475092 A
- US 5585499 A
- US 5846545 A
- US 5328470 A
- US 4873316 A
- US 4666828 A
- US 4801531 A
- US 5192659 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A

- WO 725901 A
- WO 7261386 A
- WO 726387 A

- WO 727860 A
- WO 2003018612 A
- WO 9627007 A

**Non-patent literature cited in the description**

- **BOGUSKI MS.** *Science,* 1999, vol. 286, 453-455
- **SAMBROOK et al.** Molecular cloning, A laboratory manual. Cold Spring Harbor press, 1989
- **LIANG et al.** *Science,* 1992, vol. 257, 967-971
- **ADAMS et al.** *Nature,* 1995, vol. 377, 3-173
- **HILLIER et al.** *Genome Res.,* 1996, vol. 6, 807-828
- **LIANG ; PARDEE.** *Science,* 1992, vol. 257, 967-70
- **WELSH et al.** *Nucleic Acids Res.,* 1992, vol. 20, 4965-70
- **KATO.** *Nucleic Acids Res.,* 1995, vol. 18, 3685-90
- **VALCULESCU et al.** *Science,* 1995, vol. 270, 484-8
- **ASHBURNER ; GOODMAN.** *Curr. Opin. Genet Dev.,* 1997, vol. 7, 750-756
- **KARP.** *Trends Biochem. Sci.,* 1998, vol. 23, 114-116
- **ETZOLD et al.** *Methods Enzymol,* 1996, vol. 266, t14-t28
- **SCHULER et al.** *Methods Enzymol.,* 1996, vol. 266, 141-162
- **CHUNG ; WONG.** *Trends Biotech.,* 1999, vol. 17, 351-355
- **SOREK.** *Genome Res.,* 2002, vol. 12, 1060-1067
- *Krevintseva Trends Genet.,* 2003, vol. 19 (3), 124-8
- **KEEGAN.** *Nat Rev. Genet.,* 2001, vol. 2, 869-78
- **SCHAUB.** *Biobhimie,* 2002, vol. 84, 791-803
- **ADLER.** *Curr. Opin. Genet Dev.,* 1994, vol. 4, 316-22
- **MAKELA TP, KOSKINEN PJ ; YASTRIK I ; ALITALO K.** *Science,* 17 April 1992, vol. 256 (5055), 373-7
- **TAMURA N ; GARBERS DL.** GC-B1. *J. Biol. Chem.,* 2003, vol. 278 (49), 48880-9
- **YAN et al.** *J Biol Chem.,* 2001, vol. 276 (14), 10870-8
- the signaling reported for soluble IL-6R. *Kallen Biochim Biophys Acta.,* 11 November 2002, vol. 1592 (3), 323-43
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat'l. Acad. Sci. USA,* 1988, vol. 85, 2444

- **ALTSCHUL et al.** *J. Mol. Biol.,* 1990, vol. 215, 403-410
- **SUTTON G. et al.** *Genome Science and Technology,* 1995, vol. 1, 9-19
- **BONFIELD JK. et al.** *Nucleic Acids Res.,* 1995, vol. 23, 4992-4999
- **HUANG X. et al.** *Genomics,* 1996, vol. 33, 21-31
- **LAURENCE CB. et al.** *Genomics,* 1994, vol. 23, 192-201
- **SWINDELL SR. ; PLASTERER JN.** *Methods Mol. Biol.,* 1997, vol. 70, 75-89
- **RONEN FELDNIAN ; IDO DAGAN ; HAYM HIRSH.** Mining Text Using Keyword Distributions. *Proceedings of the 1995 Workshop on Knowledge Discovery in Databases,* 1995
- Finding Associations in Collections of Text. **RONEN FELDMAN ; HAYM HIRSH.** Machine Learning and Data Mining: Methods and Applications. John Wiley & Sons, Ltd, 1997
- Technology Text Mining, Turning Information Into Knowledge: A White Paper from IBM. 17 February 1998
- **EINAT HAZKANI-COVO ; EREZ LEVANON ; GALIT ROTMAN ; DAN GRAUR ; AMIT NOVIK.** Evolution of multicellularity in metazoa: comparative analysis of the subcellular localization of proteins in Saccharomyces, Drosophila and Caenorhabditis. *Cell Biology International,* 2004
- **BURSET et al.** *Nucleic Acids Res.,* 2000, vol. 28, 4364-75
- **SHOSHAN et al.** Proceeding of SPIE. 2001, vol. 4266, 86-95
- **R. SOREK ; G. AST ; D. GRAUR.** *Genome Res.*
- **CROFT et al.** *Nat. Genet,* 2000, vol. 24, 340-1
- **MUNEER et al.** *Genomic,* 2002, vol. 79, 344-8
- **KRAWZCZAK et al.** *Hum. Genet.,* 1992, vol. 90, 41-54
- **LIU et al.** *Nature Genet.,* 2001, vol. 27, 55-58
- *Nature,* 2001, vol. 409, 860-921
- **MODREK et al.** *Nucleic Acids Res.,* 2001, vol. 29, 2850-2859
- **ROSS, R.J.M.** *Growth hormone & IGF Research,* 1999, vol. 9, 42-46
- *Caballero Dis Markers,* 2001, vol. 17 (2), 67-75
- Vaccine Design (the subunit and adjuvant approach). Plenum Press, 1995
- **ROLLAND.** *Crit Rev. Therap. Drug Carrier Systems,* 1998, vol. 15, 143-198

- **FISHER-HOCH et al.** *Proc. Natl. Acad. Sci. USA,* 1989, vol. 86, 317-321
- **FLEXNER et al.** *Ann. N.Y Acad. Sci.,* 1989, vol. 569, 86-103
- **FLEXNER et al.** *Vaccine,* 1990, vol. 8, 17-21
- **BERKNER.** *Biotechniques,* 1988, vol. 6, 616-627
- **ROSENFELD et al.** *Science,* 1991, vol. 252, 431-434
- **KOLLS et al.** *Proc. Natl. Acad. Sci. USA,* 1994, vol. 91, 215-219
- **KASS-EISLER et al.** *Proc. Natl. Acad. Sci. USA,* 1993, vol. 90, 11498-11502
- **GUZMAN et al.** *Circulation,* 1993, vol. 88, 2838-2848
- **GUZMAN et al.** *Cir. Res.,* 1993, vol. 73, 1202-1207
- **ULMER et al.** *Science,* 1993, vol. 259, 1745-1749
- **COHEN.** *Science,* 1993, vol. 259, 1691-1692
- **ZITVOGEL et al.** *Nature Med.,* 1998, vol. 4, 594-600
- *Biochemistry,* 27 February 2001, vol. 40 (8), 2572-9
- **TOULMÉ.** *Nature Biotech.,* 2001, vol. 19, 17
- **FARIA et al.** *Nature Biotech.,* 2001, vol. 19, 40-44
- **HYRUP et al.** *Bioorganic & Medicinal Chemistry,* 1996, vol. 4, 5-23
- **PERRY-O'KEEFE et al.** *Proc. Natl. Acad. Sci. USA,* 1996, vol. 93, 14670-675
- **LUFT.** *J Mol Med,* 1998, vol. 76 (2), 75-6
- **KRONENWETT et al.** *Blood,* 1998, vol. 91 (3), 852-62
- **RAJUR et al.** *Bioconjug Chem,* 1997, vol. 8 (6), 935-40
- **LAVIGNE et al.** *Biochem Biophys Res Commun,* 1997, vol. 237 (3), 566-71
- **AOKI et al.** *Biochem Biophys Res Commun,* 1997, vol. 231 (3), 540-5
- **WALTON et al.** *Biotechnol Bioeng,* 1999, vol. 65 (1), 1-9
- **MATVEEVA et al.** *Nature Biotechnology,* 1998, vol. 16, 1374-1375
- **HOLMUND et al.** *Curr Opin Mol Ther,* 1999, vol. 1 (3), 372-85
- **GERWITZ.** *Curr Opin Mol Ther,* 1999, vol. 1 (3), 297-306
- **UNO et al.** *Cancer Res,* 2001, vol. 61 (21), 7855-60
- **GAULTIER et al.** *Nucleic Acids Res.,* 1987, vol. 15, 6625-6641
- **INOUE et al.** *Nucleic Acids Res.,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **KROL et al.** *Bio-Techniques,* 1988, vol. 6, 958-976
- **HUTVAGNER ; ZAMORE.** *Curr. Opin. Genetics and Development,* 2002, vol. 12, 225-232
- **BERNSTEIN.** *Nature,* 2001, vol. 409, 363-366
- **HAMMOND et al.** *Nat Rev. Gen.,* 2001, vol. 2, 110-119
- **SHARP.** *Genes. Dev.,* 2001, vol. 15, 485-90
- **HAMMOND et al.** *Nat. Rev. Gen.,* 2001, vol. 2, 110-119
- **TUSCHL.** *ChemBiochem.,* 2001, vol. 2, 239-245
- **CULLEN.** *Nat. Immunol.,* 2002, vol. 3, 597-599
- **BRANTL.** *Biochem. Biophys. Act.,* 2002, vol. 1575, 15-25
- **TUSCHL.** *ChemBiochem.,* vol. 2, 239-245
- **BREAKER, R.R. ; JOYCE, G.** *Chemistry and Biology,* 1995, vol. 2, 655
- **SANTORO, S.W. ; JOYCE, G.F.** *Proc. Natl, Acad. Sci. USA,* 1997, vol. 943, 4262
- **SANTORO, S.W. ; JOYCE, G.F.** *Proc. Natl, Acad. Sci. USA*
- **KHACHIGIAN, LM.** *Curr Opin Mol Ther,* 2002, vol. 4, 119-21
- **ITOH et al.** *Ann Meeting Am Soc Gen Ther,* 2002, www.asgt.org
- **SAMBROOK et al.** Molecular Cloning, A Laboratory Manual. Cold Spring Harbor Laboratory Press, 1989
- Current Protocols in Molecular Biology. John Wiley & Sons, 1995
- **RUTHER et al.** *EMBO J.,* 1983, vol. 2, 1791
- **SMITH ; JOHNSON.** *Gene,* 1988, vol. 67, 31-40
- **PINKERT et al.** *Genes Dev.,* 1987, vol. 1, 268-277
- **CALAME ; EATON.** *Adv. Immunol.,* 1988, vol. 43, 235-275
- **WINOTO ; BALTIMORE.** *EMBO J.,* 1989, vol. 8, 729-733
- **BANERJI et al.** *Cell,* 1982, vol. 33, 729-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379
- **CAMPES ; TILGHMAN.** *Genes Dev.,* 1989, vol. 3, 537-546
- **GOSSEN ; BUJARD.** *Proc.-Natl. Acad. Sci. USA,* 1992, vol. 89, 5547

- **PAILLARD.** *Human Gene Therapy,* 1989, vol. 9, 983
- **WADA et al.** *Nucleic Acids Res.,* 1992, vol. 20, 2111-2118
- **SMITH et al.** *J. Virol.,* 1983, vol. 46, 584
- **GOEDDEL.** Gene Expression Technology: Methods in Enzymology. Academic Press, 1990, vol. 185
- **AUSUBEL et al.** Current Protocols in Molecular Biology. John Wiley & Sons, 1994
- **POUWELS et al.** Cloning Vectors: A Laboratory Manual. 1985
- **MULLIGAN et al.** *Proc. Natl. Acad. Sci. USA,* 1981, vol. 78, 2072
- **COLBERRE-GARAPIN et al.** *J. Mol. Biol.,* 1981, vol. 150, 1
- **SANTERRE et al.** *Gene,* 1981, vol. 30, 147
- **FISHER.** Laboratory Techniques In Biochemistry And Molecular Biology. Elsevier, 1980
- **JANKNECHT et al.** *Proc. Natl. Acad. Sci. USA,* 1981, vol. 88, 8972
- Solid Phase Peptide Synthesis. The Pierce Chemical Co, 1984
- **LANZA.** *Nature Biotechnol.,* 1996, vol. 14, 1107
- **JOKI et al.** *Nature Biotechnol.,* 2001, vol. 19, 35
- **ARKIN ; YOURVAN.** *Proc. Natl. Acad. Sci. USA,* 1992, vol. 89, 7811-7815
- **DELGRAVE et al.** *Protein Engineering,* 1993, vol. 6, 327-331
- **KABAT et al.** Sequences of Proteins of Immunological Interest. NIH Publication No. 91-3242, 1991
- **CHOTHIA.** *J. Mol. Biol.,* 1987, vol. 196, 901-917
- **WARD et al.** *Nature,* 1989, vol. 341, 544-546
- **HUSE et al.** *Science,* 1989, vol. 246, 1275
- **BIRD et al.** *Science,* 1988, vol. 242, 423-426
- **HUSTON et al.** *Proc. Natl. Acad. Sci. USA,* 1988, vol. 85, 5879-5883
- **COLCHER et al.** *Ann. NY Acad. Sci.,* 1999, vol. 880, 263-80
- **REITER.** *Clin. Cancer Res.,* 1996, vol. 2, 245-52
- **FUCHS et al.** *Bio/Technology,* 1991, vol. 9, 1370-1372
- **HAY et al.** *Human Antibody Hybridomas,* 1992, vol. 3, 81-85
- **HUSE et al.** *Science,* 1989, vol. 246, 1275-1281
- **GRIFFTHS et al.** *EMBO J,* 1993, vol. 12, 725-734
- **HAWKINS et al.** *J. Mol Biol,* 1992, vol. 226, 889-896
- **CLACKSON et al.** *Nature,* 1991, vol. 352, 624-628
- **GRAM et al.** *Proc. Natl. Acad. Sci. USA,* 1992, vol. 89, 3576-3580
- **GARRAD et al.** *Bio/Technology,* 1991, vol. 9, 1373-1377
- **HOOGENBOOM et al.** *Nucl. Acids Res.,* 1991, vol. 19, 4133-4137
- **BARBAS et al.** *Proc. Natl. Acad. Sci. USA,* 1991, vol. 88, 7978-7982
- **LONBERG et al.** *Nature,* 1994, vol. 368, 856-859
- **GREEN et al.** *Nature Genet.,* 1994, vol. 7, 13-21
- **MORRISON et al.** *Proc. Natl. Acad. Sci. USA,* 1994, vol. 81, 6851-6855
- **BRUGGEMAN et al.** *Immunol.,* 1993, vol. 7, 33-40
- **TUAILLON et al.** *Proc. Natl. Acad. Sci. USA,* 1993, vol. 90, 3720-3724
- **BRUGGEMAN et al.** *Eur. J. Immunol,* 1991, vol. 21, 1323-1326
- **BETTER et al.** *Science,* 1988, vol. 240, 1041-1043
- **LIU et al.** *Proc. Natl. Acad. Sci. USA,* 1987, vol. 84, 3439-3443
- **LIU et al.** *J. Immunol.,* 1987, vol. 139, 3521-3526
- **SUN et al.** *Proc. Natl. Acad. Sci. USA,* 1987, vol. 84, 214-218
- **NISHIMURA et al.** *Cancer Res.,* 1987, vol. 47, 999-1005
- **WOOD et al.** *Nature,* 1985, vol. 314, 446-449
- **SHAW et al.** *J. Natl. Cancer Inst.,* 1988, vol. 80, 1553-1559
- **MORRISON et al.** *Proc. Natl. Acad. Sci. USA,* 1984, vol. 81, 6851
- **NEUBERGER et al.** *Nature,* 1984, vol. 312, 604
- **TAKEDA et al.** *Nature,* 1984, vol. 314, 452
- **WINNAKER.** From Genes to Clones. Verlagsgesellschaft, 1987
- **MORRISON.** *Science,* 1985, vol. 229, 1202-1207
- **OI et al.** *BioTechniques,* 1986, vol. 4, 214
- **JONES et al.** *Nature,* 1986, vol. 321, 552-525
- **VERHOEYAN et al.** *Science,* 1988, vol. 239, 1534
- **BEIDLER et al.** *J. Immunol.,* 1988, vol. 141, 4053-4060
- *Eur. Heart J.,* 2002, vol. 23 (21), 1670-7
- *Proc. Natl. Acad. Sci. USA.,* 2000, vol. 97 (4), 1495-500
- *J. Biol. Chem.,* 2000, vol. 275 (43), 33850-60
- *J. Biol. Chem.,* 2000, vol. 275 (43), 33 850-60
- **RAO D. S. et al.** *Mol. Cell Biol.,* 2001, vol. 21 (22), 7796-806
- **SAMBRANO G. R. et al.** *Nature,* 2001, vol. 413 (6851), 26-7

- **KOIVISTO U. M. et al.** *Cell,* 2001, vol. 105 (5), 575-85
- **SIMON A. et al.** *Ned Tijdschr Geneeskd,* 2001, vol. 145 (2), 77-8
- **DOMBROWICZ D. et al.** *J. Exp. Med.,* 2001, vol. 193 (1), 25-34
- **STANKIEWICZ P et al.** *Am. J. Med. Genet.,* 2001, vol. 103 (2), 166-71
- *Hum. Mutat,* 2000, vol. 16 (1), 23-30
- *Oncogene,* 1999, vol. 18 (35), 4968-73
- *Am. J. Cardiol.,* 2002, vol. 90 (5A), 11G-18G
- *Blood.,* 2001, vol. 98 (13), 3650-7
- *Ann. Emerg. Med.,* 2003, vol. 42 (3), 343-50
- *Oral. Dis.,* 2003, vol. 9 (3), 119-28
- *J. Surg. Res.,* 2003, vol. 113 (1), 102-8
- *Am. J. Pathol.,* 2003, vol. 163 (3), 845-58
- *Front. Biosci.,* 1999, vol. 1 (4), D9-21
- *Science,* 2003, vol. 302 (5646), 819-22
- *J. Neurol.,* 2003, vol. 250 (3), IIII25-III29
- *Nat. Genet.,* 2003, vol. 35 (2), 125-7
- *Cancer Res.,* 2003, vol. 63 (17), 5428-37
- *Lab. Invest.,* 2003, vol. 83 (9), 1255-65
- *Am. J. Pathol.,* 2003, vol. 163 (4), 1645-52
- *Arch. Virol.,* 2003, vol. 148 (9), 1851-62
- *Nat. Genet.,* 2003, vol. 35 (2), 165-70
- *J. Natl. Cancer Inst.,* 2003, vol. 95 (17), 1263-5
- *Carcinogenesis,* 2003, vol. 24 (7), 1281-2
- *J. Thorac. Cardiovasc. Surg.,* 2003, vol. 126 (2), 344-57
- *J. Immunol.,* 2003, vol. 171 (3), 1556-63
- *World J. Gastroenterol.,* 2002, vol. 8 (5), 901-7
- **GOODMAN P.A. et al.** *Oncogene,* 2001, vol. 20 (30), 3969-78
- **BOULTWOOD J.** *J. Clin. Pathol.,* 2001, vol. 54 (7), 512-6
- **ZANELLA A. et al.** *Br. J. Haematol.,* 2001, vol. 113 (1), 43-8
- **HOUTEN S. M. et al.** *Eur. J. Hum. Genet.,* 2001, vol. 9 (4), 253-9
- **GUZMAN M. L. et al.** *Blood,* 2001, vol. 97 (7), 2177-9
- **SCHULLER E. et al.** *Life Sci.,* 2001, vol. 69 (3), 263-70
- **MASLIAH E. et al.** *Exp. Neurol.,* 1995, vol. 136 (2), 107-22
- **GRIFFITHS I. R. et al.** *Neuropathol. Appl. Neurobiol.,* 1989, vol. 15 (1), 63-74
- **ASTOR P. et al.** *Ann. Neurol.,* 2001, vol. 49 (2), 263-7
- **WHITE R. L.** *Pathol. Biol. (Paris),* 1997, vol. 45 (3), 240-4
- **KOTTLER M. L.** *J. Clin. Endocrinol. Metab.,* 2000, vol. 85 (9), 3002-8
- **PUEL A. ; LEONARD W. J.** *Curr. Opin. Immunol.,* 2000, vol. 12 (4), 468-7
- **MOHN A.R et al.** *Cell,* 1999, vol. 98 (4), 427-36
- **ZHAO Y. X. et al.** *Arthritis Rheum.,* 1999, vol. 42 (8), 1662-72
- **AHESHWARI H. G. et al.** *J. Clin. Endocrinol. Metab.,* 1998, vol. 83 (11), 4065-74
- *Urology,* 2003, vol. 62 (5), 55-62
- *Invest. New Drugs.,* 2001, vol. 21 (4), 435-43
- *JAMA,* 2003, vol. 290 (16), 2149-58
- *Am. J. Med. Genet.,* 2003, vol. 123B (1), 64-9
- *Dis. Colon Rectum,* 2003, vol. 46 (11), 1498-507
- *N Engl. J. Med.,* 2003, vol. 349 (17), 1673-4
- *J. Chem Neuroanat.,* 2003, vol. 26 (2), 143-51
- *Hum. Mol. Genet.,* 2003, vol. 21
- *Mol. Biol. Evol.,* 2003, vol. 31
- *J. Neuropathol. Exp. Neurol.,* 2003, vol. 62 (7), 751-64
- *Antioxid Redox Signal.,* 2003, vol. 5, 337-48
- *J. Neurochem.,* 2003, vol. 86 (2), 394-404
- *Hum. Genet.,* 2003, vol. 6
- *Neurol Sci.,* 2003, vol. 24 (3), 159-60
- *J. Neurol.,* 2003, vol. 250 (3), III25-III29
- *J. Hum. Genet,* 2003, vol. 48 (8), 415-9
- *J. Mol. Neurosci.,* 2003, vol. 20 (3), 283-6
- *J. Alzheimers Dis.,* 2003, vol. 5 (3), 171-7
- *Semin. Oncol.,* 2003, vol. 30 (5), 709-16
- *J Neurochem.,* 2003, vol. 87 (1), 248-56
- *EMBO J.,* 2003, vol. 22 (20), 5435-5445
- *Crit Care Med.,* 2002, vol. 30 (5), 214-9
- *Eur. J. Surg.,* 2002, vol. 168 (4), 204-14
- **WESTERBERG L. et al.** *Blood,* 2001, vol. 98 (4), 1086-94
- **TANG M. L. ; FISCUS L. C.** *Pulm. Pharmacol. Ther.,* 2001, vol. 14 (3), 203-10
- **FUKUCHI M. et al.** *J. Am. Coll. Cardiol.,* 2001, vol. 37 (5), 1436-42
- **ROBBINS P. B. et al.** *Proc. Natl. Acad. Sci.,* 2001, vol. 98 (9), 5193-8
- **ROLF B. et al.** *Brain Res.,* 2001, vol. 891 (1-2), 247-52
- *Trends Cell Biol.,* 2002, vol. 12 (12), 585-91
- *Neuron,* 2003, vol. 40 (1), 25-40
- *Trends Biochem. Sci.,* 2003, vol. 28 (10), 558-65
- *Med. Genet.,* 2003, vol. 40 (9), 671-5
- **FIJEN C. A. et al.** *Clin. Exp. Immunol.,* 2000, vol. 120 (2), 338-45
- **GRISCELLI C. et al.** *Immunodefic. Rev.,* 1989, vol. 1 (2), 135-53
- **PAVIC I. et al.** *J. Gen. Virol.,* 1993, vol. 74, 2215-23
- **EVANS C. A. et al.** *Neurogenetics,* 2001, vol. 3 (2), 69-78
- **BAIOCCHI R. A. et al.** *J. Clin. Invest.,* 2001, vol. 108 (6), 887-94
- **HIRAIWA H. ; CHOU J. Y.** *DNA Cell Biol.,* 2001, vol. 20 (8), 447-53
- **MCNEISH J. et al.** *Proc. Natl. Acad. Sci.,* 2000, vol. 97 (8), 4245-50
- **TANG N. L. et al.** *Hum. Mol. Genet.,* 1999, vol. 8 (4), 655-60

- **PAYNE A. S. et al.** *Proc. Natl. Acad. Sci.,* 1998, vol. 95 (18), 10854-9
- **NEWBURY-ECOB R.** *J. Med. Genet.,* 1998, vol. 35 (1), 49-53
- *Oncogene,* 2003, vol. 22 (38), 6005-12
- *Oncologist,* 2003, vol. 8 (5), 411-24
- *J. Med. Invest.,* 2003, vol. 50 (3-4), 126-35
- **PARKINSON.** *Annu. Rev. Phytopathol.,* 04 September 2003, vol. 41, 641-67
- **PARKINSON.** *FASEB J.,* 04 September 2003
- *Am. J. Cardiol.,* 2003, vol. 92 (4B), 10K-16K
- *JAMA,* 2003, vol. 290 (13), 1721-8
- *JAMA,* 2003, vol. 290 (13), 1713-20
- *Diabetes,* 2003, vol. 52 (9), 2274-8
- *J. Neurosci.,* 2003, vol. 23 (24), 8471-9
- **PARKINSON.** *J. Neurosci.,* 2003, vol. 23 (23), 8302-9
- *Lancet,* 2003, vol. 362 (9385), 712
- *Clin. Neurophysiol.,* 2003, vol. 114 (9), 1724-8
- *J. Pathol.,* 2003, vol. 201 (1), 37-45
- *Cancer Res.,* 2003, vol. 63 (16), 4952-9
- *Eur. J. Cancer.,* 2003, vol. 39 (13), 1899-903
- *Neurology,* 2003, vol. 61 (3), 404-6
- *Urology,* 2003, vol. 61 (4), 845-50
- *J. Cutan. Pathol.,* 2002, vol. 29 (7), 430
- *Cancer.,* 2002, vol. 94 (6), 1777-86
- *Clin. Cancer Res.,* 2001, vol. 7 (8), 2415-24
- *Breast Cancer Res. Treat,* 2001, vol. 65 (1), 11-21
- *J. Am. Soc. Nephrol.,* 2002, vol. 13 (2), 322-31
- *Eur. J. Immunol.,* 2001, vol. 31 (4), 1221-7
- *J. Cell Physiol.,* 2000, vol. 182 (2), 303-9
- *J. Neurosci.,* 2003, vol. 23 (25), 8788-99
- *Neurobiol. Dis.,* 2003, vol. 14 (1), 146-56
- *J. Neurosci.,* 2003, vol. 23 (17), 6956-64
- *Am. J. PathoL,* 2003, vol. 163 (2), 609-19
- *Cancer Res.,* 2003, vol. 63 (15), 4299-304
- *Semin. Thromb. Hemost,* 2003, vol. 29 (3), 247-58
- *Proc. Natl. Acad. Sci. U S A.,* 2003, vol. 100 (16), 9506-11
- *J. Med. Genet.,* 2003, vol. 40 (10), 733-40
- *J. Neuropathol. Exp. Neurol.,* 2003, vol. 62 (9), 968-75
- *J. Neurochem.,* 2003, vol. 87 (2), 427-36
- *N. Engl. J. Med.,* 2003, vol. 349 (16), 1526-33
- *JAMA,* 2003, vol. 290, 1721-8
- *Dig. Dis. Sci.,* 2003, vol. 48 (9), 1832-8
- *J. Vasc. Surg.,* 2003, vol. 38 (4), 827-32
- *Oncogene,* 2003, vol. 22 (43), 6699-703
- *Br. J, Haematol.,* 2003, vol. 123 (2), 288-96
- *Circulation,* 2003, vol. 108 (15), 1839-44
- *Ophthalmology,* 2003, vol. 110 (10), 2040-4
- *Am. J. Ophthalmol.,* 2003, vol. 136 (4), 729-32
- *BMC Gastroenterology,* 06 November 2003, vol. 3, 31
- *Int. J. Neurosci.,* 2003, vol. 13 (12), 1705-1717
- *Int. J. Neurosci.,* 2003, vol. 113 (11), 1579-1591
- *Ann. Neurol.,* 2003, vol. 54 (4), 494-509
- *Other Motor Neuron Disord.,* 2003, vol. 4 (2), 96-9
- *J. Nippon. Med. Sch.,* 2003, vol. 70 (5), 384-92
- *Endocrinology,* 2003, vol. 144 (10), 4478-83
- *Mol. Pathol.,* 2003, vol. 56 (5), 302-4
- *Neurosci. Lett.,* 2003, vol. 350 (2), 105-8
- *Cancer,* 2003, vol. 98 (9), 1842-8
- *Cancer,* 2003, vol. 98 (9), 1822-9
- *J. Neurol.,* 2003, vol. 250 (3), III15-III24
- *J. NeuroL,* 2003, vol. 250 (3), III2-III10
- *Pancreas,* 2003, vol. 27 (4), 291-6
- *Eur. J. Pediatr.,* 1997, vol. 156 (12), 935-8
- *BMJ.,* 2003, vol. 327 (7421), 974-71
- *Ann. Intern. Med.,* October 2003, vol. 139 (8), 670-82
- *J. Med. Genet,* 2003, vol. 40 (10), 761-6
- *Am. J. Hum. Genet.,* 2001, vol. 69 (5), 1002-12
- *Lancet,* 2003, vol. 362 (9390), 1112-9
- *J. Cell Biol.,* 2003, vol. 162 (6), 1111-22
- *Cancer Res.,* 2003, vol. 63 (16), 4836-41
- *Neuromuscul. Disord.,* 2003, vol. 13 (7-8), 579-88
- *Neuromuscul. Disord.,* 2003, vol. 13 (6), 456-67
- *J. Alzheimers Dis.,* 2003, vol. 5 (3), 209-28
- *J. Am. Coll. Cardiol.,* 2003, vol. 42 (2), 319-27
- *Proteomics,* 2003, vol. 3 (6), 979-90
- *Cardiovasc. Res.,* 2003, vol. 60 (1), 205-13
- *Rheum. Dis. Clin. North Am.,* 2003, vol. 29 (3), 631-45
- *Curr. Opin. Clin. Nutr. Metab. Care.,* 2003, vol. 6 (4), 435-9
- *Neurovirol.,* 2003, vol. 9 (2), 274-83
- *Dev. Ophthalmol.,* 2003, vol. 37, 109-25
- *J. Virol. Methods.,* 2003, vol. 109 (1), 75-83
- *FEMS Immunol. Med. Microbiol.,* 2003, vol. 35 (2), 125-30
- *J. Exp. Med.,* 2003, vol. 197 (5), 633-42
- **SHANE R. et al.** *Brain Res.,* 2001, vol. 907 (1-2), 109-16
- **ALKAYED N. J. et al.** *J. Neurosci.,* 2001, vol. 21 (19), 7543-50
- **ELHADD T .A. et al.** *J. Clin. Endocrinol. Metab.,* 2001, vol. 86 (9), 4223-32
- **HONG S. et al.** *Nat. Med.,* 2001, vol. 7 (9), 1052-6
- **UDY G. B. et al.** *Proc. Natl. Acad. Sci. USA,* 1997, vol. 94 (14), 7239-44
- **PADBERG F. et al.** *J. Neuroimmunol.,* 1999, vol. 99 (2), 218-23
- **SUZUKI A. et al.** *J Exp Med,* 2001, vol. 193 (4), 471-81
- **ZHOU Y. et al.** *Oncology,* 2001, vol. 60 (4), 330-8

- **WATANABE G. et al.** *Cancer J.,* 2001, vol. 7 (2), 132-9
- *Cell Cycle,* 2003, vol. 2 (2), 99-104
- *Bioessays.,* 2001, vol. 23 (8), 671-3
- *Biochim. Biophys. Acta,* 1997, vol. 1354 (3), 241-51
- *J. Cell Biol.,* 2001, vol. 152 (1), 75-85
- *Front Biosci.,* 2000, vol. 5, D244-57
- *Biol. Chem.,* 1999, vol. 380 (2), 117-28
- *Clin. Exp. Immunol.,* 1997, vol. 109 (3), 488-94
- *Tumori,* 2003, vol. 89 (3), 278-84
- *Prostate,* 2003, vol. 57 (1), 80-92
- *J. Pathol.,* 2003, vol. 200 (5), 640-6
- *Curr. Hematol. Rep.,* 2003, vol. 2 (4), 335-40
- *J. Thromb. Haemost.,* 2003, vol. 1 (7), 1381-90
- *Trends Cardiovasc. Med.,* 2003, vol. 13 (5), 188-95
- *Neurosci. Lett.,* 2003, vol. 342 (1-2), 41-4
- *Am. J. Ophthalmol.,* 2003, vol. 136 (4), 678-87
- *Proc. Natl. Acad. Sci. USA.,* 2003, vol. 100 (18), 10347-52
- *J. Neurosci.,* 2003, vol. 23 (17), 6914-27
- *Brain Res Bull.,* 2003, vol. 61 (4), 427-35
- *Anticancer Res.,* 2003, vol. 23 (4), 3419-26
- *Neuromuscul Disord.,* 2003, vol. 13 (7-8), 559-67
- *J. Pathol.,* 2003, vol. 200 (5), 553-60
- **CAMPOS Y. et al.** *Ann. Neurol.,* 2001, vol. 50 (3), 409-13
- **GUTSTEIN D. E. et al.** *Circulation,* 2001, vol. 104 (10), 1194-9
- **DIEZ-JUAN A. ; ANDRES V.** *FASEB J.,* 2001, vol. 15 (11), 1989-95
- **ESWORTHY R. S. et al.** *Am. J. Physiol. Gastrointest. Liver Physiol.,* 2001, vol. 281 (3), G848-55
- **YASUTOMO K. et al.** *Nat. Genet.,* 2001, vol. 28 (4), 313-4
- *Pancreas,* 2003, vol. 27 (4), 281-5
- **HANDE et al.** *Hum. Mol. Genet.,* 2001, vol. 10 (5), 519-28
- **DELANY et al.** *J. Clin. Invest.,* 2000, vol. 105 (7), 915-23
- **HOLMBECK et al.** *Cell,* 1999, vol. 99 (1), 81-92
- **MATSUKI et al.** *Proc. Natl. Acad. Sci. USA,* 1998, vol. 95 (3), 1044-9
- **SIMPKINS et al.** *Am. J. Med.,* 1997, vol. 103 (3A), 19S-25S
- **KRENN V. et al.** *Histol Histopathol,* July 2000, vol. 15 (3), 791
- **JAN VOSWINKEL et al.** *Arthritis Res,* 2001, vol. 3 (3), 189
- **ERIKSON J. et al.** *Immunol Res,* 1998, vol. 17 (1-2), 49
- **RENAUDINEAU Y. et al.** *Clin Diagn Lab Immunol.,* March 1999, vol. 6 (2), 156
- **CHAN OT. et al.** *Immunol Rev,* June 1999, vol. 169, 107
- **ZIMMET P.** *Diabetes Res Clin Pract,* October 1996, vol. 34, 125
- **ORGIAZZI J.** *Endocrinol Metab Clin North Am,* June 2000, vol. 29 (2), 339
- **BRALEY-MULLEN H. ; YU S.** *J Immunol,* 15 December 2000, vol. 165 (12), 7262
- **TOYODAN. et al.** *Nippon Rinsho,* August 1999, vol. 57 (8), 1810
- **MITSUMA T.** *Nippon Rinsho.,* August 1999, vol. 57 (8), 1759
- **GARZA KM. et al.** *J Reprod Immunol,* February 1998, vol. 37 (2), 87
- **DIEKMAN AB. et al.** *Am J Reprod Immunol.,* March 2000, vol. 43 (3), 134
- **TINCANI A. et al.** *Lupus,* 1998, vol. 7 (2), 107-9
- **CROSS AH. et al.** *J Neuroimmunol,* 01 January 2001, vol. 112 (1-2), 1
- **ORON L. et al.** *J Neural Transm,* 1997, vol. 49, 77
- **INFANTE AJ. ; KRAIG E.** *Int Rev Immunol,* 1999, vol. 18 (1-2), 83
- **KORNBERG AJ.** *J Clin Neurosci.,* May 2000, vol. 7 (3), 191
- **KUSUNOKI S.** *Am J Med Sci.,* April 2000, vol. 319 (4), 234
- **TAKAMORI M.** *Am J Med Sci.,* April 2000, vol. 319 (4), 204
- **ANTOINE JC. ; HONNORAT J.** *Rev Neurol (Paris),* January 2000, vol. 156 (1), 23
- **NOBILE-ORAZIO E. et al.** *Electroencephalogr Clin Neurophysiol,* 1999, vol. 50, 419
- **VINCENT A. et al.** *Ann N Y Acad Sci.,* 13 May 1998, vol. 841, 482
- **MATSUURA E. et al.** *Lupus,* 1998, vol. 7 (2), 13S
- **VAARALA O.** *Lupus.,* 1998, vol. 7 (2), 132
- **PRAPROTNIK S. et al.** *Wien Klin Wochenschr,* 25 August 2000, vol. 112 (15-16), 660
- **LACROIX-DESMAZES S. et al.** *Semin Thromb Hemost.,* 2000, vol. 26 (2), 157
- **NOEL LH.** *Ann Med Interne (Paris),* May 2000, vol. 151 (3), 178
- **FLAMHOLZ R. et al.** *J Clin Apheresis,* 1999, vol. 14 (4), 171
- **WALLUKAT G. et al.** *Am J Cardiol.,* 17 June 1999, vol. 83 (12A), 75H
- **MOCCIA F.** *Ann Ital Med Int.,* April 1999, vol. 14 (2), 114
- **EFREMOV DG. et al.** *Leuk Lymphoma,* January 1998, vol. 28 (3-4), 285
- **GARCIA HEROLA A. et al.** *Gastroenterol Hepatol.,* January 2000, vol. 23 (1), 16
- **LANDAU YE. ; SHOENFELD Y.** *Harefuah,* 16 January 2000, vol. 138 (2), 122
- **FEIST E. et al.** *Int Arch Allergy Immunol,* September 2000, vol. 123 (1), 92

- **ZAULI D. et al.** *Biomed Pharmacother,* June 1999, vol. 53 (5-6), 234
- **MANNS MP.** *J Hepatol,* August 2000, vol. 33 (2), 326
- **STRASSBURG CP. et al.** *Eur J Gastroenterol Hepatol.,* June 1999, vol. 11 (6), 595
- **TISCH R ; MCDEVITT HO.** *Proc Natl Acad Sci U S A,* 18 January 1994, vol. 91 (2), 437
- **DATTA SK.** *Lupus,* 1998, vol. 7 (9), 591
- **CASTANO L. ; EISENBARTH GS.** *Ann. Rev. Immunol.,* vol. 8, 647
- **SAKATA S. et al.** *Mol Cell Endocrinol,* March 1993, vol. 92 (1), 77
- **ALEXANDER RB. et al.** *Urology,* December 1997, vol. 50 (6), 893
- **HARA T. et al.** *Blood,* 01 March 1991, vol. 77 (5), 1127
- **SODERSTROM M. et al.** *J Neurol Neurosurg Psychiatry,* May 1994, vol. 57 (5), 544
- **OSHIMA M. et al.** *Eur J Immunol,* December 1990, vol. 20 (12), 2563
- **HIEMSTRA HS. et al.** *Proc Natl Acad Sci U S A,* 27 March 2001, vol. 98 (7), 3988
- **CUNHA-NETO E et al.** *J Clin Invest,* 15 October 1996, vol. 98 (8), 1709
- **SEMPLE JW. et al.** *Blood,* 15 May 1996, vol. 87 (10), 4245
- **CAPOROSSI AP. et al.** *Viral Immunol,* 1998, vol. 11 (1), 9
- **SALLAH S. et al.** *Ann Hematol,* March 1997, vol. 74 (3), 139
- **FRANCO A. et al.** *Clin Immunol Immunopathol,* March 1990, vol. 54 (3), 382
- **JONES DE.** *Clin Sci (Colch),* November 1996, vol. 91 (5), 551
- **KELLY CJ.** *J Am Soc Nephrol,* August 1990, vol. 1 (2), 140
- **YOO TJ. et al.** *Cell Immunol,* August 1994, vol. 157 (1), 249
- **GLODDEK B. et al.** *Ann N Y Acad Sci,* 29 December 1997, vol. 830, 266
- **MATSUURA E. et al.** *Lupus.,* 1998, vol. 7 (2), 135
- **VAARALA O.** *Lupus.,* 1998, vol. 7 (2), S132
- **NOEL LH.** *Ann Med Interne (Paris),* May 2000, vol. 151 (3), 178
- **CUNHA-NETO E. et al.** *J Clin Invest,* 15 October 1996, vol. 98 (8), 1709
- **CAPOROSSI AP et al.** *Viral Immunol,* 1998, vol. 11 (1), 9
- **TISCH R ; MCDEVITT HO.** *Proc Natl Acad Sci units S A,* 18 January 1994, vol. 91 (2), 437
- **TOYODA N. et al.** *Nippon Rinsho,* August 1999, vol. 57 (8), 1810
- **ORON L. et al.** *J Neural Transm Suppl.,* 1997, vol. 49, 77
- **INFANTE AJ ; KRAIG E.** *Int Rev Immunol,* 1999, vol. 18 (1-2), 83
- **HIEMSTRA HS. et al.** *Proc Natl Acad Sci units S A,* 27 March 2001, vol. 98 (7), 3988
- **ZUCKERMANN et al.** *J. Med. Chem.,* 1994, vol. 37, 2678-85
- **LAM.** *Anticancer Drug Des.,* 1997, vol. 12, 145
- **DEWITT et al.** *Proc. Natl. Acad. Sci. USA,* 1993, vol. 90, 6909
- **ERB et al.** *Proc. Natl. Acad. Sci. USA,* 1994, vol. 91, 11422
- **ZUCKERMANN et al.** *J. Med. Chem.,* 1994, vol. 37, 2678
- **CHO et al.** *Science,* 1993, 1303
- **GALLOP et al.** *J. Med. Chem.,* 1994, vol. 37, 1233
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci. USA,* 1990, vol. 87, 6378-6382
- **FELICI.** *J. Mol. Biol.,* 1991, vol. 222, 301-310
- **MCCONNELL et al.** *Science,* 1992, vol. 257, 1906-1912
- **SJOLANDER ; URBANICZKY.** *Anal. Chem.,* 1991, vol. 63, 2338-2345
- **SZABO et al.** *Curr. Opin. Struct. Biol.,* 1995, vol. 5, 699-705
- **RIVAS ; MINTON.** *Trends Biochem Sci,* 1993, vol. 18, 284-7
- Current Protocols in Molecular Biology. J. Wiley & Sons, 1999
- **HEEGAARD.** *J. Mol. Recognit.,* 1998, vol. 11, 141-148
- **HAGE ; TWEED.** *J. Chromatogr. Biomed. Sci. Appl.,* 1997, vol. 699, 499-525
- **ZERVOS et al.** *Cell,* 1993, vol. 72, 223-232
- **MADURA et al.** *J. Biol. Chem.,* 1993, vol. 268, 12046-12054
- **BARTEL et al.** *Biotechniques,* 1993, vol. 14, 920-924
- **IWABUCHI et al.** *Oncogene,* 1993, vol. 8, 1693-1696
- **CRONIN et al.** *Human Mutation,* 1996, vol. 7, 244-255
- **KOZAL et al.** *Nature Medicine,* 1996, vol. 2, 753-759
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177

- **LIZARDI et al.** *Bio/Technology,* 1988, vol. 6, 1197
- **MYERS et al.** *Science,* 1985, vol. 230, 1242
- **COTTON et al.** *Proc. Natl. Acad. Sci. USA,* 1988, vol. 85, 4397
- **SALEEBA et al.** *Methods Enzymol.,* 1992, vol. 217, 286-295
- **HSU et al.** *Carcinogenesis,* 1994, vol. 15, 1657-1662
- **ORITA et al.** *Proc. Natl. Acad. Sci. USA,* 1989, vol. 86, 2766
- **COTTON.** *Mutat. Res.,* 1993, vol. 285, 125-144
- **HAYASHI.** *Genet. Anal. Tech. Appl.,* 1992, vol. 9, 73-79
- **KEEN et al.** *Trends Genet.,* 1991, 5
- **MYERS et al.** *Nature,* 1985, vol. 313, 495
- **ROSENBAUM ; REISSNER.** *Biophys. Che,* 1987, vol. 265, 12753
- **POINT et al.** *Nature,* 1986, vol. 324, 163
- **SAIKI et al.** *Proc. Natl. Acad. Sci. USA,* 1989, vol. 86, 6230
- **XU et al.** *Nature Biotechnol.,* 2001, vol. 19, 148
- **GIBBS et al.** *Nucleic Acids Res.,* 1989, vol. 17, 2437-2448
- **PROSSNER.** *Tibtech.,* 1993, vol. 11, 238
- **BARANY.** *Proc. Natl. Acad. Sci. USA,* 1991, vol. 88, 189
- **GOLUB et al.** *Science,* 1999, vol. 286, 531
- **DE WILDT et al.** *Nature Biotech.,* 2000, vol. 18, 89-994
- **LUEKING et al.** *Anal. Biochem.,* 1999, vol. 270, 103-111
- **GE.** *Nucleic Acids Res.,* 2001, vol. 28 (e3), I-VII
- **MACBEATH ; SCHREIBER.** *Science,* 2000, vol. 289, 1760-1763
- **KOOMEN et al.** *J. Mass Spectrom.,* 2000, vol. 35, 258-264
- **JAMES.** *AIDS Treatment News Archive,* 1994, vol. 209
- **HATTIS et al.** *Env. Health Perspect.,* 1991, vol. 90, 229-238
- **SCHENTAG.** *Am. J. Health-Syst. Pharm.,* 1999, vol. 56 (3), S21-S24
- **NICOLAU.** *Am. J. Health-Syst. Pharm.,* 1991, vol. 56 (3), S16-S20
- **MCLEOD et al.** *Eur. J. Cancer,* 1999, vol. 35, 1650-1652
- **EICHELBAUM et al.** *Clin. Exp. Pharmacol. Physiol.,* 1996, vol. 23, 983-985
- **LINDER et al.** *Clin. Chem.,* 1997, vol. 43, 254-266
- **CRUIKSHANK et al.** *J. Acquired Immune Deficiency Syndromes and Human Retrovirology,* 1997, vol. 14, 193
- **CHEN et al.** *Proc. Natl. Acad. Sci. USA,* 1994, vol. 91, 3054-3057
- **VAICKUS.** *Crit Rev. in Oncol./Hemotol.,* 1991, vol. 11, 267-97
- **OSBORNE et al.** *Curr. Opin. Chem. Biol.,* 1997, vol. 1, 5-9
- **PATEL.** *Curr. Opin. Chem. Biol.,* 1997, vol. 1, 32-46
- **HERLYN.** *Ann. Med.,* 1991, vol. 31, 66-78
- **BHATTACHARYA-CHATTERJEE ; FOON.** *Cancer Treat. Res.,* 1998, vol. 94, 51-68
- **MARASCO et al.** *Proc. Natl. Acad. Sci. USA,* 1993, vol. 90, 7889-7893
- **PINKERT et al.** *Genes Dev.,* 1997, vol. 1, 268-277
- **CALAME et al.** *Adv. Immunol.,* 1988, vol. 43, 235-275
- **WINOTO et al.** *EMBO J.,* 1989, vol. 8, 729-733
- **BANERJI et al.** *Cell,* 1983, 33729-740
- **BYME et al.** *Proc. Natl. Acad. Sci. USA,* 1989, vol. 86, 5473-5477
- **EDLUNCH et al.** *Science,* 1985, vol. 230, 912-916
- **TONKINSON et al.** *Cancer Investigation,* 1996, vol. 14 (1), 54-65
- **TAYLOR.** *Nat. Biotechnol.,* 1999, vol. 17, 1097-1100
- **MERCATANTE.** *J. Biol. Chem.,* 2001, vol. 276, 16411-16417
- **KARRAS.** *Mol. Pharmacol.,* 2000, vol. 58, 380-387
- **GILES.** *Antisense Acid Drug Dev.,* 1999, vol. 9, 213-220
- **SAZANI ; KOLE.** *Progress in Moleclular and Subcellular Biology,* 2003, vol. 31, 217-239
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985

- Transcription and Translation. 1984
- Animal Cell Culture. 1986
- Immobilized Cells and Enzymes. IRL Press, 1986
- **PERBAL, B.** A Practical Guide to Molecular Cloning. 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **BOGUSKI ; SCHULER.** *Nat. Genet.,* 1995, vol. 10, 369-71
- **AUDIC ; CLAVERIE.** *Genome Res.,* 1997, vol. 7, 986-995
- **HUMINIECKI ; BICKNELL.** *Genome Res.,* 2000, vol. 10, 1796-1806
- **KAWAMOTO et al.** *Genome Res.,* 2000, vol. 10, 1817-1827
- **VALCULESCU et al.** *Science,* 1995, vol. 270, 484-487
- **CARON et al.** *Science,* 2001, vol. 291, 1289-1292
- **SCHENA et al.** *Science,* 1995, vol. 270, 467-470
- **SHOSHAN et al.** *Proc. SPIE Microarrays: Optical Technologies and Informatics,* 2001, vol. 4266, 86-95
- **MATLOUBIAN.** *Nat. Immunol.,* 2000, vol. 1, 298-304
- **DAVID et al.** *J. Biol. Chem.,* 2002, vol. 277, 18084-18090
- **SOREK et al.** *Genome Res.,* 2002, vol. 12, 1060-7
- **CAMARGO et al.** *Proc. Natl. Acad. Sci. USA,* 2001, vol. 98, 12103-12108
- **DONG B ; HOROWITZ DS ; KOBAYASHI R ; KRAINER AR.** *Nucleic Acids Res,* 1993, vol. 21 (17), 4085-92
- **DWIGHT et al.** *Nucleic Acids Res.,* 2002, vol. 30, 69-72
- *Nucleic Acids Res.,* 2002, vol. 30, 106-108
- PharmaProject. PJB Publications Ltd, 2003
- **MOHLER et al.** *J. Immunology,* vol. 151, 1548-1561
- *SWISS-PROT and TrEMBL release,* 18 December 2002
- *Medline databases,* 06 April 2001
- *Gene Ontology Consortium,* 22 October 2003
- **HOLM L ; SANDER C.** Removing near-neighbor redundancy from large protein sequence collections. *Bioinformatics,* June 1998, vol. 14 (5), 423-9
- **BASU et al.** *International Journal of molecular Medicine,* 2002, vol. 10, 3-10
- **LAYTON J. E.** *Growth Factors,* 1992, vol. 6, 17-186
- **YOUNG et al.** *Protein Science,* 1997, vol. 6, 1228-1236
- **LAYTON et al.** *Journal of Biological Chemistry,* 1999, vol. 274 (25), 17445-17451
- **BISHOP et al.** *Journal of Biological Chemistry,* 2001, vol. 276 (36), 33465-33470
- **HUBEL et al.** *Ann Hematol.,* 2003, vol. 82, 207-213
- **KUGA et al.** *Biochemical and biophysical research communications,* 1989, vol. 159 (1), 103-111
- **CLARK-LEWIS et al.** *The Journal of Immunology,* 1988, vol. 141 (3), 881-889
- **COSENZA et al.** *Protein Science,* 2000, vol. 9, 916-926
- **VANDERSPEK et al.** *Cytokine,* 2002, vol. 17 (5), 227-233
- **GORGUN et al.** *Cytokine,* 2002, vol. 20 (1), 17-22
- **FRY et al.** *Blood,* 2002, vol. 99 (11), 3892-3904
- **OLOFSON et al.** *Proc. Natl. Acad. Sci. USA.,* 1996, vol. 93, 2576-2581
- **CLAUSS M.** *Seminars in Thrombosis and Hemostasis,* 2000, vol. 26 (5), 561-569
- **SCOTNEY et al.** *Clinical and experimental Pharmacology and physiology,* 2002, vol. 29, 1024-1029
- **OLOFSSON et al.** *Clinical and experimental Pharmacology,* 1998, vol. 95 (20), 11709-11714
- **SHINKARUK et al.** *Curr. Med. Chem - Anti Cancer Agents,* 2003, vol. 3 (2), 95-117
- **OLOFSSON et al.** *Current Opinion in Biotechnology,* 1999, vol. 10, 528-535
- **ADAMCOVA.** *Physiol. Res.,* 1999, vol. 48, 235-247
- **EINAT HAZKANI-COVO ; EREZ LEVANON ; GALIT ROTMAN ; DAN GRAUR ; AMIT NOVIK.** Evolution of multicellularity in metazoa: comparative analysis of the subcellular localization of proteins in Saccharomyces, Drosophila and Caenorhabditis. *Cell Biology International,* 2004, vol. 28 (3), 171-8
- **MULDER et al.** The InterPro Database, 2003 brings increased coverage and new features. *Nucleic Acids Res.,* 2003, vol. 31, 315-318
- **BATEMAN A et al.** The Pfam protein families database. *Nucleic Acids Res.,* 2004, vol. 32, 138-41
- **LETUNIC I et al.** SMART 40: towards genomic data integration. *Nucleic Acids Res.,* 2004, vol. 32, 142-4
- **HAFT DH et al.** The TIGRFAMs database of protein families. *Nucleic Acids Res.,* 2003, vol. 31, 371-373
- **WU CH et al.** The Protein Information Resource. *Nucleic Acids Res.,* 2003, vol. 31, 345-347

- **GOUGH J et al.** Assignment of homology to genome sequences using a library of Hidden Markov Models that represent all proteins of known structure. *Journal Molecular BioL,* 2001, vol. 313, 903-919

- **EDGAR et al.** *Nucleic Acids Research,* 2002, vol. 30 (1), 207-210
- **SU et al.** *Proc Natl Acad Sci U S A.,* 09 April 2004, vol. 101, 6062-7